(19) Europäisches Patentamt
European Patent Office
Office européen des brevets

(11) EP 3 973 981 A1

(12) EUROPEAN PATENT APPLICATION

(43) Date of publication:
**30.03.2022 Bulletin 2022/13**

(21) Application number: **21189885.3**

(22) Date of filing: **17.05.2013**

(51) International Patent Classification (IPC):
***A61K 38/17*** *(2006.01)*

(52) Cooperative Patent Classification (CPC):
**C07K 14/43518; A61P 19/02; A61P 25/04; A61P 29/00; A61P 43/00**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.05.2012 US 201261648871 P**
**18.09.2012 US 201261702538 P**
**14.03.2013 US 201361781276 P**
**15.03.2013 US 201313833555**

(62) Document number(s) of the earlier application(s) in accordance with Art. 76 EPC:
**13791454.5 / 2 852 397**

(71) Applicant: **Janssen Biotech, Inc.**
**Horsham, PA 19044 (US)**

(72) Inventors:
- **ECKERT, William**
**San Diego, 92121 (US)**
- **FLINSPACH, Mack**
**San Diego, 92121 (US)**
- **HUNTER, Michael**
**San Diego, 92121 (US)**
- **LIU, Yi**
**San Diego, 92121 (US)**
- **NEFF, Robert**
**San Diego, 92121 (US)**
- **WICKENDEN, Alan**
**San Diego, 92121 (US)**
- **GIBBS, Alan**
**Spring House, 19477 (US)**

(74) Representative: **Carpmaels & Ransford LLP**
**One Southampton Row**
**London WC1B 5HA (GB)**

Remarks:
This application was filed on 05.08.2021 as a divisional application to the application mentioned under INID code 62.

# (54) HUWENTOXIN-IV VARIANTS AND METHODS OF USE

(57) The present invention relates to Huwentoxin-IV variants, polynucleotides encoding them, methods of making and using the foregoing, and methods of alleviating pain with peptide inhibitors of Nav1.7.

EP 3 973 981 A1

SEQUENCE LISTING

```
<110>  Flinspach, Mack
       Eckert, William
       Gibbs, Alan
       Hunter, Michael
       Liu, Yi
       Neff, Robert
       Wickenden, Alan

<120>  Huwentoxin-IV variants and methods of use

<130>  JBI5008WOPCT

<140>  PCT/US2013/041572
<141>  2013-05-17

<150>  61/648,871
<151>  2012-05-18

<150>  61/702,538
<151>  2012-09-18

<150>  61/781,276
<151>  2013-03-14

<150>  13/833,555
<151>  2013-03-15

<160>  357

<170>  PatentIn version 3.5

<210>  1
<211>  35
<212>  PRT
<213>  Ornithoctonus huwena


<220>
<221>  MOD_RES
<222>  (35)..(35)
<223>  AMIDATION

<400>  1

Glu Cys Leu Glu Ile Phe Lys Ala Cys Asn Pro Ser Asn Asp Gln Cys
1               5                   10                  15


Cys Lys Ser Ser Lys Leu Val Cys Ser Arg Lys Thr Arg Trp Cys Lys
            20                  25                  30


Tyr Gln Ile
        35


<210>  2
<211>  39
<212>  PRT
<213>  Ornithoctonus huwena
```

<400> 2

Gly Pro Glu Cys Leu Glu Ile Phe Lys Ala Cys Asn Pro Ser Asn Asp
1 5 10 15

Gln Cys Cys Lys Ser Ser Lys Leu Val Cys Ser Arg Lys Thr Arg Trp
20 25 30

Cys Lys Tyr Gln Ile Gly Lys
35

<210> 3
<211> 39
<212> PRT
<213> Artificial sequence

<220>
<223> Huwentoxin-IV variant

<400> 3

Gly Pro Asn Cys Leu Arg Ile Phe Lys Ala Cys Asn Pro Ser Asn Asp
1 5 10 15

Gln Cys Cys Lys Ser Ser Lys Leu Val Cys Ser Arg Lys Thr Arg Trp
20 25 30

Cys Lys Trp Ser Ile Gly Lys
35

<210> 4
<211> 39
<212> PRT
<213> Artificial sequence

<220>
<223> Huwentoxin-IV variant

<400> 4

Gly Pro Asn Cys Leu Arg Ile Phe Lys Ala Cys Asn Pro Ser Asn Asp
1 5 10 15

Gln Cys Cys Lys Ser Ser Lys Leu Val Cys Ser Arg Lys Thr Arg Trp
20 25 30

Cys Lys Tyr Ser Ile Gly Lys
35

<210> 5
<211> 39
<212> PRT
<213> Artificial sequence

<220>
<223> Huwentoxin-IV variant

<400> 5

Gly Pro Asn Cys Leu Arg Ile Phe Lys Ala Cys Asn Pro Ser Asn Asp
1 5 10 15

Gln Cys Cys Lys Ser Ser Lys Leu Val Cys Ser Lys Lys Thr Arg Trp
20 25 30

Cys Lys Tyr Ser Ile Gly Lys
35

<210> 6
<211> 39
<212> PRT
<213> Artificial sequence

<220>
<223> Huwentoxin-IV variant

<400> 6

Gly Pro Glu Cys Leu Glu Ile Phe Lys Ala Cys Asn Pro Ser Asn Asp
1 5 10 15

Gln Cys Cys Lys Ser Ser Lys Leu Val Cys Ser Arg Lys Thr Arg Trp
20 25 30

Cys Lys Tyr Phe Ile Gly Lys
35

<210> 7
<211> 39
<212> PRT
<213> Artificial sequence

<220>
<223> Huwentoxin-IV variant

<400> 7

Gly Pro Asn Cys Leu Glu Ile Phe Lys Ala Cys Asn Pro Ser Asn Asp
1 5 10 15

Gln Cys Cys Lys Ser Ser Lys Leu Val Cys Ser Arg Lys Thr Arg Trp
20 25 30

Cys Lys Trp Gln Ile Ile Lys
35

<210> 8
<211> 39

<212> PRT
<213> Artificial sequence

<220>
<223> Huwentoxin-IV variant

<400> 8

```
Gly Pro Glu Cys Leu Arg Ile Phe Lys Ala Cys Asn Pro Ser Asn Asp
1               5                   10                  15

Gln Cys Cys Lys Ser Ser Lys Leu Val Cys Ser Arg Lys Thr Arg Trp
            20                  25                  30

Cys Lys Trp Ser Ile Gly Lys
        35
```

<210> 9
<211> 39
<212> PRT
<213> Artificial sequence

<220>
<223> Huwentoxin-IV variant

<400> 9

```
Gly Pro Glu Cys Leu Gln Ile Phe Lys Ala Cys Asn Pro Ser Asn Asp
1               5                   10                  15

Gln Cys Cys Lys Ser Ser Lys Leu Val Cys Ser Arg Lys Thr Arg Trp
            20                  25                  30

Cys Lys Tyr Gln Ile Gly Lys
        35
```

<210> 10
<211> 39
<212> PRT
<213> Artificial sequence

<220>
<223> Huwentoxin-IV variant

<400> 10

```
Gly Pro Glu Cys Leu Glu Ile Phe Lys Ala Cys Asn Pro Ser Asn Asp
1               5                   10                  15

Gln Cys Cys Lys Ser Ser Lys Leu Val Cys Ser Arg Lys Thr Arg Trp
            20                  25                  30

Cys Lys Tyr Ser Ile Gly Lys
        35
```

<210> 11
<211> 39
<212> PRT
<213> Artificial sequence

<220>
<223> Huwentoxin-IV variant

<400> 11

Gly Pro Glu Cys Leu Glu Ile Phe Lys Ala Cys Asn Pro Ser Asn Asp
1 5 10 15

Asn Cys Cys Lys Ser Ser Lys Leu Val Cys Ser Arg Lys Thr Arg Trp
20 25 30

Cys Lys Tyr Gln Ile Gly Lys
35

<210> 12
<211> 39
<212> PRT
<213> Artificial sequence

<220>
<223> Huwentoxin-IV variant

<400> 12

Gly Pro Asn Cys Leu Arg Ile Phe Lys Ala Cys Asn Pro Ser Asn Asp
1 5 10 15

Gln Cys Cys Lys Ser Ser Lys Leu Val Cys Ser Arg Lys Thr Arg Trp
20 25 30

Cys Lys Tyr Gln Ile Ile Lys
35

<210> 13
<211> 39
<212> PRT
<213> Artificial sequence

<220>
<223> Huwentoxin-IV variant

<400> 13

Gly Pro Glu Cys Leu Glu Ile Phe Lys Ala Cys Asn Pro Ser Asn Asp
1 5 10 15

Gln Cys Cys Lys Ser Ser Lys Leu Val Cys Ser Arg Lys Thr Arg Trp
20 25 30

```
Cys Lys Trp Gln Ile Gly Lys
        35


<210>  14
<211>  39
<212>  PRT
<213>  Artificial sequence

<220>
<223>  Huwentoxin-IV variant

<400>  14

Gly Pro Glu Cys Leu Glu Ile Phe Lys Ala Cys Asn Pro Ser Asn Asp
1               5                   10                  15


Gln Cys Cys Lys Ser Ser Arg Leu Val Cys Ser Arg Lys Thr Arg Trp
            20                  25                  30


Cys Lys Tyr Gln Ile Gly Lys
        35


<210>  15
<211>  39
<212>  PRT
<213>  Artificial sequence

<220>
<223>  Huwentoxin-IV variant

<400>  15

Gly Pro Glu Cys Leu Glu Ile Phe Lys Arg Cys Asn Pro Ser Asn Asp
1               5                   10                  15


Gln Cys Cys Lys Ser Ser Lys Leu Val Cys Ser Arg Lys Thr Arg Trp
            20                  25                  30


Cys Lys Tyr Gln Ile Gly Lys
        35


<210>  16
<211>  39
<212>  PRT
<213>  Artificial sequence

<220>
<223>  Huwentoxin-IV variant

<400>  16

Gly Pro Asn Cys Leu Arg Ile Phe Lys Ala Cys Asn Pro Ser Asn Asp
1               5                   10                  15


Gln Cys Cys Lys Ser Ser Lys Leu Val Cys Ser Lys Lys Thr Arg Trp
```

20 25 30

Cys Lys Tyr Ser Ile Ile Lys
35

<210> 17
<211> 39
<212> PRT
<213> Artificial sequence

<220>
<223> Huwentoxin-IV variant

<400> 17

Gly Pro Asn Cys Leu Arg Ile Phe Lys Ala Cys Asn Pro Ser Asn Asp
1 5 10 15

Gln Cys Cys Lys Ser Ser Lys Leu Val Cys Ser Arg Lys Thr Arg Trp
20 25 30

Cys Lys Trp Gln Ile Ile Lys
35

<210> 18
<211> 39
<212> PRT
<213> Artificial sequence

<220>
<223> Huwentoxin-IV variant

<400> 18

Gly Pro Glu Cys Leu Arg Ile Phe Lys Ala Cys Asn Pro Ser Asn Asp
1 5 10 15

Gln Cys Cys Lys Ser Ser Lys Leu Val Cys Ser Arg Lys Thr Arg Trp
20 25 30

Cys Lys Tyr Gln Ile Gly Lys
35

<210> 19
<211> 39
<212> PRT
<213> Artificial sequence

<220>
<223> Huwentoxin-IV variant

<400> 19

Gly Pro Asn Cys Leu Glu Ile Phe Lys Ala Cys Asn Pro Ser Asn Asp
1 5 10 15

Gln Cys Cys Lys Ser Ser Lys Leu Val Cys Ser Arg Lys Thr Arg Trp
20 25 30

Cys Lys Trp Ser Ile Gly Lys
35

<210> 20
<211> 39
<212> PRT
<213> Artificial sequence

<220>
<223> Huwentoxin-IV variant

<400> 20

Gly Pro Glu Cys Leu Glu Ile Phe Lys Ala Cys Asn Pro Ser Asn Asp
1 5 10 15

Gln Cys Cys Lys Ser Asn Lys Leu Val Cys Ser Arg Lys Thr Arg Trp
20 25 30

Cys Lys Tyr Gln Ile Gly Lys
35

<210> 21
<211> 39
<212> PRT
<213> Artificial sequence

<220>
<223> Huwentoxin-IV variant

<400> 21

Gly Pro Glu Cys Leu Glu Ile Phe Lys Ala Cys Asn Pro Ser Asn Asp
1 5 10 15

Gln Cys Cys Lys Gln Ser Lys Leu Val Cys Ser Arg Lys Thr Arg Trp
20 25 30

Cys Lys Tyr Gln Ile Gly Lys
35

<210> 22
<211> 39
<212> PRT
<213> Artificial sequence

<220>
<223> Huwentoxin-IV variant

<400> 22

Gly Pro Asn Cys Leu Arg Ile Phe Lys Ala Cys Asn Pro Ser Asn Asp
1               5                   10                  15

Gln Cys Cys Lys Ser Ser Lys Leu Val Cys Ser Arg Lys Thr Arg Trp
            20                  25                  30

Cys Lys Tyr Gln Ile Gly Lys
        35

<210>  23
<211>  39
<212>  PRT
<213>  Artificial sequence

<220>
<223>  Huwentoxin-IV variant

<400>  23

Gly Pro Glu Cys Leu Glu Ile Phe Lys Ala Cys Asn Pro Ser Ala Asp
1               5                   10                  15

Gln Cys Cys Lys Ser Ser Lys Leu Val Cys Ser Arg Lys Thr Arg Trp
            20                  25                  30

Cys Lys Tyr Gln Ile Gly Lys
        35

<210>  24
<211>  39
<212>  PRT
<213>  Artificial sequence

<220>
<223>  Huwentoxin-IV variant

<400>  24

Gly Pro Glu Cys Leu Glu Ile Phe Lys Ala Cys Asn Pro Ser Asn Asp
1               5                   10                  15

Gln Cys Cys Lys Ser Ser Lys Leu Val Cys Ile Arg Lys Thr Arg Trp
            20                  25                  30

Cys Lys Tyr Gln Ile Gly Lys
        35

<210>  25
<211>  39
<212>  PRT
<213>  Artificial sequence

<220>

<223> Huwentoxin-IV variant

<400> 25

```
Gly Pro Glu Cys Leu Glu Ile Phe Lys Ala Cys Asn Pro Ser Asn Asp
1               5                   10                  15

Gln Cys Cys Lys Ser Ser Lys Leu Val Cys Ser Arg Lys Thr Arg Trp
            20                  25                  30

Cys Lys Tyr Gln Ile Gly Arg
        35
```

<210> 26
<211> 39
<212> PRT
<213> Artificial sequence

<220>
<223> Huwentoxin-IV variant

<400> 26

```
Gly Pro Glu Cys Leu Asn Ile Phe Lys Ala Cys Asn Pro Ser Asn Asp
1               5                   10                  15

Gln Cys Cys Lys Ser Ser Lys Leu Val Cys Ser Arg Lys Thr Arg Trp
            20                  25                  30

Cys Lys Tyr Gln Ile Gly Lys
        35
```

<210> 27
<211> 39
<212> PRT
<213> Artificial sequence

<220>
<223> Huwentoxin-IV variant

<400> 27

```
Gly Pro Glu Cys Leu Glu Ile Phe Lys Ala Cys Asn Pro Ser Asn Asp
1               5                   10                  15

Gln Cys Cys Lys Pro Ser Lys Leu Val Cys Ser Arg Lys Thr Arg Trp
            20                  25                  30

Cys Lys Tyr Gln Ile Gly Lys
        35
```

<210> 28
<211> 39
<212> PRT

<213> Artificial sequence

<220>
<223> Huwentoxin-IV variant

<400> 28

Gly Pro Glu Cys Leu Glu Ile Phe Lys Ala Cys Asn Pro Ser Asn Asp
1 5 10 15

Gln Cys Cys Lys Ser Asp Lys Leu Val Cys Ser Arg Lys Thr Arg Trp
20 25 30

Cys Lys Tyr Gln Ile Gly Lys
35

<210> 29
<211> 39
<212> PRT
<213> Artificial sequence

<220>
<223> Huwentoxin-IV variant

<400> 29

Gly Pro Glu Cys Leu Glu Ile Phe Lys Ala Cys Asn Pro Ser Asn Asp
1 5 10 15

Gln Cys Cys Lys Ser Arg Lys Leu Val Cys Ser Arg Lys Thr Arg Trp
20 25 30

Cys Lys Tyr Gln Ile Gly Lys
35

<210> 30
<211> 39
<212> PRT
<213> Artificial sequence

<220>
<223> Huwentoxin-IV variant

<400> 30

Gly Pro Glu Cys Leu Glu Ile Phe Lys Ala Cys Asn Pro Ser Asn Asp
1 5 10 15

Arg Cys Cys Lys Ser Ser Lys Leu Val Cys Ser Arg Lys Thr Arg Trp
20 25 30

Cys Lys Tyr Gln Ile Gly Lys
35

<210> 31
<211> 39
<212> PRT
<213> Artificial sequence

<220>
<223> Huwentoxin-IV variant

<400> 31

```
Gly Pro Glu Cys Leu Glu Ile Phe Lys Ala Cys Asn Pro Ser Asn Asp
1               5                   10                  15

Gln Cys Cys Lys Ser Ser Lys Leu Val Cys Ser Arg Lys Thr Arg Trp
            20                  25                  30

Cys Lys Tyr Leu Ile Gly Lys
        35
```

<210> 32
<211> 39
<212> PRT
<213> Artificial sequence

<220>
<223> Huwentoxin-IV variant

<400> 32

```
Gly Pro Glu Cys Leu Arg Ile Phe Lys Ala Cys Asn Pro Ser Asn Asp
1               5                   10                  15

Gln Cys Cys Lys Ser Ser Lys Leu Val Cys Ser Arg Lys Thr Arg Trp
            20                  25                  30

Cys Lys Tyr Ser Ile Ile Lys
        35
```

<210> 33
<211> 39
<212> PRT
<213> Artificial sequence

<220>
<223> Huwentoxin-IV variant

<400> 33

```
Gly Pro Glu Cys Leu Glu Ile Phe Lys Ala Cys Asn Pro Ser Asn Asp
1               5                   10                  15

Gln Cys Cys Lys Ser Ser Phe Leu Val Cys Ser Arg Lys Thr Arg Trp
            20                  25                  30

Cys Lys Tyr Gln Ile Gly Lys
```

35

<210> 34
<211> 39
<212> PRT
<213> Artificial sequence

<220>
<223> Huwentoxin-IV variant

<400> 34

Gly Pro Glu Cys Leu Glu Ile Phe Lys Ala Cys Asn Pro Ser Asn Asp
1 5 10 15

Gln Cys Cys Lys Ser Ser His Leu Val Cys Ser Arg Lys Thr Arg Trp
20 25 30

Cys Lys Tyr Gln Ile Gly Lys
35

<210> 35
<211> 39
<212> PRT
<213> Artificial sequence

<220>
<223> Huwentoxin-IV variant

<400> 35

Gly Pro Glu Cys Leu Glu Ile Phe Lys Ala Cys Asn Pro Ser Asn Asp
1 5 10 15

Gln Cys Cys Lys Ser Pro Lys Leu Val Cys Ser Arg Lys Thr Arg Trp
20 25 30

Cys Lys Tyr Gln Ile Gly Lys
35

<210> 36
<211> 39
<212> PRT
<213> Artificial sequence

<220>
<223> Huwentoxin-IV variant

<400> 36

Gly Pro Asn Cys Leu Arg Ile Phe Lys Ala Cys Asn Pro Ser Asn Asp
1 5 10 15

Gln Cys Cys Lys Ser Ser Lys Leu Val Cys Ser Lys Lys Thr Arg Trp
20 25 30

```
Cys Lys Tyr Gln Ile Ile Lys
        35


<210>  37
<211>  39
<212>  PRT
<213>  Artificial sequence

<220>
<223>  Huwentoxin-IV variant

<400>  37

Gly Pro Glu Cys Leu Glu Ile Phe Lys Ala Cys Asn Pro Ser Asn Asp
1               5                   10                  15


Gln Cys Cys Lys Ser Ser Asn Leu Val Cys Ser Arg Lys Thr Arg Trp
            20                  25                  30


Cys Lys Tyr Gln Ile Gly Lys
        35


<210>  38
<211>  39
<212>  PRT
<213>  Artificial sequence

<220>
<223>  Huwentoxin-IV variant

<400>  38

Gly Pro Glu Cys Leu Glu Ile Phe Lys Ala Cys Asn Pro Ser Asn Asp
1               5                   10                  15


Gln Cys Cys Tyr Ser Ser Lys Leu Val Cys Ser Arg Lys Thr Arg Trp
            20                  25                  30


Cys Lys Tyr Gln Ile Gly Lys
        35


<210>  39
<211>  39
<212>  PRT
<213>  Artificial sequence

<220>
<223>  Huwentoxin-IV variant

<400>  39

Gly Pro Glu Cys Leu Glu Tyr Phe Lys Ala Cys Asn Pro Ser Asn Asp
1               5                   10                  15
```

Gln Cys Cys Lys Ser Ser Lys Leu Val Cys Ser Arg Lys Thr Arg Trp
20 25 30

Cys Lys Tyr Gln Ile Gly Lys
35

<210> 40
<211> 39
<212> PRT
<213> Artificial sequence

<220>
<223> Huwentoxin-IV variant

<400> 40

Gly Pro Asn Cys Leu Arg Ile Phe Lys Ala Cys Asn Pro Ser Asn Asp
1 5 10 15

Gln Cys Cys Lys Ser Ser Lys Leu Val Cys Ser Arg Lys Thr Arg Trp
20 25 30

Cys Lys Tyr Ser Ile Ile Lys
35

<210> 41
<211> 39
<212> PRT
<213> Artificial sequence

<220>
<223> Huwentoxin-IV variant

<400> 41

Gly Pro Glu Cys Leu Glu Ile Phe Lys Ala Cys Asn Pro Ser Asn Asp
1 5 10 15

Gln Cys Cys Lys Arg Ser Lys Leu Val Cys Ser Arg Lys Thr Arg Trp
20 25 30

Cys Lys Tyr Gln Ile Gly Lys
35

<210> 42
<211> 39
<212> PRT
<213> Artificial sequence

<220>
<223> Huwentoxin-IV variant

<400> 42

Gly Pro Pro Cys Leu Glu Ile Phe Lys Ala Cys Asn Pro Ser Asn Asp
1               5                   10                  15

Gln Cys Cys Lys Ser Ser Lys Leu Val Cys Ser Arg Lys Thr Arg Trp
            20                  25                  30

Cys Lys Tyr Gln Ile Gly Lys
        35

<210>  43
<211>  39
<212>  PRT
<213>  Artificial sequence

<220>
<223>  Huwentoxin-IV variant

<400>  43

Gly Pro Glu Cys Leu Glu Ile Phe Lys Ala Cys Asn Pro Ser Asn Asp
1               5                   10                  15

Gln Cys Cys Lys Ser Ser Lys Arg Val Cys Ser Arg Lys Thr Arg Trp
            20                  25                  30

Cys Lys Tyr Gln Ile Gly Lys
        35

<210>  44
<211>  39
<212>  PRT
<213>  Artificial sequence

<220>
<223>  Huwentoxin-IV variant

<400>  44

Gly Pro Glu Cys Leu His Ile Phe Lys Ala Cys Asn Pro Ser Asn Asp
1               5                   10                  15

Gln Cys Cys Lys Ser Ser Lys Leu Val Cys Ser Arg Lys Thr Arg Trp
            20                  25                  30

Cys Lys Tyr Gln Ile Gly Lys
        35

<210>  45
<211>  39
<212>  PRT
<213>  Artificial sequence

<220>
<223>  Huwentoxin-IV variant

<400> 45

Gly Pro Glu Cys Leu Arg Ile Phe Lys Ala Cys Asn Pro Ser Asn Asp
1 5 10 15

Gln Cys Cys Lys Ser Ser Lys Leu Val Cys Ser Lys Lys Thr Arg Trp
20 25 30

Cys Lys Tyr Gln Ile Gly Lys
35

<210> 46
<211> 39
<212> PRT
<213> Artificial sequence

<220>
<223> Huwentoxin-IV variant

<400> 46

Gly Pro Glu Cys Leu Glu Ile Phe Lys Ala Cys Asn Pro Ser Asn Asp
1 5 10 15

Gln Cys Cys Lys Ser Ser Lys Leu Val Cys Ser Lys Lys Thr Arg Trp
20 25 30

Cys Lys Trp Gln Ile Gly Lys
35

<210> 47
<211> 39
<212> PRT
<213> Artificial sequence

<220>
<223> Huwentoxin-IV variant

<400> 47

Gly Pro Glu Cys Leu Glu Ile Phe Lys Ala Cys Asn Pro Ser Asn Asp
1 5 10 15

Gln Cys Cys Lys Ser Ser Lys Leu Val Cys Ser Arg Lys Thr Arg Trp
20 25 30

Cys Lys Tyr Gln Ile Gly Phe
35

<210> 48
<211> 39
<212> PRT
<213> Artificial sequence

<220>
<223> Huwentoxin-IV variant

<400> 48

Gly Pro Glu Cys Leu Arg Ile Phe Lys Ala Cys Asn Pro Ser Asn Asp
1               5                   10                  15

Gln Cys Cys Lys Ser Ser Lys Leu Val Cys Ser Lys Lys Thr Arg Trp
            20                  25                  30

Cys Lys Trp Gln Ile Ile Lys
        35

<210> 49
<211> 39
<212> PRT
<213> Artificial sequence

<220>
<223> Huwentoxin-IV variant

<400> 49

Gly Pro Glu Cys Leu Glu Ile Phe Lys Ala Cys Asn Pro Ser Asn Asp
1               5                   10                  15

Gln Cys Cys Lys Ser Ser Lys Leu Val Cys Ser Arg Lys Thr Arg Trp
            20                  25                  30

Cys Lys Tyr Thr Ile Gly Lys
        35

<210> 50
<211> 39
<212> PRT
<213> Artificial sequence

<220>
<223> Huwentoxin-IV variant

<400> 50

Gly Pro Arg Cys Leu Glu Ile Phe Lys Ala Cys Asn Pro Ser Asn Asp
1               5                   10                  15

Gln Cys Cys Lys Ser Ser Lys Leu Val Cys Ser Arg Lys Thr Arg Trp
            20                  25                  30

Cys Lys Tyr Gln Ile Gly Lys
        35

<210> 51

<211> 39
<212> PRT
<213> Artificial sequence

<220>
<223> Huwentoxin-IV variant

<400> 51

Gly Pro Glu Cys Leu Glu Ile Phe Lys Ala Cys Asn Pro Ser Asn Asp
1 5 10 15

Gln Cys Cys Arg Ser Ser Lys Leu Val Cys Ser Arg Lys Thr Arg Trp
20 25 30

Cys Lys Tyr Gln Ile Gly Lys
35

<210> 52
<211> 39
<212> PRT
<213> Artificial sequence

<220>
<223> Huwentoxin-IV variant

<400> 52

Gly Pro Glu Cys Leu Arg Ile Phe Lys Ala Cys Asn Pro Ser Asn Asp
1 5 10 15

Gln Cys Cys Lys Ser Ser Lys Leu Val Cys Ser Arg Lys Thr Arg Trp
20 25 30

Cys Lys Tyr Gln Ile Ile Lys
35

<210> 53
<211> 39
<212> PRT
<213> Artificial sequence

<220>
<223> Huwentoxin-IV variant

<400> 53

Gly Pro Glu Cys Leu Glu Ile Phe Lys Ala Cys Asn Pro Ser Asn Asp
1 5 10 15

Gln Cys Cys Lys Ser Tyr Lys Leu Val Cys Ser Arg Lys Thr Arg Trp
20 25 30

Cys Lys Tyr Gln Ile Gly Lys
35

<210> 54
<211> 39
<212> PRT
<213> Artificial sequence

<220>
<223> Huwentoxin-IV variant

<400> 54

Gly Pro Glu Cys Leu Glu Ile Phe Lys Glu Cys Asn Pro Ser Asn Asp
1 5 10 15

Gln Cys Cys Lys Ser Ser Lys Leu Val Cys Ser Arg Lys Thr Arg Trp
20 25 30

Cys Lys Tyr Gln Ile Gly Lys
35

<210> 55
<211> 39
<212> PRT
<213> Artificial sequence

<220>
<223> Huwentoxin-IV variant

<400> 55

Gly Pro Glu Cys Leu Glu Ile Phe Lys Ala Cys Asn Pro Ser Asn Asp
1 5 10 15

Gln Cys Cys Lys Ser Ser Lys Leu Val Cys Ser Arg Lys Thr Trp Trp
20 25 30

Cys Lys Tyr Gln Ile Gly Lys
35

<210> 56
<211> 39
<212> PRT
<213> Artificial sequence

<220>
<223> Huwentoxin-IV variant

<400> 56

Gly Pro Glu Cys Leu Glu Ile Phe Trp Ala Cys Asn Pro Ser Asn Asp
1 5 10 15

Gln Cys Cys Lys Ser Ser Lys Leu Val Cys Ser Arg Lys Thr Arg Trp
20 25 30

```
Cys Lys Tyr Gln Ile Gly Lys
        35


<210>  57
<211>  39
<212>  PRT
<213>  Artificial sequence

<220>
<223>  Huwentoxin-IV variant

<400>  57

Gly Pro Glu Cys Leu Glu Ile Phe Lys Ala Cys Asn Pro Ser Asn Asp
1               5                   10                  15


Gln Cys Cys Lys Ser Ser Lys Leu Val Cys Ser Arg Trp Thr Arg Trp
            20                  25                  30


Cys Lys Tyr Gln Ile Gly Lys
        35


<210>  58
<211>  39
<212>  PRT
<213>  Artificial sequence

<220>
<223>  Huwentoxin-IV variant

<400>  58

Gly Pro Glu Cys Leu Glu Ile Phe Lys Ala Cys Asn Pro Ser Asn Asp
1               5                   10                  15


Gln Cys Cys Lys Ser Ser Lys Leu Val Cys Ser Arg Lys Thr Arg Trp
            20                  25                  30


Cys Lys Tyr Gln Ile Ile Lys
        35


<210>  59
<211>  39
<212>  PRT
<213>  Artificial sequence

<220>
<223>  Huwentoxin-IV variant

<400>  59

Gly Pro Lys Cys Leu Glu Ile Phe Lys Ala Cys Asn Pro Ser Asn Asp
1               5                   10                  15
```

Gln Cys Cys Lys Ser Ser Lys Leu Val Cys Ser Arg Lys Thr Arg Trp
20 25 30

Cys Lys Tyr Gln Ile Gly Lys
35

<210> 60
<211> 39
<212> PRT
<213> Artificial sequence

<220>
<223> Huwentoxin-IV variant

<400> 60

Gly Pro Glu Cys Leu Glu Ile Phe Lys Ala Cys Asn Pro Ser Asn Asp
1 5 10 15

Gln Cys Cys Lys Ser Ser Lys Leu Val Cys Ser Arg Lys Thr His Trp
20 25 30

Cys Lys Tyr Gln Ile Gly Lys
35

<210> 61
<211> 39
<212> PRT
<213> Artificial sequence

<220>
<223> Huwentoxin-IV variant

<400> 61

Gly Pro Glu Cys Leu Glu Ile Phe Lys Ala Cys Asn Pro Ser Gln Asp
1 5 10 15

Gln Cys Cys Lys Ser Ser Lys Leu Val Cys Ser Arg Lys Thr Arg Trp
20 25 30

Cys Lys Tyr Gln Ile Gly Lys
35

<210> 62
<211> 39
<212> PRT
<213> Artificial sequence

<220>
<223> Huwentoxin-IV variant

<400> 62

Gly Pro Glu Cys Leu Tyr Ile Phe Lys Ala Cys Asn Pro Ser Asn Asp

1 5 10 15

Gln Cys Cys Lys Ser Ser Lys Leu Val Cys Ser Arg Lys Thr Arg Trp
20 25 30

Cys Lys Tyr Gln Ile Gly Lys
35

<210> 63
<211> 39
<212> PRT
<213> Artificial sequence

<220>
<223> Huwentoxin-IV variant

<400> 63

Gly Pro Glu Cys Leu Glu Ile Phe Lys Ala Cys Asn Pro Ser Asn Asp
1 5 10 15

Gln Cys Cys Lys Ser Ser Lys Leu Val Cys Ser Arg Lys Thr Arg Trp
20 25 30

Cys Lys Tyr Arg Ile Gly Lys
35

<210> 64
<211> 39
<212> PRT
<213> Artificial sequence

<220>
<223> Huwentoxin-IV variant

<400> 64

Gly Pro Glu Cys Leu Glu Ile Phe Lys Ala Cys Asn Pro Ser Asn Asp
1 5 10 15

Gln Cys Cys Lys Ile Ser Lys Leu Val Cys Ser Arg Lys Thr Arg Trp
20 25 30

Cys Lys Tyr Gln Ile Gly Lys
35

<210> 65
<211> 39
<212> PRT
<213> Artificial sequence

<220>
<223> Huwentoxin-IV variant

<400> 65

Gly Pro Glu Cys Leu Arg Ile Phe Lys Ala Cys Asn Pro Ser Asn Asp
1 5 10 15

Gln Cys Cys Lys Ser Ser Lys Leu Val Cys Ser Lys Lys Thr Arg Trp
20 25 30

Cys Lys Trp Gln Ile Gly Lys
35

<210> 66
<211> 39
<212> PRT
<213> Artificial sequence

<220>
<223> Huwentoxin-IV variant

<400> 66

Gly Pro Glu Cys Leu Glu Ile Phe Lys Ala Cys Asn Pro Ser Asn Asp
1 5 10 15

Gln Cys Cys Lys Leu Ser Lys Leu Val Cys Ser Arg Lys Thr Arg Trp
20 25 30

Cys Lys Tyr Gln Ile Gly Lys
35

<210> 67
<211> 39
<212> PRT
<213> Artificial sequence

<220>
<223> Huwentoxin-IV variant

<400> 67

Gly Pro Asn Cys Leu Glu Ile Phe Lys Ala Cys Asn Pro Ser Asn Asp
1 5 10 15

Gln Cys Cys Lys Ser Ser Lys Leu Val Cys Ser Arg Lys Thr Arg Trp
20 25 30

Cys Lys Tyr Gln Ile Gly Lys
35

<210> 68
<211> 39
<212> PRT
<213> Artificial sequence

<220>
<223> Huwentoxin-IV variant

<400> 68

Gly Pro Glu Cys Leu Glu Ile Phe Lys Ala Cys Asn Pro Ser Asn Asp
1 5 10 15

Gln Cys Cys Lys Ser Ser Ser Leu Val Cys Ser Arg Lys Thr Arg Trp
20 25 30

Cys Lys Tyr Gln Ile Gly Lys
35

<210> 69
<211> 39
<212> PRT
<213> Artificial sequence

<220>
<223> Huwentoxin-IV variant

<400> 69

Gly Pro Glu Cys Leu Glu Ile Phe Arg Ala Cys Asn Pro Ser Asn Asp
1 5 10 15

Gln Cys Cys Lys Ser Ser Lys Leu Val Cys Ser Arg Lys Thr Arg Trp
20 25 30

Cys Lys Tyr Gln Ile Gly Lys
35

<210> 70
<211> 39
<212> PRT
<213> Artificial sequence

<220>
<223> Huwentoxin-IV variant

<400> 70

Gly Pro Glu Cys Ser Glu Ile Phe Lys Ala Cys Asn Pro Ser Asn Asp
1 5 10 15

Gln Cys Cys Lys Ser Ser Lys Leu Val Cys Ser Arg Lys Thr Arg Trp
20 25 30

Cys Lys Tyr Gln Ile Gly Lys
35

<210> 71
<211> 39

<212> PRT
<213> Artificial sequence

<220>
<223> Huwentoxin-IV variant

<400> 71

```
Gly Pro His Cys Leu Glu Ile Phe Lys Ala Cys Asn Pro Ser Asn Asp
1               5                   10                  15

Gln Cys Cys Lys Ser Ser Lys Leu Val Cys Ser Arg Lys Thr Arg Trp
            20                  25                  30

Cys Lys Tyr Gln Ile Gly Lys
        35
```

<210> 72
<211> 39
<212> PRT
<213> Artificial sequence

<220>
<223> Huwentoxin-IV variant

<400> 72

```
Gly Pro Glu Cys Leu Glu Ile Phe Lys Ala Cys Asn Pro Ser Asn Asp
1               5                   10                  15

Gln Cys Cys Lys Ser Ser Lys Leu Val Cys Ser Trp Lys Thr Arg Trp
            20                  25                  30

Cys Lys Tyr Gln Ile Gly Lys
        35
```

<210> 73
<211> 39
<212> PRT
<213> Artificial sequence

<220>
<223> Huwentoxin-IV variant

<400> 73

```
Gly Pro Ile Cys Leu Glu Ile Phe Lys Ala Cys Asn Pro Ser Asn Asp
1               5                   10                  15

Gln Cys Cys Lys Ser Ser Lys Leu Val Cys Ser Arg Lys Thr Arg Trp
            20                  25                  30

Cys Lys Tyr Gln Ile Gly Lys
        35
```

<210>  74
<211>  39
<212>  PRT
<213>  Artificial sequence

<220>
<223>  Huwentoxin-IV variant

<400>  74

Gly Pro Glu Cys Leu Glu Ile Phe Lys Ala Cys Asn Pro Ser Trp Asp
1               5                   10                  15

Gln Cys Cys Lys Ser Ser Lys Leu Val Cys Ser Arg Lys Thr Arg Trp
            20                  25                  30

Cys Lys Tyr Gln Ile Gly Lys
        35

<210>  75
<211>  39
<212>  PRT
<213>  Artificial sequence

<220>
<223>  Huwentoxin-IV variant

<400>  75

Gly Pro Glu Cys Leu Arg Ile Phe Lys Ala Cys Asn Pro Ser Asn Asp
1               5                   10                  15

Gln Cys Cys Lys Ser Ser Lys Leu Val Cys Ser Lys Lys Thr Arg Trp
            20                  25                  30

Cys Lys Tyr Gln Ile Ile Lys
        35

<210>  76
<211>  39
<212>  PRT
<213>  Artificial sequence

<220>
<223>  Huwentoxin-IV variant

<400>  76

Gly Pro Glu Cys Leu Glu Ile Phe Lys Ala Cys Asn Pro Ser Asn Asp
1               5                   10                  15

Gln Cys Cys Lys Ser Ser Lys Leu Val Cys Ser Arg Lys Thr Arg Trp
            20                  25                  30

Cys Lys Thr Gln Ile Gly Lys
35

<210> 77
<211> 39
<212> PRT
<213> Artificial sequence

<220>
<223> Huwentoxin-IV variant

<400> 77

Gly Pro Glu Cys Leu Asp Ile Phe Lys Ala Cys Asn Pro Ser Asn Asp
1 5 10 15

Gln Cys Cys Lys Ser Ser Lys Leu Val Cys Ser Arg Lys Thr Arg Trp
20 25 30

Cys Lys Tyr Gln Ile Gly Lys
35

<210> 78
<211> 39
<212> PRT
<213> Artificial sequence

<220>
<223> Huwentoxin-IV variant

<400> 78

Gly Pro Glu Cys Leu Glu Ile Phe Lys Ala Cys Asn Pro Ser Asn Asp
1 5 10 15

Gln Cys Cys Lys Ser Ser Lys Leu Val Cys Ser Arg Lys Thr Arg Trp
20 25 30

Cys Lys Tyr Gln Ile Leu Lys
35

<210> 79
<211> 39
<212> PRT
<213> Artificial sequence

<220>
<223> Huwentoxin-IV variant

<400> 79

Gly Pro Glu Cys Leu Glu Ile Phe Lys Ala Cys Asn Pro Ser Asn Asp
1 5 10 15

Tyr Cys Cys Lys Ser Ser Lys Leu Val Cys Ser Arg Lys Thr Arg Trp

20 25 30

Cys Lys Tyr Gln Ile Gly Lys
35

<210> 80
<211> 39
<212> PRT
<213> Artificial sequence

<220>
<223> Huwentoxin-IV variant

<400> 80

Gly Pro Glu Cys Leu Glu Ile Phe Lys Ala Cys Asn Pro Ser Asn Asp
1 5 10 15

Gln Cys Cys Lys Ser Ser Lys Leu Val Cys Ser Arg Lys Thr Arg Trp
20 25 30

Cys Lys Tyr Tyr Ile Gly Lys
35

<210> 81
<211> 39
<212> PRT
<213> Artificial sequence

<220>
<223> Huwentoxin-IV variant

<400> 81

Gly Pro Glu Cys Leu Glu Ile Phe Lys Ala Cys Asn Pro Ser Asn Asp
1 5 10 15

Gln Cys Cys Lys Lys Ser Lys Leu Val Cys Ser Arg Lys Thr Arg Trp
20 25 30

Cys Lys Tyr Gln Ile Gly Lys
35

<210> 82
<211> 39
<212> PRT
<213> Artificial sequence

<220>
<223> Huwentoxin-IV variant

<400> 82

Gly Pro Glu Cys Leu Glu Ile Phe Lys Ala Cys Asn Pro Ser Asn Asp
1 5 10 15

Gln Cys Cys Lys Ser Ser Trp Leu Val Cys Ser Arg Lys Thr Arg Trp
20 25 30

Cys Lys Tyr Gln Ile Gly Lys
35

<210> 83
<211> 39
<212> PRT
<213> Artificial sequence

<220>
<223> Huwentoxin-IV variant

<400> 83

Gly Pro Asn Cys Leu Glu Ile Phe Lys Ala Cys Asn Pro Ser Asn Asp
1 5 10 15

Gln Cys Cys Lys Ser Ser Lys Leu Val Cys Ser Arg Lys Thr Arg Trp
20 25 30

Cys Lys Trp Ser Ile Ile Lys
35

<210> 84
<211> 39
<212> PRT
<213> Artificial sequence

<220>
<223> Huwentoxin-IV variant

<400> 84

Gly Pro Asn Cys Leu Arg Ile Phe Lys Ala Cys Asn Pro Ser Asn Asp
1 5 10 15

Gln Cys Cys Lys Ser Ser Lys Leu Val Cys Ser Lys Lys Thr Arg Trp
20 25 30

Cys Lys Trp Ser Ile Gly Lys
35

<210> 85
<211> 39
<212> PRT
<213> Artificial sequence

<220>
<223> Huwentoxin-IV variant

<400> 85

```
Gly Pro Glu Cys Leu Leu Ile Phe Lys Ala Cys Asn Pro Ser Asn Asp
1               5                   10                  15

Gln Cys Cys Lys Ser Ser Lys Leu Val Cys Ser Arg Lys Thr Arg Trp
            20                  25                  30

Cys Lys Tyr Gln Ile Gly Lys
        35
```

<210>  86
<211>  39
<212>  PRT
<213>  Artificial sequence

<220>
<223>  Huwentoxin-IV variant

<400>  86

```
Gly Pro Gly Cys Leu Glu Ile Phe Lys Ala Cys Asn Pro Ser Asn Asp
1               5                   10                  15

Gln Cys Cys Lys Ser Ser Lys Leu Val Cys Ser Arg Lys Thr Arg Trp
            20                  25                  30

Cys Lys Tyr Gln Ile Gly Lys
        35
```

<210>  87
<211>  39
<212>  PRT
<213>  Artificial sequence

<220>
<223>  Huwentoxin-IV variant

<400>  87

```
Gly Pro Glu Cys Leu Glu Ile Phe Lys Ala Cys Asn Pro Ser Asn Asp
1               5                   10                  15

Thr Cys Cys Lys Ser Ser Lys Leu Val Cys Ser Arg Lys Thr Arg Trp
            20                  25                  30

Cys Lys Tyr Gln Ile Gly Lys
        35
```

<210>  88
<211>  39
<212>  PRT
<213>  Artificial sequence

<220>

<223> Huwentoxin-IV variant

<400> 88

Gly Pro Glu Cys Leu Glu Ile Phe Lys Ala Cys Asn Pro Ser Asn Asp
1               5                   10                  15

Gln Cys Cys Lys Val Ser Lys Leu Val Cys Ser Arg Lys Thr Arg Trp
            20                  25                  30

Cys Lys Tyr Gln Ile Gly Lys
        35

<210> 89
<211> 39
<212> PRT
<213> Artificial sequence

<220>
<223> Huwentoxin-IV variant

<400> 89

Gly Pro Glu Cys Leu Glu Ile Phe Lys Ala Cys Asn Pro Ser Asn Asp
1               5                   10                  15

Gln Cys Cys Lys Ser Ser Lys Leu Val Cys Ser Arg Lys Thr Arg Trp
            20                  25                  30

Cys Lys Tyr Gln Ile Trp Lys
        35

<210> 90
<211> 39
<212> PRT
<213> Artificial sequence

<220>
<223> Huwentoxin-IV variant

<400> 90

Gly Pro Glu Cys Leu Glu Ile Phe Lys Ala Cys Asn Pro Ser Asn Asp
1               5                   10                  15

Gln Cys Cys Lys Ser Ser Lys Leu Thr Cys Ser Arg Lys Thr Arg Trp
            20                  25                  30

Cys Lys Tyr Gln Ile Gly Lys
        35

<210> 91
<211> 39
<212> PRT

<213> Artificial sequence

<220>
<223> Huwentoxin-IV variant

<400> 91

Gly Pro Asn Cys Leu Arg Ile Phe Lys Ala Cys Asn Pro Ser Asn Asp
1 5 10 15

Gln Cys Cys Lys Ser Ser Lys Leu Val Cys Ser Lys Lys Thr Arg Trp
20 25 30

Cys Lys Trp Gln Ile Gly Lys
35

<210> 92
<211> 39
<212> PRT
<213> Artificial sequence

<220>
<223> Huwentoxin-IV variant

<400> 92

Gly Pro Glu Cys Leu Glu Ile Phe Lys Ala Cys Asn Pro Ser Asn Asp
1 5 10 15

Gln Cys Cys Lys Ser Ser Lys Leu Val Cys Ser Arg Lys Thr Arg Trp
20 25 30

Cys Lys Tyr Gln Ile Gly Gly
35

<210> 93
<211> 39
<212> PRT
<213> Artificial sequence

<220>
<223> Huwentoxin-IV variant

<400> 93

Gly Pro Gln Cys Leu Glu Ile Phe Lys Ala Cys Asn Pro Ser Asn Asp
1 5 10 15

Gln Cys Cys Lys Ser Ser Lys Leu Val Cys Ser Arg Lys Thr Arg Trp
20 25 30

Cys Lys Tyr Gln Ile Gly Lys
35

<210> 94
<211> 39
<212> PRT
<213> Artificial sequence

<220>
<223> Huwentoxin-IV variant

<400> 94

Gly Pro Glu Cys Leu Arg Ile Phe Lys Ala Cys Asn Pro Ser Asn Asp
1 5 10 15

Gln Cys Cys Lys Ser Ser Lys Leu Val Cys Ser Arg Lys Thr Arg Trp
20 25 30

Cys Lys Trp Gln Ile Ile Lys
35

<210> 95
<211> 39
<212> PRT
<213> Artificial sequence

<220>
<223> Huwentoxin-IV variant

<400> 95

Gly Pro Glu Cys Leu Arg Ile Phe Lys Ala Cys Asn Pro Ser Asn Asp
1 5 10 15

Gln Cys Cys Lys Ser Ser Lys Leu Val Cys Ser Arg Lys Thr Arg Trp
20 25 30

Cys Lys Trp Ser Ile Ile Lys
35

<210> 96
<211> 39
<212> PRT
<213> Artificial sequence

<220>
<223> Huwentoxin-IV variant

<400> 96

Gly Pro Glu Cys Leu Glu Ile Phe Lys Ala Cys Asn Pro Ser Asn Asp
1 5 10 15

Gln Cys Cys Lys Ser Ser Lys Leu Val Cys Ser Arg Lys Thr Arg Trp
20 25 30

Cys Lys Tyr Gln Ile Lys Lys

35

<210> 97
<211> 39
<212> PRT
<213> Artificial sequence

<220>
<223> Huwentoxin-IV variant

<400> 97

Gly Pro Glu Cys Leu Glu Ile Phe Lys Ala Cys Asn Pro Ser Asn Asp
1 5 10 15

Gln Cys Cys Lys Asn Ser Lys Leu Val Cys Ser Arg Lys Thr Arg Trp
20 25 30

Cys Lys Tyr Gln Ile Gly Lys
35

<210> 98
<211> 39
<212> PRT
<213> Artificial sequence

<220>
<223> Huwentoxin-IV variant

<400> 98

Gly Pro Glu Cys Leu Glu Ile Phe Lys Ala Cys Asn Pro Ser Asn Asp
1 5 10 15

Gln Cys Cys Lys Ser Thr Lys Leu Val Cys Ser Arg Lys Thr Arg Trp
20 25 30

Cys Lys Tyr Gln Ile Gly Lys
35

<210> 99
<211> 39
<212> PRT
<213> Artificial sequence

<220>
<223> Huwentoxin-IV variant

<400> 99

Gly Pro Glu Cys Leu Glu Ile Phe Lys Ala Cys Asn Pro Ser Asn Asp
1 5 10 15

Gln Cys Cys Lys Ser Ser Leu Leu Val Cys Ser Arg Lys Thr Arg Trp
20 25 30

Cys Lys Tyr Gln Ile Gly Lys
35

<210> 100
<211> 39
<212> PRT
<213> Artificial sequence

<220>
<223> Huwentoxin-IV variant

<400> 100

Gly Pro Glu Cys Leu Glu Ile Phe Lys Ala Cys Asn Pro Ser Asn Asp
1 5 10 15

Gln Cys Cys Lys Ser His Lys Leu Val Cys Ser Arg Lys Thr Arg Trp
20 25 30

Cys Lys Tyr Gln Ile Gly Lys
35

<210> 101
<211> 39
<212> PRT
<213> Artificial sequence

<220>
<223> Huwentoxin-IV variant

<400> 101

Gly Pro Glu Cys Leu Glu Ile Phe Lys Ala Cys Asn Pro Ser Asn Asp
1 5 10 15

Ala Cys Cys Lys Ser Ser Lys Leu Val Cys Ser Arg Lys Thr Arg Trp
20 25 30

Cys Lys Tyr Gln Ile Gly Lys
35

<210> 102
<211> 39
<212> PRT
<213> Artificial sequence

<220>
<223> Huwentoxin-IV variant

<400> 102

Gly Pro Asn Cys Leu Glu Ile Phe Lys Ala Cys Asn Pro Ser Asn Asp
1 5 10 15

Gln Cys Cys Lys Ser Ser Lys Leu Val Cys Ser Arg Lys Thr Arg Trp
20 25 30

Cys Lys Tyr Ser Ile Gly Lys
35

<210> 103
<211> 39
<212> PRT
<213> Artificial sequence

<220>
<223> Huwentoxin-IV variant

<400> 103

Gly Pro Glu Cys Leu Glu Ile Phe Lys Ala Cys Asn Pro Ser Asn Asp
1 5 10 15

Gln Cys Cys Lys Ser Ser Lys Leu Val Cys Ser Arg Lys Thr Arg Trp
20 25 30

Cys Lys Tyr Gln Ile Gly His
35

<210> 104
<211> 39
<212> PRT
<213> Artificial sequence

<220>
<223> Huwentoxin-IV variant

<400> 104

Gly Pro Glu Cys Leu Glu Ile Phe Lys Ala Cys Asn Pro Ser Asn Asp
1 5 10 15

Gln Cys Cys Lys Tyr Ser Lys Leu Val Cys Ser Arg Lys Thr Arg Trp
20 25 30

Cys Lys Tyr Gln Ile Gly Lys
35

<210> 105
<211> 39
<212> PRT
<213> Artificial sequence

<220>
<223> Huwentoxin-IV variant

<400> 105

```
Gly Pro Glu Cys Leu Glu Ile Phe Lys Ala Cys Asn Pro Ser Asn Asp
1               5                   10                  15

Gln Cys Cys Ile Ser Ser Lys Leu Val Cys Ser Arg Lys Thr Arg Trp
            20                  25                  30

Cys Lys Tyr Gln Ile Gly Lys
        35
```

```
<210>  106
<211>  39
<212>  PRT
<213>  Artificial sequence

<220>
<223>  Huwentoxin-IV variant

<400>  106

Gly Pro Glu Cys Leu Glu Ile Phe Lys Ala Cys Asn Pro Ser Asn Asp
1               5                   10                  15

Gln Cys Cys Lys Phe Ser Lys Leu Val Cys Ser Arg Lys Thr Arg Trp
            20                  25                  30

Cys Lys Tyr Gln Ile Gly Lys
        35
```

```
<210>  107
<211>  39
<212>  PRT
<213>  Artificial sequence

<220>
<223>  Huwentoxin-IV variant

<400>  107

Gly Pro Glu Cys Leu Glu Ile Phe Lys Ala Cys Asn Pro Ser Thr Asp
1               5                   10                  15

Gln Cys Cys Lys Ser Ser Lys Leu Val Cys Ser Arg Lys Thr Arg Trp
            20                  25                  30

Cys Lys Tyr Gln Ile Gly Lys
        35
```

```
<210>  108
<211>  39
<212>  PRT
<213>  Artificial sequence

<220>
<223>  Huwentoxin-IV variant
```

<400> 108

Gly Pro Glu Cys Leu Glu Ile Phe Lys Ala Cys Asn Pro Ser Asp Asp
1 5 10 15

Gln Cys Cys Lys Ser Ser Lys Leu Val Cys Ser Arg Lys Thr Arg Trp
20 25 30

Cys Lys Tyr Gln Ile Gly Lys
35

<210> 109
<211> 39
<212> PRT
<213> Artificial sequence

<220>
<223> Huwentoxin-IV variant

<400> 109

Gly Pro Glu Cys Leu Glu Ile Phe Lys Ala Cys Asn Pro Ser Asn Asp
1 5 10 15

Gln Cys Cys Lys Ser Ser Lys Leu Val Cys Ser Arg Lys Thr Arg Lys
20 25 30

Cys Lys Tyr Gln Ile Gly Lys
35

<210> 110
<211> 39
<212> PRT
<213> Artificial sequence

<220>
<223> Huwentoxin-IV variant

<400> 110

Gly Pro Glu Cys Leu Glu Ile Phe Lys Ala Cys Asn Pro Ser Asn Asp
1 5 10 15

Gln Cys Cys Lys Ser Ser Lys Leu Val Cys Ser Arg Lys Thr Arg Trp
20 25 30

Cys Lys Ile Gln Ile Gly Lys
35

<210> 111
<211> 39
<212> PRT
<213> Artificial sequence

<220>
<223>  Huwentoxin-IV variant

<400>  111

Gly Pro Glu Cys Leu Glu Ile Phe Lys Ala Cys Asn Pro Ser Asn Asp
1               5                   10                  15

Gln Cys Cys Lys Ser Ser Lys Leu Val Cys Ser Arg Lys Thr Arg Trp
            20                  25                  30

Cys Lys Tyr Ala Ile Gly Lys
        35

<210>  112
<211>  39
<212>  PRT
<213>  Artificial sequence

<220>
<223>  Huwentoxin-IV variant

<400>  112

Gly Pro Asn Cys Leu Glu Ile Phe Lys Ala Cys Asn Pro Ser Asn Asp
1               5                   10                  15

Gln Cys Cys Lys Ser Ser Lys Leu Val Cys Ser Arg Lys Thr Arg Trp
            20                  25                  30

Cys Lys Tyr Ser Ile Ile Lys
        35

<210>  113
<211>  39
<212>  PRT
<213>  Artificial sequence

<220>
<223>  Huwentoxin-IV variant

<400>  113

Gly Pro Glu Cys Leu Glu Ile Phe Lys Ala Cys Asn Pro Ser Asn Asp
1               5                   10                  15

Trp Cys Cys Lys Ser Ser Lys Leu Val Cys Ser Arg Lys Thr Arg Trp
            20                  25                  30

Cys Lys Tyr Gln Ile Gly Lys
        35

<210>  114

<211> 39
<212> PRT
<213> Artificial sequence

<220>
<223> Huwentoxin-IV variant

<400> 114

```
Gly Pro Leu Cys Leu Glu Ile Phe Lys Ala Cys Asn Pro Ser Asn Asp
1               5                   10                  15

Gln Cys Cys Lys Ser Ser Lys Leu Val Cys Ser Arg Lys Thr Arg Trp
            20                  25                  30

Cys Lys Tyr Gln Ile Gly Lys
        35
```

<210> 115
<211> 39
<212> PRT
<213> Artificial sequence

<220>
<223> Huwentoxin-IV variant

<400> 115

```
Gly Pro Ser Cys Leu Glu Ile Phe Lys Ala Cys Asn Pro Ser Asn Asp
1               5                   10                  15

Gln Cys Cys Lys Ser Ser Lys Leu Val Cys Ser Arg Lys Thr Arg Trp
            20                  25                  30

Cys Lys Tyr Gln Ile Gly Lys
        35
```

<210> 116
<211> 39
<212> PRT
<213> Artificial sequence

<220>
<223> Huwentoxin-IV variant

<400> 116

```
Gly Pro Glu Cys Leu Pro Ile Phe Lys Ala Cys Asn Pro Ser Asn Asp
1               5                   10                  15

Gln Cys Cys Lys Ser Ser Lys Leu Val Cys Ser Arg Lys Thr Arg Trp
            20                  25                  30

Cys Lys Tyr Gln Ile Gly Lys
        35
```

<210> 117
<211> 39
<212> PRT
<213> Artificial sequence

<220>
<223> Huwentoxin-IV variant

<400> 117

Gly Pro Glu Cys Leu Glu Ile Phe Lys Ala Cys Asn Pro Ser Asn Asp
1 5 10 15

Gln Cys Cys Thr Ser Ser Lys Leu Val Cys Ser Arg Lys Thr Arg Trp
20 25 30

Cys Lys Tyr Gln Ile Gly Lys
35

<210> 118
<211> 39
<212> PRT
<213> Artificial sequence

<220>
<223> Huwentoxin-IV variant

<400> 118

Gly Pro Glu Cys Leu Glu Ile Phe Lys Tyr Cys Asn Pro Ser Asn Asp
1 5 10 15

Gln Cys Cys Lys Ser Ser Lys Leu Val Cys Ser Arg Lys Thr Arg Trp
20 25 30

Cys Lys Tyr Gln Ile Gly Lys
35

<210> 119
<211> 39
<212> PRT
<213> Artificial sequence

<220>
<223> Huwentoxin-IV variant

<400> 119

Gly Pro Glu Cys Asn Glu Ile Phe Lys Ala Cys Asn Pro Ser Asn Asp
1 5 10 15

Gln Cys Cys Lys Ser Ser Lys Leu Val Cys Ser Arg Lys Thr Arg Trp
20 25 30

Cys Lys Tyr Gln Ile Gly Lys
35

<210> 120
<211> 39
<212> PRT
<213> Artificial sequence

<220>
<223> Huwentoxin-IV variant

<400> 120

Gly Pro Glu Cys Leu Glu Ile Phe Lys Ala Cys Arg Pro Ser Asn Asp
1 5 10 15

Gln Cys Cys Lys Ser Ser Lys Leu Val Cys Ser Arg Lys Thr Arg Trp
20 25 30

Cys Lys Tyr Gln Ile Gly Lys
35

<210> 121
<211> 39
<212> PRT
<213> Artificial sequence

<220>
<223> Huwentoxin-IV variant

<400> 121

Gly Pro Glu Cys Leu Glu Ile Phe Lys Ala Cys Asn Pro Ser Asn Asp
1 5 10 15

Gln Cys Cys Lys Ser Ser Lys Leu Val Cys Ser Arg Lys Thr Arg Trp
20 25 30

Cys Lys Tyr Gln Ile Gly Trp
35

<210> 122
<211> 39
<212> PRT
<213> Artificial sequence

<220>
<223> Huwentoxin-IV variant

<400> 122

Gly Pro Glu Cys Leu Glu Ile Phe Lys Ala Cys Asn Pro Ser Asn Asp
1 5 10 15

Gln Cys Cys Lys Ser Ser Lys Leu Ala Cys Ser Arg Lys Thr Arg Trp
20 25 30

Cys Lys Tyr Gln Ile Gly Lys
35

<210> 123
<211> 39
<212> PRT
<213> Artificial sequence

<220>
<223> Huwentoxin-IV variant

<400> 123

Gly Pro Glu Cys Leu Glu Ile Phe Lys Ala Cys Asn Pro Ser Tyr Asp
1 5 10 15

Gln Cys Cys Lys Ser Ser Lys Leu Val Cys Ser Arg Lys Thr Arg Trp
20 25 30

Cys Lys Tyr Gln Ile Gly Lys
35

<210> 124
<211> 39
<212> PRT
<213> Artificial sequence

<220>
<223> Huwentoxin-IV variant

<400> 124

Gly Pro Glu Cys Leu Glu Ile Phe Lys Ala Cys Asn Pro Ser Asn Asp
1 5 10 15

Gln Cys Cys Lys Ser Ser Gln Leu Val Cys Ser Arg Lys Thr Arg Trp
20 25 30

Cys Lys Tyr Gln Ile Gly Lys
35

<210> 125
<211> 39
<212> PRT
<213> Artificial sequence

<220>
<223> Huwentoxin-IV variant

<400> 125

Gly Pro Glu Cys Leu Glu Ile Phe Lys Ala Cys Asn Pro Ser Asn Asp

1 5 10 15

Gln Cys Cys Lys Ala Ser Lys Leu Val Cys Ser Arg Lys Thr Arg Trp
20 25 30

Cys Lys Tyr Gln Ile Gly Lys
35

<210> 126
<211> 39
<212> PRT
<213> Artificial sequence

<220>
<223> Huwentoxin-IV variant

<400> 126

Gly Pro Glu Cys Leu Glu Ile Phe Lys Ala Cys Asn Pro Ser Asn Asp
1 5 10 15

Gln Cys Cys Lys Ser Ile Lys Leu Val Cys Ser Arg Lys Thr Arg Trp
20 25 30

Cys Lys Tyr Gln Ile Gly Lys
35

<210> 127
<211> 39
<212> PRT
<213> Artificial sequence

<220>
<223> Huwentoxin-IV variant

<400> 127

Gly Pro Glu Cys Leu Glu Ile Phe Lys Ala Cys Asn Pro Ser Asn Asp
1 5 10 15

Gln Cys Cys Lys Ser Ser Lys Leu Val Cys Ser Arg Lys Thr Arg Trp
20 25 30

Cys Lys Trp Ser Ile Ile Lys
35

<210> 128
<211> 39
<212> PRT
<213> Artificial sequence

<220>
<223> Huwentoxin-IV variant

<400> 128

Gly Pro Thr Cys Leu Glu Ile Phe Lys Ala Cys Asn Pro Ser Asn Asp
1 5 10 15

Gln Cys Cys Lys Ser Ser Lys Leu Val Cys Ser Arg Lys Thr Arg Trp
20 25 30

Cys Lys Tyr Gln Ile Gly Lys
35

<210> 129
<211> 39
<212> PRT
<213> Artificial sequence

<220>
<223> Huwentoxin-IV variant

<400> 129

Gly Pro Asn Cys Leu Glu Ile Phe Lys Ala Cys Asn Pro Ser Asn Asp
1 5 10 15

Gln Cys Cys Lys Ser Ser Lys Leu Val Cys Ser Lys Lys Thr Arg Trp
20 25 30

Cys Lys Trp Ser Ile Gly Lys
35

<210> 130
<211> 39
<212> PRT
<213> Artificial sequence

<220>
<223> Huwentoxin-IV variant

<400> 130

Gly Pro Glu Cys Leu Glu Ile Phe Lys Ala Cys Asn Pro Arg Asn Asp
1 5 10 15

Gln Cys Cys Lys Ser Ser Lys Leu Val Cys Ser Arg Lys Thr Arg Trp
20 25 30

Cys Lys Tyr Gln Ile Gly Lys
35

<210> 131
<211> 39
<212> PRT
<213> Artificial sequence

<220>
<223> Huwentoxin-IV variant

<400> 131

Gly Pro Glu Cys Leu Glu Ile Phe Lys Ala Cys Asn Pro Ser Asn Asp
1 5 10 15

Gln Cys Cys Lys Ser Trp Lys Leu Val Cys Ser Arg Lys Thr Arg Trp
20 25 30

Cys Lys Tyr Gln Ile Gly Lys
35

<210> 132
<211> 39
<212> PRT
<213> Artificial sequence

<220>
<223> Huwentoxin-IV variant

<400> 132

Gly Pro Asn Cys Leu Arg Ile Phe Lys Ala Cys Asn Pro Ser Asn Asp
1 5 10 15

Gln Cys Cys Lys Ser Ser Lys Leu Val Cys Ser Arg Lys Thr Arg Trp
20 25 30

Cys Lys Trp Gln Ile Gly Lys
35

<210> 133
<211> 39
<212> PRT
<213> Artificial sequence

<220>
<223> Huwentoxin-IV variant

<400> 133

Gly Pro Glu Cys Leu Glu Ile Phe Lys Ala Cys Asn Pro Ser Asn Asp
1 5 10 15

Gln Cys Cys Lys Ser Ser Lys Leu Val Cys Ser Arg Tyr Thr Arg Trp
20 25 30

Cys Lys Tyr Gln Ile Gly Lys
35

<210> 134
<211> 39

<212> PRT
<213> Artificial sequence

<220>
<223> Huwentoxin-IV variant

<400> 134

Gly Pro Glu Cys Leu Glu Ile Phe Lys Ala Cys Asn Pro Ser Asn Asp
1 5 10 15

Gln Cys Cys Lys Ser Val Lys Leu Val Cys Ser Arg Lys Thr Arg Trp
20 25 30

Cys Lys Tyr Gln Ile Gly Lys
35

<210> 135
<211> 39
<212> PRT
<213> Artificial sequence

<220>
<223> Huwentoxin-IV variant

<400> 135

Gly Pro Glu Cys Leu Glu Ile Phe Lys Ala Cys Asn Pro Ser Asn Asp
1 5 10 15

Gln Cys Cys Lys Ser Ser Lys Leu Leu Cys Ser Arg Lys Thr Arg Trp
20 25 30

Cys Lys Tyr Gln Ile Gly Lys
35

<210> 136
<211> 39
<212> PRT
<213> Artificial sequence

<220>
<223> Huwentoxin-IV variant

<400> 136

Gly Pro Glu Cys Leu Glu Ile Phe Lys Ala Cys Asn Pro Ser Asn Asp
1 5 10 15

Gln Cys Cys Lys Ser Ser Lys Leu Val Cys Ser Arg Lys Thr Arg Trp
20 25 30

Cys Lys Tyr Ser Ile Ile Lys
35

<210> 137
<211> 39
<212> PRT
<213> Artificial sequence

<220>
<223> Huwentoxin-IV variant

<400> 137

```
Gly Pro Glu Cys Leu Glu Ile Phe Lys Ala Cys Asn Pro Ser Gln Asp
1               5                   10                  15

Gln Cys Cys Lys Ser Ser Lys Leu Val Cys Ser Arg Lys Thr Lys Trp
            20                  25                  30

Cys Lys Tyr Gln Ile Gly Arg
        35
```

<210> 138
<211> 39
<212> PRT
<213> Artificial sequence

<220>
<223> Huwentoxin-IV variant

<400> 138

```
Gly Pro Glu Cys Leu Glu Ile Phe Lys Ala Cys Asn Pro Ser Asn Asp
1               5                   10                  15

Gln Cys Cys Lys Ser Ser Lys Leu Val Cys Ser Lys Lys Thr Arg Trp
            20                  25                  30

Cys Lys Trp Ser Ile Gly Lys
        35
```

<210> 139
<211> 39
<212> PRT
<213> Artificial sequence

<220>
<223> Huwentoxin-IV variant

<400> 139

```
Gly Pro Phe Cys Leu Glu Ile Phe Lys Ala Cys Asn Pro Ser Asn Asp
1               5                   10                  15

Gln Cys Cys Lys Ser Ser Lys Leu Val Cys Ser Arg Lys Thr Arg Trp
            20                  25                  30
```

Cys Lys Tyr Gln Ile Gly Lys
        35

<210>  140
<211>  39
<212>  PRT
<213>  Artificial sequence

<220>
<223>  Huwentoxin-IV variant

<400>  140

Gly Pro Glu Cys Leu Glu Ile Phe Lys Ala Cys Asn Pro Ser Asn Asp
1               5                   10                  15

Gln Cys Cys Ser Ser Ser Lys Leu Val Cys Ser Arg Lys Thr Arg Trp
            20                  25                  30

Cys Lys Tyr Gln Ile Gly Lys
        35

<210>  141
<211>  39
<212>  PRT
<213>  Artificial sequence

<220>
<223>  Huwentoxin-IV variant

<400>  141

Gly Pro Glu Cys Leu Glu Ile Phe Lys Ala Cys Asn Pro Ser Asn Asp
1               5                   10                  15

Gln Cys Cys Gln Ser Ser Lys Leu Val Cys Ser Arg Lys Thr Arg Trp
            20                  25                  30

Cys Lys Tyr Gln Ile Gly Lys
        35

<210>  142
<211>  39
<212>  PRT
<213>  Artificial sequence

<220>
<223>  Huwentoxin-IV variant

<400>  142

Gly Pro Glu Cys Leu Glu Ile Phe Lys Ala Cys Asn Pro Ser Ile Asp
1               5                   10                  15

Gln Cys Cys Lys Ser Ser Lys Leu Val Cys Ser Arg Lys Thr Arg Trp

20 25 30

Cys Lys Tyr Gln Ile Gly Lys
35

<210> 143
<211> 39
<212> PRT
<213> Artificial sequence

<220>
<223> Huwentoxin-IV variant

<400> 143

Gly Pro Glu Cys Leu Glu Ile Phe Lys Ala Cys Asn Pro Ser Asn Asp
1 5 10 15

Gln Cys Cys Lys Ser Ser Lys Pro Val Cys Ser Arg Lys Thr Arg Trp
20 25 30

Cys Lys Tyr Gln Ile Gly Lys
35

<210> 144
<211> 39
<212> PRT
<213> Artificial sequence

<220>
<223> Huwentoxin-IV variant

<400> 144

Gly Pro Glu Cys Leu Glu Ile Phe Lys Ala Cys Val Pro Ser Asn Asp
1 5 10 15

Gln Cys Cys Lys Ser Ser Lys Leu Val Cys Ser Arg Lys Thr Arg Trp
20 25 30

Cys Lys Tyr Gln Ile Gly Lys
35

<210> 145
<211> 39
<212> PRT
<213> Artificial sequence

<220>
<223> Huwentoxin-IV variant

<400> 145

Gly Pro Glu Cys Leu Glu Ile Phe Ser Ala Cys Asn Pro Ser Asn Asp
1 5 10 15

Gln Cys Cys Lys Ser Ser Lys Leu Val Cys Ser Arg Lys Thr Arg Trp
20 25 30

Cys Lys Tyr Gln Ile Gly Lys
35

<210> 146
<211> 39
<212> PRT
<213> Artificial sequence

<220>
<223> Huwentoxin-IV variant

<400> 146

Gly Pro Glu Cys Leu Glu Ile Phe Lys Ala Cys Asn Pro Ser Asn Asp
1 5 10 15

Gln Cys Cys Lys Ser Ser Lys Tyr Val Cys Ser Arg Lys Thr Arg Trp
20 25 30

Cys Lys Tyr Gln Ile Gly Lys
35

<210> 147
<211> 39
<212> PRT
<213> Artificial sequence

<220>
<223> Huwentoxin-IV variant

<400> 147

Gly Pro Glu Cys Trp Glu Ile Phe Lys Ala Cys Asn Pro Ser Asn Asp
1 5 10 15

Gln Cys Cys Lys Ser Ser Lys Leu Val Cys Ser Arg Lys Thr Arg Trp
20 25 30

Cys Lys Tyr Gln Ile Gly Lys
35

<210> 148
<211> 39
<212> PRT
<213> Artificial sequence

<220>
<223> Huwentoxin-IV variant

<400> 148

Gly Pro Glu Cys Leu Glu Ile Phe Lys Ala Cys Asn Pro Ser Asn Asp
1 5 10 15

Gln Cys Cys Gly Ser Ser Lys Leu Val Cys Ser Arg Lys Thr Arg Trp
20 25 30

Cys Lys Tyr Gln Ile Gly Lys
35

<210> 149
<211> 39
<212> PRT
<213> Artificial sequence

<220>
<223> Huwentoxin-IV variant

<400> 149

Gly Pro Glu Cys Leu Glu Ile Phe Lys Ala Cys Asn Pro Ser Asn Asp
1 5 10 15

Gln Cys Cys Lys Ser Ser Lys Leu Val Cys Ser Arg Lys Thr Asn Trp
20 25 30

Cys Lys Tyr Gln Ile Gly Lys
35

<210> 150
<211> 39
<212> PRT
<213> Artificial sequence

<220>
<223> Huwentoxin-IV variant

<400> 150

Gly Pro Glu Cys Leu Glu Ile Phe Lys Ala Cys Asn Pro Ser Asn Asp
1 5 10 15

Gln Cys Cys Lys Ser Ser Lys Leu Val Cys Ser Arg Lys Thr Arg Trp
20 25 30

Cys Lys Tyr Gln Ile Arg Lys
35

<210> 151
<211> 39
<212> PRT
<213> Artificial sequence

<220>

<223> Huwentoxin-IV variant

<400> 151

Gly Pro Glu Cys Leu Glu Ile Phe Lys Ala Cys Asn Pro Ser Asn Asp
1 5 10 15

Gln Cys Cys Lys Ser Ser Lys Leu Arg Cys Ser Arg Lys Thr Arg Trp
20 25 30

Cys Lys Tyr Gln Ile Gly Lys
35

<210> 152
<211> 39
<212> PRT
<213> Artificial sequence

<220>
<223> Huwentoxin-IV variant

<400> 152

Gly Pro Glu Cys Leu Glu Ile Phe Lys Ala Cys Asn Pro Ser Asn Asp
1 5 10 15

Gln Cys Cys Leu Ser Ser Lys Leu Val Cys Ser Arg Lys Thr Arg Trp
20 25 30

Cys Lys Tyr Gln Ile Gly Lys
35

<210> 153
<211> 39
<212> PRT
<213> Artificial sequence

<220>
<223> Huwentoxin-IV variant

<400> 153

Gly Pro Glu Cys Leu Glu Ile Phe Lys Ala Cys Asn Pro Ser Asn Asp
1 5 10 15

Gln Cys Cys Lys Ser Ser Gly Leu Val Cys Ser Arg Lys Thr Arg Trp
20 25 30

Cys Lys Tyr Gln Ile Gly Lys
35

<210> 154
<211> 39
<212> PRT

<213> Artificial sequence

<220>
<223> Huwentoxin-IV variant

<400> 154

Gly Pro Glu Cys Leu Glu Ile Phe Lys Ala Cys Asn Pro Ser Asn Asp
1 5 10 15

Gln Cys Cys Lys Ser Ser Lys Leu Val Cys Ser Arg Lys Thr Arg Trp
20 25 30

Cys Lys Tyr Lys Ile Gly Lys
35

<210> 155
<211> 39
<212> PRT
<213> Artificial sequence

<220>
<223> Huwentoxin-IV variant

<400> 155

Gly Pro Asn Cys Leu Glu Ile Phe Lys Ala Cys Asn Pro Ser Asn Asp
1 5 10 15

Gln Cys Cys Lys Ser Ser Lys Leu Val Cys Ser Arg Lys Thr Arg Trp
20 25 30

Cys Lys Tyr Gln Ile Ile Lys
35

<210> 156
<211> 39
<212> PRT
<213> Artificial sequence

<220>
<223> Huwentoxin-IV variant

<400> 156

Gly Pro Glu Cys Phe Glu Ile Phe Lys Ala Cys Asn Pro Ser Asn Asp
1 5 10 15

Gln Cys Cys Lys Ser Ser Lys Leu Val Cys Ser Arg Lys Thr Arg Trp
20 25 30

Cys Lys Tyr Gln Ile Gly Lys
35

<210> 157
<211> 39
<212> PRT
<213> Artificial sequence

<220>
<223> Huwentoxin-IV variant

<400> 157

```
Gly Pro Glu Cys Leu Glu Val Phe Lys Ala Cys Asn Pro Ser Asn Asp
1               5                   10                  15

Gln Cys Cys Lys Ser Ser Lys Leu Val Cys Ser Arg Lys Thr Arg Trp
            20                  25                  30

Cys Lys Tyr Gln Ile Gly Lys
        35
```

<210> 158
<211> 39
<212> PRT
<213> Artificial sequence

<220>
<223> Huwentoxin-IV variant

<400> 158

```
Gly Pro Asn Cys Leu Glu Ile Phe Lys Ala Cys Asn Pro Ser Asn Asp
1               5                   10                  15

Gln Cys Cys Lys Ser Ser Lys Leu Val Cys Ser Lys Lys Thr Arg Trp
            20                  25                  30

Cys Lys Tyr Ser Ile Gly Lys
        35
```

<210> 159
<211> 39
<212> PRT
<213> Artificial sequence

<220>
<223> Huwentoxin-IV variant

<400> 159

```
Gly Pro Glu Cys Leu Glu Ile Phe Lys Ala Cys Asn Pro Ser Asn Asp
1               5                   10                  15

Gln Cys Cys Lys Ser Gln Lys Leu Val Cys Ser Arg Lys Thr Arg Trp
            20                  25                  30

Cys Lys Tyr Gln Ile Gly Lys
```

35

<210> 160
<211> 39
<212> PRT
<213> Artificial sequence

<220>
<223> Huwentoxin-IV variant

<400> 160

Gly Pro Asp Cys Leu Glu Ile Phe Lys Ala Cys Asn Pro Ser Asn Asp
1 5 10 15

Gln Cys Cys Lys Ser Ser Lys Leu Val Cys Ser Arg Lys Thr Arg Trp
20 25 30

Cys Lys Tyr Gln Ile Gly Lys
35

<210> 161
<211> 39
<212> PRT
<213> Artificial sequence

<220>
<223> Huwentoxin-IV variant

<400> 161

Gly Pro Tyr Cys Leu Glu Ile Phe Lys Ala Cys Asn Pro Ser Asn Asp
1 5 10 15

Gln Cys Cys Lys Ser Ser Lys Leu Val Cys Ser Arg Lys Thr Arg Trp
20 25 30

Cys Lys Tyr Gln Ile Gly Lys
35

<210> 162
<211> 39
<212> PRT
<213> Artificial sequence

<220>
<223> Huwentoxin-IV variant

<400> 162

Gly Pro Glu Cys Leu Glu Ile Phe Lys Ala Cys Asn Pro Ser Asn Asp
1 5 10 15

Gln Cys Cys Lys Ser Ser Ala Leu Val Cys Ser Arg Lys Thr Arg Trp
20 25 30

Cys Lys Tyr Gln Ile Gly Lys
        35

<210> 163
<211> 39
<212> PRT
<213> Artificial sequence

<220>
<223> Huwentoxin-IV variant

<400> 163

Gly Pro Glu Cys Leu Glu Ile Phe Lys Ala Cys Asn Pro Val Asn Asp
1               5                   10                  15

Gln Cys Cys Lys Ser Ser Lys Leu Val Cys Ser Arg Lys Thr Arg Trp
            20                  25                  30

Cys Lys Tyr Gln Ile Gly Lys
        35

<210> 164
<211> 39
<212> PRT
<213> Artificial sequence

<220>
<223> Huwentoxin-IV variant

<400> 164

Gly Pro Glu Cys Leu Glu Ile Phe Lys Ala Cys Asn Pro Ser Asn Asp
1               5                   10                  15

Gln Cys Cys Lys Ser Ser Lys Leu Val Cys Ser Ala Lys Thr Arg Trp
            20                  25                  30

Cys Lys Tyr Gln Ile Gly Lys
        35

<210> 165
<211> 39
<212> PRT
<213> Artificial sequence

<220>
<223> Huwentoxin-IV variant

<400> 165

Gly Pro Glu Cys Leu Glu Ile Phe Lys Ala Cys Asn Pro Ser Asn Asp
1               5                   10                  15

Asp Cys Cys Lys Ser Ser Lys Leu Val Cys Ser Arg Lys Thr Arg Trp
20 25 30

Cys Lys Tyr Gln Ile Gly Lys
35

<210> 166
<211> 39
<212> PRT
<213> Artificial sequence

<220>
<223> Huwentoxin-IV variant

<400> 166

Gly Pro Glu Cys Leu Glu Ile Phe Lys Ala Cys Asn Pro Ser His Asp
1 5 10 15

Gln Cys Cys Lys Ser Ser Lys Leu Val Cys Ser Arg Lys Thr Arg Trp
20 25 30

Cys Lys Tyr Gln Ile Gly Lys
35

<210> 167
<211> 39
<212> PRT
<213> Artificial sequence

<220>
<223> Huwentoxin-IV variant

<400> 167

Gly Pro Glu Cys Leu Glu Ile Phe Lys Phe Cys Asn Pro Ser Asn Asp
1 5 10 15

Gln Cys Cys Lys Ser Ser Lys Leu Val Cys Ser Arg Lys Thr Arg Trp
20 25 30

Cys Lys Tyr Gln Ile Gly Lys
35

<210> 168
<211> 39
<212> PRT
<213> Artificial sequence

<220>
<223> Huwentoxin-IV variant

<400> 168

Gly Pro Glu Cys Leu Glu Ile Phe Lys Ala Cys Asn Pro Ser Asn Asp
1               5                   10                  15

Gln Cys Cys Lys Ser Ser Lys Phe Val Cys Ser Arg Lys Thr Arg Trp
            20                  25                  30

Cys Lys Tyr Gln Ile Gly Lys
        35

<210>  169
<211>  39
<212>  PRT
<213>  Artificial sequence

<220>
<223>  Huwentoxin-IV variant

<400>  169

Gly Pro Glu Cys Leu Glu Ile Phe Lys Ala Cys Asn Pro Ser Asn Asp
1               5                   10                  15

Gln Cys Cys Lys Ser Ser Lys Leu Val Cys Ser Arg Lys Thr Arg Trp
            20                  25                  30

Cys Lys Tyr Gln Ile Phe Lys
        35

<210>  170
<211>  39
<212>  PRT
<213>  Artificial sequence

<220>
<223>  Huwentoxin-IV variant

<400>  170

Gly Pro Glu Cys Leu Glu Ile Phe Lys Ala Cys Asn Pro Ser Asn Asp
1               5                   10                  15

Gln Cys Cys Lys Ser Ser Lys Leu Val Cys Ser Arg Lys Thr Arg Trp
            20                  25                  30

Cys Lys Tyr Gln Ile Gly Val
        35

<210>  171
<211>  39
<212>  PRT
<213>  Artificial sequence

<220>
<223>  Huwentoxin-IV variant

<400> 171

Gly Pro Glu Cys Leu Glu Ile Phe Lys Ala Cys Asn Pro Ser Asn Asp
1 5 10 15

Gln Cys Cys Lys Ser Ser Lys Leu Val Cys Ser Arg Lys Thr Arg Trp
20 25 30

Cys Lys Tyr Gln Ile Gly Tyr
35

<210> 172
<211> 39
<212> PRT
<213> Artificial sequence

<220>
<223> Huwentoxin-IV variant

<400> 172

Gly Pro Glu Cys Leu Glu Ile Phe Lys Ala Cys Asn Pro Ser Gly Asp
1 5 10 15

Gln Cys Cys Lys Ser Ser Lys Leu Val Cys Ser Arg Lys Thr Arg Trp
20 25 30

Cys Lys Tyr Gln Ile Gly Lys
35

<210> 173
<211> 39
<212> PRT
<213> Artificial sequence

<220>
<223> Huwentoxin-IV variant

<400> 173

Gly Pro Asn Cys Leu Arg Ile Phe Lys Ala Cys Asn Pro Ser Asn Asp
1 5 10 15

Gln Cys Cys Lys Ser Ser Lys Leu Val Cys Ser Lys Lys Thr Arg Trp
20 25 30

Cys Lys Tyr Gln Ile Gly Lys
35

<210> 174
<211> 39
<212> PRT
<213> Artificial sequence

<220>
<223> Huwentoxin-IV variant

<400> 174

Gly Pro Glu Cys Leu Glu Ile Phe Lys Ala Cys Asn Pro Ser Asn Asp
1               5                   10                  15

Gln Cys Cys Lys Ser Ser Lys Leu Val Cys Ser Arg Lys Thr Lys Trp
            20                  25                  30

Cys Lys Tyr Gln Ile Gly Lys
        35

<210> 175
<211> 39
<212> PRT
<213> Artificial sequence

<220>
<223> Huwentoxin-IV variant

<400> 175

Gly Pro Glu Cys Leu Glu Ile Phe Lys Ala Cys Asn Pro Ser Asn Asp
1               5                   10                  15

Gln Cys Cys Lys Ser Ser Lys Leu Val Cys Ser Lys Lys Thr Arg Trp
            20                  25                  30

Cys Lys Tyr Gln Ile Gly Lys
        35

<210> 176
<211> 39
<212> PRT
<213> Artificial sequence

<220>
<223> Huwentoxin-IV variant

<400> 176

Gly Pro Glu Cys Leu Glu Ile Phe Lys Ala Cys Ser Pro Ser Asn Asp
1               5                   10                  15

Gln Cys Cys Lys Ser Ser Lys Leu Val Cys Ser Arg Lys Thr Arg Trp
            20                  25                  30

Cys Lys Tyr Gln Ile Gly Lys
        35

<210> 177

<211> 39
<212> PRT
<213> Artificial sequence

<220>
<223> Huwentoxin-IV variant

<400> 177

Gly Pro Glu Cys Leu Glu Ile Phe Lys Ala Cys Asn Pro Ser Asn Asp
1               5                   10                  15

Gln Cys Cys Lys Ser Ser Lys Leu Val Cys Ser Ile Lys Thr Arg Trp
            20                  25                  30

Cys Lys Tyr Gln Ile Gly Lys
        35

<210> 178
<211> 39
<212> PRT
<213> Artificial sequence

<220>
<223> Huwentoxin-IV variant

<400> 178

Gly Pro Glu Cys Leu Glu Ile Phe Lys Ala Cys Asn Pro Ser Asn Asp
1               5                   10                  15

Gln Cys Cys Lys Ser Ser Lys Leu Val Cys Ser Lys Lys Thr Arg Trp
            20                  25                  30

Cys Lys Tyr Ser Ile Ile Lys
        35

<210> 179
<211> 39
<212> PRT
<213> Artificial sequence

<220>
<223> Huwentoxin-IV variant

<400> 179

Gly Pro Glu Cys Leu Ile Ile Phe Lys Ala Cys Asn Pro Ser Asn Asp
1               5                   10                  15

Gln Cys Cys Lys Ser Ser Lys Leu Val Cys Ser Arg Lys Thr Arg Trp
            20                  25                  30

Cys Lys Tyr Gln Ile Gly Lys
        35

<210> 180
<211> 39
<212> PRT
<213> Artificial sequence

<220>
<223> Huwentoxin-IV variant

<400> 180

Gly Pro Glu Cys Leu Glu Ile Phe Lys Ala Cys Asn Pro Ser Asn Asp
1               5                   10                  15

Gln Cys Cys Lys Ser Ser Lys Leu Val Cys Ser Arg Lys Thr Gly Trp
            20                  25                  30

Cys Lys Tyr Gln Ile Gly Lys
        35

<210> 181
<211> 39
<212> PRT
<213> Artificial sequence

<220>
<223> Huwentoxin-IV variant

<400> 181

Gly Pro Glu Cys Leu Glu Ile Phe Lys Ala Cys Asn Pro Ser Asn Asp
1               5                   10                  15

Gln Cys Cys Lys Ser Ser Lys Leu Val Cys Ser Arg Lys Thr Gly Trp
            20                  25                  30

Cys Lys Tyr Gln Ile Gly Lys
        35

<210> 182
<211> 39
<212> PRT
<213> Artificial sequence

<220>
<223> Huwentoxin-IV variant

<400> 182

Gly Pro Glu Cys Leu Glu Ile Phe Lys Ala Cys Asn Pro Ser Asn Asp
1               5                   10                  15

Gln Cys Cys Lys Ser Ser Lys Leu Val Cys Ser Arg Lys Thr Arg Trp
            20                  25                  30

Cys Lys Tyr Gly Ile Gly Lys
35

<210> 183
<211> 39
<212> PRT
<213> Artificial sequence

<220>
<223> Huwentoxin-IV variant

<400> 183

Gly Pro Glu Cys Leu Glu Ile Phe Lys Ala Cys Asn Pro Ser Asn Asp
1 5 10 15

Gln Cys Cys Lys Ser Ser Lys Leu Val Cys Ser Arg Lys Val Arg Trp
20 25 30

Cys Lys Tyr Gln Ile Gly Lys
35

<210> 184
<211> 39
<212> PRT
<213> Artificial sequence

<220>
<223> Huwentoxin-IV variant

<400> 184

Gly Pro Glu Cys Leu Glu Ile Phe Lys Ala Cys Asn Pro Ser Asn Asp
1 5 10 15

Gln Cys Cys Lys Ser Ser Lys Leu Val Cys Ser Arg Lys Thr Arg Trp
20 25 30

Cys Lys Tyr Gln Ile His Lys
35

<210> 185
<211> 39
<212> PRT
<213> Artificial sequence

<220>
<223> Huwentoxin-IV variant

<400> 185

Gly Pro Glu Cys Leu Glu Ile Phe Lys Ala Cys Phe Pro Ser Asn Asp
1 5 10 15

Gln Cys Cys Lys Ser Ser Lys Leu Val Cys Ser Arg Lys Thr Arg Trp
20 25 30

Cys Lys Tyr Gln Ile Gly Lys
35

<210> 186
<211> 39
<212> PRT
<213> Artificial sequence

<220>
<223> Huwentoxin-IV variant

<400> 186

Gly Pro Glu Cys Leu Glu Ile Phe Lys Ala Cys Tyr Pro Ser Asn Asp
1 5 10 15

Gln Cys Cys Lys Ser Ser Lys Leu Val Cys Ser Arg Lys Thr Arg Trp
20 25 30

Cys Lys Tyr Gln Ile Gly Lys
35

<210> 187
<211> 39
<212> PRT
<213> Artificial sequence

<220>
<223> Huwentoxin-IV variant

<400> 187

Gly Pro Glu Cys Leu Glu Ile Phe Lys Ala Cys Asn Pro Ser Asn Asp
1 5 10 15

Gln Cys Cys Lys Ser Ser Lys Leu Val Cys Ser Arg Lys Thr Arg Trp
20 25 30

Cys Lys Tyr Gln Ile Val Lys
35

<210> 188
<211> 39
<212> PRT
<213> Artificial sequence

<220>
<223> Huwentoxin-IV variant

<400> 188

Gly Pro Glu Cys Leu Glu Ile Phe Lys Ala Cys Asn Pro Ser Asn Asp

1 5 10 15

Glu Cys Cys Lys Ser Ser Lys Leu Val Cys Ser Arg Lys Thr Arg Trp
20 25 30

Cys Lys Tyr Gln Ile Gly Lys
35

<210> 189
<211> 39
<212> PRT
<213> Artificial sequence

<220>
<223> Huwentoxin-IV variant

<400> 189

Gly Pro Glu Cys Leu Glu Ile Phe Lys Ala Cys Asn Pro Ser Asn Asp
1 5 10 15

Gln Cys Cys Pro Ser Ser Lys Leu Val Cys Ser Arg Lys Thr Arg Trp
20 25 30

Cys Lys Tyr Gln Ile Gly Lys
35

<210> 190
<211> 39
<212> PRT
<213> Artificial sequence

<220>
<223> Huwentoxin-IV variant

<400> 190

Gly Pro Glu Cys Leu Glu Ile Phe Lys Ala Cys Asn Pro Ser Val Asp
1 5 10 15

Gln Cys Cys Lys Ser Ser Lys Leu Val Cys Ser Arg Lys Thr Arg Trp
20 25 30

Cys Lys Tyr Gln Ile Gly Lys
35

<210> 191
<211> 39
<212> PRT
<213> Artificial sequence

<220>
<223> Huwentoxin-IV variant

<400> 191

Gly Pro Glu Cys Leu Glu Ile Phe Lys Ala Cys Asn Val Ser Asn Asp
1               5                   10                  15

Gln Cys Cys Lys Ser Ser Lys Leu Val Cys Ser Arg Lys Thr Arg Trp
            20                  25                  30

Cys Lys Tyr Gln Ile Gly Lys
        35

<210> 192
<211> 39
<212> PRT
<213> Artificial sequence

<220>
<223> Huwentoxin-IV variant

<400> 192

Gly Pro Asn Cys Leu Glu Ile Phe Lys Ala Cys Asn Pro Ser Asn Asp
1               5                   10                  15

Gln Cys Cys Lys Ser Ser Lys Leu Val Cys Ser Lys Lys Thr Arg Trp
            20                  25                  30

Cys Lys Tyr Ser Ile Ile Lys
        35

<210> 193
<211> 39
<212> PRT
<213> Artificial sequence

<220>
<223> Huwentoxin-IV variant

<400> 193

Gly Pro Glu Cys Leu Glu Ile Phe Lys Ala Cys Asn Pro Ser Asn Asp
1               5                   10                  15

Gln Cys Cys Lys Ser Ser Lys Lys Val Cys Ser Arg Lys Thr Arg Trp
            20                  25                  30

Cys Lys Tyr Gln Ile Gly Lys
        35

<210> 194
<211> 39
<212> PRT
<213> Artificial sequence

<220>
<223> Huwentoxin-IV variant

<400> 194

Gly Pro Glu Cys Leu Glu Ile Phe Lys Ala Cys Asn Pro Phe Asn Asp
1 5 10 15

Gln Cys Cys Lys Ser Ser Lys Leu Val Cys Ser Arg Lys Thr Arg Trp
20 25 30

Cys Lys Tyr Gln Ile Gly Lys
35

<210> 195
<211> 39
<212> PRT
<213> Artificial sequence

<220>
<223> Huwentoxin-IV variant

<400> 195

Gly Pro Glu Cys Leu Glu Ile Phe Lys Ala Cys Asn Pro Ser Asn Asp
1 5 10 15

Lys Cys Cys Lys Ser Ser Lys Leu Val Cys Ser Arg Lys Thr Arg Trp
20 25 30

Cys Lys Tyr Gln Ile Gly Lys
35

<210> 196
<211> 39
<212> PRT
<213> Artificial sequence

<220>
<223> Huwentoxin-IV variant

<400> 196

Gly Pro Glu Cys Leu Glu Ile Phe Lys Ala Cys Asn Pro Ser Asn Asp
1 5 10 15

Gln Cys Cys Lys Ser Ser Lys Leu Val Cys Ser Lys Lys Thr Arg Trp
20 25 30

Cys Lys Tyr Ser Ile Gly Lys
35

<210> 197
<211> 39

<212> PRT
<213> Artificial sequence

<220>
<223> Huwentoxin-IV variant

<400> 197

```
Gly Pro Glu Cys Leu Glu Ile Phe Lys Ala Cys Asn Pro Ser Asn Asp
1               5                   10                  15

Gln Cys Cys Lys Ser Ser Lys Leu Val Cys Ser Arg Lys Thr Arg Trp
            20                  25                  30

Cys Lys Tyr Val Ile Gly Lys
        35
```

<210> 198
<211> 39
<212> PRT
<213> Artificial sequence

<220>
<223> Huwentoxin-IV variant

<400> 198

```
Gly Pro Glu Cys Leu Glu Ile Phe Lys Ala Cys Asn Pro Ser Asn Asp
1               5                   10                  15

Gln Cys Cys Lys Ser Ser Lys Leu Val Cys Ser Arg Ala Thr Arg Trp
            20                  25                  30

Cys Lys Tyr Gln Ile Gly Lys
        35
```

<210> 199
<211> 39
<212> PRT
<213> Artificial sequence

<220>
<223> Huwentoxin-IV variant

<400> 199

```
Gly Pro Glu Cys Leu Glu Ile Phe Lys Ala Cys Asn Pro Ser Asn Asp
1               5                   10                  15

Gln Cys Cys Lys Ser Ser Thr Leu Val Cys Ser Arg Lys Thr Arg Trp
            20                  25                  30

Cys Lys Tyr Gln Ile Gly Lys
        35
```

<210> 200
<211> 39
<212> PRT
<213> Artificial sequence

<220>
<223> Huwentoxin-IV variant

<400> 200

Gly Pro Glu Cys Leu Glu Ile Phe Lys Ala Cys Asn Pro Gln Asn Asp
1 5 10 15

Gln Cys Cys Lys Ser Ser Lys Leu Val Cys Ser Arg Lys Thr Arg Trp
20 25 30

Cys Lys Tyr Gln Ile Gly Lys
35

<210> 201
<211> 39
<212> PRT
<213> Artificial sequence

<220>
<223> Huwentoxin-IV variant

<400> 201

Gly Pro Glu Cys Leu Glu Ile Phe Lys Ala Cys Asn Pro Ser Asn Asp
1 5 10 15

Gln Cys Cys Lys Ser Ser Lys Leu Val Cys Ser Arg Lys Thr Ala Trp
20 25 30

Cys Lys Tyr Gln Ile Gly Lys
35

<210> 202
<211> 39
<212> PRT
<213> Artificial sequence

<220>
<223> Huwentoxin-IV variant

<400> 202

Gly Pro Glu Cys Leu Glu Ile Phe Lys Ala Cys Asn Pro Ser Asn Asp
1 5 10 15

Gln Cys Cys Lys Ser Ser Lys Leu Val Cys Ser Arg Lys Thr Arg Trp
20 25 30

Cys Lys Tyr Gln Ile Gly Pro
        35

<210>  203
<211>  39
<212>  PRT
<213>  Artificial sequence

<220>
<223>  Huwentoxin-IV variant

<400>  203

Gly Pro Glu Cys Leu Glu Ile Phe Lys Ala Cys Asn Pro Ser Ser Asp
1               5                   10                  15

Gln Cys Cys Lys Ser Ser Lys Leu Val Cys Ser Arg Lys Thr Arg Trp
            20                  25                  30

Cys Lys Tyr Gln Ile Gly Lys
        35

<210>  204
<211>  39
<212>  PRT
<213>  Artificial sequence

<220>
<223>  Huwentoxin-IV variant

<400>  204

Gly Pro Glu Cys Leu Glu Ile Phe Lys Ala Cys Asn Pro Ser Asn Asp
1               5                   10                  15

Leu Cys Cys Lys Ser Ser Lys Leu Val Cys Ser Arg Lys Thr Arg Trp
            20                  25                  30

Cys Lys Tyr Gln Ile Gly Lys
        35

<210>  205
<211>  39
<212>  PRT
<213>  Artificial sequence

<220>
<223>  Huwentoxin-IV variant

<400>  205

Gly Pro Glu Cys Leu Glu Ile Phe Lys Val Cys Asn Pro Ser Asn Asp
1               5                   10                  15

Gln Cys Cys Lys Ser Ser Lys Leu Val Cys Ser Arg Lys Thr Arg Trp

20 25 30

Cys Lys Tyr Gln Ile Gly Lys
35

<210> 206
<211> 39
<212> PRT
<213> Artificial sequence

<220>
<223> Huwentoxin-IV variant

<400> 206

Gly Pro Glu Cys Leu Glu Ile Phe Lys Ala Cys Glu Pro Ser Asn Asp
1 5 10 15

Gln Cys Cys Lys Ser Ser Lys Leu Val Cys Ser Arg Lys Thr Arg Trp
20 25 30

Cys Lys Tyr Gln Ile Gly Lys
35

<210> 207
<211> 39
<212> PRT
<213> Artificial sequence

<220>
<223> Huwentoxin-IV variant

<400> 207

Gly Pro Glu Cys Leu Glu Ile Phe Lys Ala Cys Asn Pro Ser Pro Asp
1 5 10 15

Gln Cys Cys Lys Ser Ser Lys Leu Val Cys Ser Arg Lys Thr Arg Trp
20 25 30

Cys Lys Tyr Gln Ile Gly Lys
35

<210> 208
<211> 39
<212> PRT
<213> Artificial sequence

<220>
<223> Huwentoxin-IV variant

<400> 208

Gly Pro Glu Cys Arg Glu Ile Phe Lys Ala Cys Asn Pro Ser Asn Asp
1 5 10 15

Gln Cys Cys Lys Ser Ser Lys Leu Val Cys Ser Arg Lys Thr Arg Trp
20 25 30

Cys Lys Tyr Gln Ile Gly Lys
35

<210> 209
<211> 39
<212> PRT
<213> Artificial sequence

<220>
<223> Huwentoxin-IV variant

<400> 209

Gly Pro Glu Cys Leu Glu Ile Phe Lys Ala Cys Lys Pro Ser Asn Asp
1 5 10 15

Gln Cys Cys Lys Ser Ser Lys Leu Val Cys Ser Arg Lys Thr Arg Trp
20 25 30

Cys Lys Tyr Gln Ile Gly Lys
35

<210> 210
<211> 39
<212> PRT
<213> Artificial sequence

<220>
<223> Huwentoxin-IV variant

<400> 210

Gly Pro Glu Cys Leu Glu Ile Phe Lys Ala Cys Asn Pro Ser Asn Asp
1 5 10 15

Gln Cys Cys Lys Gly Ser Lys Leu Val Cys Ser Arg Lys Thr Arg Trp
20 25 30

Cys Lys Tyr Gln Ile Gly Lys
35

<210> 211
<211> 39
<212> PRT
<213> Artificial sequence

<220>
<223> Huwentoxin-IV variant

<400> 211

Gly Pro Glu Cys Leu Glu Ile Phe Lys Ala Cys Asn Pro Ser Asn Asp
1               5                   10                  15

Gln Cys Cys Lys Ser Ser Lys Leu Val Cys Ser Arg Lys Thr Arg Trp
            20                  25                  30

Cys Lys Tyr Gln Ile Gly Asn
        35

<210>  212
<211>  39
<212>  PRT
<213>  Artificial sequence

<220>
<223>  Huwentoxin-IV variant

<400>  212

Gly Pro Glu Cys Leu Glu Ile Phe Lys Ala Cys Asn Pro Ser Leu Asp
1               5                   10                  15

Gln Cys Cys Lys Ser Ser Lys Leu Val Cys Ser Arg Lys Thr Arg Trp
            20                  25                  30

Cys Lys Tyr Gln Ile Gly Lys
        35

<210>  213
<211>  39
<212>  PRT
<213>  Artificial sequence

<220>
<223>  Huwentoxin-IV variant

<400>  213

Gly Pro Glu Cys Leu Glu Ile Phe Lys Ala Cys Asn Pro Ser Asn Pro
1               5                   10                  15

Gln Cys Cys Lys Ser Ser Lys Leu Val Cys Ser Arg Lys Thr Arg Trp
            20                  25                  30

Cys Lys Tyr Gln Ile Gly Lys
        35

<210>  214
<211>  39
<212>  PRT
<213>  Artificial sequence

<220>

<223> Huwentoxin-IV variant

<400> 214

Gly Pro Glu Cys Leu Glu Ile Phe Lys Ala Cys Asn Pro Ser Asn Asp
1 5 10 15

Gln Cys Cys Lys Ser Ser Lys Leu Val Cys Ser Arg Lys Thr Arg Trp
20 25 30

Cys Lys Tyr Ile Ile Gly Lys
35

<210> 215
<211> 39
<212> PRT
<213> Artificial sequence

<220>
<223> Huwentoxin-IV variant

<400> 215

Gly Pro Glu Cys Leu Glu Ile Phe Lys Ala Cys Asn Pro Ser Asn Asp
1 5 10 15

Gln Cys Cys Lys Ser Ser Lys Leu Val Cys Ser Gly Lys Thr Arg Trp
20 25 30

Cys Lys Tyr Gln Ile Gly Lys
35

<210> 216
<211> 39
<212> PRT
<213> Artificial sequence

<220>
<223> Huwentoxin-IV variant

<400> 216

Gly Pro Glu Cys Leu Glu Ile Phe Lys Ala Cys Asn Pro Ser Asn Asp
1 5 10 15

Gln Cys Cys Lys Ser Ser Lys Leu Val Cys Ser Arg Lys Thr Leu Trp
20 25 30

Cys Lys Tyr Gln Ile Gly Lys
35

<210> 217
<211> 39
<212> PRT

<213> Artificial sequence

<220>
<223> Huwentoxin-IV variant

<400> 217

Gly Pro Glu Cys Leu Glu Ile Phe Lys Ala Cys Asn Pro Ser Asn Asp
1               5                   10                  15

Gln Cys Cys Lys Ser Ser Asp Leu Val Cys Ser Arg Lys Thr Arg Trp
            20                  25                  30

Cys Lys Tyr Gln Ile Gly Lys
        35

<210> 218
<211> 39
<212> PRT
<213> Artificial sequence

<220>
<223> Huwentoxin-IV variant

<400> 218

Gly Pro Glu Cys Leu Glu Ile Phe Lys Ala Cys Asn Pro Ser Asn Trp
1               5                   10                  15

Gln Cys Cys Lys Ser Ser Lys Leu Val Cys Ser Arg Lys Thr Arg Trp
            20                  25                  30

Cys Lys Tyr Gln Ile Gly Lys
        35

<210> 219
<211> 39
<212> PRT
<213> Artificial sequence

<220>
<223> Huwentoxin-IV variant

<400> 219

Gly Pro Glu Cys Leu Glu Ile Phe Lys Ala Cys Asn Pro Ser Asn Asp
1               5                   10                  15

Gln Cys Cys Lys Ser Ser Lys Trp Val Cys Ser Arg Lys Thr Arg Trp
            20                  25                  30

Cys Lys Tyr Gln Ile Gly Lys
        35

<210> 220
<211> 39
<212> PRT
<213> Artificial sequence

<220>
<223> Huwentoxin-IV variant

<400> 220

```
Gly Pro Glu Cys Leu Glu Ile Phe Lys Ala Cys Asn Pro Ser Asn Asp
1               5                   10                  15

Gln Cys Cys Lys Ser Ser Lys Leu Lys Cys Ser Arg Lys Thr Arg Trp
            20                  25                  30

Cys Lys Tyr Gln Ile Gly Lys
        35
```

<210> 221
<211> 39
<212> PRT
<213> Artificial sequence

<220>
<223> Huwentoxin-IV variant

<400> 221

```
Gly Pro Glu Cys Leu Glu Ile Phe Gln Ala Cys Asn Pro Ser Asn Asp
1               5                   10                  15

Gln Cys Cys Lys Ser Ser Lys Leu Val Cys Ser Arg Lys Thr Arg Trp
            20                  25                  30

Cys Lys Tyr Gln Ile Gly Lys
        35
```

<210> 222
<211> 39
<212> PRT
<213> Artificial sequence

<220>
<223> Huwentoxin-IV variant

<400> 222

```
Gly Pro Asn Cys Leu Glu Ile Phe Lys Ala Cys Asn Pro Ser Asn Asp
1               5                   10                  15

Gln Cys Cys Lys Ser Ser Lys Leu Val Cys Ser Lys Lys Thr Arg Trp
            20                  25                  30

Cys Lys Tyr Gln Ile Gly Lys
```

35

<210> 223
<211> 39
<212> PRT
<213> Artificial sequence

<220>
<223> Huwentoxin-IV variant

<400> 223

Gly Pro Glu Cys Leu Glu Ile Phe Lys Ala Cys Asn Pro Ser Asn Asp
1 5 10 15

Gln Cys Cys Lys Ser Ser Lys Leu Val Cys Ser Arg Lys Thr Arg Tyr
20 25 30

Cys Lys Tyr Gln Ile Gly Lys
35

<210> 224
<211> 39
<212> PRT
<213> Artificial sequence

<220>
<223> Huwentoxin-IV variant

<400> 224

Gly Pro Glu Cys Leu Glu Ile Phe Lys Ala Cys Asn Pro Ser Asn Asp
1 5 10 15

Pro Cys Cys Lys Ser Ser Lys Leu Val Cys Ser Arg Lys Thr Arg Trp
20 25 30

Cys Lys Tyr Gln Ile Gly Lys
35

<210> 225
<211> 39
<212> PRT
<213> Artificial sequence

<220>
<223> Huwentoxin-IV variant

<400> 225

Gly Pro Glu Cys Leu Glu Ile Phe Lys Ala Cys Asn Pro Ser Asn Asp
1 5 10 15

Gln Cys Cys Lys Ser Ser Lys Leu Val Cys Ser Lys Lys Thr Arg Trp
20 25 30

Cys Lys Tyr Gln Ile Ile Lys
35

<210> 226
<211> 39
<212> PRT
<213> Artificial sequence

<220>
<223> Huwentoxin-IV variant

<400> 226

Gly Pro Glu Cys Leu Glu Ile Phe Lys Ala Cys Asn Pro Ser Asn Asp
1 5 10 15

Gln Cys Cys Lys Ser Ser Lys Leu Val Cys Ser Thr Lys Thr Arg Trp
20 25 30

Cys Lys Tyr Gln Ile Gly Lys
35

<210> 227
<211> 39
<212> PRT
<213> Artificial sequence

<220>
<223> Huwentoxin-IV variant

<400> 227

Gly Pro Glu Cys Leu Glu Ile Phe Lys Ala Cys Asn Pro Ser Asn Asp
1 5 10 15

Gln Cys Cys Lys Ser Ser Lys Leu Val Cys Ser Arg Lys Thr Asp Trp
20 25 30

Cys Lys Tyr Gln Ile Gly Lys
35

<210> 228
<211> 39
<212> PRT
<213> Artificial sequence

<220>
<223> Huwentoxin-IV variant

<400> 228

Gly Pro Glu Cys Leu Glu Ile Phe Lys Ala Cys Asn Pro Ser Asn Asp
1               5                   10                  15

Gln Cys Cys Phe Ser Ser Lys Leu Val Cys Ser Arg Lys Thr Arg Trp
            20                  25                  30

Cys Lys Tyr Gln Ile Gly Lys
        35

<210> 229
<211> 39
<212> PRT
<213> Artificial sequence

<220>
<223> Huwentoxin-IV variant

<400> 229

Gly Pro Glu Cys Leu Glu Ile Phe Lys Ala Cys Asn Pro Ser Asn Asp
1               5                   10                  15

Gln Cys Cys Lys Ser Ser Lys Leu Val Cys Ser His Lys Thr Arg Trp
            20                  25                  30

Cys Lys Tyr Gln Ile Gly Lys
        35

<210> 230
<211> 39
<212> PRT
<213> Artificial sequence

<220>
<223> Huwentoxin-IV variant

<400> 230

Gly Pro Glu Cys Leu Glu Ile Phe Lys Ala Cys Asn Pro Ser Asn Gln
1               5                   10                  15

Gln Cys Cys Lys Ser Ser Lys Leu Val Cys Ser Arg Lys Thr Arg Trp
            20                  25                  30

Cys Lys Tyr Gln Ile Gly Lys
        35

<210> 231
<211> 39
<212> PRT
<213> Artificial sequence

<220>
<223> Huwentoxin-IV variant

<400> 231

Gly Pro Glu Cys Leu Glu Ile Phe Lys Ala Cys Asn Pro Ser Asn Asp
1 5 10 15

Gln Cys Cys Lys Ser Ser Lys Leu Val Cys Ser Val Lys Thr Arg Trp
20 25 30

Cys Lys Tyr Gln Ile Gly Lys
35

<210> 232
<211> 39
<212> PRT
<213> Artificial sequence

<220>
<223> Huwentoxin-IV variant

<400> 232

Gly Pro Glu Cys Leu Glu Ile Phe Lys Ala Cys Asn Pro Ser Asn Asp
1 5 10 15

Gln Cys Cys Lys Ser Ser Lys Ala Val Cys Ser Arg Lys Thr Arg Trp
20 25 30

Cys Lys Tyr Gln Ile Gly Lys
35

<210> 233
<211> 39
<212> PRT
<213> Artificial sequence

<220>
<223> Huwentoxin-IV variant

<400> 233

Gly Pro Glu Cys Leu Glu Ile Phe Lys Ala Cys Asn Pro Ser Asn Gly
1 5 10 15

Gln Cys Cys Lys Ser Ser Lys Leu Val Cys Ser Arg Lys Thr Arg Trp
20 25 30

Cys Lys Tyr Gln Ile Gly Lys
35

<210> 234
<211> 39
<212> PRT
<213> Artificial sequence

<220>
<223> Huwentoxin-IV variant

<400> 234

Gly Pro Glu Cys Leu Glu Ile Phe Lys Ala Cys Asn Pro Ser Asn Asp
1 5 10 15

Gln Cys Cys Lys Ser Ser Lys Leu Val Cys Ser Arg Lys Thr Arg Trp
20 25 30

Cys Lys Tyr Gln Ile Gly Ser
35

<210> 235
<211> 39
<212> PRT
<213> Artificial sequence

<220>
<223> Huwentoxin-IV variant

<400> 235

Gly Pro Glu Cys Leu Glu Ile Phe Lys Ala Cys Asn Pro Ser Asn Asp
1 5 10 15

Val Cys Cys Lys Ser Ser Lys Leu Val Cys Ser Arg Lys Thr Arg Trp
20 25 30

Cys Lys Tyr Gln Ile Gly Lys
35

<210> 236
<211> 39
<212> PRT
<213> Artificial sequence

<220>
<223> Huwentoxin-IV variant

<400> 236

Gly Pro Glu Cys Leu Glu Ile Met Lys Ala Cys Asn Pro Ser Asn Asp
1 5 10 15

Gln Cys Cys Lys Ser Ser Lys Leu Val Cys Ser Arg Lys Thr Arg Trp
20 25 30

Cys Lys Tyr Gln Ile Gly Lys
35

<210> 237

```
<211>  39
<212>  PRT
<213>  Artificial sequence

<220>
<223>  Huwentoxin-IV variant

<400>  237

Gly Pro Glu Cys Leu Glu Ile Phe Lys Ala Cys Asn Pro Ser Asn Asp
1               5                   10                  15

Gln Cys Cys Lys Ser Ser Lys Leu Val Cys Ser Arg Lys Thr Arg Trp
            20                  25                  30

Cys Lys Tyr Gln Ile Gln Lys
        35


<210>  238
<211>  39
<212>  PRT
<213>  Artificial sequence

<220>
<223>  Huwentoxin-IV variant

<400>  238

Gly Pro Glu Cys Leu Glu Ile Phe Lys Ala Cys Asn Pro Ser Asn Asp
1               5                   10                  15

Gln Cys Cys Lys Ser Ser Lys Leu Val Cys Ser Arg Lys Thr Arg Trp
            20                  25                  30

Cys Lys Tyr Gln Ile Gly Thr
        35


<210>  239
<211>  39
<212>  PRT
<213>  Artificial sequence

<220>
<223>  Huwentoxin-IV variant

<400>  239

Gly Pro Glu Cys Leu Glu Ile Phe Lys Ala Cys Asn Pro Ser Asn Asp
1               5                   10                  15

Gln Cys Cys Trp Ser Ser Lys Leu Val Cys Ser Arg Lys Thr Arg Trp
            20                  25                  30

Cys Lys Tyr Gln Ile Gly Lys
        35
```

<210> 240
<211> 39
<212> PRT
<213> Artificial sequence

<220>
<223> Huwentoxin-IV variant

<400> 240

Gly Pro Glu Cys Leu Glu Ile Phe Lys Ala Cys Asn Pro Ser Asn Asp
1 5 10 15

Gln Cys Cys Lys Ser Ser Lys Leu Val Cys Ser Arg Lys Thr Arg Trp
20 25 30

Cys Lys Tyr Trp Ile Gly Lys
35

<210> 241
<211> 39
<212> PRT
<213> Artificial sequence

<220>
<223> Huwentoxin-IV variant

<400> 241

Gly Pro Glu Cys Leu Glu Ile Phe Lys Ala Cys Asn Pro Ser Asn Asp
1 5 10 15

Gln Cys Cys Lys Ser Ser Lys Leu Val Cys Ser Arg Lys Thr Arg Trp
20 25 30

Cys Lys Trp Ser Ile Gly Lys
35

<210> 242
<211> 39
<212> PRT
<213> Artificial sequence

<220>
<223> Huwentoxin-IV variant

<400> 242

Gly Pro Glu Cys Leu Ala Ile Phe Lys Ala Cys Asn Pro Ser Asn Asp
1 5 10 15

Gln Cys Cys Lys Ser Ser Lys Leu Val Cys Ser Arg Lys Thr Arg Trp
20 25 30

Cys Lys Tyr Gln Ile Gly Lys
        35

<210> 243
<211> 39
<212> PRT
<213> Artificial sequence

<220>
<223> Huwentoxin-IV variant

<400> 243

Gly Pro Ala Cys Leu Glu Ile Phe Lys Ala Cys Asn Pro Ser Asn Asp
1               5                   10                  15

Gln Cys Cys Lys Ser Ser Lys Leu Val Cys Ser Arg Lys Thr Arg Trp
            20                  25                  30

Cys Lys Tyr Gln Ile Gly Lys
        35

<210> 244
<211> 39
<212> PRT
<213> Artificial sequence

<220>
<223> Huwentoxin-IV variant

<400> 244

Gly Pro Glu Cys Leu Glu Ile Phe Lys Ala Cys Asn Pro Ser Asn Asp
1               5                   10                  15

Gln Cys Cys Ala Ser Ser Lys Leu Val Cys Ser Arg Lys Thr Arg Trp
            20                  25                  30

Cys Lys Tyr Gln Ile Gly Lys
        35

<210> 245
<211> 39
<212> PRT
<213> Artificial sequence

<220>
<223> Huwentoxin-IV variant

<400> 245

Gly Pro Glu Cys Leu Glu Ile Phe Lys Trp Cys Asn Pro Ser Asn Asp
1               5                   10                  15

Gln Cys Cys Lys Ser Ser Lys Leu Val Cys Ser Arg Lys Thr Arg Trp
20 25 30

Cys Lys Tyr Gln Ile Gly Lys
35

<210> 246
<211> 39
<212> PRT
<213> Artificial sequence

<220>
<223> Huwentoxin-IV variant

<400> 246

Gly Pro Glu Cys Leu Glu Ile Phe Lys Ala Cys Asn Pro Ser Asn Asp
1 5 10 15

Gln Cys Cys Lys Ser Ser Lys Leu Val Cys Ser Arg Lys Ala Arg Trp
20 25 30

Cys Lys Tyr Gln Ile Gly Lys
35

<210> 247
<211> 39
<212> PRT
<213> Artificial sequence

<220>
<223> Huwentoxin-IV variant

<400> 247

Gly Pro Glu Cys Leu Glu Ile Phe Lys Ala Cys Asn Pro Ser Asn Asp
1 5 10 15

Gln Cys Cys Lys Ser Ala Lys Leu Val Cys Ser Arg Lys Thr Arg Trp
20 25 30

Cys Lys Tyr Gln Ile Gly Lys
35

<210> 248
<211> 39
<212> PRT
<213> Artificial sequence

<220>
<223> Huwentoxin-IV variant

<400> 248

Gly Pro Glu Cys Leu Glu Ile Phe Lys Ala Cys Asn Pro Ser Asn Asp

1 5 10 15

Gln Cys Cys Lys Ser Ser Lys Leu Val Cys Ser Arg Lys Thr Arg Trp
20 25 30

Cys Lys Tyr Gln Ile Gly Ala
35

<210> 249
<211> 39
<212> PRT
<213> Artificial sequence

<220>
<223> Huwentoxin-IV variant

<400> 249

Gly Pro Glu Cys Leu Glu Ile Phe Lys Ala Cys Ala Pro Ser Asn Asp
1 5 10 15

Gln Cys Cys Lys Ser Ser Lys Leu Val Cys Ser Arg Lys Thr Arg Trp
20 25 30

Cys Lys Tyr Gln Ile Gly Lys
35

<210> 250
<211> 39
<212> PRT
<213> Artificial sequence

<220>
<223> Huwentoxin-IV variant

<400> 250

Gly Pro Glu Cys Leu Glu Ile Phe Lys Ala Cys Asn Pro Ala Asn Asp
1 5 10 15

Gln Cys Cys Lys Ser Ser Lys Leu Val Cys Ser Arg Lys Thr Arg Trp
20 25 30

Cys Lys Tyr Gln Ile Gly Lys
35

<210> 251
<211> 39
<212> PRT
<213> Artificial sequence

<220>
<223> Huwentoxin-IV variant

<400> 251

Gly Pro Glu Cys Leu Glu Ile Phe Ala Ala Cys Asn Pro Ser Asn Asp
1 5 10 15

Gln Cys Cys Lys Ser Ser Lys Leu Val Cys Ser Arg Lys Thr Arg Trp
20 25 30

Cys Lys Tyr Gln Ile Gly Lys
35

<210> 252
<211> 39
<212> PRT
<213> Artificial sequence

<220>
<223> Huwentoxin-IV variant

<400> 252

Gly Pro Glu Cys Leu Glu Ala Phe Lys Ala Cys Asn Pro Ser Asn Asp
1 5 10 15

Gln Cys Cys Lys Ser Ser Lys Leu Val Cys Ser Arg Lys Thr Arg Trp
20 25 30

Cys Lys Tyr Gln Ile Gly Lys
35

<210> 253
<211> 39
<212> PRT
<213> Artificial sequence

<220>
<223> Huwentoxin-IV variant

<400> 253

Gly Pro Glu Cys Ala Glu Ile Phe Lys Ala Cys Asn Pro Ser Asn Asp
1 5 10 15

Gln Cys Cys Lys Ser Ser Lys Leu Val Cys Ser Arg Lys Thr Arg Trp
20 25 30

Cys Lys Tyr Gln Ile Gly Lys
35

<210> 254
<211> 39
<212> PRT
<213> Artificial sequence

<220>
<223> Huwentoxin-IV variant

<400> 254

Gly Pro Glu Cys Leu Glu Ile Phe Lys Ala Cys Asn Pro Ser Asn Asp
1 5 10 15

Gln Cys Cys Lys Ser Ser Lys Leu Val Cys Ser Arg Lys Thr Arg Trp
20 25 30

Cys Lys Tyr Gln Ile Ala Lys
35

<210> 255
<211> 39
<212> PRT
<213> Artificial sequence

<220>
<223> Huwentoxin-IV variant

<400> 255

Gly Pro Glu Cys Leu Glu Ile Ala Lys Ala Cys Asn Pro Ser Asn Asp
1 5 10 15

Gln Cys Cys Lys Ser Ser Lys Leu Val Cys Ser Arg Lys Thr Arg Trp
20 25 30

Cys Lys Tyr Gln Ile Gly Lys
35

<210> 256
<211> 39
<212> PRT
<213> Artificial sequence

<220>
<223> Huwentoxin-IV variant

<400> 256

Gly Pro Glu Cys Leu Glu Ile Phe Lys Ala Cys Asn Pro Ser Asn Asp
1 5 10 15

Gln Cys Cys Lys Ser Ser Lys Leu Val Cys Ser Arg Lys Thr Arg Trp
20 25 30

Cys Lys Tyr Gln Ala Gly Lys
35

<210> 257
<211> 39

<212> PRT
<213> Artificial sequence

<220>
<223> Huwentoxin-IV variant

<400> 257

Gly Pro Glu Cys Leu Glu Ile Phe Lys Ala Cys Asn Pro Ser Asn Asp
1 5 10 15

Gln Cys Cys Lys Ser Ser Lys Leu Val Cys Ser Arg Lys Thr Arg Trp
20 25 30

Cys Lys Ala Gln Ile Gly Lys
35

<210> 258
<211> 39
<212> PRT
<213> Artificial sequence

<220>
<223> Huwentoxin-IV variant

<400> 258

Gly Pro Glu Cys Leu Glu Ile Phe Lys Ala Cys Asn Ala Ser Asn Asp
1 5 10 15

Gln Cys Cys Lys Ser Ser Lys Leu Val Cys Ser Arg Lys Thr Arg Trp
20 25 30

Cys Lys Tyr Gln Ile Gly Lys
35

<210> 259
<211> 39
<212> PRT
<213> Artificial sequence

<220>
<223> Huwentoxin-IV variant

<400> 259

Gly Pro Glu Cys Leu Glu Ile Phe Lys Ala Cys Asn Pro Ser Asn Ala
1 5 10 15

Gln Cys Cys Lys Ser Ser Lys Leu Val Cys Ser Arg Lys Thr Arg Trp
20 25 30

Cys Lys Tyr Gln Ile Gly Lys
35

<210> 260
<211> 39
<212> PRT
<213> Artificial sequence

<220>
<223> Huwentoxin-IV variant

<400> 260

Gly Pro Glu Cys Leu Glu Ile Phe Lys Ala Cys Asn Pro Ser Asn Asp
1               5                   10                  15

Gln Cys Cys Lys Ser Ser Lys Leu Val Cys Ala Arg Lys Thr Arg Trp
            20                  25                  30

Cys Lys Tyr Gln Ile Gly Lys
        35

<210> 261
<211> 39
<212> PRT
<213> Artificial sequence

<220>
<223> Huwentoxin-IV variant

<400> 261

Gly Pro Glu Cys Leu Glu Ile Phe Lys Ala Cys Asn Pro Ser Asn Asp
1               5                   10                  15

Gln Cys Cys Lys Ser Ser Lys Leu Val Cys Ser Arg Lys Thr Arg Ala
            20                  25                  30

Cys Lys Tyr Gln Ile Gly Lys
        35

<210> 262
<211> 39
<212> PRT
<213> Artificial sequence

<220>
<223> Huwentoxin-IV variant

<400> 262

Gly Pro Glu Cys Leu Glu Ile Phe Lys Ala Cys Asn Pro Ser Asn Asp
1               5                   10                  15

Gln Cys Cys Lys Ser Ser Lys Leu Val Cys Ser Arg Lys Thr Arg Trp
            20                  25                  30

Cys Ala Tyr Gln Ile Gly Lys
35

<210> 263
<211> 1977
<212> PRT
<213> Homo sapiens

<400> 263

Met Ala Met Leu Pro Pro Pro Gly Pro Gln Ser Phe Val His Phe Thr
1 5 10 15

Lys Gln Ser Leu Ala Leu Ile Glu Gln Arg Ile Ala Glu Arg Lys Ser
20 25 30

Lys Glu Pro Lys Glu Glu Lys Lys Asp Asp Asp Glu Glu Ala Pro Lys
35 40 45

Pro Ser Ser Asp Leu Glu Ala Gly Lys Gln Leu Pro Phe Ile Tyr Gly
50 55 60

Asp Ile Pro Pro Gly Met Val Ser Glu Pro Leu Glu Asp Leu Asp Pro
65 70 75 80

Tyr Tyr Ala Asp Lys Lys Thr Phe Ile Val Leu Asn Lys Gly Lys Thr
85 90 95

Ile Phe Arg Phe Asn Ala Thr Pro Ala Leu Tyr Met Leu Ser Pro Phe
100 105 110

Ser Pro Leu Arg Arg Ile Ser Ile Lys Ile Leu Val His Ser Leu Phe
115 120 125

Ser Met Leu Ile Met Cys Thr Ile Leu Thr Asn Cys Ile Phe Met Thr
130 135 140

Met Asn Asn Pro Pro Asp Trp Thr Lys Asn Val Glu Tyr Thr Phe Thr
145 150 155 160

Gly Ile Tyr Thr Phe Glu Ser Leu Val Lys Ile Leu Ala Arg Gly Phe
165 170 175

Cys Val Gly Glu Phe Thr Phe Leu Arg Asp Pro Trp Asn Trp Leu Asp
180 185 190

Phe Val Val Ile Val Phe Ala Tyr Leu Thr Glu Phe Val Asn Leu Gly
195 200 205

```
Asn Val Ser Ala Leu Arg Thr Phe Arg Val Leu Arg Ala Leu Lys Thr
    210                 215                 220

Ile Ser Val Ile Pro Gly Leu Lys Thr Ile Val Gly Ala Leu Ile Gln
225                 230                 235                 240

Ser Val Lys Lys Leu Ser Asp Val Met Ile Leu Thr Val Phe Cys Leu
                245                 250                 255

Ser Val Phe Ala Leu Ile Gly Leu Gln Leu Phe Met Gly Asn Leu Lys
            260                 265                 270

His Lys Cys Phe Arg Asn Ser Leu Glu Asn Asn Glu Thr Leu Glu Ser
        275                 280                 285

Ile Met Asn Thr Leu Glu Ser Glu Glu Asp Phe Arg Lys Tyr Phe Tyr
    290                 295                 300

Tyr Leu Glu Gly Ser Lys Asp Ala Leu Leu Cys Gly Phe Ser Thr Asp
305                 310                 315                 320

Ser Gly Gln Cys Pro Glu Gly Tyr Thr Cys Val Lys Ile Gly Arg Asn
                325                 330                 335

Pro Asp Tyr Gly Tyr Thr Ser Phe Asp Thr Phe Ser Trp Ala Phe Leu
            340                 345                 350

Ala Leu Phe Arg Leu Met Thr Gln Asp Tyr Trp Glu Asn Leu Tyr Gln
        355                 360                 365

Gln Thr Leu Arg Ala Ala Gly Lys Thr Tyr Met Ile Phe Phe Val Val
    370                 375                 380

Val Ile Phe Leu Gly Ser Phe Tyr Leu Ile Asn Leu Ile Leu Ala Val
385                 390                 395                 400

Val Ala Met Ala Tyr Glu Glu Gln Asn Gln Ala Asn Ile Glu Glu Ala
                405                 410                 415

Lys Gln Lys Glu Leu Glu Phe Gln Gln Met Leu Asp Arg Leu Lys Lys
            420                 425                 430

Glu Gln Glu Glu Ala Glu Ala Ile Ala Ala Ala Ala Ala Glu Tyr Thr
        435                 440                 445

Ser Ile Arg Arg Ser Arg Ile Met Gly Leu Ser Glu Ser Ser Ser Glu
    450                 455                 460
```

Thr Ser Lys Leu Ser Ser Lys Ser Ala Lys Glu Arg Arg Asn Arg Arg
465 470 475 480

Lys Lys Lys Asn Gln Lys Lys Leu Ser Ser Gly Glu Glu Lys Gly Asp
485 490 495

Ala Glu Lys Leu Ser Lys Ser Glu Ser Glu Asp Ser Ile Arg Arg Lys
500 505 510

Ser Phe His Leu Gly Val Glu Gly His Arg Arg Ala His Glu Lys Arg
515 520 525

Leu Ser Thr Pro Asn Gln Ser Pro Leu Ser Ile Arg Gly Ser Leu Phe
530 535 540

Ser Ala Arg Arg Ser Ser Arg Thr Ser Leu Phe Ser Phe Lys Gly Arg
545 550 555 560

Gly Arg Asp Ile Gly Ser Glu Thr Glu Phe Ala Asp Asp Glu His Ser
565 570 575

Ile Phe Gly Asp Asn Glu Ser Arg Arg Gly Ser Leu Phe Val Pro His
580 585 590

Arg Pro Gln Glu Arg Arg Ser Ser Asn Ile Ser Gln Ala Ser Arg Ser
595 600 605

Pro Pro Met Leu Pro Val Asn Gly Lys Met His Ser Ala Val Asp Cys
610 615 620

Asn Gly Val Val Ser Leu Val Asp Gly Arg Ser Ala Leu Met Leu Pro
625 630 635 640

Asn Gly Gln Leu Leu Pro Glu Gly Thr Thr Asn Gln Ile His Lys Lys
645 650 655

Arg Arg Cys Ser Ser Tyr Leu Leu Ser Glu Asp Met Leu Asn Asp Pro
660 665 670

Asn Leu Arg Gln Arg Ala Met Ser Arg Ala Ser Ile Leu Thr Asn Thr
675 680 685

Val Glu Glu Leu Glu Glu Ser Arg Gln Lys Cys Pro Pro Trp Trp Tyr
690 695 700

Arg Phe Ala His Lys Phe Leu Ile Trp Asn Cys Ser Pro Tyr Trp Ile
705 710 715 720

Lys Phe Lys Lys Cys Ile Tyr Phe Ile Val Met Asp Pro Phe Val Asp
725 730 735

Leu Ala Ile Thr Ile Cys Ile Val Leu Asn Thr Leu Phe Met Ala Met
740 745 750

Glu His His Pro Met Thr Glu Glu Phe Lys Asn Val Leu Ala Ile Gly
755 760 765

Asn Leu Val Phe Thr Gly Ile Phe Ala Ala Glu Met Val Leu Lys Leu
770 775 780

Ile Ala Met Asp Pro Tyr Glu Tyr Phe Gln Val Gly Trp Asn Ile Phe
785 790 795 800

Asp Ser Leu Ile Val Thr Leu Ser Leu Val Glu Leu Phe Leu Ala Asp
805 810 815

Val Glu Gly Leu Ser Val Leu Arg Ser Phe Arg Leu Leu Arg Val Phe
820 825 830

Lys Leu Ala Lys Ser Trp Pro Thr Leu Asn Met Leu Ile Lys Ile Ile
835 840 845

Gly Asn Ser Val Gly Ala Leu Gly Asn Leu Thr Leu Val Leu Ala Ile
850 855 860

Ile Val Phe Ile Phe Ala Val Val Gly Met Gln Leu Phe Gly Lys Ser
865 870 875 880

Tyr Lys Glu Cys Val Cys Lys Ile Asn Asp Asp Cys Thr Leu Pro Arg
885 890 895

Trp His Met Asn Asp Phe Phe His Ser Phe Leu Ile Val Phe Arg Val
900 905 910

Leu Cys Gly Glu Trp Ile Glu Thr Met Trp Asp Cys Met Glu Val Ala
915 920 925

Gly Gln Ala Met Cys Leu Ile Val Tyr Met Met Val Met Val Ile Gly
930 935 940

Asn Leu Val Val Leu Asn Leu Phe Leu Ala Leu Leu Leu Ser Ser Phe
945 950 955 960

Ser Ser Asp Asn Leu Thr Ala Ile Glu Glu Asp Pro Asp Ala Asn Asn

965 970 975

Leu Gln Ile Ala Val Thr Arg Ile Lys Lys Gly Ile Asn Tyr Val Lys
980 985 990

Gln Thr Leu Arg Glu Phe Ile Leu Lys Ala Phe Ser Lys Lys Pro Lys
995 1000 1005

Ile Ser Arg Glu Ile Arg Gln Ala Glu Asp Leu Asn Thr Lys Lys
1010 1015 1020

Glu Asn Tyr Ile Ser Asn His Thr Leu Ala Glu Met Ser Lys Gly
1025 1030 1035

His Asn Phe Leu Lys Glu Lys Asp Lys Ile Ser Gly Phe Gly Ser
1040 1045 1050

Ser Val Asp Lys His Leu Met Glu Asp Ser Asp Gly Gln Ser Phe
1055 1060 1065

Ile His Asn Pro Ser Leu Thr Val Thr Val Pro Ile Ala Pro Gly
1070 1075 1080

Glu Ser Asp Leu Glu Asn Met Asn Ala Glu Glu Leu Ser Ser Asp
1085 1090 1095

Ser Asp Ser Glu Tyr Ser Lys Val Arg Leu Asn Arg Ser Ser Ser
1100 1105 1110

Ser Glu Cys Ser Thr Val Asp Asn Pro Leu Pro Gly Glu Gly Glu
1115 1120 1125

Glu Ala Glu Ala Glu Pro Met Asn Ser Asp Glu Pro Glu Ala Cys
1130 1135 1140

Phe Thr Asp Gly Cys Val Arg Arg Phe Ser Cys Cys Gln Val Asn
1145 1150 1155

Ile Glu Ser Gly Lys Gly Lys Ile Trp Trp Asn Ile Arg Lys Thr
1160 1165 1170

Cys Tyr Lys Ile Val Glu His Ser Trp Phe Glu Ser Phe Ile Val
1175 1180 1185

Leu Met Ile Leu Leu Ser Ser Gly Ala Leu Ala Phe Glu Asp Ile
1190 1195 1200

```
Tyr Ile  Glu Arg Lys Lys Thr  Ile Lys Ile Ile Leu  Glu Tyr Ala
    1205                 1210                 1215

Asp Lys  Ile Phe Thr Tyr Ile  Phe Ile Leu Glu Met  Leu Leu Lys
    1220                 1225                 1230

Trp Ile  Ala Tyr Gly Tyr Lys  Thr Tyr Phe Thr Asn  Ala Trp Cys
    1235                 1240                 1245

Trp Leu  Asp Phe Leu Ile Val  Asp Val Ser Leu Val  Thr Leu Val
    1250                 1255                 1260

Ala Asn  Thr Leu Gly Tyr Ser  Asp Leu Gly Pro Ile  Lys Ser Leu
    1265                 1270                 1275

Arg Thr  Leu Arg Ala Leu Arg  Pro Leu Arg Ala Leu  Ser Arg Phe
    1280                 1285                 1290

Glu Gly  Met Arg Val Val Val  Asn Ala Leu Ile Gly  Ala Ile Pro
    1295                 1300                 1305

Ser Ile  Met Asn Val Leu Leu  Val Cys Leu Ile Phe  Trp Leu Ile
    1310                 1315                 1320

Phe Ser  Ile Met Gly Val Asn  Leu Phe Ala Gly Lys  Phe Tyr Glu
    1325                 1330                 1335

Cys Ile  Asn Thr Thr Asp Gly  Ser Arg Phe Pro Ala  Ser Gln Val
    1340                 1345                 1350

Pro Asn  Arg Ser Glu Cys Phe  Ala Leu Met Asn Val  Ser Gln Asn
    1355                 1360                 1365

Val Arg  Trp Lys Asn Leu Lys  Val Asn Phe Asp Asn  Val Gly Leu
    1370                 1375                 1380

Gly Tyr  Leu Ser Leu Leu Gln  Val Ala Thr Phe Lys  Gly Trp Thr
    1385                 1390                 1395

Ile Ile  Met Tyr Ala Ala Val  Asp Ser Val Asn Val  Asp Lys Gln
    1400                 1405                 1410

Pro Lys  Tyr Glu Tyr Ser Leu  Tyr Met Tyr Ile Tyr  Phe Val Val
    1415                 1420                 1425

Phe Ile  Ile Phe Gly Ser Phe  Phe Thr Leu Asn Leu  Phe Ile Gly
    1430                 1435                 1440
```

Val Ile Ile Asp Asn Phe Asn Gln Gln Lys Lys Lys Leu Gly Gly
1445 1450 1455

Gln Asp Ile Phe Met Thr Glu Glu Gln Lys Lys Tyr Tyr Asn Ala
1460 1465 1470

Met Lys Lys Leu Gly Ser Lys Lys Pro Gln Lys Pro Ile Pro Arg
1475 1480 1485

Pro Gly Asn Lys Ile Gln Gly Cys Ile Phe Asp Leu Val Thr Asn
1490 1495 1500

Gln Ala Phe Asp Ile Ser Ile Met Val Leu Ile Cys Leu Asn Met
1505 1510 1515

Val Thr Met Met Val Glu Lys Glu Gly Gln Ser Gln His Met Thr
1520 1525 1530

Glu Val Leu Tyr Trp Ile Asn Val Val Phe Ile Ile Leu Phe Thr
1535 1540 1545

Gly Glu Cys Val Leu Lys Leu Ile Ser Leu Arg His Tyr Tyr Phe
1550 1555 1560

Thr Val Gly Trp Asn Ile Phe Asp Phe Val Val Val Ile Ile Ser
1565 1570 1575

Ile Val Gly Met Phe Leu Ala Asp Leu Ile Glu Thr Tyr Phe Val
1580 1585 1590

Ser Pro Thr Leu Phe Arg Val Ile Arg Leu Ala Arg Ile Gly Arg
1595 1600 1605

Ile Leu Arg Leu Val Lys Gly Ala Lys Gly Ile Arg Thr Leu Leu
1610 1615 1620

Phe Ala Leu Met Met Ser Leu Pro Ala Leu Phe Asn Ile Gly Leu
1625 1630 1635

Leu Leu Phe Leu Val Met Phe Ile Tyr Ala Ile Phe Gly Met Ser
1640 1645 1650

Asn Phe Ala Tyr Val Lys Lys Glu Asp Gly Ile Asn Asp Met Phe
1655 1660 1665

Asn Phe Glu Thr Phe Gly Asn Ser Met Ile Cys Leu Phe Gln Ile
1670 1675 1680

Thr Thr Ser Ala Gly Trp Asp Gly Leu Leu Ala Pro Ile Leu Asn
1685 1690 1695

Ser Lys Pro Pro Asp Cys Asp Pro Lys Lys Val His Pro Gly Ser
1700 1705 1710

Ser Val Glu Gly Asp Cys Gly Asn Pro Ser Val Gly Ile Phe Tyr
1715 1720 1725

Phe Val Ser Tyr Ile Ile Ile Ser Phe Leu Val Val Val Asn Met
1730 1735 1740

Tyr Ile Ala Val Ile Leu Glu Asn Phe Ser Val Ala Thr Glu Glu
1745 1750 1755

Ser Thr Glu Pro Leu Ser Glu Asp Asp Phe Glu Met Phe Tyr Glu
1760 1765 1770

Val Trp Glu Lys Phe Asp Pro Asp Ala Thr Gln Phe Ile Glu Phe
1775 1780 1785

Ser Lys Leu Ser Asp Phe Ala Ala Ala Leu Asp Pro Pro Leu Leu
1790 1795 1800

Ile Ala Lys Pro Asn Lys Val Gln Leu Ile Ala Met Asp Leu Pro
1805 1810 1815

Met Val Ser Gly Asp Arg Ile His Cys Leu Asp Ile Leu Phe Ala
1820 1825 1830

Phe Thr Lys Arg Val Leu Gly Glu Ser Gly Glu Met Asp Ser Leu
1835 1840 1845

Arg Ser Gln Met Glu Glu Arg Phe Met Ser Ala Asn Pro Ser Lys
1850 1855 1860

Val Ser Tyr Glu Pro Ile Thr Thr Thr Leu Lys Arg Lys Gln Glu
1865 1870 1875

Asp Val Ser Ala Thr Val Ile Gln Arg Ala Tyr Arg Arg Tyr Arg
1880 1885 1890

Leu Arg Gln Asn Val Lys Asn Ile Ser Ser Ile Tyr Ile Lys Asp
1895 1900 1905

Gly Asp Arg Asp Asp Asp Leu Leu Asn Lys Lys Asp Met Ala Phe

```
    1910                1915                1920

Asp Asn  Val Asn Glu Asn Ser  Ser Pro Glu Lys Thr  Asp Ala Thr
    1925                1930                1935

Ser Ser  Thr Thr Ser Pro Pro  Ser Tyr Asp Ser Val  Thr Lys Pro
    1940                1945                1950

Asp Lys  Glu Lys Tyr Glu Gln  Asp Arg Thr Glu Lys  Glu Asp Lys
    1955                1960                1965

Gly Lys  Asp Ser Lys Glu Ser  Lys Lys
    1970                1975


<210>  264
<211>  2005
<212>  PRT
<213>  Homo sapiens

<400>  264

Met Ala Gln Ser Val Leu Val Pro Pro Gly Pro Asp Ser Phe Arg Phe
1               5                   10                  15

Phe Thr Arg Glu Ser Leu Ala Ala Ile Glu Gln Arg Ile Ala Glu Glu
            20                  25                  30

Lys Ala Lys Arg Pro Lys Gln Glu Arg Lys Asp Glu Asp Asp Glu Asn
        35                  40                  45

Gly Pro Lys Pro Asn Ser Asp Leu Glu Ala Gly Lys Ser Leu Pro Phe
    50                  55                  60

Ile Tyr Gly Asp Ile Pro Pro Glu Met Val Ser Val Pro Leu Glu Asp
65                  70                  75                  80

Leu Asp Pro Tyr Tyr Ile Asn Lys Lys Thr Phe Ile Val Leu Asn Lys
                85                  90                  95

Gly Lys Ala Ile Ser Arg Phe Ser Ala Thr Pro Ala Leu Tyr Ile Leu
            100                 105                 110

Thr Pro Phe Asn Pro Ile Arg Lys Leu Ala Ile Lys Ile Leu Val His
        115                 120                 125

Ser Leu Phe Asn Met Leu Ile Met Cys Thr Ile Leu Thr Asn Cys Val
    130                 135                 140

Phe Met Thr Met Ser Asn Pro Pro Asp Trp Thr Lys Asn Val Glu Tyr
```

145 150 155 160

Thr Phe Thr Gly Ile Tyr Thr Phe Glu Ser Leu Ile Lys Ile Leu Ala
165 170 175

Arg Gly Phe Cys Leu Glu Asp Phe Thr Phe Leu Arg Asp Pro Trp Asn
180 185 190

Trp Leu Asp Phe Thr Val Ile Thr Phe Ala Tyr Val Thr Glu Phe Val
195 200 205

Asp Leu Gly Asn Val Ser Ala Leu Arg Thr Phe Arg Val Leu Arg Ala
210 215 220

Leu Lys Thr Ile Ser Val Ile Pro Gly Leu Lys Thr Ile Val Gly Ala
225 230 235 240

Leu Ile Gln Ser Val Lys Lys Leu Ser Asp Val Met Ile Leu Thr Val
245 250 255

Phe Cys Leu Ser Val Phe Ala Leu Ile Gly Leu Gln Leu Phe Met Gly
260 265 270

Asn Leu Arg Asn Lys Cys Leu Gln Trp Pro Pro Asp Asn Ser Ser Phe
275 280 285

Glu Ile Asn Ile Thr Ser Phe Phe Asn Asn Ser Leu Asp Gly Asn Gly
290 295 300

Thr Thr Phe Asn Arg Thr Val Ser Ile Phe Asn Trp Asp Glu Tyr Ile
305 310 315 320

Glu Asp Lys Ser His Phe Tyr Phe Leu Glu Gly Gln Asn Asp Ala Leu
325 330 335

Leu Cys Gly Asn Ser Ser Asp Ala Gly Gln Cys Pro Glu Gly Tyr Ile
340 345 350

Cys Val Lys Ala Gly Arg Asn Pro Asn Tyr Gly Tyr Thr Ser Phe Asp
355 360 365

Thr Phe Ser Trp Ala Phe Leu Ser Leu Phe Arg Leu Met Thr Gln Asp
370 375 380

Phe Trp Glu Asn Leu Tyr Gln Leu Thr Leu Arg Ala Ala Gly Lys Thr
385 390 395 400

Tyr Met Ile Phe Phe Val Leu Val Ile Phe Leu Gly Ser Phe Tyr Leu
405 410 415

Ile Asn Leu Ile Leu Ala Val Val Ala Met Ala Tyr Glu Glu Gln Asn
420 425 430

Gln Ala Thr Leu Glu Glu Ala Glu Gln Lys Glu Ala Glu Phe Gln Gln
435 440 445

Met Leu Glu Gln Leu Lys Lys Gln Gln Glu Glu Ala Gln Ala Ala Ala
450 455 460

Ala Ala Ala Ser Ala Glu Ser Arg Asp Phe Ser Gly Ala Gly Gly Ile
465 470 475 480

Gly Val Phe Ser Glu Ser Ser Ser Val Ala Ser Lys Leu Ser Ser Lys
485 490 495

Ser Glu Lys Glu Leu Lys Asn Arg Arg Lys Lys Lys Lys Gln Lys Glu
500 505 510

Gln Ser Gly Glu Glu Glu Lys Asn Asp Arg Val Arg Lys Ser Glu Ser
515 520 525

Glu Asp Ser Ile Arg Arg Lys Gly Phe Arg Phe Ser Leu Glu Gly Ser
530 535 540

Arg Leu Thr Tyr Glu Lys Arg Phe Ser Ser Pro His Gln Ser Leu Leu
545 550 555 560

Ser Ile Arg Gly Ser Leu Phe Ser Pro Arg Arg Asn Ser Arg Ala Ser
565 570 575

Leu Phe Ser Phe Arg Gly Arg Ala Lys Asp Ile Gly Ser Glu Asn Asp
580 585 590

Phe Ala Asp Asp Glu His Ser Thr Phe Glu Asp Asn Asp Ser Arg Arg
595 600 605

Asp Ser Leu Phe Val Pro His Arg His Gly Glu Arg Arg His Ser Asn
610 615 620

Val Ser Gln Ala Ser Arg Ala Ser Arg Val Leu Pro Ile Leu Pro Met
625 630 635 640

Asn Gly Lys Met His Ser Ala Val Asp Cys Asn Gly Val Val Ser Leu
645 650 655

Val Gly Gly Pro Ser Thr Leu Thr Ser Ala Gly Gln Leu Leu Pro Glu
660 665 670

Gly Thr Thr Thr Glu Thr Glu Ile Arg Lys Arg Arg Ser Ser Ser Tyr
675 680 685

His Val Ser Met Asp Leu Leu Glu Asp Pro Thr Ser Arg Gln Arg Ala
690 695 700

Met Ser Ile Ala Ser Ile Leu Thr Asn Thr Met Glu Glu Leu Glu Glu
705 710 715 720

Ser Arg Gln Lys Cys Pro Pro Cys Trp Tyr Lys Phe Ala Asn Met Cys
725 730 735

Leu Ile Trp Asp Cys Cys Lys Pro Trp Leu Lys Val Lys His Leu Val
740 745 750

Asn Leu Val Val Met Asp Pro Phe Val Asp Leu Ala Ile Thr Ile Cys
755 760 765

Ile Val Leu Asn Thr Leu Phe Met Ala Met Glu His Tyr Pro Met Thr
770 775 780

Glu Gln Phe Ser Ser Val Leu Ser Val Gly Asn Leu Val Phe Thr Gly
785 790 795 800

Ile Phe Thr Ala Glu Met Phe Leu Lys Ile Ile Ala Met Asp Pro Tyr
805 810 815

Tyr Tyr Phe Gln Glu Gly Trp Asn Ile Phe Asp Gly Phe Ile Val Ser
820 825 830

Leu Ser Leu Met Glu Leu Gly Leu Ala Asn Val Glu Gly Leu Ser Val
835 840 845

Leu Arg Ser Phe Arg Leu Leu Arg Val Phe Lys Leu Ala Lys Ser Trp
850 855 860

Pro Thr Leu Asn Met Leu Ile Lys Ile Ile Gly Asn Ser Val Gly Ala
865 870 875 880

Leu Gly Asn Leu Thr Leu Val Leu Ala Ile Ile Val Phe Ile Phe Ala
885 890 895

Val Val Gly Met Gln Leu Phe Gly Lys Ser Tyr Lys Glu Cys Val Cys
900 905 910

```
Lys Ile Ser Asn Asp Cys Glu Leu Pro Arg Trp His Met His Asp Phe
        915                 920                 925

Phe His Ser Phe Leu Ile Val Phe Arg Val Leu Cys Gly Glu Trp Ile
    930                 935                 940

Glu Thr Met Trp Asp Cys Met Glu Val Ala Gly Gln Thr Met Cys Leu
945                 950                 955                 960

Thr Val Phe Met Met Val Met Val Ile Gly Asn Leu Val Val Leu Asn
                965                 970                 975

Leu Phe Leu Ala Leu Leu Leu Ser Ser Phe Ser Ser Asp Asn Leu Ala
            980                 985                 990

Ala Thr Asp Asp Asp Asn Glu Met  Asn Asn Leu Gln Ile  Ala Val Gly
        995                 1000                 1005

Arg Met  Gln Lys Gly Ile Asp  Phe Val Lys Arg Lys  Ile Arg Glu
    1010                 1015                 1020

Phe Ile  Gln Lys Ala Phe Val  Arg Lys Gln Lys Ala  Leu Asp Glu
    1025                 1030                 1035

Ile Lys  Pro Leu Glu Asp Leu  Asn Asn Lys Lys Asp  Ser Cys Ile
    1040                 1045                 1050

Ser Asn  His Thr Thr Ile Glu  Ile Gly Lys Asp Leu  Asn Tyr Leu
    1055                 1060                 1065

Lys Asp  Gly Asn Gly Thr Thr  Ser Gly Ile Gly Ser  Ser Val Glu
    1070                 1075                 1080

Lys Tyr  Val Val Asp Glu Ser  Asp Tyr Met Ser Phe  Ile Asn Asn
    1085                 1090                 1095

Pro Ser  Leu Thr Val Thr Val  Pro Ile Ala Val Gly  Glu Ser Asp
    1100                 1105                 1110

Phe Glu  Asn Leu Asn Thr Glu  Glu Phe Ser Ser Glu  Ser Asp Met
    1115                 1120                 1125

Glu Glu  Ser Lys Glu Lys Leu  Asn Ala Thr Ser Ser  Ser Glu Gly
    1130                 1135                 1140

Ser Thr  Val Asp Ile Gly Ala  Pro Ala Glu Gly Glu  Gln Pro Glu
```

1145 1150 1155

Val Glu Pro Glu Glu Ser Leu Glu Pro Glu Ala Cys Phe Thr Glu
1160 1165 1170

Asp Cys Val Arg Lys Phe Lys Cys Cys Gln Ile Ser Ile Glu Glu
1175 1180 1185

Gly Lys Gly Lys Leu Trp Trp Asn Leu Arg Lys Thr Cys Tyr Lys
1190 1195 1200

Ile Val Glu His Asn Trp Phe Glu Thr Phe Ile Val Phe Met Ile
1205 1210 1215

Leu Leu Ser Ser Gly Ala Leu Ala Phe Glu Asp Ile Tyr Ile Glu
1220 1225 1230

Gln Arg Lys Thr Ile Lys Thr Met Leu Glu Tyr Ala Asp Lys Val
1235 1240 1245

Phe Thr Tyr Ile Phe Ile Leu Glu Met Leu Leu Lys Trp Val Ala
1250 1255 1260

Tyr Gly Phe Gln Val Tyr Phe Thr Asn Ala Trp Cys Trp Leu Asp
1265 1270 1275

Phe Leu Ile Val Asp Val Ser Leu Val Ser Leu Thr Ala Asn Ala
1280 1285 1290

Leu Gly Tyr Ser Glu Leu Gly Ala Ile Lys Ser Leu Arg Thr Leu
1295 1300 1305

Arg Ala Leu Arg Pro Leu Arg Ala Leu Ser Arg Phe Glu Gly Met
1310 1315 1320

Arg Val Val Val Asn Ala Leu Leu Gly Ala Ile Pro Ser Ile Met
1325 1330 1335

Asn Val Leu Leu Val Cys Leu Ile Phe Trp Leu Ile Phe Ser Ile
1340 1345 1350

Met Gly Val Asn Leu Phe Ala Gly Lys Phe Tyr His Cys Ile Asn
1355 1360 1365

Tyr Thr Thr Gly Glu Met Phe Asp Val Ser Val Val Asn Asn Tyr
1370 1375 1380

Ser Glu Cys Lys Ala Leu Ile Glu Ser Asn Gln Thr Ala Arg Trp
1385 1390 1395

Lys Asn Val Lys Val Asn Phe Asp Asn Val Gly Leu Gly Tyr Leu
1400 1405 1410

Ser Leu Leu Gln Val Ala Thr Phe Lys Gly Trp Met Asp Ile Met
1415 1420 1425

Tyr Ala Ala Val Asp Ser Arg Asn Val Glu Leu Gln Pro Lys Tyr
1430 1435 1440

Glu Asp Asn Leu Tyr Met Tyr Leu Tyr Phe Val Ile Phe Ile Ile
1445 1450 1455

Phe Gly Ser Phe Phe Thr Leu Asn Leu Phe Ile Gly Val Ile Ile
1460 1465 1470

Asp Asn Phe Asn Gln Gln Lys Lys Lys Phe Gly Gly Gln Asp Ile
1475 1480 1485

Phe Met Thr Glu Glu Gln Lys Lys Tyr Tyr Asn Ala Met Lys Lys
1490 1495 1500

Leu Gly Ser Lys Lys Pro Gln Lys Pro Ile Pro Arg Pro Ala Asn
1505 1510 1515

Lys Phe Gln Gly Met Val Phe Asp Phe Val Thr Lys Gln Val Phe
1520 1525 1530

Asp Ile Ser Ile Met Ile Leu Ile Cys Leu Asn Met Val Thr Met
1535 1540 1545

Met Val Glu Thr Asp Asp Gln Ser Gln Glu Met Thr Asn Ile Leu
1550 1555 1560

Tyr Trp Ile Asn Leu Val Phe Ile Val Leu Phe Thr Gly Glu Cys
1565 1570 1575

Val Leu Lys Leu Ile Ser Leu Arg Tyr Tyr Tyr Phe Thr Ile Gly
1580 1585 1590

Trp Asn Ile Phe Asp Phe Val Val Val Ile Leu Ser Ile Val Gly
1595 1600 1605

Met Phe Leu Ala Glu Leu Ile Glu Lys Tyr Phe Val Ser Pro Thr
1610 1615 1620

Leu Phe Arg Val Ile Arg Leu Ala Arg Ile Gly Arg Ile Leu Arg
1625 1630 1635

Leu Ile Lys Gly Ala Lys Gly Ile Arg Thr Leu Leu Phe Ala Leu
1640 1645 1650

Met Met Ser Leu Pro Ala Leu Phe Asn Ile Gly Leu Leu Leu Phe
1655 1660 1665

Leu Val Met Phe Ile Tyr Ala Ile Phe Gly Met Ser Asn Phe Ala
1670 1675 1680

Tyr Val Lys Arg Glu Val Gly Ile Asp Asp Met Phe Asn Phe Glu
1685 1690 1695

Thr Phe Gly Asn Ser Met Ile Cys Leu Phe Gln Ile Thr Thr Ser
1700 1705 1710

Ala Gly Trp Asp Gly Leu Leu Ala Pro Ile Leu Asn Ser Gly Pro
1715 1720 1725

Pro Asp Cys Asp Pro Asp Lys Asp His Pro Gly Ser Ser Val Lys
1730 1735 1740

Gly Asp Cys Gly Asn Pro Ser Val Gly Ile Phe Phe Phe Val Ser
1745 1750 1755

Tyr Ile Ile Ile Ser Phe Leu Val Val Val Asn Met Tyr Ile Ala
1760 1765 1770

Val Ile Leu Glu Asn Phe Ser Val Ala Thr Glu Glu Ser Ala Glu
1775 1780 1785

Pro Leu Ser Glu Asp Asp Phe Glu Met Phe Tyr Glu Val Trp Glu
1790 1795 1800

Lys Phe Asp Pro Asp Ala Thr Gln Phe Ile Glu Phe Ala Lys Leu
1805 1810 1815

Ser Asp Phe Ala Asp Ala Leu Asp Pro Pro Leu Leu Ile Ala Lys
1820 1825 1830

Pro Asn Lys Val Gln Leu Ile Ala Met Asp Leu Pro Met Val Ser
1835 1840 1845

Gly Asp Arg Ile His Cys Leu Asp Ile Leu Phe Ala Phe Thr Lys
1850 1855 1860

Arg Val Leu Gly Glu Ser Gly Glu Met Asp Ala Leu Arg Ile Gln
1865 1870 1875

Met Glu Glu Arg Phe Met Ala Ser Asn Pro Ser Lys Val Ser Tyr
1880 1885 1890

Glu Pro Ile Thr Thr Thr Leu Lys Arg Lys Gln Glu Glu Val Ser
1895 1900 1905

Ala Ile Ile Ile Gln Arg Ala Tyr Arg Arg Tyr Leu Leu Lys Gln
1910 1915 1920

Lys Val Lys Lys Val Ser Ser Ile Tyr Lys Lys Asp Lys Gly Lys
1925 1930 1935

Glu Cys Asp Gly Thr Pro Ile Lys Glu Asp Thr Leu Ile Asp Lys
1940 1945 1950

Leu Asn Glu Asn Ser Thr Pro Glu Lys Thr Asp Met Thr Pro Ser
1955 1960 1965

Thr Thr Ser Pro Pro Ser Tyr Asp Ser Val Thr Lys Pro Glu Lys
1970 1975 1980

Glu Lys Phe Glu Lys Asp Lys Ser Glu Lys Glu Asp Lys Gly Lys
1985 1990 1995

Asp Ile Arg Glu Ser Lys Lys
2000 2005

<210> 265
<211> 37
<212> PRT
<213> Artificial sequence

<220>
<223> Huwentoxin-IV variant

<220>
<221> MISC_FEATURE
<222> (1)..(1)
<223> Xaa1 is any amino acid

<220>
<221> MISC_FEATURE
<222> (3)..(5)
<223> Xaa is any amino acid

<220>
<221> MISC_FEATURE

<222> (7)..(8)
<223> Xaa is any amino acid

<220>
<221> MISC_FEATURE
<222> (10)..(15)
<223> Xaa is any amino acid

<220>
<221> MISC_FEATURE
<222> (18)..(23)
<223> Xaa is any amino acid

<220>
<221> MISC_FEATURE
<222> (25)..(30)
<223> Xaa is any amino acid

<220>
<221> MISC_FEATURE
<222> (33)..(34)
<223> Xaa is any amino acid

<220>
<221> MISC_FEATURE
<222> (36)..(37)
<223> Xaa is any amino acid or deleted

<400> 265

```
Xaa Cys Xaa Xaa Xaa Phe Xaa Xaa Cys Xaa Xaa Xaa Xaa Xaa Xaa Cys
1               5                   10                  15

Cys Xaa Xaa Xaa Xaa Xaa Xaa Cys Xaa Xaa Xaa Xaa Xaa Xaa Cys Lys
            20                  25                  30

Xaa Xaa Ile Xaa Xaa
        35
```

<210> 266
<211> 30
<212> PRT
<213> Artificial sequence

<220>
<223> Linker

<400> 266

```
Gly Gly Gly Gly Ser Gly Gly Gly Gly Ser Gly Gly Gly Gly Ser Gly
1               5                   10                  15

Gly Gly Gly Ser Gly Gly Gly Gly Ser Gly Gly Gly Gly Ser
            20                  25                  30
```

<210> 267
<211> 37
<212> PRT

<213> Artificial sequence

<220>
<223> Reference huwentoxin-IV sequence

<400> 267

Glu Cys Leu Glu Ile Phe Lys Ala Cys Asn Pro Ser Asn Asp Gln Cys
1 5 10 15

Cys Lys Ser Ser Lys Leu Val Cys Ser Arg Lys Thr Arg Trp Cys Lys
20 25 30

Tyr Gln Ile Gly Lys
35

<210> 268
<211> 35
<212> PRT
<213> Ornithoctonus huwena

<400> 268

Glu Cys Leu Glu Ile Phe Lys Ala Cys Asn Pro Ser Asn Asp Gln Cys
1 5 10 15

Cys Lys Ser Ser Lys Leu Val Cys Ser Arg Lys Thr Arg Trp Cys Lys
20 25 30

Tyr Gln Ile
35

<210> 269
<211> 20
<212> PRT
<213> Artificial sequence

<220>
<223> Linker

<400> 269

Gly Gly Gly Gly Ser Gly Gly Gly Gly Ser Gly Gly Gly Gly Ser Gly
1 5 10 15

Gly Gly Gly Ser
20

<210> 270
<211> 8
<212> PRT
<213> Artificial sequence

<220>
<223> Linker

<400> 270

Leu Glu Val Leu Phe Gln Gly Pro
1 5

<210> 271
<211> 111
<212> DNA
<213> Artificial sequence

<220>
<223> Huwentoxin-IV variant

<400> 271
aactgcttac ggatttttaa ggcatgcaac ccttcaaatg accagtgctg caagagctcg 60

aaattagttt gcagtcgaaa aaccaggtgg tgtaaatgga gcataggtaa a 111

<210> 272
<211> 111
<212> DNA
<213> Artificial sequence

<220>
<223> Huwentoxin-IV variant

<400> 272
aactgcttac ggatttttaa ggcatgcaac ccttcaaatg accagtgctg caagagctcg 60

aaattagttt gcagtcgaaa aaccaggtgg tgtaaataca gcataggtaa a 111

<210> 273
<211> 111
<212> DNA
<213> Artificial sequence

<220>
<223> Huwentoxin-IV variant

<400> 273
aactgcttac ggatttttaa ggcatgcaac ccttcaaatg accagtgctg caagagctcg 60

aaattagttt gcagtaagaa aaccaggtgg tgtaaataca gcataggtaa a 111

<210> 274
<211> 111
<212> DNA
<213> Artificial sequence

<220>
<223> Huwentoxin-IV variant

<400> 274
gagtgcttag agatttttaa ggcatgcaac ccttcaaatg accagtgctg caagagctcg 60

aaattagttt gcagtcgaaa aaccaggtgg tgtaaatact ttataggtaa a 111

<210> 275
<211> 111
<212> DNA
<213> Artificial sequence

<220>
<223> Huwentoxin-IV variant

<400> 275
aactgcttag agatttttaa ggcatgcaac ccttcaaatg accagtgctg caagagctcg 60

aaattagttt gcagtcgaaa aaccaggtgg tgtaaatggc aaataatcaa a 111

<210> 276
<211> 37
<212> PRT
<213> Artificial sequence

<220>
<223> Huwentoxin-IV variant

<220>
<221> MISC_FEATURE
<222> (1)..(1)
<223> Xaa is any amino acid

<220>
<221> MISC_FEATURE
<222> (3)..(5)
<223> Xaa is any amino acid

<220>
<221> MISC_FEATURE
<222> (7)..(8)
<223> Xaa is any amino acid

<220>
<221> MISC_FEATURE
<222> (10)..(15)
<223> Xaa is any amino acid

<220>
<221> MISC_FEATURE
<222> (18)..(23)
<223> Xaa is any amino acid

<220>
<221> MISC_FEATURE
<222> (25)..(27)
<223> Xaa is any amino acid

<220>
<221> MISC_FEATURE
<222> (29)..(29)
<223> Xaa is any amino acid

<220>
<221> MISC_FEATURE
<222> (34)..(35)
<223> Xaa is any amino acid

<220>
<221> MISC_FEATURE
<222> (36)..(37)
<223> Xaa is any amino acid or deleted

<400> 276

Xaa Cys Xaa Xaa Xaa Phe Xaa Xaa Cys Xaa Xaa Xaa Xaa Xaa Xaa Cys
1 5 10 15

Cys Xaa Xaa Xaa Xaa Xaa Xaa Cys Xaa Xaa Xaa Thr Xaa Trp Cys Lys
20 25 30

Tyr Xaa Xaa Xaa Xaa
35

<210> 277
<211> 39
<212> PRT
<213> Artificial sequence

<220>
<223> Huwentoxin-IV variant

<400> 277

Gly Pro Asn Cys Leu Glu Ile Phe Lys Ala Cys Asn Pro Ser Asn Asp
1 5 10 15

Gln Cys Cys Lys Ser Ser Phe Leu Val Cys Ser Lys Lys Thr Arg Trp
20 25 30

Cys Lys Tyr Ser Ile Ile Lys
35

<210> 278
<211> 39
<212> PRT
<213> Artificial sequence

<220>
<223> Huwentoxin-IV variant

<400> 278

Gly Pro Asn Cys Leu Glu Ile Phe Lys Ala Cys Asn Pro Ser Asn Asp
1 5 10 15

Gln Cys Cys Lys Ser Asn Lys Leu Val Cys Ser Lys Lys Thr Arg Trp
20 25 30

Cys Lys Tyr Ser Ile Ile Lys
35

<210> 279
<211> 39
<212> PRT
<213> Artificial sequence

<220>
<223> Huwentoxin-IV variant

<400> 279

```
Gly Pro Asn Cys Leu Arg Ile Phe Lys Ala Cys Asn Pro Ser Asn Asp
1               5                   10                  15

Gln Cys Cys Lys Ser Ser Lys Leu Val Cys Ser Asp Lys Thr Arg Trp
            20                  25                  30

Cys Lys Trp Ser Ile Gly Lys
        35
```

<210> 280
<211> 39
<212> PRT
<213> Artificial sequence

<220>
<223> Huwentoxin-IV variant

<400> 280

```
Gly Pro Asn Cys Leu Arg Ile Phe Lys Ala Cys Asn Pro Ser Asn Asp
1               5                   10                  15

Gln Cys Cys Lys Ser Ser Lys Leu Val Cys Ser Arg His Thr Arg Trp
            20                  25                  30

Cys Lys Trp Ser Ile Gly Lys
        35
```

<210> 281
<211> 39
<212> PRT
<213> Artificial sequence

<220>
<223> Huwentoxin-IV variant

<400> 281

```
Gly Pro Asn Cys Leu Glu Ile Phe Lys Ala Cys Asn Pro Ser Asn Asp
1               5                   10                  15

Gln Cys Cys Lys Ser Ser Lys Leu Val Cys Ser Lys Lys Thr Arg Trp
            20                  25                  30

Cys Lys Tyr Ser Ile Ile Lys
```

35

<210> 282
<211> 39
<212> PRT
<213> Artificial sequence

<220>
<223> Huwentoxin-IV variant

<400> 282

Gly Pro Asn Cys Leu Glu Ile Phe Lys Ala Cys Asn Pro Ser Asn Asp
1 5 10 15

Gln Cys Cys Lys Ser Ser Lys Leu Val Cys Ser Lys Lys Thr Arg Trp
20 25 30

Cys Lys Tyr Ser Ile Ile Phe
35

<210> 283
<211> 39
<212> PRT
<213> Artificial sequence

<220>
<223> Huwentoxin-IV variant

<400> 283

Gly Pro Asn Cys Leu Glu Ile Phe Lys Ala Cys Asn Pro Ser Asn Asp
1 5 10 15

Gln Cys Cys Lys Ser Ser Lys Leu Val Cys Ser Lys Lys Thr Arg Trp
20 25 30

Cys Lys Tyr Ser Ile Ile Arg
35

<210> 284
<211> 39
<212> PRT
<213> Artificial sequence

<220>
<223> Huwentoxin-IV variant

<400> 284

Gly Pro Asn Cys Leu Glu Ile Phe Lys Ala Cys Asn Pro Ser Asn Asp
1 5 10 15

Gln Cys Cys Lys Ser Ser Lys Leu Val Cys Ile Lys Lys Thr Arg Trp
20 25 30

Cys Lys Tyr Ser Ile Ile Lys
        35

<210>  285
<211>  39
<212>  PRT
<213>  Artificial sequence

<220>
<223>  Huwentoxin-IV variant

<400>  285

Gly Pro Asn Cys Leu Glu Ile Phe Lys Ala Cys Asn Pro Ser Asn Asp
1               5                   10                  15

Gln Cys Cys Lys Ser Ser Asn Leu Val Cys Ser Lys Lys Thr Arg Trp
            20                  25                  30

Cys Lys Tyr Ser Ile Ile Lys
        35

<210>  286
<211>  39
<212>  PRT
<213>  Artificial sequence

<220>
<223>  Huwentoxin-IV variant

<400>  286

Gly Pro Asn Cys Leu Glu Ile Phe Lys Ala Cys Asn Pro Ser Asn Asp
1               5                   10                  15

Gln Cys Cys Lys Ser Ser His Leu Val Cys Ser Lys Lys Thr Arg Trp
            20                  25                  30

Cys Lys Tyr Ser Ile Ile Lys
        35

<210>  287
<211>  39
<212>  PRT
<213>  Artificial sequence

<220>
<223>  Huwentoxin-IV variant

<400>  287

Gly Pro Asn Cys Leu Glu Ile Phe Lys Ala Cys Asn Pro Ser Asn Asp
1               5                   10                  15

Gln Cys Cys Lys Ser Ser Phe Leu Val Cys Ser Lys Lys Thr Arg Trp
20 25 30

Cys Lys Tyr Ser Ile Ile Lys
35

<210> 288
<211> 39
<212> PRT
<213> Artificial sequence

<220>
<223> Huwentoxin-IV variant

<400> 288

Gly Pro Asn Cys Leu Glu Ile Phe Lys Ala Cys Asn Pro Ser Asn Asp
1 5 10 15

Gln Cys Cys Lys Ser Ser Arg Leu Val Cys Ser Lys Lys Thr Arg Trp
20 25 30

Cys Lys Tyr Ser Ile Ile Lys
35

<210> 289
<211> 39
<212> PRT
<213> Artificial sequence

<220>
<223> Huwentoxin-IV variant

<400> 289

Gly Pro Asn Cys Leu Glu Ile Phe Lys Ala Cys Asn Pro Ser Asn Asp
1 5 10 15

Gln Cys Cys Lys Ser Ser Lys Arg Val Cys Ser Lys Lys Thr Arg Trp
20 25 30

Cys Lys Tyr Ser Ile Ile Lys
35

<210> 290
<211> 39
<212> PRT
<213> Artificial sequence

<220>
<223> Huwentoxin-IV variant

<400> 290

Gly Pro Asn Cys Leu Glu Ile Phe Lys Ala Cys Asn Pro Ser Asn Asp
1               5                   10                  15

Gln Cys Cys Lys Pro Ser Lys Leu Val Cys Ser Lys Lys Thr Arg Trp
            20                  25                  30

Cys Lys Tyr Ser Ile Ile Lys
        35

<210> 291
<211> 39
<212> PRT
<213> Artificial sequence

<220>
<223> Huwentoxin-IV variant

<400> 291

Gly Pro Asn Cys Leu Glu Ile Phe Lys Ala Cys Asn Pro Ser Asn Asp
1               5                   10                  15

Gln Cys Cys Tyr Ser Ser Lys Leu Val Cys Ser Lys Lys Thr Arg Trp
            20                  25                  30

Cys Lys Tyr Ser Ile Ile Lys
        35

<210> 292
<211> 39
<212> PRT
<213> Artificial sequence

<220>
<223> Huwentoxin-IV variant

<400> 292

Gly Pro Asn Cys Leu Glu Ile Phe Lys Ala Cys Asn Pro Ser Asn Asp
1               5                   10                  15

Gln Cys Cys Lys Ser Asn Lys Leu Val Cys Ser Lys Lys Thr Arg Trp
            20                  25                  30

Cys Lys Tyr Ser Ile Ile Lys
        35

<210> 293
<211> 39
<212> PRT
<213> Artificial sequence

<220>
<223> Huwentoxin-IV variant

<400> 293

Gly Pro Asn Cys Leu Glu Ile Phe Lys Ala Cys Asn Pro Ser Asn Asp
1               5                   10                  15

Gln Cys Cys Lys Ser Pro Lys Leu Val Cys Ser Lys Lys Thr Arg Trp
            20                  25                  30

Cys Lys Tyr Ser Ile Ile Lys
        35

<210> 294
<211> 39
<212> PRT
<213> Artificial sequence

<220>
<223> Huwentoxin-IV variant

<400> 294

Gly Pro Asn Cys Leu Glu Ile Phe Lys Ala Cys Asn Pro Ser Asn Asp
1               5                   10                  15

Gln Cys Cys Lys Ser Arg Lys Leu Val Cys Ser Lys Lys Thr Arg Trp
            20                  25                  30

Cys Lys Tyr Ser Ile Ile Lys
        35

<210> 295
<211> 39
<212> PRT
<213> Artificial sequence

<220>
<223> Huwentoxin-IV variant

<400> 295

Gly Pro Asn Cys Leu Asn Ile Phe Lys Arg Cys Asn Pro Ser Asn Asp
1               5                   10                  15

Gln Cys Cys Lys Ser Ser Lys Leu Val Cys Ser Lys Lys Thr Arg Trp
            20                  25                  30

Cys Lys Tyr Ser Ile Ile Lys
        35

<210> 296
<211> 39
<212> PRT
<213> Artificial sequence

<220>
<223> Huwentoxin-IV variant

<400> 296

Gly Pro Asn Cys Leu Asn Ile Phe Lys Arg Cys Asn Pro Ser Asn Asp
1 5 10 15

Gln Cys Cys Lys Ser Ser Lys Leu Val Cys Ser Lys Lys Thr Arg Trp
20 25 30

Cys Lys Tyr Ser Ile Ile Lys
35

<210> 297
<211> 39
<212> PRT
<213> Artificial sequence

<220>
<223> Huwentoxin-IV variant

<400> 297

Gly Pro Asn Cys Leu His Ile Phe Lys Gln Cys Asn Pro Ser Asn Asp
1 5 10 15

Gln Cys Cys Lys Ser Ser Lys Leu Val Cys Ser Lys Lys Thr Arg Trp
20 25 30

Cys Lys Tyr Ser Ile Ile Lys
35

<210> 298
<211> 39
<212> PRT
<213> Artificial sequence

<220>
<223> Huwentoxin-IV variant

<400> 298

Gly Pro Asn Cys Leu His Ile Phe Lys Gln Cys Asn Pro Ser Asn Asp
1 5 10 15

Gln Cys Cys Lys Ser Ser Lys Leu Val Cys Ser Lys Lys Thr Arg Trp
20 25 30

Cys Lys Tyr Ser Ile Ile Lys
35

<210> 299

<211> 39
<212> PRT
<213> Artificial sequence

<220>
<223> Huwentoxin-IV variant

<400> 299

Gly Pro Asn Cys Leu Glu Ile Phe Lys Ala Cys Asn Pro Ser Asn Asp
1 5 10 15

Arg Cys Cys Lys Ser Ser Lys Leu Val Cys Ser Lys Lys Thr Arg Trp
20 25 30

Cys Lys Tyr Ser Ile Ile Lys
35

<210> 300
<211> 39
<212> PRT
<213> Artificial sequence

<220>
<223> Huwentoxin-IV variant

<400> 300

Gly Pro Asn Cys Leu Glu Ile Phe Lys Ala Cys Asn Pro Ser Ala Asp
1 5 10 15

Gln Cys Cys Lys Ser Ser Lys Leu Val Cys Ser Lys Lys Thr Arg Trp
20 25 30

Cys Lys Tyr Ser Ile Ile Lys
35

<210> 301
<211> 39
<212> PRT
<213> Artificial sequence

<220>
<223> Huwentoxin-IV variant

<400> 301

Gly Pro Asn Cys Leu Glu Ile Phe Lys Gln Cys Asn Pro Ser Asn Asp
1 5 10 15

Gln Cys Cys Lys Ser Ser Lys Leu Val Cys Ser Lys Lys Thr Arg Trp
20 25 30

Cys Lys Tyr Ser Ile Ile Lys
35

<210> 302
<211> 39
<212> PRT
<213> Artificial sequence

<220>
<223> Huwentoxin-IV variant

<400> 302

Gly Pro Asn Cys Leu Glu Ile Phe Lys Asn Cys Asn Pro Ser Asn Asp
1 5 10 15

Gln Cys Cys Lys Ser Ser Lys Leu Val Cys Ser Lys Lys Thr Arg Trp
20 25 30

Cys Lys Tyr Ser Ile Ile Lys
35

<210> 303
<211> 39
<212> PRT
<213> Artificial sequence

<220>
<223> Huwentoxin-IV variant

<400> 303

Gly Pro Asn Cys Leu Gln Ile Phe Lys Ala Cys Asn Pro Ser Asn Asp
1 5 10 15

Gln Cys Cys Lys Ser Ser Lys Leu Val Cys Ser Lys Lys Thr Arg Trp
20 25 30

Cys Lys Tyr Ser Ile Ile Lys
35

<210> 304
<211> 39
<212> PRT
<213> Artificial sequence

<220>
<223> Huwentoxin-IV variant

<400> 304

Gly Pro Asn Cys Leu Glu Ile Phe Lys Ala Cys Asn Pro Ser Asn Asp
1 5 10 15

Asn Cys Cys Lys Ser Ser Lys Leu Val Cys Ser Lys Lys Thr Arg Trp
20 25 30

Cys Lys Tyr Ser Ile Ile Lys
35

<210> 305
<211> 39
<212> PRT
<213> Artificial sequence

<220>
<223> Huwentoxin-IV variant

<400> 305

Gly Pro Asn Cys Leu Glu Ile Phe Lys Ala Cys Asn Pro Ser Asn Asp
1 5 10 15

Gln Cys Cys Lys Arg Ser Lys Leu Val Cys Ser Lys Lys Thr Arg Trp
20 25 30

Cys Lys Tyr Ser Ile Ile Lys
35

<210> 306
<211> 39
<212> PRT
<213> Artificial sequence

<220>
<223> Huwentoxin-IV variant

<400> 306

Gly Pro Asn Cys Leu Glu Ile Phe Lys Ala Cys Asn Pro Ser Asn Asp
1 5 10 15

Gln Cys Cys Lys Ser Val Lys Leu Val Cys Ser Lys Lys Thr Arg Trp
20 25 30

Cys Lys Tyr Ser Ile Ile Lys
35

<210> 307
<211> 39
<212> PRT
<213> Artificial sequence

<220>
<223> Huwentoxin-IV variant

<400> 307

Gly Pro Asn Cys Leu Arg Ile Phe Lys Ala Cys Asn Pro Ser Asn Asp
1 5 10 15

Gln Cys Cys Lys Ser Ser Lys Leu Val Cys Ser Arg Lys Thr Arg Trp
20 25 30

Cys Lys Trp Ser Ile Gly Lys
35

<210> 308
<211> 39
<212> PRT
<213> Artificial sequence

<220>
<223> Huwentoxin-IV variant

<400> 308

Gly Pro Asn Cys Leu Arg Ile Phe Lys Ala Cys Asn Pro Ser Asn Asp
1 5 10 15

Gln Cys Cys Lys Gln Ser Lys Leu Val Cys Ser Arg Lys Thr Arg Trp
20 25 30

Cys Lys Trp Ser Ile Gly Lys
35

<210> 309
<211> 39
<212> PRT
<213> Artificial sequence

<220>
<223> Huwentoxin-IV variant

<400> 309

Gly Pro Asn Cys Leu Arg Ile Phe Lys Ala Cys Asn Pro Ser Asn Asp
1 5 10 15

Asp Cys Cys Lys Ser Ser Lys Leu Val Cys Ser Arg Lys Thr Arg Trp
20 25 30

Cys Lys Trp Ser Ile Gly Lys
35

<210> 310
<211> 39
<212> PRT
<213> Artificial sequence

<220>
<223> Huwentoxin-IV variant

<400> 310

Gly Pro Asn Cys Leu Arg Ile Phe Lys Ala Cys Asn Pro Ser Asn Asp

1               5                   10                  15

Gln Cys Cys Gln Ser Ser Lys Leu Val Cys Ser Arg Lys Thr Arg Trp
            20                  25                  30

Cys Lys Trp Ser Ile Gly Lys
        35

<210> 311
<211> 39
<212> PRT
<213> Artificial sequence

<220>
<223> Huwentoxin-IV variant

<400> 311

Gly Pro Asn Cys Leu Arg Ile Phe Lys Ala Cys Asn Pro Ser Asn Ser
1               5                   10                  15

Gln Cys Cys Lys Ser Ser Lys Leu Val Cys Ser Arg Lys Thr Arg Trp
            20                  25                  30

Cys Lys Trp Ser Ile Gly Lys
        35

<210> 312
<211> 39
<212> PRT
<213> Artificial sequence

<220>
<223> Huwentoxin-IV variant

<400> 312

Gly Pro Asn Cys Leu Arg Ile Phe Lys Ala Cys Asn Pro Ser Asn Gln
1               5                   10                  15

Gln Cys Cys Lys Ser Ser Lys Leu Val Cys Ser Arg Lys Thr Arg Trp
            20                  25                  30

Cys Lys Trp Ser Ile Gly Lys
        35

<210> 313
<211> 39
<212> PRT
<213> Artificial sequence

<220>
<223> Huwentoxin-IV variant

<400> 313

Gly Pro Asn Cys Leu Arg Ile Phe Lys Ala Cys Asn Pro Ser Asn Asp
1 5 10 15

Pro Cys Cys Lys Ser Ser Lys Leu Val Cys Ser Arg Lys Thr Arg Trp
20 25 30

Cys Lys Trp Ser Ile Gly Lys
35

<210> 314
<211> 39
<212> PRT
<213> Artificial sequence

<220>
<223> Huwentoxin-IV variant

<400> 314

Gly Pro Asn Cys Leu Arg Ile Phe Lys Ala Cys Asn Pro Ser Asn Asp
1 5 10 15

Gln Cys Cys Lys Ser Ser Lys Leu Val Cys Ser Arg Lys Thr Arg Trp
20 25 30

Cys Ala Trp Ser Ile Gly Lys
35

<210> 315
<211> 39
<212> PRT
<213> Artificial sequence

<220>
<223> Huwentoxin-IV variant

<400> 315

Gly Pro Asn Cys Leu Arg Ile Phe Lys Ala Cys Asn Pro Ser Asn Asp
1 5 10 15

Gln Cys Cys Lys Ser Ser Lys Leu Val Cys Ser Arg Lys Thr Arg Trp
20 25 30

Cys Trp Trp Ser Ile Gly Lys
35

<210> 316
<211> 39
<212> PRT
<213> Artificial sequence

<220>
<223> Huwentoxin-IV variant

<400> 316

Gly Pro Asn Cys Leu Arg Ile Phe Lys Ala Cys Asn Pro Ser Asn Asp
1 5 10 15

Gln Cys Cys Lys Ser Ser Lys Leu Val Cys Ser Arg Ala Thr Arg Trp
20 25 30

Cys Lys Trp Ser Ile Gly Lys
35

<210> 317
<211> 39
<212> PRT
<213> Artificial sequence

<220>
<223> Huwentoxin-IV variant

<400> 317

Gly Pro Asn Cys Leu Arg Ile Phe Lys Ala Cys Asn Pro Ser Asn Asp
1 5 10 15

Gln Cys Cys Lys Ser Ser Lys Leu Val Cys Ser Asp Lys Thr Arg Trp
20 25 30

Cys Lys Trp Ser Ile Gly Lys
35

<210> 318
<211> 39
<212> PRT
<213> Artificial sequence

<220>
<223> Huwentoxin-IV variant

<400> 318

Gly Pro Asn Cys Leu Arg Ile Phe Lys Ala Cys Asn Pro Ser Asn Asp
1 5 10 15

Gln Cys Cys Lys Ser Ser Lys Leu Val Cys Ser Arg His Thr Arg Trp
20 25 30

Cys Lys Trp Ser Ile Gly Lys
35

<210> 319
<211> 39

<212> PRT
<213> Artificial sequence

<220>
<223> Huwentoxin-IV variant

<400> 319

Gly Pro Asn Cys Leu Arg Ile Phe Lys Ala Cys Asn Pro Ser Asn Asp
1 5 10 15

Gln Cys Cys Lys Ser Ser Lys Leu Val Cys Ser His Lys Thr Arg Trp
20 25 30

Cys Lys Trp Ser Ile Gly Lys
35

<210> 320
<211> 39
<212> PRT
<213> Artificial sequence

<220>
<223> Huwentoxin-IV variant

<400> 320

Gly Pro Asn Cys Leu Arg Ile Phe Lys Ala Cys Asn Pro Ser Asn Asp
1 5 10 15

Gln Cys Cys Lys Ser Ser Lys Leu Val Cys Ser Arg Lys Thr Arg Trp
20 25 30

Cys Lys Trp Ser Ile Gly Arg
35

<210> 321
<211> 39
<212> PRT
<213> Artificial sequence

<220>
<223> Huwentoxin-IV variant

<400> 321

Gly Pro Asn Cys Leu Arg Ile Phe Lys Ala Cys Asn Pro Ser Asn Asp
1 5 10 15

Gln Cys Cys Lys Ser Ser Lys Leu Val Cys Ser Arg Lys Thr Arg Trp
20 25 30

Cys Lys Trp Ser Ile Gly Pro
35

<210> 322
<211> 39
<212> PRT
<213> Artificial sequence

<220>
<223> Huwentoxin-IV variant

<400> 322

Gly Pro Asn Cys Leu Arg Ile Phe Lys Ala Cys Asn Pro Ser Asn Asp
1 5 10 15

Gln Cys Cys Trp Ser Ser Lys Leu Val Cys Ser Arg Lys Thr Arg Trp
20 25 30

Cys Lys Trp Ser Ile Gly Lys
35

<210> 323
<211> 39
<212> PRT
<213> Artificial sequence

<220>
<223> Huwentoxin-IV variant

<400> 323

Gly Pro Asn Cys Leu Arg Ile Phe Lys Ala Cys Asn Pro Ser Asn Leu
1 5 10 15

Gln Cys Cys Lys Ser Ser Lys Leu Val Cys Ser Arg Lys Thr Arg Trp
20 25 30

Cys Lys Trp Ser Ile Gly Lys
35

<210> 324
<211> 39
<212> PRT
<213> Artificial sequence

<220>
<223> Huwentoxin-IV variant

<400> 324

Gly Pro Asn Cys Leu Arg Ile Phe Lys Ala Cys Asn Pro Ser Asn Asp
1 5 10 15

Trp Cys Cys Lys Ser Ser Lys Leu Val Cys Ser Arg Lys Thr Arg Trp
20 25 30

Cys Lys Trp Ser Ile Gly Lys
35

<210> 325
<211> 39
<212> PRT
<213> Artificial sequence

<220>
<223> Huwentoxin-IV variant

<400> 325

Gly Pro Asn Cys Leu Arg Ile Phe Lys Ala Cys Asn Pro Ser Asn Asp
1 5 10 15

Gln Cys Cys Lys Ser Ser Lys Leu Val Cys Ser Arg Lys Thr Arg Trp
20 25 30

Cys Lys Trp Ser Ile Val Lys
35

<210> 326
<211> 39
<212> PRT
<213> Artificial sequence

<220>
<223> Huwentoxin-IV variant

<400> 326

Gly Pro Asn Cys Leu Arg Ile Phe Lys Ala Cys Asn Pro Ser Asn Asp
1 5 10 15

Gln Cys Cys Lys Ser Ser Lys Leu Val Cys Ser Arg Lys Thr Gly Trp
20 25 30

Cys Lys Trp Ser Ile Gly Lys
35

<210> 327
<211> 39
<212> PRT
<213> Artificial sequence

<220>
<223> Huwentoxin-IV variant

<400> 327

Gly Pro Asn Cys Leu Arg Ile Phe Lys Ala Cys Asn Pro Ser Asn Asp
1 5 10 15

Gln Cys Cys Lys Ser Ser Lys Leu Val Cys Ser Arg Lys Thr Trp Trp

20 25 30

Cys Lys Trp Ser Ile Gly Lys
35

<210> 328
<211> 39
<212> PRT
<213> Artificial sequence

<220>
<223> Huwentoxin-IV variant

<400> 328

Gly Pro Asn Cys Leu Arg Ile Phe Lys Ala Cys Asn Pro Ser Asn Asp
1 5 10 15

Gln Cys Cys Lys Ser Ser Lys Leu Val Cys Ser Arg Lys Thr Arg Trp
20 25 30

Cys Lys Trp Ser Ile Thr Lys
35

<210> 329
<211> 39
<212> PRT
<213> Artificial sequence

<220>
<223> Huwentoxin-IV variant

<400> 329

Gly Pro Asn Cys Leu Arg Ile Phe Lys Ala Cys Asn Pro Ser Asn Asp
1 5 10 15

Gln Cys Cys Lys Ser Ser Lys Leu Val Cys Ser Arg Lys Thr Arg Lys
20 25 30

Cys Lys Trp Ser Ile Gly Lys
35

<210> 330
<211> 39
<212> PRT
<213> Artificial sequence

<220>
<223> Huwentoxin-IV variant

<400> 330

Gly Pro Asn Cys Leu Arg Ile Phe Lys Ala Cys Asn Pro Ser Asn Asp
1 5 10 15

```
Gln Cys Cys Lys Ser Ser Lys Leu Val Cys Ser Arg Lys Thr Leu Trp
            20                  25                  30

Cys Lys Trp Ser Ile Gly Lys
        35
```

<210> 331
<211> 39
<212> PRT
<213> Artificial sequence

<220>
<223> Huwentoxin-IV variant

<400> 331

```
Gly Pro Asn Cys Leu Arg Ile Phe Lys Ala Cys Asn Pro Ser Asn Asp
1               5                   10                  15

Gln Cys Cys Lys Ser Ser Lys Leu Val Cys Ser Arg Lys Thr Arg Tyr
            20                  25                  30

Cys Lys Trp Ser Ile Gly Lys
        35
```

<210> 332
<211> 39
<212> PRT
<213> Artificial sequence

<220>
<223> Huwentoxin-IV variant

<400> 332

```
Gly Pro Asn Cys Leu Arg Ile Phe Lys Ala Cys Asn Pro Ser Asn Asp
1               5                   10                  15

Gln Cys Cys Lys Ser Ser Lys Leu Val Cys Ser Pro Lys Thr Arg Trp
            20                  25                  30

Cys Lys Trp Ser Ile Gly Lys
        35
```

<210> 333
<211> 39
<212> PRT
<213> Artificial sequence

<220>
<223> Huwentoxin-IV variant

<400> 333

Gly Pro Asn Cys Leu Arg Ile Phe Lys Ala Cys Asn Pro Ser Asn Asp
1               5                   10                  15

Gln Cys Cys Lys Ser Ser Lys Ala Val Cys Ser Arg Lys Thr Arg Trp
            20                  25                  30

Cys Lys Trp Ser Ile Gly Lys
        35

<210>  334
<211>  39
<212>  PRT
<213>  Artificial sequence

<220>
<223>  Huwentoxin-IV variant

<400>  334

Gly Pro Asn Cys Leu Arg Ile Phe Lys Ala Cys Asn Pro Ser Asn Asp
1               5                   10                  15

Gln Cys Cys Lys Ser Ser Lys Trp Val Cys Ser Arg Lys Thr Arg Trp
            20                  25                  30

Cys Lys Trp Ser Ile Gly Lys
        35

<210>  335
<211>  39
<212>  PRT
<213>  Artificial sequence

<220>
<223>  Huwentoxin-IV variant

<400>  335

Gly Pro Asn Cys Leu Arg Ile Phe Lys Ala Cys Asn Pro Ser Asn Asp
1               5                   10                  15

Gln Cys Cys Lys Ser Ser Lys Leu Val Cys Ser Arg Lys Leu Arg Trp
            20                  25                  30

Cys Lys Trp Ser Ile Gly Lys
        35

<210>  336
<211>  39
<212>  PRT
<213>  Artificial sequence

<220>

<223> Huwentoxin-IV variant

<400> 336

Gly Pro Asn Cys Leu Arg Ile Phe Lys Ala Cys Asn Pro Ser Asn Asp
1               5                   10                  15

Gln Cys Cys Lys Ser Ser Lys Leu Val Cys Ser Arg Pro Thr Arg Trp
            20                  25                  30

Cys Lys Trp Ser Ile Gly Lys
        35

<210> 337
<211> 39
<212> PRT
<213> Artificial sequence

<220>
<223> Huwentoxin-IV variant

<400> 337

Gly Pro Asn Cys Leu Arg Ile Phe Lys Ala Cys Asn Pro Ser Asn Asp
1               5                   10                  15

Gln Cys Cys Lys Ser Ser Lys Leu Val Cys Ser Val Lys Thr Arg Trp
            20                  25                  30

Cys Lys Trp Ser Ile Gly Lys
        35

<210> 338
<211> 39
<212> PRT
<213> Artificial sequence

<220>
<223> Huwentoxin-IV variant

<400> 338

Gly Pro Asn Cys Leu Arg Ile Phe Lys Ala Cys Asn Pro Ser Asn Asp
1               5                   10                  15

Gln Cys Cys Lys Ser Ser Lys Leu Val Cys Ser Gly Lys Thr Arg Trp
            20                  25                  30

Cys Lys Trp Ser Ile Gly Lys
        35

<210> 339
<211> 39
<212> PRT

<213> Artificial sequence

<220>
<223> Huwentoxin-IV variant

<400> 339

Gly Pro Asn Cys Leu Arg Ile Phe Lys Ala Cys Asn Pro Ser Asn Asp
1               5                   10                  15

Gln Cys Cys Lys Ser Ser Trp Leu Val Cys Ser Arg Lys Thr Arg Trp
            20                  25                  30

Cys Lys Trp Ser Ile Gly Lys
        35

<210> 340
<211> 39
<212> PRT
<213> Artificial sequence

<220>
<223> Huwentoxin-IV variant

<400> 340

Gly Pro Asn Cys Leu Arg Ile Phe Lys Ala Cys Asn Pro Ser Asn Asp
1               5                   10                  15

Gln Cys Cys Lys Ser Ser Lys Leu Val Cys Ser Ile Lys Thr Arg Trp
            20                  25                  30

Cys Lys Trp Ser Ile Gly Lys
        35

<210> 341
<211> 39
<212> PRT
<213> Artificial sequence

<220>
<223> Huwentoxin-IV variant

<400> 341

Gly Pro Asn Cys Leu Arg Ile Phe Lys Ala Cys Asn Pro Ser Asn Asp
1               5                   10                  15

Gln Cys Cys Lys Ser Ser Lys Leu Val Cys Ser Arg Trp Thr Arg Trp
            20                  25                  30

Cys Lys Trp Ser Ile Gly Lys
        35

<210> 342
<211> 39
<212> PRT
<213> Artificial sequence

<220>
<223> Huwentoxin-IV variant

<400> 342

Gly Pro Asn Cys Leu Arg Ile Phe Lys Ala Cys Asn Pro Ser Asn Asp
1 5 10 15

Gln Cys Cys Lys Ser Ser Lys Leu Val Cys Ser Ala Lys Thr Arg Trp
20 25 30

Cys Lys Trp Ser Ile Gly Lys
35

<210> 343
<211> 39
<212> PRT
<213> Artificial sequence

<220>
<223> Huwentoxin-IV variant

<400> 343

Gly Pro Asn Cys Leu Arg Ile Phe Lys Ala Cys Asn Pro Ser Asn Asp
1 5 10 15

Gln Cys Cys Lys Ser Trp Lys Leu Val Cys Ser Arg Lys Thr Arg Trp
20 25 30

Cys Lys Trp Ser Ile Gly Lys
35

<210> 344
<211> 39
<212> PRT
<213> Artificial sequence

<220>
<223> Huwentoxin-IV variant

<400> 344

Gly Pro Asn Cys Leu Arg Ile Phe Lys Ala Cys Asn Pro Ser Asn Asp
1 5 10 15

Glu Cys Cys Lys Ser Ser Lys Leu Val Cys Ser Arg Lys Thr Arg Trp
20 25 30

Cys Lys Trp Ser Ile Gly Lys

35

<210> 345
<211> 39
<212> PRT
<213> Artificial sequence

<220>
<223> Huwentoxin-IV variant

<400> 345

Gly Pro Asn Cys Leu Arg Ile Phe Lys Ala Cys Asn Pro Ser Asn Asp
1 5 10 15

Gln Cys Cys Pro Ser Ser Lys Leu Val Cys Ser Arg Lys Thr Arg Trp
20 25 30

Cys Lys Trp Ser Ile Gly Lys
35

<210> 346
<211> 39
<212> PRT
<213> Artificial sequence

<220>
<223> Huwentoxin-IV variant

<400> 346

Gly Pro Asn Cys Leu Arg Ile Phe Lys Ala Cys Asn Pro Ser Asn Asp
1 5 10 15

Val Cys Cys Lys Ser Ser Lys Leu Val Cys Ser Arg Lys Thr Arg Trp
20 25 30

Cys Lys Trp Ser Ile Gly Lys
35

<210> 347
<211> 39
<212> PRT
<213> Artificial sequence

<220>
<223> Huwentoxin-IV variant

<400> 347

Gly Pro Asn Cys Leu Arg Ile Phe Lys Ala Cys Asn Pro Ser Asn Gly
1 5 10 15

Gln Cys Cys Lys Ser Ser Lys Leu Val Cys Ser Arg Lys Thr Arg Trp
20 25 30

Cys Lys Trp Ser Ile Gly Lys
35

<210> 348
<211> 39
<212> PRT
<213> Artificial sequence

<220>
<223> Huwentoxin-IV variant

<400> 348

Gly Pro Asn Cys Leu Arg Ile Phe Lys Ala Cys Asn Pro Ser Asn Asp
1 5 10 15

Gln Cys Cys Phe Ser Ser Lys Leu Val Cys Ser Arg Lys Thr Arg Trp
20 25 30

Cys Lys Trp Ser Ile Gly Lys
35

<210> 349
<211> 39
<212> PRT
<213> Artificial sequence

<220>
<223> Huwentoxin-IV variant

<400> 349

Gly Pro Asn Cys Leu Arg Ile Phe Lys Ala Cys Asn Pro Ser Asn Asp
1 5 10 15

Gln Cys Cys Lys Ser Ser Lys Leu Val Cys Ser Arg Lys Thr Arg Trp
20 25 30

Cys Lys Trp Ser Ile Gly Thr
35

<210> 350
<211> 39
<212> PRT
<213> Artificial sequence

<220>
<223> Huwentoxin-IV variant

<400> 350

Gly Pro Asn Cys Leu Arg Ile Phe Lys Ala Cys Asn Pro Ser Asn Asp
1 5 10 15

Gln Cys Cys Lys Ser Ser Lys Leu Val Cys Ser Arg Lys Thr Arg Trp
20 25 30

Cys Lys Trp Ser Ile Gly Gln
35

<210> 351
<211> 39
<212> PRT
<213> Artificial sequence

<220>
<223> Huwentoxin-IV variant

<400> 351

Gly Pro Asn Cys Leu Arg Ile Phe Lys Ala Cys Asn Pro Ser Asn Asp
1 5 10 15

Gln Cys Cys Lys Ser Ser Lys Leu Val Cys Ser Arg Lys Thr Arg Trp
20 25 30

Cys Lys Trp Ser Ile Gly Ser
35

<210> 352
<211> 39
<212> PRT
<213> Artificial sequence

<220>
<223> Huwentoxin-IV variant

<400> 352

Gly Pro Asn Cys Leu Arg Ile Met Lys Ala Cys Asn Pro Ser Asn Asp
1 5 10 15

Gln Cys Cys Lys Ser Ser Lys Leu Val Cys Ser Arg Lys Thr Arg Trp
20 25 30

Cys Lys Trp Ser Ile Gly Lys
35

<210> 353
<211> 39
<212> PRT
<213> Artificial sequence

<220>
<223> Huwentoxin-IV variant

<400> 353

Gly Pro Asn Cys Leu Arg Ile Phe Gln Ala Cys Asn Pro Ser Asn Asp
1               5                   10                  15

Gln Cys Cys Lys Ser Ser Lys Leu Val Cys Ser Arg Lys Thr Arg Trp
            20                  25                  30

Cys Lys Trp Ser Ile Gly Lys
        35

<210> 354
<211> 39
<212> PRT
<213> Artificial sequence

<220>
<223> Huwentoxin-IV variant

<400> 354

Gly Pro Asn Cys Leu Arg Ile Phe Lys Ala Cys Asn Arg Ser Asn Asp
1               5                   10                  15

Gln Cys Cys Lys Ser Ser Lys Leu Val Cys Ser Arg Lys Thr Arg Trp
            20                  25                  30

Cys Lys Trp Ser Ile Gly Lys
        35

<210> 355
<211> 39
<212> PRT
<213> Artificial sequence

<220>
<223> Huwentoxin-IV variant

<400> 355

Gly Pro Asn Cys Leu Arg Ile Phe Lys Ala Cys Asn Pro Ser Asn Asp
1               5                   10                  15

Gln Cys Cys Lys Gln Ser Lys Leu Val Cys Ser Arg Lys Thr Arg Trp
            20                  25                  30

Cys Lys Trp Ser Ile Gly Lys
        35

<210> 356
<211> 30
<212> PRT
<213> Thrixopelma pruriens

<400> 356

```
Tyr Cys Gln Lys Trp Met Trp Thr Cys Asp Ser Glu Arg Lys Cys Cys
1               5                   10                  15

Glu Gly Met Val Cys Arg Leu Trp Cys Lys Lys Lys Leu Trp
            20                  25                  30


<210>  357
<211>  16
<212>  PRT
<213>  Conus kinoshitai


<220>
<221>  MOD_RES
<222>  (16)..(16)
<223>  AMIDATION

<400>  357

Cys Cys Asn Cys Ser Ser Lys Trp Cys Arg Asp His Ser Arg Cys Cys
1               5                   10                  15
```

## Description

### Cross-reference to earlier applications

This application claims the benefit of and U.S. Provisional Application No. 61/781,276, filed 14 March 2013, and U.S. Patent Application No. 13/833,555, filed March 15, 2013, which claims the benefit of U.S. Provisional Application No. 61/702,538, filed 18 September, 2012, and U.S. Provisional Application No. 61/648,871, filed 18 May, 2012, the entire contents of which are incorporated herein by reference.

### Field of the Invention

The present invention relates to Huwentoxin-IV variants, polynucleotides encoding them, methods of making and using the foregoing, and methods of alleviating pain with peptide inhibitors of Nav1.7.

### Background of the Invention

Voltage-gated sodium channels (VGSC) are present in all excitable cells including cardiac and skeletal muscle cells and central and peripheral neurons. In neuronal cells, sodium channels are responsible for amplifying sub-threshold depolarizations and generating the rapid upstroke of the action potential. As such, sodium channels are essential to the initiation and propagation of electrical signals in the nervous system. Aberrant sodium channel function is thought to underlie a variety of medical disorders (Hübner and Jentsch, Hum Mol Genet 11:2435-45, 2002) including epilepsy (Yogeeswari et al., Curr Drug Targets 5:589-602, 2004), arrhythmia (Tfelt-Hansen et al., J Cardiovasc Electrophysiol 21:107-15, 2010) myotonia (Cannon and Bean, J Clin Invest 120:80-3, 2010), and pain (Cregg et al., J Physiol 588:1897-904, 2010). Sodium channels are typically a complex of various subunits, the principle one being the pore-forming alpha-subunit, which is alone sufficient for function.

Nine known members of the family of voltage-gated sodium channel (VGSC) alpha subunits exist in humans, Nav1.1 - Nav1.9. The Nav1.x subfamily can be pharmacologically subdivided into tetrodotoxin (TTX) -sensitive or TTX-resistant. Nav1.7, (also named as PN1, SCN9A or hNE) is TTX-sensitive and is primarily expressed in peripheral sympathetic and sensory neurons. Nav1.7 accumulates at nerve fiber endings and amplifies small sub-threshold depolarizations and acts as a threshold channel that regulates excitability.

Nav1.7 function is implicated in various pain states, including acute, inflammatory and/or neuropathic pain. In man, gain of function mutations of Nav1.7 have been linked to primary erythermalgia (PE), a disease characterized by burning pain and inflammation of the extremities (Yang et al., J Med Genet 41:171-4, 2004), and paroxysmal extreme pain disorder (PEPD)(Fertleman et al., Neuron 52:767-74, 2006). Consistent with this observation, non-selective sodium channel blockers lidocaine, mexiletine and carbamazepine can provide symptomatic relief in these painful disorders (Legroux-Crespel et al., Ann Dermatol Venereol 130:429-33, 2003; Fertleman et al., Neuron 52:767-74, 2006).

Loss-of-function mutations of SNC9A in humans cause congenital indifference to pain (CIP), a rare autosomal recessive disorder characterized by a complete indifference or insensitivity to painful stimuli (Cox et al., Nature 444:894-8, 2006; Goldberg et al, Clin Genet 71:311-9, 2007; Ahmad et al., Hum Mol Genet 16:2114-21, 2007).

Single nucleotide polymorphisms in the coding region of SCN9A have been associated with increased nociceptor excitability and pain sensitivity. For example, a polymorphism rs6746030 resulting in R1150W substitution in human Nav1.7 has been associated with osteoarthritis pain, lumbar discectomy pain, phantom pain, and pancreatitis pain (Reimann et al., Proc Natl Acad Sci USA 107:5148-53, 2010). DRG neurons expressing the R1150W Nav1.7 display increased firing frequency in response to depolarization (Estacion et al., Ann Neurol 66:862-6, 2009). A disabling form of fibromyalgia has been associated with SCN9A sodium channel polymorphism rs6754031, indicating that some patients with severe fibromyalgia may have a dorsal root ganglia sodium channelopathy (Vargas-Alarcon et al., BMC Musculoskelet Disord 13:23, 2012).

In mice, deletion of the SCN9A gene in nociceptive neurons lead to reduction in mechanical and thermal pain thresholds and reduction or abolition of inflammatory pain responses (Nassar et al., Proc Natl Acad Sci USA 101:12706-11, 2004). Ablating Nav1.7 gene expression in all sensory neurons abolished mechanical pain, inflammatory pain and reflex withdrawal responses to heat. Deleting SCN9A in both sensory and sympathetic neurons abolished mechanical, thermal and neuropathic pain, and recapitulated the pain-free phenotype seen in humans with SCN9A loss-of-function mutations (Minett et al., Nat Commun 3:791, 2012). Nav1.7 inhibitors or blockers may therefore be useful in the treatment of a wide range of pain associated with various disorders.

Spider venoms are known to contain a large number of sodium channel blocking peptides, including Huwentoxin-IV (HwTx-IV) (Peng et al., J Biol Chem 277:47564-71, 2002), Protoxin-I, Protoxin-II (Middleton et al., Biochemistry 41:14734-47, 2002) and Phrixotoxin-III (Bosmans et al., Mol Pharmacol 69:419-29, 2006). Huwentoxin-IV (HWTx-IV), from the Chinese bird spider *Ornithoctonus huwena,* is a potent blocker of Nav1.7 and other TTX-sensitive voltage-gated sodium channels and likely functions as a gating modifier by trapping the voltage sensor of domain II in an inward, closed conformation (Xiao et al., J Biol Chem 283:27300-13, 2008). Protoxin-II, due to its favorable potency and selectivity profile, has been the subject of various *in vivo* studies aimed at demonstrating analgesia, none of which have reported success without disrupting the perineurium. Only through subversion of the blood-nerve barrier via desheathing of cutaneous nerves (Schmalhofer et al., Mol Pharm 74:1476-1484, 2008) or perineurial injection of hypertonic saline leading to down-regulation of tight junction protein claudin-1 (Hackel et. al., PNAS 109:29 E2018-27, 2012) was any efficacy observed for Protoxin-II. There is a need for identification of additional Nav1.7 blockers for treatment of a wide range of pain indications. In particular, there is a need for new Nav1.7 blockers with selectivity for Nav1.7 over other VGSC isoforms.

## Brief Description of the Drawings

**Figure 1 a) and b)** shows $IC_{50}$ values for inhibition of veratridine-induced membrane depolarization for Nav1.7 for generated Huwentoxin-IV variants having specific substitutions at designated residue positions. Reference Huwentoxin-IV residue corresponds to residues in polypeptide of SEQ ID NO: 267. Substitutions highlighted in gray result in variants having $IC_{50}$ values $\leq 300\times10^{-9}$ M. Values beginning with > indicate that the particular variant was inactive at the concentration indicated.

**Figure 2 a) and b)** shows $IC_{50}$ values for inhibition of veratridine-induced membrane depolarization for Nav1.2 for generated Huwentoxin-IV variants having specific substitutions at designated residue positions. Reference Huwentoxin-IV residue corresponds to residues in polypeptide of SEQ ID NO: 267. Values beginning with > indicate that the particular variant was inactive at the concentration indicated.

**Figure 3 a) and b)** shows selectivity of generated Huwentoxin-IV variants as ratios of $IC_{50}$ values for Nav1.2 to $IC_{50}$ values for Nav1.7 for each variant having specific substitutions at designated residue positions ($IC_{50}$ values calculated for inhibition of veratridine-induced membrane depolarization).

Reference Huwentoxin-IV residue corresponds to residues in polypeptide of SEQ ID NO: 267. Substitutions highlighted in gray result in variants having $IC_{50}$ (Nav1.2)/$IC_{50}$ (Nav1.7) ratio equal or over 5.0. Values beginning with > indicate that the particular variant was inactive at the concentration indicated. "Inact" indicates that the peptide was inactive on Nav1.7.

**Figure 4** shows sequences of Huwentoxin-IV variants having $EC_{50}$ for Nav1.7 $\leq 300\times10^{-9}$ M ($IC_{50}$ values calculated for inhibition of veratridine-induced membrane depolarization).

**Figure 5** shows sequences of Huwentoxin-IV variants that are at least 5-fold more selective for Nav1.7 than Nav1.2, assessed using the $IC_{50}$ (Nav1.2)/$IC_{50}$ (Nav1.7) ratio, of are inactive at Nav1.2 ($IC_{50}$ values calculated for inhibition of veratridine-induced membrane depolarization).

**Figure 6** shows $IC_{50}$ values and selectivity for select Huwentoxin-IV variants in whole cell patch-clamp assay (QPatch).

**Figure 7** shows line graph of Randall-Selitto paw pressure thresholds in grams (g) before (Pre) and 5, 10, 20, 30, 45 and 60 minutes following dorsal hind paw injections of vehicle (n=9) or a) 0.3 nmoles, b) 3 nmoles or c) 30 nmoles of huwentoxin IV (n=9) in rat. Data are represented as mean $\pm$ s.e.m using Two-way ANOVA with Bonferroni post-tests. NS = not significant; **=p<0.01; ***=p<0.001.

**Figure 8** shows mean area under the curve (AUC) of gram thresholds for huwentoxin IV-treated rats with subtraction (for each individual huwentoxin IV-treated rat) of the mean AUC for vehicle-treated animals. Using one-way ANOVA, there was a significant effect of dose (p<0.001) demonstrating dose-dependent responses. Bonferroni post tests showed significant differences between each dose group, **=p<0.01; ***=p<0.001.

**Figure 9** shows various Huwentoxin-IV alanine mutants that cause significant (>10x) reduction in function (QPatch) colored by average per residue C-alpha (CA) atoms root mean square deviation (RMSD) calculated from their respective molecular dynamics simulations (50 ns each). The CA RMSDs are colored on a gradient from 0.5 Å in red to 2.2 Å in blue. (a) WT (b) F6A, (c) P11A, (d) D14A, (e) L22A, (f) S25A, (g)W30A, (h) K32A and (i) Y33A Huwentoxin-IV mutants.

**Figure 10** shows various Huwentoxin-IV alanine mutants that appear to cause isoform specific changes in function (QPatch) colored by average per residue CA RMSD calculated from their respective molecular dynamics simulations (50 ns each). The CA RMSDs are colored on a gradient from 0.5 Å in red to 2.2 Å in blue. (a) K18A, (b) R26A, (c) K27A.

**Figure 11** shows NMR solution structure of recombinant Huwentoxin-IV (SEQ ID NO:1). The NMR structure reveals 5 residues in HwTx-IV (F6, T28, W30, K32 and Y33) that form a twisted β-sheet (cyan) to create a polar-aryl face, a putative interacting surface between Huwentoxin-IV and Nav1.7.

**Figure 12** shows Homology model of the domain 2 (DII) voltage sensing domain (VSD) of hNav1.7 with Huwentoxin-IV docked.

Based on this model, Huwentoxin-IV docks in a grove made by segments S1-S2 and S3-S4. Huwentoxin-IV (SEQ ID NO:1) residues K32 and W30 are predicted to interact with Nav1.7 (SEQ ID NO:263) residues E811 and M750, respectively.

**Figure 13** shows A) sequences and B) $IC_{50}$ values for NV1D2168 (SEQ ID NO: 102) variants. TETRA: FLIPR® Tetra; QP: QPatch.

**Figure 14** shows A) sequences and B) $IC_{50}$ values for NV1D2163 (SEQ ID NO: 3) variants. TETRA: FLIPR® Tetra; QP: QPatch.

**Figure 15** shows local administration of A) 3 nmoles and B) 30 nmoles Huwentoxin-IV (HwTx-IV) provides analgesic effect in a rat model of nociceptive pain as measured by an increase in paw pressure threshold after HwTx-IV administration. C) Mean area under the curve (AUC) of paw pressure response for HwTx-IV at indicated concentrations. In C),**p<0.01; ***p< 0.001.

**Figure 16** shows local administration of A) 0.3 nmoles; B) 3 nmoles; C) 30 nmoles Protoxin-II (ProTx-II) provides analgesic effect in a rat model of nociceptive pain as measured by an increase in paw pressure threshold after ProTx-II administration. D) Mean area under the curve (AUC) of paw pressure response for HwTx-IV at indicated concentrations. In C), *p<0.05; **p< 0.01.

**Figure 17** shows reduced A) tactile allodynia and B) thermal allodynia in rat model of monoarthritis induced by 50% intraplantar CFA in rats. C) Intraplantar ProTx-II administration significantly reduced Complete Freund's Adjuvant (CFA)-induced tactile allodynia.

**Figure 18** A) Tactile allodynia is induced by 100% intraplantar CFA but not with 50% CFA in mice. B) Intraplantar ProTx-II administration significantly reduced tactile allodynia in the 100% CFA-treated animals; C) gabapentin also reduced tactile allodynia albeit to a lesser extent than ProTx-II.

**Figure 19** shows reduced A) tactile allodynia and B) thermal allodynia in a mouse model of CFA-induced inflammatory pain in animals treated with ProTx-II administered via mini-pump at 228 μg/mouse/day for 3 days.

## Summary of the Invention

One embodiment of the invention is an isolated Huwentoxin-IV variant comprising a sequence $X_1CX_2X_3X_4FX_5X_6CX_7X_8X_9X_{10}X_{11}X_{12}CCX_{13}X_{14}X_{15}X_{16}X_{17}X_{18}CX_{19}X_{20}X_{21}X_{22}X_{23}$ $X_{24}CKX_{25}X_{26}IX_{27}X_{28}$ (SEQ ID NO: 265); wherein $X_1$, $X_2$, $X_3$, $X_4$, $X_5$, $X_6$, $X_7$, $X_8$, $X_9$, $X_{10}$, $X_{11}$, $X_{12}$, $X_{13}$, $X_{14}$, $X_{15}$, $X_{16}$, $X_{17}$, $X_{18}$, $X_{19}$, $X_{20}$, $X_{21}$, $X_{22}$, $X_{23}$, $X_{24}$, $X_{25}$, and $X_{26}$ are any amino acid; $X_{27}$ and $X_{28}$ are any amino acid or deleted; and the Huwentoxin-IV variant has an $IC_{50}$ value about $300\times10^{-9}$ M or less for human Nav1.7 (SEQ ID NO: 263), with the proviso that the Huwentoxin-IV variant is not a polypeptide comprising a sequence shown in SEQ ID NO: 1.

Another embodiment of the invention is an isolated Huwentoxin-IV variant comprising a sequence $X_1CX_2X_3X_4FX_5X_6CX_7X_8X_9X_{10}X_{11}X_{12}CCX_{13}X_{14}X_{15}X_{16}X_{17}X_{18}CX_{19}X_{20}X_{21}X_{22}X_{23}$ $X_{24}CKX_{25}X_{26}IX_{27}X_{28}$ (SEQ ID NO: 265); wherein $X_1$, $X_2$, $X_3$, $X_4$, $X_5$, $X_6$, $X_7$, $X_8$, $X_9$, $X_{10}$, $X_{11}$, $X_{12}$, $X_{13}$, $X_{14}$, $X_{15}$, $X_{16}$, $X_{17}$, $X_{18}$, $X_{19}$, $X_{20}$, $X_{21}$, $X_{22}$, $X_{23}$, $X_{24}$, $X_{25}$, and $X_{26}$ are any amino acid; $X_{27}$ and $X_{28}$ are any amino acid or deleted; and the Huwentoxin-IV variant selectively inhibits Nav1.7, with the proviso that the Huwentoxin-IV variant is not a polypeptide comprising a sequence shown in SEQ ID NO: 1.

Another embodiment of the invention is an isolated Huwentoxin-IV variant comprising a sequence $X_1CX_2X_3X_4FX_5X_6CX_7X_8X_9X_{10}X_{11}X_{12}CCX_{13}X_{14}X_{15}X_{16}X_{17}X_{18}CX_{19}X_{20}X_{21}TX_{22}WCKYX_{23}X_{24}X_{25}X_{26}$ (SEQ ID NO: 276); wherein $X_1$, $X_2$, $X_3$, $X_4$, $X_5$, $X_6$, $X_7$, $X_8$, $X_9$, $X_{10}$, $X_{11}$, $X_{12}$, $X_{13}$, $X_{14}$, $X_{15}$, $X_{16}$, $X_{17}$, $X_{18}$, $X_{19}$, $X_{20}$, $X_{21}$, $X_{22}$, $X_{23}$ and $X_{24}$ are any amino acid; $X_{25}$ and $X_{26}$ are any amino acid or deleted; and the Huwentoxin-IV variant has an $IC_{50}$ value about $300\times10^{-9}$ M or less for human Nav1.7 (SEQ ID NO: 263), with the proviso that the Huwentoxin-IV variant is not a polypeptide comprising a sequence shown in SEQ ID NO: 1.

Another embodiment of the invention is an isolated polynucleotide encoding the Huwentoxin-IV variants of the invention.

Another embodiment of the invention is a vector comprising the isolated polynucleotides of the invention.

Another embodiment of the invention is a host cell comprising a vector of the invention.

Another embodiment of the invention is a method of producing the isolated Huwentoxin-IV variant polypeptide of the invention comprising culturing the host cell of the invention and recovering the Huwentoxin-IV variant polypeptide by the host cell.

Another embodiment of the invention is a pharmaceutical composition comprising the isolated Huwentoxin-IV variant of the invention and a pharmaceutically acceptable excipient.

Another embodiment of the invention is a method of treating pain in a subject, comprising administering to the subject an effective amount of the Huwentoxin-IV variant of the invention to treat pain, other disorders of sensory or sympathetic neuron dysfunction.

Another embodiment of the invention is a method of alleviating Nav1.7-mediated pain by administering a therapeutically effective amount of a peptide inhibitor of Nav1.7 in a subject in need thereof for a time sufficient to treat or alleviate the Nav1.7-mediated pain.

In the other aspects of the invention, the peptide inhibitor of Nav1.7 is administered peripherally.

In the other aspects of the invention, the peptide inhibitor of Nav1.7 is Protoxin-II or Huwentoxin-IV or variants thereof.

## Detailed Description of the Invention

All publications, including but not limited to patents and patent applications, cited in this specification are herein incorporated by reference as though fully set forth.

As used herein and in the embodiments, the singular forms "a," "and," and "the" include plural reference unless the context clearly dictates otherwise.

Unless defined otherwise, all technical and scientific terms used herein have the same meaning as commonly understood by one of ordinary skill in the art to which an invention belongs. Although any compositions and methods similar or equivalent to those described herein can be used in the practice or testing of the invention, exemplary compositions and methods are described herein.

The term "polypeptide" means a molecule that comprises at least two amino acid residues linked by a peptide bond to form a polypeptide. Small polypeptides of less than 50 amino acids may be referred to as "peptides". Polypeptides may also be referred as "proteins".

The term "polynucleotide" means a molecule comprising a chain of nucleotides covalently linked by a sugar-phosphate backbone or other equivalent covalent chemistry. Double and single-stranded DNAs and RNAs are typical examples of polynucleotides.

The term "complementary sequence" means a second isolated polynucleotide sequence that is antiparallel to a first isolated polynucleotide sequence and that comprises nucleotides complementary to the nucleotides in the first polynucleotide sequence.

The term "vector" means a polynucleotide capable of being duplicated within a biological system or that can be moved between such systems. Vector polynucleotides typically contain elements, such as origins of replication, polyadenylation signal or selection markers, that function to facilitate the duplication or maintenance of these polynucleotides in a biological system. Examples of such biological systems may include a cell, virus, animal, plant, and reconstituted biological systems utilizing biological components capable of duplicating a vector. The polynucleotides comprising a vector may be DNA or RNA molecules or hybrids of these.

The term "expression vector" means a vector that can be utilized in a biological system or a reconstituted biological system to direct the translation of a polypeptide encoded by a polynucleotide sequence present in the expression vector.

The term "wild type Huwentoxin-IV" or "wild type HwTx-IV" as used herein refers to Chinese bird spider *Ornithoctonus huwena* Huwentoxin-IV polypeptide having a sequence shown in SEQ ID NO: 1 (ECLEIFKACNPSNDQCCKSSKLVCSRKTRWCKYQI). The term "recombinant Huwentoxin-IV" or recombinant HwTx-IV" as used herein refers to the recombinantly expressed Huwentoxin-IV having a sequence shown in SEQ ID NO: 2 (GPECLEIFKACNPSNDQCCKSSKLVCSRKTRWCKYQIGK). Recombinant Huwentoxin-IV incorporates a two amino acid N- and C-terminal tail when compared to the wild type Huwentoxin-IV. The term "reference Huwentoxin-IV" refers to a polypeptide sequence of SEQ ID NO: 267 (ECLEIFKACNPSNDQCCKSSKLVCSRKTRWCKYQIGK). Throughout the specification, residue numbering is according to SEQ ID NO: 267. For example, "F6" in the specification refers to Phenylalanine residues at position 6 of SEQ ID NO: 267.

The term "variant" as used herein refers to a polypeptide or a polynucleotide that differs from the wild type Huwentoxin-IV polypeptide of SEQ ID NO: 1 or the wild type Huwentoxin-IV polynucleotide of SEQ ID NO: 268 sequence by one or more modifications for example, substitutions, insertions or deletions of nucleotides or amino acids.

"Nav1.7" (also called as SCN9A, hNE, PN1) as used herein refers to the well known sodium channel protein type 9 subunit alpha having a sequence shown in GenBank accession number NP_002968.1 and in SEQ ID NO: 263.

"Nav1.2" as used herein refers to the well known sodium channel protein type 2 subunit alpha (SCN2A) having a sequence shown in GenBank accession number NP_001035232.1 and in SEQ ID NO: 264.

"Blocks activity" or "inhibits activity" as used herein refers to an ability of Huwentoxin-IV variants to reduce membrane depolarization induced by veratridine (3-Veratroylveracevine) by at least 10%, 20%, 30%, 40%, 50%, 60%, 70%, 75%, 80%, 85%, 90%, 95% or 100% in an *in vitro* membrane depolarization assay using FRET (fluorescence resonance energy transfer), where veratridine-induced depolarization is measured as a reduction in FRET signal using DISBAC2(3) ([bis-(1,3-diethylthiobarbituric acid) trimethine oxonol]) as an acceptor and PTS18 (trisodium 8-octadecyloxypyrene-1,3,6-trisulfonate) as a donor by exciting the donor at 390-420 nm and measuring FRET at 515-575 nm using cell lines stably expressing Nav1.7.

The term "Protoxin-II" or "ProTx-II" as used herein refers to the tarantula *Thrixopelma pruriens* (Peruvian green velvet tarantula) toxin peptide having the amino acid sequence YCQKWMWTCDSERKCCEGMVCRLWCKKKLW-COOH (SEQ ID NO: 356) as described in Middleton et al., Biochemistry 41(50):14734-47, 2002. ProTx-II is a potent and selective Nav1.7 inhibitor *in vitro* with a reported $IC_{50}$ value of 0.3 nM and selectivity of over 100-fold when compared to other Nav1.x subtypes (Schmalhofer et al., Mol Pharmacol 74:1476-1484, 2008).

The term "μ-conotoxin KIIIA" or "conotoxin KIIIA" as used herein refers to the *Conus kinoshitai* toxin having the sequence CCNCSSKWCRDHSRCC-$NH_2$ (SEQ ID NO: 357) as described in Zhang et al., J Biol Chem 282(42):30699-706, 2007.

"Nav1.7 inhibitor" or "peptide inhibitor of Nav1.7" or "blocker of Nav1.7" as used herein refers to a peptide that inhibits, reduces or blocks Nav1.7 channel activity. Peptide inhibitors of Nav1.7 can be tested for their Nav1.7 blocking activity using electrophysiological assays known in the art and assays disclosed herein. For example see Clare et al., drug Discovery Today 5:506-520, 2000.

The present invention provides isolated Huwentoxin-IV (HwTx-IV) variant polypeptides that inhibit Nav1.7, polynucleotides encoding them, vectors, host cells, and methods of using the polynucleotides and polypeptides of the invention. The variants of the invention may be more potent or more selective towards Nav1.7 when compared to the recombinant Huwentoxin-IV polypeptide. The polypeptides of the invention inhibit depolarization resulting from Nav1.7 activation, and therefore may be useful in the treatment of various conditions associated with pain and conditions associated with sensory or sympathetic neuron dysfunction. The current invention is based, at least in part, on the finding that certain residues in Huwentoxin-IV are intolerant to substitutions, specifically F6, K32 and 135, and additionally residues I5, P11, D14, S25, K27, T28, W30 and Y33 (residue numbering according to SEQ ID NO: 267) are substantially intolerant to substitutions, while other residues may be substituted to enhance potency and/or selectivity of Huwentoxin-IV variants for Nav1.7 as long as the cysteine residues at positions C2, C9, C16, C17, C24 and C31 remain intact.

One embodiment of the invention is an isolated Huwentoxin-IV variant comprising a sequence $X_1CX_2X_3X_4FX_5X_6CX_7X_8X_9X_{10}X_{11}X_{12}CCX_{13}X_{14}X_{15}X_{16}X_{17}X_{18}CX_{19}X_{20}X_{21}X_{22}X_{23}$ $X_{24}CKX_{25}X_{26}IX_{27}X_{28}$ (SEQ ID NO: 265); wherein

a) $X_1$, $X_2$, $X_3$, $X_4$, $X_5$, $X_6$, $X_7$, $X_8$, $X_9$, $X_{10}$, $X_{11}$, $X_{12}$, $X_{13}$, $X_{14}$, $X_{15}$, $X_{16}$, $X_{17}$, $X_{18}$, $X_{19}$, $X_{20}$, $X_{21}$, $X_{22}$, $X_{23}$, $X_{24}$, $X_{25}$, and $X_{26}$ are any amino acid;
b) $X_{27}$ and $X_{28}$ are any amino acid or deleted; and
c) the Huwentoxin-IV variant has an $IC_{50}$ value about $300\times10^{-9}$ M or less for human Nav1.7 (SEQ ID NO: 263), with the proviso that the Huwentoxin-IV variant is not a polypeptide comprising a sequence shown in SEQ ID NOs: 1.

The Huwentoxin-IV variants of the invention are equally potent or more potent Nav1.7 inhibitors when compared to recombinant Huwentoxin-IV (SEQ ID NO: 2). Recombinant Huwentoxin-IV has an $IC_{50}$ value of about $160\times10^{-9}$ M for human Nav1.7 in a veratridine-induced depolarization inhibition assay measuring decline in FRET (fluorescence resonance energy transfer) in cells stably expressing Nav1.7 using FLIPR® Tetra instrument (Molecular Devices). A Huwentoxin-IV variant is "equally potent or more potent" Nav1.7 inhibitor when the $IC_{50}$ value in the assay described above is about $300\times10^{-9}$ M or less. This $IC_{50}$ value is set higher than the measured $IC_{50}$ for the recombinantly expressed Huwentoxin-IV due to the intrinsic variability (1/2 log) of the assay itself. For clarity, an $IC_{50}$ of $300\times10^{-9}$ M is identical to $IC_{50}$ of $3.0\times10^{-7}$ M.

The Huwentoxin-IV variants of the invention retain the native disulfide bridges between C2-C17, C9-C24 and C16-C31 in addition to invariant residues F6, K32 and 135 (residue numbering according to SEQ ID NO: 267), while the remaining residues can be substituted with any amino acid as long as the resulting variant in the above Nav1.7 inhibition assay has an $IC_{50}$ of about $300\times10^{-9}$ M or less.

The Huwentoxin-IV variant polypeptides of the invention may be produced by chemical synthesis, such as solid phase peptide synthesis, on an automated peptide synthesizer. Alternatively, the polypeptides of the invention can be obtained from polynucleotides encoding the polypeptides by the use of cell-free expression systems such as reticulocyte lysate based expression systems, or by standard recombinant expression systems. Those skilled in the art will recognize other techniques for obtaining the polypeptides of the invention. In an exemplary method, the Huwentoxin-IV variants of the invention are generated by expressing them as human serum albumin (HSA) fusion proteins utilizing a glycine-rich linker such as $(GGGGS)_4$ (SEQ ID NO: 269) or $(GGGGS)_6$ (SEQ ID NO: 266) coupled to a protease cleavable linker such as a recognition sequence for HRV3C protease (Recombinant type 14 3C protease from human rhinovirus) LEVLFQGP (HRV3C linker) (SEQ ID NO: 270)). Hexahistidine or other tags may be used to facilitate purification using well known methods.

Generation of the Huwentoxin-IV variants is typically achieved at the nucleic acid level. The polynucleotides can be synthesized using chemical gene synthesis according to methods described in U.S. Pat. No. US6521427 and US6670127, utilizing degenerate oligonucleotides to generate the desired variants, or by standard PCR cloning and mutagenesis. Libraries of variants can be generated by standard cloning techniques to clone the polynucleotides encoding the Huwentoxin-IV variants into the vector for expression.

The Huwentoxin-IV variants may incorporate additional N- and/or C-terminal amino acids when compared to the wild type HwTx-IV of SEQ ID NO: 1, for example resulting from cloning and/or expression schemes. For example, cleavage from HSA after expression of the variant as HSA-(GGGGS)$_4$-HRV3C linker-HwTx-IV variant fusion protein may result in the incorporation of additional two residues to the N-terminus of each HwTx-IV variant, such as G and P. Additional residues may be incorporated to the C-terminus of the HwTx-IV variants, such as G and K to generate an endogenous amidation recognition sequence.

The HwTx-IV variants of the invention are tested for their ability to inhibit Nav1.7 using methods described herein. An exemplary assay is a veratridine-induced depolarization inhibition assay measuring decline in FRET (fluorescence resonance energy transfer) in cells stably expressing Nav1.7. Another exemplary assay employs electrophysiological recordings to measure the total influx of sodium ions ($Na^+$) across the cell membrane by way of voltage differential using well known patch clamp techniques and described herein.

In another embodiment, an isolated Huwentoxin-IV variant comprises a sequence $X_1CX_2X_3X_4FX_5X_6CX_7X_8X_9X_{10}X_{11}X_{12}CCX_{13}X_{14}X_{15}X_{16}X_{17}X_{18}CX_{19}X_{20}X_{21}X_{22}X_{23}$ $X_{24}CKX_{25}X_{26}IX_{27}X_{28}$ (SEQ ID NO: 265); wherein

a) $X_4$ is Y, V or I;
b) $X_8$ is P or V;
c) $X_{11}$ is D, P or W;
d) $X_{19}$ is S or I;
e) $X_{21}$ is Y, W, A, H or K;
f) $X_{22}$ is T or V;
g) $X_{24}$ is W or K;
h) $X_{25}$ is W, T, I or Y;
i) $X_1$, $X_2$, $X_3$, $X_5$, $X_6$, $X_7$, $X_9$, $X_{10}$, $X_{12}$, $X_{13}$, $X_{14}$, $X_{15}$, $X_{16}$, $X_{17}$, $X_{18}$, $X_{20}$, $X_{23}$ and $X_{26}$ are any amino acid;
j) $X_{27}$ and $X_{28}$ are any amino acid or deleted; and
k) the Huwentoxin-IV variant has an $IC_{50}$ value about $300\times10^{-9}$ M or less for human Nav1.7 (SEQ ID NO: 263), with the proviso that the Huwentoxin-IV variant is not a polypeptide comprising a sequence shown in SEQ ID NO: 1. In another embodiment, the isolated Huwentoxin-IV

variant comprises a sequence $X_1CX_2X_3X_4FX_5X_6CX_7X_8X_9X_{10}X_{11}X_{12}CCX_{13}X_{14}X_{15}X_{16}X_{17}X_{18}CX_{19}X_{20}X_{21}X_{22}X_{23}$ $X_{24}CKX_{25}X_{26}IX_{27}X_{28}$ (SEQ ID NO: 265); wherein

a) $X_1$ is K, R, H, D, Y, F, N, Q, S, T, G, L, I, P or E;
b) $X_2$ is R, F, W, N, S or L;
c) $X_3$ is R, H, D, Y, N, Q, L, I, P or E;
d) $X_4$ is Y, V or I;
e) $X_5$ is R, W, Q, S or K;
f) $X_6$ is R, E, Y, F, V or A;
g) $X_7$ is K, R, E, Y, F, S, V or N;
h) $X_8$ is P or V;
i) $X_9$ is R, F, Q, V or S;
j) $X_{10}$ is H, D, Y, W, Q, S, T, G, A, V, L, I, P or N;
k) $X_{11}$ is D, P or W;
l) $X_{12}$ is K, R, D, E, Y, W, N, T, A, L or Q;
m) $X_{13}$ is R, Y, Q, S, T, G, L, I, P or K;
n) $X_{14}$ is K, R, Y, F, N, Q, G, A, V, L, I, P or S;
o) $X_{15}$ is R, H, D, Y, W, N, Q, T, V, I, P or S;
p) $X_{16}$ is R, H, D, F, W, N, Q, S, T, G, A, L or K;
q) $X_{17}$ is K, R, Y, F, W, P or L;
r) $X_{18}$ is K, R, T, A, L or V;
s) $X_{19}$ is S or I;
t) $X_{20}$ is K, W, G, A, I, D or R;
u) $X_{21}$ is Y, W, A or K;
v) $X_{22}$ is T or V;
w) $X_{23}$ is K, H, W, N, G, A, L or R;
x) $X_{24}$ is W or K;
y) $X_{25}$ is W, T, I or Y;
z) $X_{26}$ is K, R, Y, F, S, T, G, A, V, L, I or Q;

aa) $X_{27}$ is K, R, H, F, W, V, L, I, G or deleted; and

bb) $X_{28}$ is R, H, Y, F, W, N, G, V, P, K or deleted; and the Huwentoxin-IV variant has an $IC_{50}$ value about $300\times10^{-9}$ M or less for human Nav1.7 (SEQ ID NO: 263), with the proviso that the Huwentoxin-IV variant is not a polypeptide comprising a sequence shown in SEQ ID NO: 1.

The Huwentoxin-IV variants of the invention may inhibit Nav1.7 with an $IC_{50}$ value of between about $12\times10^{-9}$ M to about $300\times10^{-9}$ M. Exemplary variants demonstrating the range of $IC_{50}$ values are polypeptides of SEQ ID NOs: 3-222 shown in Figure 4.

Another embodiment of the invention is an isolated Huwentoxin-IV variant comprising a sequence $X_1CX_2X_3X_4FX_5X_6CX_7X_8X_9X_{10}X_{11}X_{12}CCX_{13}X_{14}X_{15}X_{16}X_{17}X_{18}CX_{19}X_{20}X_{21}X_{22}X_{23}$ $X_{24}CKX_{25}X_{26}IX_{27}X_{28}$ (SEQ ID NO: 265); wherein

a) $X_1$, $X_2$, $X_3$, $X_4$, $X_5$, $X_6$, $X_7$, $X_8$, $X_9$, $X_{10}$, $X_{11}$, $X_{12}$, $X_{13}$, $X_{14}$, $X_{15}$, $X_{16}$, $X_{17}$, $X_{18}$, $X_{19}$, $X_{20}$, $X_{21}$, $X_{22}$, $X_{23}$, $X_{24}$, $X_{25}$, and $X_{26}$ are any amino acid;

b) $X_{27}$ and $X_{28}$ are any amino acid or deleted; and

c) the Huwentoxin-IV variant selectively inhibits Nav1.7, with the proviso that the Huwentoxin-IV variant is not a polypeptide comprising a sequence shown in SEQ ID NO: 1.

The Huwentoxin-IV variants of the invention may be more selective towards Nav1.7 when compared to the recombinant Huwentoxin-IV (SEQ ID NO: 2). Recombinant Huwentoxin-IV has an $IC_{50}$ of about $159\times10^{-9}$ M for Nav1.7 and an $IC_{50}$ of about $342\times10^{-9}$ M for Nav1.2, and therefore the ratio of $IC_{50}$ for Nav1.2 to $IC_{50}$ for Nav1.7 about 2.143. "Selectivity" or "selective" or "more selective" or "selectively blocks" or "selectively inhibits" when used herein refers to a Huwentoxin-IV variant that has a ratio of $IC_{50}$ for Nav1.2 to $IC_{50}$ for Nav1.7 ($IC_{50}$(Nav1.2)/ $IC_{50}$(Nav1.7)) equal or over about 5.0. In addition, a Huwentoxin-IV variant "selectively inhibits" Nav1.7 in instances when the variant does not inhibit Nav1.2 at a peptide concentration of at least $0.8\times10^{-6}$ M even if the $IC_{50}$ ratio is less than 5. $IC_{50}$ for Nav1.2 can be assayed in a veratridine-induced depolarization inhibition assay using cell lines stably expressing Nav1.2 according to methods described for Nav1.7.

Residue positions in Huwentoxin-IV that can be mutagenized to improve selectivity include residues N13, D14, Q15, K18, S19, S20, K21, L22, R26, K27, R29, W30, Y33 and Q34 (residue numbering according to SEQ ID NO: 267). Exemplary substitutions to improve selectivity are N13G, N13I, Q15E, Q15W, Q15P, K18F, K18P, S19Q, R26K and R26I. Exemplary Huwentoxin-IV variants with improved selectivity are variants of SEQ ID NOs: 5, 7, 12, 13, 16, 21, 25, 45, 46, 48, 55, 57, 58, 60, 61, 72, 74,76, 78, 82, 83, 96, 109, 111, 113, 122, 127, 131, 134, 137, 141, 142, 149, 164, 165, 172, 175, 177, 178, 180, 182, 188, 189, 192, 198, 202, 204, 213, 215, 219, 223, 224, 225, 226, 227, 228, 229, 230, 231, 232, 233, 234, 235, 236, 237, 238, 239 and 240.

Residues K7, N13, D14, Q15, K18, S19, S20, K21, L22, V23, R26, K27, R29, W30, Y33, and Q34, G36 and K37 (residue number according to SEQ ID NO: 267) may be substituted to improve both potency and selectivity of the resulting Huwentoxin-IV variants (Figure 1 and 3). Exemplary substitutions increasing both potency and selectivity are R26K, Y33W, G36I, N13Q, S19Q, and K37R (residue numbering according to SEQ ID NO: 267). Exemplary variants with improved potency and selectivity are variants of SEQ ID NOs: 5, 6, 7, 12, 13, 16, 21, 25, 45, 46, 48, 55, 57, 58, 60, 61, 72, 74, 76, 78, 82, 83, 96, 109, 111, 113, 122, 127, 131, 134, 137, 141, 142, 149, 164, 165, 169, 172, 175, 177, 178, 180, 181, 182, 187, 188, 189, 192, 198, 202, 203, 204, 207, 213, 215, 216, 219 and 221.

Another embodiment of the invention is a Huwentoxin-IV variant having the amino acid sequence shown in SEQ ID NOs: 277, 278, 279, 280, 281, 282, 283, 284, 285, 286, 287, 288, 289, 290, 291, 292, 293, 294, 295, 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354 and 355.

Selectivity and/or potency of the Huwentoxin-IV variants of the invention can further be improved by selective substitutions (grafting) at positions identified to modulate selectivity and/or potency into existing variants. Exemplary variants that can further be modified and/or improved are variants NV1G387 (E1N, R26K, Q34S, G36I; NV1D2168, SEQ ID NO: 192) and NV1G327 (E1N, E4R, Y33W, Q34S; NV1D2163, SEQ ID NO: 3). NV1G387 demonstrated high selectivity towards Nav1.7. The potency of NV1G387 can be potentially improved by diversifying positions E4, A8, N13, Q15, K18, S19, S20, K21, L22, S25, K37 and G36. Exemplary substitutions are shown in Figure 13A and Figure 14A. NV1G327 demonstrated higher potency towards Nav1.7. The selectivity of NG1G327 can be potentially improved by diversifying positions F6, P11, D14, Q15, K18, S19, R26, K27, R29, K32 and Y33. Exemplary substitutions are shown in Figure 13A and Figure 14A. Those skilled in the art will recognize that substitutions at any Huwentoxin-IV variant described herein may be combined and the effect of the combination on the potency, selectivity or other characteristics can be assessed using methods described herein.

Another embodiment of the invention is an isolated Huwentoxin-IV variant comprising a sequence

$X_1CX_2X_3X_4FX_5X_6CX_7X_8X_9X_{10}X_{11}X_{12}CCX_{13}X_{14}X_{15}X_{16}X_{17}X_{18}CX_{19}X_{20}X_{21}TX_{22}WCKYX_{23}X_{24}X_{25}X_{26}$ (SEQ ID NO: 276); wherein

$X_1$, $X_2$, $X_3$, $X_4$, $X_5$, $X_6$, $X_7$, $X_8$, $X_9$, $X_{10}$, $X_{11}$, $X_{12}$, $X_{13}$, $X_{14}$, $X_{15}$, $X_{16}$, $X_{17}$, $X_{18}$, $X_{19}$, $X_{20}$, $X_{21}$, $X_{22}$, $X_{23}$ and $X_{24}$ are any amino acid;
$X_{25}$ and $X_{26}$ are any amino acid or deleted; and
the Huwentoxin-IV variant has an $IC_{50}$ value about $300\times10^{-9}$ M or less for human Nav1.7 (SEQ ID NO: 263), with the proviso that the Huwentoxin-IV variant is not a polypeptide comprising a sequence shown in SEQ ID NO: 1.

Huwentoxin-IV variant of SEQ ID NO: 276 may comprise following substitutions:

$X_4$ is Y, V or I;
$X_8$ is P or V;
$X_{11}$ is D, P or W;
$X_{19}$ is S or I;
$X_{21}$ is Y, W, A, H or K; and
$X_{24}$ is I in SEQ ID NO: 276.

Huwentoxin-IV variant of SEQ ID NO: 276 may further comprise following substitutions:

$X_1$ is K, R, H, D, Y, F, N, Q, S, T, G, L, I, P or E;
$X_2$ is R, F, W, N, S or L;
$X_3$ is R, H, D, Y, N, Q, L, I, P or E;
$X_5$ is R, W, Q, S or K;
$X_6$ is R, E, Y, F, V or A;
$X_7$ is K, R, E, Y, F, S, V or N;
$X_9$ is R, F, Q, V or S;
$X_{10}$ is H, D, Y, W, Q, S, T, G, A, V, L, I, P or N;
$X_{12}$ is K, R, D, E, Y, W, N, T, A, L or Q;
$X_{13}$ is R, Y, Q, S, T, G, L, I, P or K;
$X_{14}$ is K, R, Y, F, N, Q, G, A, V, L, I, P or S;
$X_{15}$ is R, H, D, Y, W, N, Q, T, V, I, P or S;
$X_{16}$ is R, H, D, F, W, N, Q, S, T, G, A, L or K;
$X_{17}$ is K, R, Y, F, W, P or L;
$X_{18}$ is K, R, T, A, L or V;
$X_{20}$ is K, W, G, A, I, D or R;
$X_{22}$ is K, H, W, N, G, A, L or R;
$X_{23}$ is K, R, Y, F, S, T, G, A, V, L, I or Q;
$X_{25}$ is K, R, H, F, W, V, L, I, G or deleted; and
$X_{26}$ is R, H, Y, F, W, N, G, V, P, K or deleted.

The isolated Huwentoxin-IV variant of SEQ ID NO: 276 may have an $IC_{50}$ of less than about $160\times10^{-9}$ M for human Nav1.7.

The Huwentoxin-IV variant of SEQ ID NO: 276 may bind human Nav1.7 at residues F6, T28, W30, K32 and Y33. As long as these residues are kept invariant, other residues in the Huwentoxin-IV may be altered to improve properties, such as affinity and/or selectivity using methods described herein.

Another embodiment of the invention is an isolated fusion protein comprising the Huwentoxin-IV variant of SEQ ID NOs: 3-253 or 277-355 fused with a second polypeptide. Such second polypeptides may be leader or secretory signal sequences, partially or completely synthetic sequences resulting for example from cloning steps, or tags such as hexahistidine tag.

Additional moieties may be incorporated into the Huwentoxin-IV variants of the invention such as polyethylene glycol (PEG) molecules, such as PEG5000 or PEG20000, fatty acids and fatty acid esters of different chain lengths, for example laurate, myristate, stearate, arachidate, behenate, oleate, arachidonate, octanedioic acid, tetradecanedioic acid, octadecanedioic acid, docosanedioic acid, and the like, polylysine, octane, carbohydrates (dextran, cellulose, oligo- or polysaccharides) for desired properties. These moieties may be direct fusions with the Huwentoxin-IV variant polypeptides and may be generated by standard cloning and expression techniques. Alternatively, well known chemical coupling methods may be used to attach the moieties to recombinantly produced HwTx-IV variants of the invention.

Huwentoxin-IV variants incorporating additional moieties may be compared for functionality by several well known assays. For example, pharmacokinetic properties of Huwentoxin-IV variants coupled to PEG may be evaluated in well known *in vivo* models.

Another embodiment of the invention is an isolated Huwentoxin-IV variant comprising a polypeptide sequence of SEQ ID NOs: 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 277, 278, 279, 280, 281, 282, 283, 284, 285, 286, 287, 288, 289, 290, 291, 292, 293, 294, 295, 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354 or 355.

Another embodiment of the invention is an isolated polynucleotide comprising a polynucleotide encoding the Huwentoxin-IV variant polypeptide of the invention.

The polynucleotides of the invention may be produced by chemical synthesis such as solid phase polynucleotide synthesis on an automated polynucleotide synthesizer. Alternatively, the polynucleotides of the invention may be produced by other techniques such as PCR based duplication, vector based duplication, or restriction enzyme based DNA manipulation techniques. Techniques for producing or obtaining polynucleotides of a given known sequence are well known in the art.

The polynucleotides of the invention may also comprise at least one non-coding sequence, such as transcribed but not translated sequences, termination signals, ribosome binding sites, mRNA stabilizing sequences, introns and polyadenylation signals. The polynucleotide sequences may also comprise additional sequences encoding additional amino acids. These additional polynucleotide sequences may, for example, encode a marker or well known tag sequences such as a hexa-histidine or a HA tag which facilitate the purification of fused polypeptides. Certain exemplary polynucleotides are disclosed herein, however, other polynucleotides which, given the degeneracy of the genetic code or codon preferences in a given expression system, encode the antibody antagonists of the invention are also within the scope of the invention. Exemplary polynucleotides are polynucleotides comprising a sequence shown in SEQ ID NOs: 271, 272, 273, 274 and 275.

Another embodiment of the invention is a vector comprising an isolated polynucleotide encoding the Huwentoxin-IV variants of the invention. The vectors of the invention are useful for maintaining polynucleotides, duplicating polynucleotides, or driving expression of a polypeptide encoded by a vector of the invention in biological systems, including reconstituted biological systems. Vectors may be chromosomal-, episomal- and virus-derived such as vectors derived from bacterial plasmids, bacteriophages, transposons, yeast episomes, insertion elements, yeast chromosomal elements, baculoviruses, papova viruses such as SV40, vaccinia viruses, adenoviruses, fowl pox viruses, pseudorabies viruses, picornaviruses and retroviruses and vectors derived from combinations thereof, such as cosmids and phagemids.

In one embodiment of the invention the vector is an expression vector. Expression vectors typically comprise nucleic acid sequence elements that can control, regulate, cause or permit expression of a polypeptide encoded by such a vector. Such elements may comprise transcriptional enhancer binding sites, RNA polymerase initiation sites, ribosome binding sites, and other sites that facilitate the expression of encoded polypeptides in a given expression system. Such expression systems may be cell-based, or cell-free systems well known in the art. Nucleic acid sequence elements and parent vector sequences suitable for use in the expression of encoded polypeptides are also well known. An exemplary plasmid-derived expression vector useful for expression of the polypeptides of the invention comprises an *E. coli* origin of replication, an ampicillin resistance (Amp) gene, a CMV promoter, a signal sequence, and a SV40 polyadenlyation site.

Another embodiment of the invention is an isolated host cell comprising a vector of the invention. Exemplary host cells include Archaea cells; bacterial cells such as *Streptococci, Staphylococci, Enterococci, E. coli, Streptomyces,* cyanobacteria, *B. subtilis* and S. *aureus;* fungal cells such as *Kluveromyces, Saccharomyces,* Basidomycete, *Candida albicans* or *Aspergillus;* insect cells such as *Drosophila* S2 and *Spodoptera* Sf9; animal cells such as CHO, COS, HeLa, C127, 3T3, BHK, HEK293, CV-1, Bowes melanoma and myeloma; and plant cells, such as gymnosperm or angiosperm cells. The host cells in the methods of the invention may be provided as individual cells, or populations of cells. Populations of cells may comprise an isolated or cultured population of cells or cells present in a matrix such as a tissue.

Introduction of a polynucleotide, such as a vector, into a host cell can be effected by methods well known to those skilled in the art. These methods include calcium phosphate transfection, DEAE-Dextran mediated transfection, microinjection, cationic lipid-mediated transfection and electroporation.

Another embodiment of the invention is a method for expressing the Huwentoxin-IV variant of the invention comprising the steps of providing a host cell of the invention; and culturing the host cell under conditions sufficient for the expression of at least one Huwentoxin-IV variant of the invention.

Host cells can be cultured under any conditions suitable for maintaining or propagating a given type of host cell and sufficient for expressing a polypeptide. Culture conditions, media, and related methods sufficient for the expression of polypeptides are well known in the art. For example, many mammalian cell types can be aerobically cultured at 37°C using appropriately buffered DMEM media while bacterial, yeast and other cell types may be cultured at 37°C under appropriate atmospheric conditions in LB media.

In the methods of the invention the expression of the Huwentoxin-IV variant can be confirmed using a variety of well known methods. For example, expression of a polypeptide can be confirmed using detection reagents, such as antibodies using for example FACS or immunofluorescent techniques, or using SDS-PAGE or HPLC.

Another aspect of the invention is a method of modulating the activity of Nav1.7 in a biological tissue, the method comprising contacting a biological tissue expressing Nav1.7 with a Nav1.7 modulating amount of a Huwentoxin-IV variant of the invention, or a pharmaceutically acceptable salt thereof.

**Methods of Treatment**

Huwentoxin-IV variants of the invention may be utilized in any therapy where it is desired to treat, reduce or alleviate symptoms of pain or other disorders of sensory or sympathetic neuron dysfunction.

Pain treated with the Huwentoxin-IV variants of the invention may be any type of pain, such as chronic pain, acute pain, neuropathic pain, nociceptive pain, visceral pain, back pain, pain associated with inflammatory conditions, post-operative pain, thermal pain or pain associated with disease and degeneration.

Pain treated with the Huwentoxin-IV variants of the invention may be Nav1.7-mediated pain.

Nav1.7-mediated pain as used herein refers to pain resulting at least partially from increased Nav1.7 channel activity.

The methods of the invention may be used to treat an animal patient belonging to any classification. Examples of such animals include mammals such as humans, rodents, dogs, cats and farm animals.

The pain and/or Nav1.7-mediated pain may result from one or more causes, such as peripheral neuropathy, central neuropathy, nerve compression or entrapment syndromes such as carpal tunnel syndrome, tarsus tunnel syndrome, ulnar nerve entrapment, compression radiculopathy, lumbar spinal stenosis, sciatic nerve compression, spinal root compression, intercostal neuralgia, compression radiculopathy and radicular lower back pain, spinal root lesions, neuritis, automimmune diseases, general inflammation, chronic inflammatory conditions, arthritis, rheumatic diseases, lupus, osteoarthritis, general gastrointestinal disorders, colitis, gastric ulceration, duodenal ulcers, inflammatory bowel disorders, irritable bowel syndrome, pain associated with diarrhea, inflammatory eye disorders, inflammatory or unstable bladder disorders, psoriasis, skin complaints with inflammatory components, sunburn, carditis, dermatitis, myositis, neuritis, collagen vascular diseases, inflammatory pain and associated hyperalgesia and allodynia, neuropathic pain and associated hyperalgesia and allodynia, multiple sclerosis, demyelinating diseases, diabetes, diabetic neuropathy pain, causalgia, pain resulting from amputation or abscess, phantom limb pain, fracture pain, bone injury, direct trauma, HIV infection, acquired immune deficiency syndrome ("AIDS"), small pox infection, herpes infection, exposure to toxins or other foreign particles or molecules, invasive cancer, cancer, chemotherapy, radiotherapy, hormonal therapy, burns, congenital defect, dental pain, gout pain, fibromyalgias, encephalitis, chronic alcoholism, hypothyroidism, uremia and vitamin deficiencies, trigeminal neuralgia, stroke, thalamic pain syndrome, general headache, migraine, cluster headache, tension headache, mixed- vascular and non vascular syndromes, sympathetically maintained pain, deafferentation syndromes, asthma, epithelial tissue damage or dysfunction, disturbances of visceral motility at respiratory, genitourinary, gastrointestinal or vascular regions, wounds, burns, allergic skin reactions, pruritis, vasomotor or allergic rhinitis, or bronchial disorders, dysmenorrhoea, pain during labor and delivery, dyspepsia, gastroesophageal reflux, pancreatitis, and visceralgia.

Other disorders of sensory or sympathetic neuron dysfunction that may be alleviated by peptide Nav1.7 blockers include itch, cough and asthma. In mice, global deletion of the SCN9A gene leads to complete insensitivity to histamine-induced itch (Gingras et al., American Pain Society Meeting Abstract 2013 and U.S. Pat. Publ. No. 20120185956). This finding suggests that peptide Nav1.7 blockers may have utility in the treatment of itch, which may arise from various sources, such as dermatological or inflammatory disorders; or inflammatory disorders such as renal or hepatobiliary disorders, immunological disorders, medication reactions and unknown/idiopathic conditions, including dermatitis, psoriasis, eczema, insect sting or bite. Nav1.7 is also expressed in sensory nerves innervating the airways (Muroi et al., J Physiol. 2011 Dec 1;589(Pt 23):5663-76; Muroi et al., Am J Physiol Regul Integr Comp Physiol. 2013 Apr 10), suggesting that peptide Nav1.7 blockers may be beneficial in the treatment of cough e.g., acute or chronic cough, or cough caused by irritation from gastroesophageal reflux disease, and inflammatory diseases of the airways such as asthma and allergy-related immune responses, bronchospasm, chronic obstructive pulmonary disease, chronic bronchitis, emphysema, and hiccups (hiccoughs, singultus). Silencing Nav1.7 in vivo in nodose ganglia of guinea pigs using shRNA nearly abolished the cough reflex induced by mechanical probing (Muroi et al., Am J Physiol Regul Integr Comp Physiol. 2013 Apr 10).

One aspect of the invention is a method of alleviating or treating itch, cough or asthma in a subject by administering a therapeutically effective amount of the Huwentoxin-IV variant of the invention to a subject in need thereof for a time sufficient to alleviate the itch, cough or asthma.

Huwentoxin-IV variants of the invention can be tested for their effect in reducing or alleviating pain using animal models described herein, and models such as the SNL (spinal nerve ligation) rat model of neuropathic pain, carageenan induced allodynia model, the Freund's complete adjuvant (CFA)-induced allodynia model, the thermal injury model, the formalin model and the Bennett Model and other modes as described in U.S. Pat. Appl. No. 2011/0124711A1 and U.S. Pat. No. 7,998,980. Carageenan induced allodynia and (CFA)-induced allodynia are models of inflammatory pain. The Bennett model provides an animal model for chronic pain including post-operative pain, complex regional pain syndrome, and reflex sympathetic dystrophy.

Any of the foregoing animal models may be used to evaluate the efficacy of Huwentoxin-IV variants of the invention inhibitor in treating pain associated with the animal models. The efficacy can be compared to a no treatment or placebo control. Additionally or alternatively, efficacy can be evaluated in comparison to one or more known pain relieving medicaments.

The present invention provides methods of treating Nav1.7-mediated pain using peptide inhibitors of Nav1.7. The invention is based on the surprising finding that administration of Nav1.7 blocking peptides are efficacious in treating and/or alleviating pain in various animal models of pain, contrary to what is disclosed and suggested in the literature. While peptide inhibitors of Nav1.7 are potent and/or selective towards Nav1.7 in *in vitro* cell culture models using overexpressed Nav1.7 or on isolated neurons in which the blood-nerve barrier is subverted through desheathing or hypertonic saline injection, the peptide inhibitors have proven non-efficacious in *in vivo* animal models of pain, which lack of efficacy has been reported to result from inability of the peptides to pass the blood-nerve barrier. Several publications describe lack of efficacy of Nav1.7 blocking peptides in animal models of pain or in isolated nerves. For example Hackel et al., Proc Natl Acad Sci 109:E2018-27, 2012, describes the inability of ProTx-II to inhibit action potential firing in isolated nerves unless the perineural barrier, which provides a diffusion barrier in this model, is compromised. ProTx-II was found non-efficacious in rodent models of acute and inflammatory pain; a likely explanation stated the inability of ProTx-II to cross the blood-nerve barrier (Schmalhofer et al., Mol Pharmacol 74:1476-1484, 2008). It has been proposed that Nav1.7 peptide toxin blockers have poor oral bioavailability and they are difficult to deliver to nerve endings, implying that their use as therapeutic agents remain limited (Dib-Hajj et al., Nature Rev Neuroscience 14, 49-62, 2013).

$Na_v1.7$ is expressed in the peripheral nervous system i.e., in nociceptive dorsal root ganglions (DRG), most notably in nociceptive small-diameter DRG neurons, in particular in peripheral terminals in the skin, with little representation in the brain. $Na_v1.7$ distribution (e.g. sensory ending) and physiology predispose it to a major role in transmitting painful stimuli.

One embodiment of the invention is a method of alleviating Nav1.7-mediated pain by administering a therapeutically effective amount of a peptide inhibitor of Nav1.7 to a subject in need thereof for a time sufficient to alleviate the Nav1.7-mediated pain.

The peptide inhibitors of Nav1.7 may be utilized in any therapy where it is desired to alleviate symptoms of Nav1.7-mediated pain or other disorders of sensory or sympathetic neuron dysfunction. Alleviation of pain is meant to include complete reduction as well as partial reduction of pain sensations.

In one embodiment, pain alleviated with the peptide inhibitor of Nav1.7 may be any type of Nav1.7-mediated pain, such as chronic pain, acute pain, neuropathic pain, nociceptive pain, visceral pain, back pain, pain associated with inflammatory conditions, post-operative pain, thermal pain or pain associated with disease and degeneration.

Neuropathic pain includes for example painful diabetic neuropathy (PDN), post-herpetic neuropathy (PHN) or trigeminal neuralgia (TN). Other causes of neuropathic pain include spinal cord injuries, multiple sclerosis, phantom limb pain, post-stroke pain and HIV-associated pain. Conditions such as chronic back pain, osteoarthritis and cancer may also result in the generation of neuropathicrelated pain and thus are potentially suitable for treatment with the peptide inhibitors of Nav1.7.

The peptide inhibitors of Nav1.7 can be tested for their effect in reducing or alleviating pain using animal models such as those described herein.

Any of the foregoing animal models may be used to evaluate the efficacy of the peptide inhibitors of Nav1.7 in treating or reducing pain associated with the animal models. The efficacy can be compared to a no treatment or placebo control. Additionally or alternatively, efficacy can be evaluated in comparison to one or more known pain relieving medicaments.

In another embodiment, the Nav1.7-mediated pain is associated with primary erythemalgia (PE), paraoxysmal extreme pain disorder (PEPD), osteoarthritis, rheumatoid arthritis, lumbar discectomy, pancreatitis or fibromyalgia.

Peptide inhibitors of Nav1.7 include Protoxin-II (ProTx-II) (SEQ ID NO: 356) and Huwentoxin-IV (HwTx-IV) (SEQ ID NO: 1). Protoxin-II variants (ProTx-II variants) can be used in the methods of the invention as long as they block Nav1.7 activity and preferably have a selectivity towards Nav1.7 comparable to that of ProTx-II. Such variants are described for example in U.S. Pat. Publ. No. US2011/0065647, Int. Pat. Publ. No. WO2008/088422, and Int. Pat. Publ. No. WO2012/004664. Huwenotoxin-IV variants (HwTx-IV variants) can be used in the methods of the invention as long as they block Nav1.7 activity and preferably have selectivity towards Nav1.7 comparable to that of HwTx-IV. Such variants are described for example in U.S. Provisional Pat. Appl. Serial No. 61/702,538 and as described herein.

In the methods of the invention, the peptide inhibitors of Nav1.7 may be conjugated to a second polypeptide to form a fusion protein. Such fusion proteins are for example the well known Fc fusions or fusions to human serum albumin to extend half life of the peptide inhibitors. The conjugation can be a direct conjugation of via a linker, such as a glycine-serine rich linker. Such linkers are well known in the art.

In the methods of the invention, additional moieties may be incorporated into the peptide inhibitors of Nav1.7, such as polyethylene glycol (PEG) molecules, such as PEG5000 or PEG20000, fatty acids and fatty acid esters of different chain lengths, for example laurate, myristate, stearate, arachidate, behenate, oleate, arachidonate, octanedioic acid, tetradecanedioic acid, octadecanedioic acid, docosanedioic acid, and the like, polylysine, octane, carbohydrates (dextran, cellulose, oligo- or polysaccharides) for desired properties. These moieties may be direct fusions with the peptide inhibitors of Nav1.7 and are commercially available or may be generated by known chemical synthetic routes and known chemical coupling methods may be used to attach the moieties to the peptide inhibitors of Nav1.7

The peptide inhibitors of Nav1.7 incorporating additional moieties may be compared for their Nav1.7 blocking ability and efficacy in treatment or reducing pain using well known methods and those described herein.

Other disorders of sensory or sympathetic neuron dysfunction that can be treated with the peptide inhibitors of Nav1.7, including asthma, cough, heart-burn, itch, dermatitis, bladder instability, and Reynaud's disease.

**Pharmaceutical compositions**

The Huwentoxin-IV variants of the invention or other peptide inhibitors of Nav1.7 can be formulated in a pharmaceutically acceptable vehicle or carrier. A suitable vehicle or carrier may be water for injection, physiological saline solution or artificial cerebrospinal fluid, possibly supplemented with other materials common in compositions for parenteral administration. Neutral buffered saline or saline mixed with serum albumin are further exemplary vehicles. These solutions are sterile and generally free of particulate matter, and may be sterilized by conventional, well-known sterilization techniques (e.g., filtration). The compositions may contain pharmaceutically acceptable excipients as required to approximate physiological conditions, such as pH adjusting and buffering agents, stabilizing, thickening, lubricating and coloring agents, etc. Suitable vehicles and their formulation and packaging are described, for example, in Remington: The Science and Practice of Pharmacy (21st ed., Troy, D. ed., Lippincott Williams & Wilkins, Baltimore, MD (2005) Chapters 40 and 41).

In the methods of the invention, the Huwentoxin-IV variants or the invention or other peptide inhibitors of Nav1.7 may be administered by peripheral administration. "Peripheral administration" or "administered peripherally" means introducing an agent into a subject outside of the central nervous system. Peripheral administration encompasses any route of administration other than direct administration to the spine or brain.

Peripheral administration can be local or systemic. Local administration of the peptide inhibitors of Nav1.7 may be suitable for less selective Nav1.7 inhibitors, such as Muconotoxins, family 1(HwTx-like) and family 3 (ProTx-II like). Local administration may be used to concentrate the therapeutic to the site of action, such as local administration to joints, spinal cord, surgical wounds, sites of injury/trauma, peripheral nerve fibers, various organs (GI, urogenital, etc) or inflamed tissues. Systemic administration results in delivery of a pharmaceutical composition to essentially the entire peripheral nervous system of the subject and may also result in delivery to the central nervous system depending on the properties of the composition.

Routes of peripheral administration encompass, without limitation, topical administration, intravenous or other injection, and implanted mini-pumps or other extended release devices or formulations.

Pharmaceutical compositions of the invention include formulations involving Huwentoxin-IV variants or other peptide inhibitors of Nav1.7 in sustained- or controlleddelivery formulations. These formulations may be achieved through use of for example injectable microspheres, bioerodible particles, microemulsions, nanoparticles, nanocapsules, macroemulsions, polymeric compounds (such as polyesters, polyamino acids, hydrogels, poly(lactic acid), polyglycolic acid or ethylene vinylacetate copolymers), beads or liposomes, that may provide controlled or sustained release of the Huwentoxin-IV variants or other peptide inhibitors of Nav1.7 which can be delivered via depot injection, known to those skilled in the art. For example, hyaluronic acid or implantable drug delivery device may be used, having the effect of promoting sustained duration in the circulation.

Pharmaceutical compositions of the invention may be formulated for inhalation as a dry, inhalable powder. The inhalation solutions may also be formulated with a propellant for aerosol delivery, or a nebulizer.

Pharmaceutical compositions of the invention may be formulated for oral delivery. Huwentoxin IV variants or other peptide inhibitors of Nav1.7 that that are administered in this fashion may be formulated with or without carriers customarily used in the compounding of solid dosage forms such as tablets and capsules. A capsule may be designed to release the active portion of the formulation at the point in the gastrointestinal tract when bioavailability is maximized and pre-systemic degradation is minimized. Additional agents can be included to facilitate absorption of the Huwentoxin-IV variants. Diluents, flavorings, low melting point waxes, vegetable oils, lubricants, suspending agents, tablet disintegrating agents, and binders may also be employed. A pharmaceutical composition of the invention is preferably provided to comprise an effective quantity of one or a plurality of Huwentoxin-IV variants in a mixture with non toxic excipients that are suitable for the manufacture of tablets. By dissolving the tablets in sterile water, or another appropriate vehicle, solutions may be prepared in unit- dose form. Suitable excipients include, but are not limited to, inert diluents, such as calcium carbonate, sodium carbonate or bicarbonate, lactose, or calcium phosphate; or binding agents, such as starch, gelatin, or acacia; or lubricating agents such as magnesium stearate, stearic acid, or talc.

The Huwentoxin-IV variants of the invention or other peptide inhibitors of Nav1.7 may be prepared for use for parenteral (subcutaneous, intramuscular or intravenous), intracerebral (intra-parenchymal), intracerebroventricular, intramuscular, intra-ocular, intra-arterial, intraportal, or intralesional routes; by sustained release systems or by implantation devices, or any other administration, particularly in the form of liquid solutions or suspensions; for buccal or sublingual administration such as in the form of tablets or capsules; or intranasally such as in form of powders, nasal drops or aerosols or certain agents; transdermally in a form of a gel, ointment, lotion, cream or dusting powder, suspension or patch delivery system with chemical enhancers to either modify the skin structure or to increase the drug concentration in the transdermal patch, or with agents that enable the application of formulations containing proteins and peptides onto the skin (WO98/53847), or applications of electric fields to create transient transport pathways such as electroporation, or to increase the mobility of charged drugs through the skin such as iontophoresis, or application of ultrasound such as sonophoresis (U.S. Pat. Nos. 4,309,989 and 4,767,402). The composition also may be administered locally via implantation of a membrane, sponge or another appropriate material onto which the desired molecule has been absorbed or encapsulated.

In certain embodiments, where an implantation device is used, the device may be implanted into any suitable tissue or organ, and delivery of the desired molecule may be via diffusion, timed-release bolus, or continuous administration.

The concentration of the Huwentoxin-IV variants of the invention or other peptide inhibitors of Nav1.7 in such pharmaceutical formulation can vary widely, i.e., from less than about 0.5%, usually at or at least about 1% to as much as 15%, 20%, 30%, 40%, 50%, 60% or 70% by weight and will be selected primarily based on fluid volumes, viscosities and other factors, according to the particular mode of administration selected. The Huwentoxin-IV variants of the invention or other peptide inhibitors of Nav1.7 can be lyophilized for storage and reconstituted in a suitable vehicle prior to use. This technique has been shown to be effective with conventional protein preparations. Lyophilization and reconstitution techniques are well known in the art.

An exemplary pharmaceutical compositions of the present invention may comprise Tris buffer of about pH 7.0-8.5, or acetate buffer of about pH 4.0-5.5, and may further include sorbitol, sucrose, Tween-20 and/or a suitable substitute thereof.

The appropriate therapeutically effective dose can be determined readily by those of skill in the art. Effective dose refers to an amount or dosage sufficient to produce a desired result, i.e. to partially or completely prevent, stop, inhibit, reduce, or delay the perception of pain associated with any painful medical condition. The effective amount may vary depending on the specific vehicle and Huwentoxin-IV variant or other peptide inhibitors of Nav1.7 selected, and is also dependent on a variety of factors and conditions related to the subject to be treated and the severity of the pain. For example, factors such as the age, weight and health of the subject to be administered with the pharmaceutical compositions of the invention as well as dose response curves and toxicity data obtained in preclinical animal work would be among those considered. A determined dose may, if necessary, be repeated at appropriate time intervals selected as appropriate by a physician or other person skilled in the relevant art (e.g. nurse, veterinarian, or veterinary technician) during the treatment period. The determination of an effective amount or a therapeutically effective amount for a given agent is well within the ability of those skilled in the art.

Thus, a pharmaceutical composition of the invention for intramuscular injection could be prepared to contain 1 ml sterile buffered water, and between about 1 ng to about 100 mg, about 50 ng to about 30 mg or about 5 mg to about 25 mg of a Huwentoxin-IV variant of the invention. Similarly, a pharmaceutical composition of the invention for intravenous infusion could be made up to contain about 250 ml of sterile Ringer's solution, and about 1 mg to about 30 mg or about 5 mg to about 25 mg of a Huwentoxin-IV variant of the invention or other peptide inhibitors of Nav1.7. Actual methods for preparing parenterally administrable compositions are well known and are described in more detail in, for example, "Remington's Pharmaceutical Science", 15th ed., Mack Publishing Company, Easton, PA.

The present invention will now be described with reference to the following specific, non-limiting examples.

## Example 1

### Design and generation of Huwentoxin-IV variants

Single position amino acid scanning library substituting Ala, Asp, Glu, Phe, Gly, His, Ile, Lys, Leu, Asn, Pro, Gln, Arg, Ser, Thr, Val, Trp, and Tyr at every non-cysteine residue within the wild type Huwentoxin-IV (ECLEIFKACNPSNDQCCKSSKLVCSRKTRWCKYQI; SEQ ID NO: 1) derived from the venom of the Chinese bird spider, *Ornithoctonus huwena* was generated. The Huwentoxin-IV variants were encoded as HRV3C protease cleavable human serum albumin (HSA) fusion proteins in the following format from N- to C-terminus: $His_6$-HSA-$(GGGGS)_4$-HRV3C cleavage site-Huwentoxin-IV variant. Every variant peptide, following cleavage from HSA had a residual N-terminal GP from the cleavage site as well as a C-terminal GK which is the endogenous amidation recognition sequence. The single position variants were tested in fluorescence-based screening assays measuring their ability to inhibit Veratridine-induced membrane potential and hits were confirmed in Qpatch electrophysiology. The C-terminal GK residues in the recombinantly expressed cleaved Huwentoxin-IV variants were also substituted.

Combinatorial libraries were designed to test for additive effects of select single position hits in an attempt to generate Nav1.7 antagonists with further improved potency and selectivity profile compared to the native peptide. Two combinatorial libraries were produced, one that combined E1N, E4R, R26K, Y33W, Q34S, and G36I (library NV1D7L5), the other combined N13Q, S19Q, V23R, K27Y, R29K, and K37R (library NV1D7L6).

### Construction of expression vectors

cDNAs encoding the designed Huwentoxin-IV variant polypeptides were generated using a gene assembly technology described in U.S. Pat. No. 6,521,427. Briefly, the amino acid sequences of the designed peptide variants were backtranslated to DNA sequences using human-high frequency codons. The DNA sequence of each variant gene, together with a portion of vector DNA including the DNA cloning sites, was synthesized as multiple oligonucleotides, some of which contained degenerate codons, and assembled into full-length DNA fragments. The assembled DNA fragments were amplified by PCR and PCR products were subsequently cloned as a pool. Pooled PCR products were digested with the appropriate restriction enzymes and cloned into the designed expression vector in such as manner as to fuse each toxin variant gene to the signal peptide and the fusion partner contained in the vector. Standard molecular biology techniques were used to identify a positive clone for each designed variant. The plasmid DNA from these positive clones was purified and sequence confirmed before expressing each Huwentoxin-IV peptide variant.

### Protein expression

HEK293F cells maintained in 293 Freestyle™ media (Invitrogen) were transiently transfected with plasmids encoding Huwentoxin-IV variants using Freestyle™ transfection reagent (Invitrogen) according to standard protocols. Transfected cells were placed in a humidified incubator set at 37°C and 8% $CO_2$ for 4 days shaking at 125 RPM. The supernatant was separated from the cells by centrifugation at 5,000g for 10 minutes and filtered through a 0.2μm filter and concentrated 10 and 50 fold using an Amicon Ultra Concentrator 10K (Cat #UFC901096), and centrifuging for approximately 10 minutes at 3,750g.

### Protein purification

The secreted Huwentoxin-IV variant proteins were purified via IMAC using 1ml HisTrap HP columns (GE Healthcare). The chromatography method was run using an AKTA Xpress and protein was eluted from the column using a step gradient of Imidazole. Peak fractions were pooled and digested overnight with HRV3C protease (EMD cat# 71493; 1 unit/100 μg protein). Cleaved peptide was purified via RP-HPLC using a C18(2) column (Phenomenex, cat# 00G-4252-N0). The chromatography method was run on a Dionex HPLC system and the bound peptide was eluted using a linear gradient of acetonitrile. Peak fractions were collected, pooled and lyophilized.

Lyophilized peptides were re-suspended in HEPES buffered saline, pH7.4 (10mM HEPES, 137mM NaCl, 5.4mM KCl, 5mM glucose, 2mM $CaCl_2$, 1mM $MgCl_2$). Absorbance was measured at 280 nm, and concentrations calculated using each peptide's extinction coefficient. Peptides were analyzed by nonreducing SDS-PAGE.

For scale-up, proteins were purified in IMAC using 5ml HisTrap HP columns (GE Healthcare, cat#17-5248-02). The chromatography method was run using an AKTA Explorer or FPLC and protein was eluted from the column using a step gradient of Imidazole. Peak fractions were pooled and concentrated using Amicon Ultra-15 centrifugal concentrators (Millipore, cat# UFC901096) and dialyzed overnight against 2 changes of Dulbecco's phosphate buffered saline, pH7.2 (Invitrogen, cat#14190). The fusion was then digested overnight with HRV3C (EMD cat# 71493; 1 unit/100 μg protein). The cleaved fusion was purified by IMAC using 5ml HisTrap HP columns. The peptide was collected in the flow through fraction. Pooled peptide was concentrated and polished via RP-HPLC using a C18(2) column (Phenomenex, cat# 00G-4252-N0). The chromatography method was run on an Agilent 1100 HPLC system and the bound peptide was eluted using a linear gradient of acetonitrile.

Each peak fraction was analyzed by RP-HPLC on an analytical C18(2) column (Phenomenex, cat#00G-4252-E0) using an acetonitrile linear gradient. Fractions with the same retention times were pooled and lyophilized. Lyophilized peptides were re-suspended in HEPES buffered saline, pH7.4 (10mM HEPES, 137mM NaCl, 5.4mM KCl, 5mM glucose, 2mM $CaCl_2$, 1mM $MgCl_2$). Absorbance was measured at 280 nm, and concentrations calculated using each peptide's extinction coefficient. Final peptides were analyzed by electrospray ionization mass spectrometry on a Waters system.

## Example 2. Characterization of Huwentoxin-IV variants

### Membrane depolarization assays

Ability of the generated Huwentoxin-IV variants to inhibit membrane depolarization induced by Nav1.7 agonist veratridine (3-Veratroylveracevine; Biomol, Catalog# NA125) was measured using FRET assay (fluorescence resonance energy transfer) on FLIPR® Tetra using DISBAC2(3) (Invitrogen, K1018) as an electron acceptor and PTS18 (Trisodium 8-octadecyloxypyrene-1,3,6-trisulfonate) (Sigma) as a donor by exciting the donor at 390-420 nm and measuring FRET at 515-575 nm.

HEK293F cells stably expressing the hNav1.7 channel under G418 selection (Invitrogen) were cultured in DMEM/F12 supplemented with glutamine, 10% FBS, 1% NEAAs, and 400 μg/ml G-418. 50 μl of harvested cells were plated at 25,000 cells/well into poly-lysine coated 384-well black clear bottom plates. The plates were incubated at room temperature (RT) for 15 min followed by an overnight incubation at 37°C. All incubations were done in the dark unless otherwise stated. The next day, the wells were washed 4 times with assay buffer, and resuspended in 25 μl of assay buffer (137 mM NaCl, 4mM KCl, 2mM $MgCl_2$, 2mM $CaCl_2$, 5 mM Glucose, 10 mM HEPES ). 2x stock (6 μM) of the PTS18 dye was prepared by suspending the dye in 10% pluronic F127 in DMSO at 1:1 (v/v ratio). 25 μl of the 2x PTS18 stock was added into the wells and the cells were stained for 30 min at RT, after which the dye was washed off with the assay buffer.

Huwentoxin-IV peptides were suspended at 3x their final concentration in the assay buffer containing 10 μM DISBAC2(3) and 400 μM VABSC-1 to suppress background fluorescence (Sigma, cat# 201987). 25 μl/well of the suspended Huwentoxin-IV peptides were added onto each well, and incubated for 60 minutes at RT. Depolarization was induced by 25 μM final concentration of veratridine (by adding 25 μl/well of 75mM (3x) stock solution), and the reduction in the mean intensity of FRET dye fluorescence was measured 30 seconds after adding the agonist. A 1.3X dilution of each measured Huwentoxin-IV peptide occurred after adding veratridine by convention, the concentration at the beginning of the FLIPR® Tetra assay is reported. Tetracaine, TTX, Protoxin-II and Huwentoxin-IV are established sodium channel blockers and were used as controls in each experimental series.

Fluorescence counts for each well were converted to % inhibition by normalizing the signal to the negative control (response to agonist veratridine alone) and positive control (response to veratridine in the presence of 10 μM tetracaine)

For measurements, "spatial uniformity correction" (all fluorescence traces are normalized to the average initial starting intensity) and "subtract bias value" (subtract the initial starting intensity from each trace) are turned on in FLIPR® Tetra.

For screening mode, no averaging was performed and each uploaded data point represents the response in an individual well.

For concentration-response mode, all individual data points were used in a non-linear least-squares procedure to find the best fit to a Hill function using Origin software (Microcal). $IC_{50}$ values were extrapolated from the resultant fitted curve.

The mean and standard deviations of the positive (P±dP) and negative (N±dN) controls were used to calculate the amount of block (B) in a well with a response (R) as follows:

$$B = 100\%\left(\frac{N-R}{N-P}\right)$$

The screening window (a measure of the data quality) is defined as:

$$z' = 1 - 3\left|\frac{\delta N + \delta P}{N - P}\right|$$

Assay plates were accepted if (1) the screening window based on the controls was z'>0.5, and (2) the potency of control antagonists for that day were within ±0.5 log units of their historical mean.

Selectivity of Huwentoxin-IV variants were assessed by ability of the variants to inhibit NaV1.2-induced membrane depolarization using HEK293F cells stably expressing the hNav1.2 channel under G418 selection (Invitrogen cat #11330) as described for Nav1.7, except that depolarization was induced by about 8.35 μM final concentration of veratridine (by adding 25 μl/well of 25 μM (3x) stock solution). Selectivity was measured as a ratio of $IC_{50}$(Nav1.2)/$IC_{50}$(Nav1.7).

### QPatch Assay

HEK293 cells stably expressing human Nav1.7 were cultured in DMEM/F-12 media (1:1), supplemented with 10% fetal bovine serum, 400 μg/mL Geneticin and 100 μM NEAAs (all reagents from Invitrogen). The cells were maintained at 37°C and in 5% $CO_2$ and assayed upon reaching ~70-90% confluency. Before testing in QPatch (Sophion), cells were first dissociated using 0.05% trypsin (5 min at 37°C), resuspended in CHO-S-SFM media (Life Technologies) and gently triturated to break up cell clumps. Cell density was adjusted to $1\text{-}2\times10^6$/mL with the same media and cells were transferred to a cell "hotel" in QPatch HT and used in experiments for several hours.

For giga-ohm seal formation and whole-cell patch clamp recording, the extracellular solution contained 137 mM NaCl, 5.4 mM KCl, 1 mM $MgCl_2$, 2 mM $CaCl_2$, 5 mM glucose, and 10 mM HEPES, pH = 7.4 and osmolarity = 315 mOsm. The intracellular solution contained 135 mM CsF, 10 mM CsCl, 5 mM EGTA, 5 mM NaCl and 10 mM HEPES, pH = 7.3 and osmolarity = 290 mOsm.

The voltage protocol used in the assay was as follows. From a holding potential of -75 mV, cells were first hyperpolarized to -120 mV for 2 sec and then depolarized to 0 mV for 5 ms before returning to the holding potential (-75 mV). This protocol was repeated once every 60 sec during liquid applications (see below). Cells were otherwise held at -75 mV when the above voltage protocol was not executed.

Upon establishment of the whole-cell recording configuration, a total of five applications of the extracellular solution (all containing 0.1% bovine serum albumin (BSA) with or without test compound, except for the last application, which contained 1 μM TTX without BSA) were made on to cells being recorded. The first application contained only the control buffer (5 μl). The voltage protocol was executed 10 times (for a total duration of 10 min) five sec after the application. The next three applications (5 μl each) contained a test compound (same compound at the same concentration for all three applications) or control buffer (for control cells only). Five seconds after each of these applications, the voltage protocol was again executed 10 times (also once per min). The last application contained 1 μM TTX (composed of three 10 μl sub-applications, each separated by 2 sec), five seconds after which the same voltage protocol was executed twice to obtain the baseline current.

Currents were sampled at 25 kHz and filtered at 5 kHz with an 8-pole Bessle filter. The series resistance compensation level was set at 80%. For each cell, the peak current amplitude at 0 mV for each current trace in the first four liquid applications was first subtracted from that of the last trace in the presence of TTX and then normalized to that of the last trace in the first (control buffer) application as % inhibition. To control for current rundown, this (% inhibition) value for each cell in the presence of a test compound was further normalized to the average % inhibition value for control (typically 5-6) cells in the same experiment. The mean value of the last two such values in the last compound application (i.e., the corrected % inhibition value for each concentration of a test compound) was used in concentration response calculations. All experiments were performed at room temperature (~22 °C). Data are expressed as mean±se.

For reference compounds, results obtained from QPatch using this protocol, e.g., potency/kinetics, were in good accord with that from manual patch clamp.

### Results

Library matrix for single substitution variants and their $IC_{50}$ values for Nav1.7 obtained using the depolarization assay on FLIPR® Tetra is shown in Figure 1. Library matrix for single substitution variants and the $IC_{50}$ for Nav1.2 obtained using the depolarization assay on FLIPR® Tetra is shown in Figure 2. Selectivity measured as a ratio of the obtained $IC_{50}$ for Nav1.2 to the obtained $IC_{50}$ for Nav1.7 of the single substitution variants is shown in Figure 3. Figures 4 and 5 show sequences of the variants ranked by potency on Nav1.7 (Figure 4) or selectivity (Figure 5).

Select variants were tested in whole cell patch clamp experiments. The recombinant Huwentoxin-IV and Huwentoxin-IV variants were tested against Nav1.7 and Nav1.2 stably expressed in HEK293 cells using the QPatch assay

described above. The $IC_{50}$ values obtained for each huwentoxin-IV variant for Nav1.7 and Nav1.2 using the whole cell patch clamp methods is shown in Figure 6. Selectivity of the Huwentoxin-IV variants was calculated as above using the $IC_{50}$ values obtained from the whole cell patch-clamp experiments.

Using Huwentoxin-IV as a starting point single-position amino acid scanning library was designed to identify variants with improved potency or selectivity. Select single position variants with interesting properties were included into combinatorial libraries. Single-position variants that were used in the design of the combinatorial libraries included E1N, E4R, R26K, Y33W, Q34S, G36I, N13Q, S19Q, V23R, K27Y, R29K, and K37R (residue numbering according to SEQ ID NO: 267), all of which showed improvements in potency, selectivity or both. Additional single-position variants with improved properties include R26W (SEQ ID NO: 72), K27W (SEQ ID NO: 57), Q34F (SEQ ID NO: 6) and R29W (SEQ ID NO: 55). In addition, variants (E1N,E4R,R26K,Q34S) (SEQ ID NO: 5), (E1N,E4R,R26K,Q34S,G36I) (SEQ ID NO: 16), (E4R,R26K,Y33W,G36I) (SEQ ID NO: 48), (E1N,Y33W,Q34S,G36I) (SEQ ID NO: 83), (N13Q,R29K,K37R) (SEQ ID NO: 137), (E1N,R26K,Q34S,G36I) (SEQ ID NO: 192) and (R26K,Y33W) (SEQ ID NO: 46) identified from combinatorial libraries demonstrate improved potency and/or selectivity.

## Example 3. Analgesic activity of Huwentoxin-IV following

## intraplantar administration in rats

### Methods

Male Sprague-Dawley (CD) rats (Charles River, San Diego) weighing >300 grams were used in this study. Naive animals were trained for two days prior to the day of testing (in order to reduce the variability in responses). Training consisted of performing actual tests multiple times on each animal over a duration of ~1 hour for each rat. Animals first received a mark with a Sharpie in the center of the dorsal aspect of the left paw just proximal to the toes to enable consistently testing the same site of the paw. Rats were then loosely wrapped in a towel leaving the hind paws uncovered, the left hind paw was placed in the Randall Selitto device with the maximum threshold set at 500 grams (Ugo-Basile Randall-Selitto Device, Analgesy-Meter) with the Sharpie mark just beneath the point of the cone on the test device that comes in contact with the paw and pressure was increased at a steady rate via electronic ramp with foot control until the animal responded. A 'response' for training followed the same criteria as that on the day of testing and consisted of any one of the following: 1) removal of the hind paw from the device, 2) a clear attempt at removal or 3) vocalization. Rats were tested up to 3 times consecutively unless they responded to a threshold greater than or equal to 100 grams. Over the course of the hour/day of training, 1-3 consecutive tests were made for each rat with 5-20 minutes apart.

For compound testing, trained, un-injured rats were tested once for the pre-compound thresholds. Animals were assigned into peptide- or vehicle-treated groups in such a manner as to produce comparable pre-administration threshold means. Experiments were conducted blind to treatment groups where possible. One test for each time point following injection (5, 10, 20, 30 45, 60 min) was taken and recorded.

### Material Preparation and Local Administration in Hindpaw

Amidated Huwentoxin IV (Peptides International, Louisville, KY) was received in lyophilized form and reconstituted with HEPES-buffered saline, aliquoted and frozen at -20°C. Just prior to administration in the left dorsal hind paw, aliquots were thawed and diluted to appropriate concentrations using HEPES-buffered saline as the diluent. Because stress related to handling and paw injections may itself produce an increase in paw pressure threshold (stress-induced analgesia), rats were briefly anesthetized with isoflurane for the injection (5% induction; 2-3% maintenance). Animals were injected s.c. (100 μL of peptide solution or vehicle) in the dorsal aspect of the paw with the needle inserted left of center toward the ankle such that the tip of the needle ended just underneath the Sharpie mark in the center of the dorsal paw proximal to the toes.

### Data Analysis

Gram thresholds were recorded and entered into Prism 5.01 (Graphpad Software Inc., LaJolla, CA) for graphing, generating area under the curve (AUC) values and statistical analysis. For comparison of gram values over time, a two-way ANOVA was used with a significance level of $p<0.05$. For generation of mean AUC values, the AUC for each rat in the peptide group was individually obtained and the mean AUC of the vehicle group was subtracted from it. The vehicle subtracted AUCs for each peptide-treated animal were averaged and compared either by Student's T-test or one-way ANOVA, each with a significance level of $p<0.05$.

## Results

Huwentoxin-IV administered locally into the dorsal aspect of the paw produced a dose-dependent increase in paw pressure threshold in the Randall-Selitto test. Three and 30 nmoles Huwentoxin IV, but not 0.3 nmoles, increased thresholds significantly above those observed for vehicle-treated animals (Figure 7). AUCs were significantly different between all 3 peptide treated groups (with mean vehicle AUC subtracted from the AUC for each animal) (Figure 8). Some local edema was also noted following administration of each dose of Huwentoxin-IV. Similar edema was not noted in the vehicle injected rats.

## Example 4. Molecular modeling of Huwentoxin-IV interaction

## with Nav1.7

### NMR Structure Determination

All NMR experiments were performed using Bruker Avance 600, 700, or 950 MHz spectrometers. The peptides were dissolved in aqueous buffer containing 10% $D_2O$. The buffer maintained a pH of 6.7 using 20 mM phosphate, 0.1mM dEDTA, and 0.002% $NaN_3$. All spectra were collected at 298 K, unless otherwise stated. Individual residue spin systems were assigned using TOCSY (Bax and Davis, Mag. Reson. 1985, 65, 355-360) spectra using spin-lock (MLEV) with mixing times of 75 ms. Sequential residue assignments were made from NOESY (Jeener et al., J. Chem. Phys. 1979, 71, 4546-4553; Kumar et al., Biochem. Biophys. Res. Commun. 1980, 95, 1-6) experiments collected with a mixing time of 150 ms. In addition, $^{15}$N-HSQC (Bodenhausen et al., Chem. Phys. Lett. 1980, 69, 185-189) experiments aided assignment, and Cysteine oxidation states were elucidated via $^{13}$C-HSQC spectra using routine methods (Cavanagh et al., Protein NMR Spectroscopy: Principles and Practice 1995 Academic Press). Shifted sinebell squared weighting and zero filling was applied before Fourier transformation using NMRPipe (Delaglio et al., J. Biomol. NMR 6, 277-293, 1995) during data processing. Interproton distance restraints were derived from throughspace interactions observed in the NOESY spectra, and automatically assigned by CYANA (Guntert et al., J. Mol. Biol. 273, 283-298, 1997). In addition, peptides containing W32 that showed significant (>0.2 ppm) ring current anisotropy on neighboring amino acids have aromatic side-chain restraints applied. The applications PREDITOR (Berjanskii et al., Nuc. Acid. Res. 2006, 34, W63-W69) and DANGLE (Cheung et al., J. Mag. Reson. 202, 223-233, 2010) were used to predict phi and psi angle ranges based on chemical shift data. Backbone omega angle restraints were set to 180°. Based on data derived from the NOESY and $^{13}$C-HSQC experiments disulfide bonds were fixed between, C9-C24, C2-C17, and C16-C31.

Homology models of the peptides were used as input (Cycle 1) to CYANA followed by six cycles of combined automated NOESY assignment and structure calculation. During each cycle 1000 conformers were calculated using a standard simulated annealing schedule with 10000 torsion angle dynamics steps per conformer followed by 50000 steps of energy minimization. Ensembles of 20 conformers with the lowest target function values were then used as input into an explicit water, distance restrained minimization refinement routine using MOE (Chemical Computing Group Inc., www://_chemcomp_com).

### Molecular dynamics

An NMR structure of native HwTx-IV (structure at Protein Data Bank http://_www_rcsb_org/pdb/home/home_do; pdb 1MB6) was used as the starting point to characterize the stability of HwTx-IV using molecular dynamics simulations. In addition to simulations of the native HwTx-IV, simulations were performed to discern the importance of each of the three disulfide bonds and to determine the changes in peptide stability due to single alanine point mutations. To characterize the importance of the three disulfide bonds, separate molecular dynamics simulations (total of 7 simulations) were performed with the C2-C17, C9-C24, C16-C31, C2-C17/C9-C24, C2-C17/C16-C31, C9-C24/C16-C31 and C2-C17/C9-C24/C16-C31 cysteines converted into individual cysteine residues. To determine the effects of a single alanine point mutation an *in silico* molecular dynamics alanine scan (of all non-cysteine positions) was performed (total of 28 simulations).

For each molecular dynamics simulation, the HwTx-IV was solvated in explicit water (with a minimum of 12 Å padding) and neutralized to 0.1M NaCl. The protein was minimized and equilibrated for 50 ns using NAMD 2.8 [James et al., Journal of Computational Chemistry, 26:1781-1802, 2005). CHARMM 22 CMAP [MacKerell, Jr. et al., J Comput Chem 25: 1400-1415, 2004) parameters were used for the simulations with a multiple time stepping algorithm for evaluating electrostatics with bonded interactions computed every 1 fs, short-range non-bonded interactions computed every 2 fs, and long-range interactions computed every 4 fs. Long range electrostatic forces were evaluated using the particle mesh Ewald summation method with a grid spacing of less than 1 Å. Temperature was maintained at 300K using Langevin dynamics and a constant pressure of 1 atm was maintained using a Nose-Hoover Langevin piston. Periodic boundary conditions were assumed and non-bonded interactions were calculated using scaled 1-4 exclusion with shifting starting at 8 Å and a complete cut-off at 12 Å. Following simulation, the molecular dynamics trajectories were aligned based on the backbone C-alpha (CA) atoms and the root mean square deviation (RMSD) per residue calculated over the entire simulation relative to the initial NMR structure using Visual Molecular Dynamics (VMD) (Humphrey et al., J. Molec. Graphics, 1996, vol. 14, pp. 33-38).

Homology modeling of Nav1.7 and docking of HwTx-IV

A homology model of Nav1.7 Domain 2 (DII) segments S1-S4 was built with the structure NavAb (voltage-gated Na(+) channel from Arcobacter butzleri; structure at Protein Data Bank http://_www_rcsb_org/pdb/home/home_do; pdb 3RVY) as a template using the Modeller component in Discovery Studio 3.1 (Accelrys). The model was then further refined to generate a resting state Nav1.7 structure. S4 was manually moved down into a resting state configuration, the S1-S2 and S3-S4 loops were regenerated and the entire model was energy minimized. Native HwTx-IV was manually docked into the Nav1.7 homology model based on the results of the alanine scan of HwTx-IV inhibition against Nav1.7 and on published Nav1.7 mutations that effect HwTx-IV binding (Xiao et al., J Biol Chem. 286:27301-10, 2011. Epub 2011 Jun 9.).

Following the manual docking, the entire Nav1.7 DII S1-S4 with docked HwTx-IV system was minimized and an implicit membrane molecular dynamics simulation performed using the CHARMm forcefield with Generalized Born Implicit Membrane (Discovery Studio (Spassov et al., J. Phys. Chem. B, 106, 8726-8738, 2002) to further refine the docked structure.

**Results**

**Molecular dynamic simulations**

A series of molecular dynamics simulations were conducted to help understand the molecular basis for changes in the activity of the HwTx-IV mutants that lead to significant loss of activity (F6A, P11A, D14A, L22A, S25A, W30A, K32A, Y33A) or channel selectivity (K18A, R26A and K27A) based on structural changes of the toxins alone. The previously generated NMR structure for HwTx-IV (pdb code 1MB6) was used as a template for building the various alanine mutant peptides and each toxin variant was subjected to 50 ns of molecular dynamics simulations.

The average CA RMSD of native HwTx-IV peptide was only 1.007 Å indicating a highly stable peptide. Molecular dynamics simulations revealed that only W30A (Figure 9g), F6A (Figure 9b)(which normally form a pi-pi interaction) and L22A (Figure 9e)could influence the core stability of HwTx-IV. All other loss of function mutants exerted little to no effect on core stability. On the contrary, all loss of function mutants, as well as the mutants that differentially affected Nav1.7 and Nav1.2 activity were able to influence the flexibility of the loop regions. For example, W30A (Figure 9g), F6A (Figure 9b) and L22A (Figure 9e), increased the flexibility of loops 3 and 4, K32A (Figure 9h) increased loop 3 flexibility and D14A (Figure 9d) and P11A (Figure 9c) showed a pronounced increase in loop 2 flexibility. K27A (Figure 10c) and R26A (Figure 10b) were found to increase loop 4 flexibility. K18A (Figure 10a) and S25A (Figure 9f) mutations did not impact the flexibility of any loops.

**NMR**

To gain additional insight into the structural features of HwTX-IV and to directly test some of the main predictions of the molecular dynamic simulations we determined the NMR structure of recombinant WT HwTX-IV and compared it to the structure of W30A and K32A.

Despite the complete loss of activity measured in the QPatch and binding assays, but largely in keeping with the molecular dynamic simulations, W30A and K32A exhibit a similar global structure to WT recombinant HwTX-IV. Although interproton NOESY's and backbone chemical shift values indicate W30A, K32A, and the wild type peptides have very similar folds and structure, local differences are apparent near the solvent exposed face of the twisted beta-sheet. These differences include the observation of strong ring current anisotropy within a 5 angstrom radius of W30 in the K32A and wild type peptides. This anisotropy, most notably affecting F6 and T28, is indicative of a close spatial interaction that may affect the conformation/dynamics of the β-turn as well as the orientations of the side-chains. The solution structures imply another local difference, based on side-chain geometry, a potential cation-π interaction between the protonated amine of K32 and the π electrons of Y33, available to the W30A and wild type peptides. The side-chains of the five residues involved with these local differences, F6, T28, W30, K32, Y33 all reside in close proximity to each other lending to the aforementioned intra-molecular interactions as well as to form a potential 'pharmacophore' for inter-molecular interactions with Nav1.7.

## Homology modeling and docking

In order to explore the specific interactions made between HwTx-IV and the Nav1.7 channel, a homology model of Nav1.7 domain II (DII) voltage sensor domain (VSD; segments S1-S4) was constructed using NavAB as a template. The model was further refined to produce a resting state structure into which to manually dock the HwTx-IV peptide using available SAR data along with published channel mutation data (Xiao et al., Biol Chem. 286:27301-10, 2011. Epub 2011 Jun 9.).

The published channel mutation data suggested that HwTx-IV binds in the DII voltage sensor domain with interactions with the S1-S2 and S3-S4 loops (specifically with residues E753, E811, D816, and E818). The resulting docked structure is presented in Figure 12, with the hydrophobic patch comprised of W30 and F6 along with the basic K32 residue oriented in the groove formed by Nav1.7 S1-S2 and S3-S4 loops. The docked model places the W30 and F6 hydrophobic patch interacting with the channel groove with corresponding hydrophobic residue M750. While charged interactions along the edge of the S1-S2 loop and S3-S4 loop allow the HwTx-IV to orient itself in the binding site. Specifically on S1-S2 loop, charge-charge interactions are made between K7-E753 and E4-K762 of the HwTx-IV and the Nav1.7 channel respectively. Likewise, a series of charge-charge interactions between the HwTx-IV and the S3-S4 Nav1.7 loop also occur, R26-D816, K27-818, and K32-E811.

## Example 5

## Design and generation of additional Huwentoxin-IV variants

Two grafting libraries were generated based on the obtained Huwentoxin-IV variants NV1G387 (E1N, R26K, Q34S, G36I; NV1D2168, SEQ ID NO: 192) and NV1G327 (E1N, E4R, Y33W, Q34S; NV1D2163, SEQ ID NO: 3).

Peptides were recombinantly expressed as described in Example 2, and IC50 values were measured using FLIPR® Tetra and QPatch as described in Example 2. Selectivity to voltage-gated sodium channels Nav1.1, Nav1.2, Hav1.3, Nav1.4, Nav1.5 and Nav1.7 were assessed using both methods.

The variant NV1G387 (NV1D2168) demonstrated high selectivity towards Nav1.7 (Figure 5) and was grafted with substitutions that in the original Huwentoxin-IV scan enhanced potency (Nav1.7 IC50>0.05μM). The library design for NV1G387 is shown in Table 1.

Table 1.

| Favorable potency mutations | | |
|---|---|---|
| | **E1N,R26K,Q34S,G36I** | |
| | NV1G387 | |
| | (NV1D2168) | |
| | ≤0.05 | |
| E1 | | |
| C2 | | |
| L3 | | |
| E4 | R, H,N,Q | |
| I5 | Y | |
| F6 | | |
| K7 | | |
| A8 | R, H,N,Q | |
| C9 | | |
| N10 | | |
| P11 | | |
| S12 | | |
| N13 | A | |

(continued)

| Favorable potency mutations | | |
|---|---|---|
| D14 | | |
| Q15 | R,N | |
| C16 | | |
| C17 | | |
| K18 | Y | |
| S19 | R,Q,P | |
| S20 | R,D,N,P | |
| K21 | R,H,F,N | |
| L22 | R | |
| V23 | | |
| C24 | | |
| S25 | I | |
| R26 | | |
| K27 | | |
| T28 | | |
| R29 | | |
| W30 | | |
| C31 | | |
| K32 | | |
| Y33 | W | |
| Q34 | | |
| I35 | | |
| G36 | | |
| K37 | R,F | |

The variant NV1G327 (NV1D2163) demonstrated high potency (Figure 4) and was grafted with substitutions that in the original Huwentoxin-IV scan enhanced selectivity (in this experiment defined as >5× selectivity over NaV1.2 or undefined). The library design for NV1G327 is shown in Table 2.

Table 2.

| | Favorable Selectivity Mutations | |
|---|---|---|
| | **E1N,E4R,Y33W,Q34S** | |
| | NV1G327 | |
| | (NV1D2163) | |
| | > 5x or undef. | >5x |
| E1 | | |
| C2 | | |
| L3 | | |
| E4 | | |

(continued)

| | **Favorable Selectivitv Mutations** | |
|---|---|---|
| I5 | | |
| F6 | V,M | M |
| K7 | Q | |
| A8 | | |
| C9 | | |
| N10 | | |
| P11 | R | |
| S12 | | |
| N13 | W,Q,S,G,I,P | W,Q,G,I |
| D14 | Q,S,G,L,P | Q,S,G,P |
| Q15 | D,E,W,V,P | D,E,W,V,P |
| C16 | | |
| C17 | | |
| K18 | F,W,Q,P | F,W,Q,P |
| S19 | Q | Q |
| S20 | W,V | W,V |
| K21 | W | W |
| L22 | E,W,Q,A | W,A |
| V23 | A | A |
| C24 | | |
| S25 | | |
| R26 | K,H,D,W,T,G,A,V,I,P | K,H,W,T,G,A,V,I,P |
| K27 | H,W,A,I,P | H,W,A |
| T28 | K,L | |
| R29 | H,D,W,N,G,L | H,D,W,N,G |
| W30 | K,Y | K,Y |
| C31 | | |
| K32 | W,A | |
| Y33 | | |
| Q34 | | |
| 135 | H | |
| G36 | F,T,V,I | I |
| K37 | R,Q,S,T,P | R,S,P |

Figure 13A shows the sequences and Figure 13B characteristics of mutants based on the NV1G387 (NV1D2168) scaffold. All values are $IC_{50}$ values in nM unless only a single point assay was performed. In the latter case, the percent inhibition (%I) achieved at a given peptide concentration is listed.

Figure 14A shows sequences and Figure 14B characteristics of mutants based on the NV1G327 (NV1D2163) scaffold. Values in Figure 14B are as in Figure 13B.

The Huwentoxin-IV variants from the bidirectional grafting libraries demonstrated improved selectivity and/or include variants

>NV1G559
GPNCLEIFKACNPSNDQCCKSSFLVCSKKTRWCKYSIIK (SEQ ID NO: 277)
(E1N,R26K,Q34S,G36I,grafted with K21F)
>NV1G566
GPNCLEIFKACNPSNDQCCKSNKLVCSKKTRWCKYSIIK (SEQ ID NO: 278)
(E1N,R26K,Q34S,G36I,grafted with S20N)
>NV1G611
GPNCLRIFKACNPSNDQCCKSSKLVCSDKTRWCKWSIGK (SEQ ID NO: 279)
(E1N,E4R,Y33W,Q34S, grafted with R26D)
>NV1G612
GPNCLRIFKACNPSNDQCCKSSKLVCSRHTRWCKWSIGK (SEQ ID NO: 280)
(E1N,E4R,Y33W,Q34S, grafted with K27H)

**Example 6. Local administration of Nav1.7 inhibitors provide analgesic effects in a model of nociceptive pain in rats**

The analgesic effects of three Nav1.7 blocking peptides were evaluated in rat and mouse models of acute nociceptive pain. The peptides evaluated were Huwentoxin-IV (HwTx-IV) (Peng et al., J Biol Chem 277:47564-71, 2002), Protoxin II (Middleton et al., Biochemistry 41:14734-47, 2002) and conotoxin KIIIA (Zhang et al., J Biol Chem. 2007 282(42):30699-706). These peptides were applied locally since HwTX-IV and KIIIA block several voltage-gated sodium channel isoforms and are expected to induce significant side-effects when administered systemically. The rank order of potency for Nav1.7 block for these three peptides is ProTX-II>HwTX-IV>KIIIA.

**Animals.** Male Sprague-Dawley (CD) rats (Charles River, San Diego) were ordered ~190-200 grams and used at >300 grams.

**Material Preparation and Local Administration in Hindpaw.**

Amidated Huwentoxin IV (Peptides International, Louisville, KY), Protoxin II (Peptides Institute, Japan) or KIIIA were received in lyophilized form and reconstituted with HEPES-buffered saline, aliquoted and frozen at -20°C. Just prior to administration in the left dorsal hind paw, aliquots were thawed and diluted to appropriate concentrations using HEPES-buffered saline as the diluent. Because stress related to handling and paw injections may itself produce an increase in paw pressure threshold (stress-induced analgesia), rats were briefly anesthetized with isoflurane for the injection (5% induction; 2-3% maintenance). Animals were injected s.c. ( 100 μL of peptide solution or vehicle) in the dorsal aspect of the paw with the needle inserted left of center toward the ankle such that the tip of the needle ended just underneath a mark made with an indelible marker in the center of the dorsal paw proximal to the toes.

**Randall-Selitto Test**

A Ugo-Basile Randall-Selitto Device (Analgesy-Meter) was used with the maximum threshold set at 500 grams.

**Training.** Naive animals were trained for two days prior to the day of testing (in order to reduce the variability in responses) as in commonly reported in the literature. Training consisted of performing actual tests multiple times on each animal over duration of ~1 hour for each rat. Animals first received a mark with an indelible marker in the center of the dorsal aspect of the left paw just proximal to the toes to enable consistently testing the same site of the paw. Rats were then loosely wrapped in a towel leaving the hind paws uncovered, the left hind paw was placed in the Randall-Selitto device with the indelible mark just beneath the point of the cone on the test device that comes in contact with the paw and pressure was increased at a steady rate via electronic ramp with foot control until the animal responded. A 'response' for training followed the same criteria as that on the day of testing and consisted of any one of the following: 1) removal of the hind paw from the device, 2) a clear attempt at removal or 3) vocalization. Rats were tested up to 3 times consecutively unless they responded to a threshold greater than or equal to 100 grams. Over the course of the hour/day of training, 1-3 consecutive tests were made for each rat with 5-20 minutes apart.

**Testing.** Prior to application of peptides or vehicle, trained, un-injured rats were tested once for the precompound thresholds. Animals were assigned into peptide- or vehicle-treated groups in such a manner as to produce comparable pre-administration threshold means. Experiments were conducted blind to treatment groups whenever possible (i.e. whenever starting with testing a new peptide or new dose). One test for each time point following injection (5, 10, 20, 30 45, 60 and 120 min) was taken and recorded. Responses were defined identically as responses during training (see *Training* above).

**Data Analysis.** Gram thresholds were recorded on paper and entered into Prism 5.01 (Graphpad Software Inc., LaJolla, CA) for graphing, generating area under the curve (AUC) values and statistical analysis. For comparison of gram values over time, a two-way ANOVA was used with a significance level of $p<0.05$. For generation of mean AUC values, the AUC for each rat in the peptide group was individually obtained and the mean AUC of the vehicle group was subtracted from it. Next, the vehicle subtracted AUCs for each peptide-treated animal were averaged together and compared either by Student's T-test or one-way ANOVA, each with a significance level of $p<0.05$. Responses at 120 min are not shown and were not included in the AUC calculations. Instead, values from prior to administration (Pre) and 5-60 min were used.

**Results.** Huwentoxin IV administered locally into the dorsal aspect of the paw produced a dose-dependent increase in paw pressure threshold in the Randall-Selitto test. 3 nmoles (Figure 15A) and 30 nmoles (Figure 15B) of Huwentoxin IV, but not 0.3 nmoles (not shown), increased thresholds significantly above those observed for vehicle-treated animals. Areas under the curve (AUC) (Figure 15C) were significantly different between all 3 peptide treated groups (with mean vehicle AUC subtracted from the AUC for each animal).

Protoxin II administered locally into the dorsal aspect of the paw produced a dose-dependent increase in paw pressure threshold in the Randall-Selitto test. Each dose of peptide, 0.3 nmoles (Figure 16A), 3 nmoles (Figure 16B) and 30 nmoles (Figure 16C) increased thresholds significantly above those observed for vehicle-treated animals. AUCs were significantly different except between the 0.3 and 3 nmole doses (with mean vehicle AUC subtracted from the AUC for each animal) (Figure 2D).

KIIIA administered locally into the dorsal aspect of the paw at both doses (3 and 30 nmoles) demonstrated a tendency towards increased paw pressure threshold in the Randall-Selitto test which did not reach statistical significance. There was no significant difference between the AUCs from the two doses (not shown).

The findings of this study demonstrate that ProTX-II and HwTX-IV exhibited significant analgesic effects in a rat model of acute nociceptive pain following local administration. Although KIIIA produced a trend toward analgesic activity, this did not reach the level of statistical significance. The rank order of activity (ProTX-II>HwTX-IV>KIIIA) in the pain assay matched that for Nav1.7 block in-vitro, suggesting that Nav1.7 block may have contributed to the analgesic activity.

**Example 7. Local administration of ProTx-II provides antihyperalgesic effects in a model of inflammatory pain in rats**

**Animals.** Male Sprague-Dawley rats weighing 240-295 grams (Mean/s.e.m.: 280.2 $\pm$ 3.3) at the start of the study.

**Behavioral Tests**

**Tactile Allodynia Testing**

Mechanical (tactile) allodynia was assessed by determining the median threshold at which the affected paw was withdrawn from 8 graded stimuli (von Frey filaments: 0.4, 0.6, 1.0, 2.0, 4.0, 6.0, 8.0, and 15.0 g; Stoelting, Wood Dale, IL) applied perpendicularly with sufficient force to bend slightly and held for 5-7 seconds against the plantar hindpaw through custom-made wire-mesh observation cages. Paw withdrawal during or immediately following the removal of the stimulus was considered a positive response. A 50% paw withdrawal threshold (PWT) was determined by sequentially increasing and decreasing the stimulus strength and analyzing withdrawal data using an adaptation of the Dixon up-down method (Dixon, 1980), as described in (Chaplan et al., 1994). Rats were acclimated to the wire mesh for 10 minutes prior to testing. Tactile thresholds before and on several different days following injection of Complete Freund's Adjuvant (CFA) were evaluated.

**Thermal Allodynia Testing**

Paw threshold responses to radiant heat were evaluated using a Thermal Paw Stimulator (Hargreave's Device; UCSD Anesthesiology, San Diego, CA) before and following CFA administration. Naive rats were used to set the gain and intensity of the radiant heat such that their responses were in the range of ~8-12 s latency until paw withdrawal (mean ~10 s). Cut-off is set by the device at 20 s. For each time point, 3 separate measurements on the same paw were obtained about 5 minutes apart for each animal and were averaged together.

**Monoarthritis Model Induction**

An emulsion of Complete Freund's Adjuvant (CFA; Sigma-Aldrich; Saint Louis, MO) was prepared in a 1:1 ratio with CFA and 0.9% saline. Animals were anesthetized with isoflurane 5% induction; 2-5% maintenance and 100 μL of the emulsion was injected subcutaneously into the left hind paw. On day 12 following CFA injection, the ipsilateral paw was injected with either 30 nmoles of Protoxin II (Peptides International; Louisville, Kentucky) in 100 μL HEPES-buffered saline or vehicle (100μL HEPES-buffered saline).

**Data Analysis.** Data are represented as mean ± s.e.m. Gram thresholds (tactile) and latencies to thermal paw withdrawal were recorded on paper and entered into Prism 5.01 (Graphpad Software Inc., LaJolla, CA) for graphing and statistical analysis. For comparison of threshold values over time, a two-way ANOVA with Bonferroni post hoc test was used with a significance level of $p<0.05$.

## Results

Threshold for tactile allodynia (Figure 17A) and latency for thermal allodynia (Figure 17B) were significantly reduced in the animal model of monoarthritis induced by 50% intraplantar CFA in rats. Intraplantar Protoxin II significantly increased the tactile threshold when compared to vehicle injected animals at 30 and 60 minutes after injection.

## Example 8. Local administration of ProTx-II provides antihyperalgesic effects in a mouse model of inflammatory pain

**Animals.** Male C57/bl6 mice were used weighing, at the start of the study, 24-31 grams (Mean/s.e.m.: 27.5 ± 0.3).

## Behavioral Tests

*Tactile Allodynia Testing*

Mechanical (tactile) allodynia was assessed by determining the median threshold at which the affected paw was withdrawn from 7 graded stimuli (von Frey filaments: 0.07, 0.16, 0.4, 0.6, 1.0, 2.0 & 4.0 g; Stoelting, Wood Dale, IL) applied perpendicularly with sufficient force to bend slightly and held for ~3 seconds against the center plantar hindpaw through custom-made wire-mesh observation cages. Paw withdrawal during or immediately following the removal of the stimulus was considered a positive response. A 50% paw withdrawal threshold (PWT) was determined by sequentially increasing and decreasing the stimulus strength and analyzing withdrawal data using an adaptation of the Dixon up-down method (Dixon, 1980), as described in (Chaplan et al., 1994). Mice were acclimated to the wire mesh test conditions for ~1 hour per day for 2 days prior to testing and for 30 minutes prior to testing on each day of testing. Tactile thresholds before and on several different days following injection of Complete Freund's Adjuvant (CFA) were evaluated.

## Monoarthritis Model Induction

For 50% Complete Freund's Adjuvant (CFA; Sigma-Aldrich; Saint Louis, MO), an emulsion was prepared in a 1:1 ratio with CFA and 0.9% saline (vehicle treated controls received only 0.9% saline). For 100% CFA, animals were injected with neat CFA as it arrives from the vendor and control animals were injected with 0.9% saline. Animals were anesthetized with isoflurane 5% induction; 2-5% maintenance and 20 μL was injected subcutaneously into the left hind paw using a 50 μL Hamilton syringe and a 25 gauge needle.

## Treatments

All studies were conducted blinded to treatment. On day 3 following CFA injection, either gabapentin (150 mg/kg, n=6) or vehicle (sterile water; n=6) was administered orally (4mL/kg) as a positive control with known anti-allodynic efficacy in this model and evaluated for changes in tactile threshold. Following a 6-day washout period (on day 9 following CFA) the same animals were tested with 3 nmoles Protoxin II (Peptides International, Louisville, KY) or vehicle (HEPES-buffered saline) administered intraplantar into the left, CFA-treated paw.

## Data Analysis

Data are represented as mean ± s.e.m. Gram thresholds (tactile) and latencies to thermal paw withdrawal were recorded on paper and entered into Prism 5.01 (Graphpad Software Inc., LaJolla, CA) for graphing and statistical analysis. For comparison of threshold values over time, a two-way ANOVA with Bonferroni post hoc test was used with a significance level of $p<0.05$. 100% CFA produced a longlasting, robust tactile allodynia together with local paw edema from days 1-8 post-CFA (Figure 18A). 50% CFA produced a transient allodynia peaking at 2-4 days day following CFA administration.

**Results**

ProTX-II produced a profound anti-allodynic effect following local administration to the inflamed mouse paw. Tactile thresholds were increased above baseline, indicating ProTX-II had additional analgesic effects at the administered dose. The effect of ProTX-II was more pronounced than that achieved with a positive control agent, Gabapentin in increasing CFA-induced tactile allodynia (Figure 18B and 18C).

Collectively, the findings demonstrate that local administration of ProTX-II exerted analgesic and anti-allodynic effects in models of acute and inflammatory pain in rats and mice. These results suggest that local administration of Nav1.7 blocking peptides may be beneficial in a variety of human pain states that are Nav1.7 dependent. These results also suggest that suitably selective peptides will be efficacious following systemic administration

**Example 9. Tolerability of sustained systemic administration of ProTx2**

Exposure and tolerability of ProTX-II following delivery via osmotic mini-pumps for up to seven days in mice was assessed in order to select a dose(s) for subsequent evaluation animal models of pain.

**Test Compound**

Protoxin-II ( Peptides International, Louisville, KY) was formulated in DPBS (no Calcium and Magnesium) at stock concentrations of 0.05, 0.5 and 3.8mg/mL.

**Mini Pumps**

ProTx-II or vehicle was delivered via Alzet micro-osmotic mini pumps at 0.5μl per hour for 7 days after implantation into the mouse. A pump and its flow moderator were first weighed and then filled with a 1mL syringe attached with a 27-gague blunt tipped needle. With the pump in an upright position, pumps were filled, the moderator inserted and re-weighed. Weights were recorded (empty and filled weights) to ensure that the fill volume was over 90% of the Mean Fill volume specified in the Alzet pump directions. The pumps were then placed in a 15mL conical tube filled with 0.9% saline and placed at 37°C for 5-6hours prior to implantation.

**Implantation of Mini Pumps**

Mice were given 20μl of 0.3mg/ml Buprenex prior to being anesthetized (5% induction; 2% maintenance) with isoflurane. Their backs were shaved, wiped down with isopropyl alcohol and povidone iodine, and a small incision was made between the scapulae. Using a hemostat, a small pocket was formed by spreading the subcutaneous connective tissues apart. The pump was inserted into the pocket with the flow moderator pointing away from the incision. The skin incision was then closed using 7mm staples and the animals were allowed to recover in their home cages.

**Determination of Plasma ProTX-II concentrations using QPatch.**

HEK293 cells stably expressing human Nav1.7 were cultured in DMEM/F-12 media (1:1), supplemented with 10% fetal bovine serum, 400 μg/mL Geneticin and 100 μM NEAAs (all reagents from Life Technologies). The cells were maintained at 37°C and in 5% CO2 and assayed upon reaching ~70-90% confluency. Before testing in QPatch (Sophion), cells were first dissociated using 0.05% trypsin (5 min at 37°C), resuspended in CHO-S-SFM media (Life Technologies) and gently triturated to break up cell clumps. Cell density was adjusted to $1\text{-}2\times10^6$/mL with the same media and cells were transferred to a cell "hotel" in QPatch HT and used in experiments for several hours.

The voltage protocol used in the assay was as follows. From a holding potential of -75 mV, cells were first hyperpolarized to -120 mV for 2 sec and then depolarized to 0 mV for 5 ms before returning to the holding potential (-75 mV). This protocol was repeated once every 60 sec during liquid applications (see below). Cells were otherwise held at -75 mV when the above voltage protocol was not executed.

For giga-ohm seal formation, the extracellular solution contained 137 mM NaCl, 5.4 mM KCl, 1 mM $MgCl_2$, 2 mM $CaCl_2$, 5 mM glucose, and 10 mM HEPES, pH = 7.4 and osmolarity = 315 mOsm. The intracellular solution contained 135 mM CsF, 10 mM CsCl, 5 mM EGTA, 5 mM NaCl and 10 mM HEPES, pH = 7.3 and osmolarity = 290 mOsm.

For whole-cell patch clamp recording, plasma from control or test (vehicle- or peptide-dosed) rodents were first diluted (10-1000 fold) in the above extracellular solution and these plasma-containing buffers were subsequently used as the extracellular solution. The intracellular solution remained the same as above.

Upon establishment of the whole-cell recording configuration, a total of five applications of a plasma-containing (except for the last application, which was 1 μM TTX in the extracellular solution that contained no plasma) extracellular solution were made onto each cell being recorded. The first application (5 μl) contained only the control plasma (i.e. plasma-containing buffer). The voltage protocol was executed 10 times (for a total duration of 10 min) five sec after the application. The next three applications (5 μl each) contained either plasma (diluted by the same factor as that in the first application for the control plasma) from a vehicle- or peptide-dosed rodent, or, in the case of control cells, the same control plasma as in the first application. As a positive control, a known concentration (300 nM) of synthetic protoxin-II was spiked into the 10×-diluted control plasma-containing buffer, which was further serial-diluted to obtain lower concentrations (i.e., 3-, 10-, 30- and 100-fold diluted concentrations) of the control peptide in the other plasma-containing buffers (i.e., buffers with plasma diluted by 30, 100, 300 and 1000 fold). Five seconds after each of these three applications, the voltage protocol was again executed 10 times (also once per min). The last application contained 1 μM TTX (composed of three 10 μl sub-applications, each separated by 2 sec), five seconds after which the same voltage protocol was executed twice to obtain the baseline current.

Currents were sampled at 25 kHz and filtered at 5 kHz with an 8-pole Bessle filter. The series resistance compensation level was set at 80%. For each cell, the peak current amplitude at 0 mV for each current trace in the first four liquid applications was first subtracted from that of the last trace in the presence of TTX and then normalized to that of the last trace in the first (control buffer) application as % inhibition. To control for current rundown, this (% inhibition) value for each cell in the presence of a test plasma-containing buffer was further normalized to the average % inhibition value for control (typically 5-6) cells (tested with buffer that contained only control plasma that was diluted by the same factor as that for the test plasma) in the same experiment. The mean value of the last two such values in the last (i.e., 4th overall) plasma application was used in concentration response calculations. The ProTx-II concentrations in the undiluted plasma was calculated by comparing the level of channel inhibition in the presence of serial-diluted plasma (from ProTx-II-dose rodents) buffers with that from (diluted) control plasmas in the presence of spiked (i.e., known) concentrations of ProTx-II. All experiments were performed at room temperature (~22 °C). Data are expressed as mean±se.

**Results Summary**

Plasma concentrations for each dose group at various time points after pump implantation are shown in Table 3. Plasma concentrations were below the limit of detection (~5nM) at all time points for the two lower doses. Plasma concentrations were 50 - 83nM for the higher dose and were similar at all time points within the dose group (suggesting steady state was reached within 2 days). All doses were well tolerated with no abnormal behavior noted at any dose or time point.

Table 3.

| Species | Cpd | Pump Type | Conc in pump (ug/ul) | Flow rate (ul/h) | Total dose (ug/day) | Duration (d) | Est Cp (nM) | Behav Obs |
|---|---|---|---|---|---|---|---|---|
| Mouse | ProTX -II | 7 day | 0.05 | 0.5 | 0.6 | 2 | BLOQ | norma l |
| Mouse | ProTX -II | 7 day | 0.05 | 0.5 | 0.6 | 5 | BLOQ | norma l |
| Mouse | ProTX -II | 7 day | 0.05 | 0.5 | 0.6 | 7 | BLOQ | norma l |
| Mouse | ProTX -II | 7 day | 0.5 | 0.5 | 6 | 2 | BLOQ | norma l |
| Mouse | ProTX -II | 7 day | 0.5 | 0.5 | 6 | 5 | BLOQ | norma l |
| Mouse | ProTX -II | 7 day | 0.5 | 0.5 | 6 | 7 | BLOQ | norma l |
| Mouse | ProTX -II | 7 day | 3.8 | 0.5 | 45.6 | 2 | 50 | norma l |

(continued)

| Species | Cpd | Pump Type | Conc in pump (ug/ul) | Flow rate (ul/h) | Total dose (ug/day) | Duration (d) | Est Cp (nM) | Behav Obs |
|---|---|---|---|---|---|---|---|---|
| Mouse | ProTX -II | 7 day | 3.8 | 0.5 | 45.6 | 5 | 55 | norma l |
| Mouse | ProTX -II | 7 day | 3.8 | 0.5 | 45.6 | 7 | 83 | norma l |

ProTX-II was well tolerated at doses up to 45.6ug/day for 7 days in mice. Given that a maximally tolerated dose was not identified in this study, we decided to evaluate a 5 fold higher dose (228ug/d) in a pain study.

**Example 10. Delivery of ProTx-II using mini-pumps provides anti-allodynic effects in a mouse model of inflammatory** pain.

**Animals.** Male C57Bl/6 mice, ordered from Charles River and housed individually, were used for this study.

## Behavioral Tests

### Tactile Allodynia Testing

Mechanical (tactile) allodynia was assessed by determining the median threshold at which the affected paw was withdrawn from 7 graded stimuli (von Frey filaments: 0.07, 0.16, 0.4, 0.6, 1, 2, 4 g; Stoelting, Wood Dale, IL) applied perpendicularly with sufficient force to bend slightly and held for 3 seconds against the plantar hindpaw through custom-made wire-mesh observation cages. Paw withdrawal during or immediately following the removal of the stimulus was considered a positive response. A paw withdrawal threshold (PWT), recorded in grams, was determined by sequentially increasing and decreasing the stimulus strength and analyzing withdrawal data using an adaptation of the Dixon up-down method (Dixon, 1980), as described in (Chaplan et al., 1994). Mice were acclimated to the wire mesh for 30 minutes prior to testing. Tactile thresholds before and on several different days following injection of 100% Complete Freund's Adjuvant (CFA) were evaluated. Behavioral testing was done completely blinded. A separate investigator, than the one doing the testing, organized the Pre-Threshold values to homogenize them prior to Baseline testing.

### Thermal (Hargreaves) Allodynia Testing

A modified Hargreaves box was used to measure thermal allodynia (Hargreaves et al., 1988, Pain, 32:77-88; Dirig et al., 1997, J Neurosci. Methods, 76:183-191). This box consists of Plexiglas chambers with a raised glass floor maintained at a constant temperature (28°C). The thermal nociceptive stimulus originates from a projection bulb below the glass surface, and the stimulus is delivered separately to one hind paw at a time with a 20 second cutoff time. A constant amperage was used throughout the study, which resulted in Pre-test paw withdrawal latencies between ~8-12 seconds when averaged over 3 read-outs taken 5 minutes apart. The animals were allowed to habituate on the glass surface for 10 minutes before paw withdrawal latencies (PWL) in seconds, were recorded.

CFA

Animals were anesthetized with isoflurane 5% induction and a 2% maintenance and 20 μL of 100% Complete Freund's Adjuvant (CFA; Sigma-Aldrich; Saint Louis, MO was injected subcutaneously into the left hind paw using a 25gauge needle attached to a 1mL syringe.

### Test Compound

Protoxin-II ( Peptides International) was formulated in DPBS (no Calcium and Magnesium) at a stock concentration of 9.5mg/mL.

### Mini Pumps

Alzet micro-osmotic mini pumps (Durect Corporation Model 1003D) were used. These pumps delivered the test compound and vehicle at 1.0μl per hour for 3 days after implantation into the mouse. A pump and its flow moderator were first weighed and then filled with a 1mL syringe attached with a 27-gague blunt tipped needle. With the pump in an upright position the pump was filled, the flow moderator inserted and re-weighed. Weights were recorded (empty and filled weights) to ensure that the fill volume was over 90% of the Mean Fill volume specified in the Alzet pump directions (92μL per instruction sheet). The pumps were then placed in a 15mL conical tube filled with 0.9% saline and placed in 37°C for 5-6hours prior to implantation.

### Implantation of Mini Pumps

Mice were given 20μl of 0.3mg/ml Buprenex prior to being anesthetized (5% induction; 2% maintenance) with isoflurane. Their backs were shaved, wiped down with isopropyl alcohol and povidone iodine, and a small incision was made between the scapulae. Using a hemostat, a small pocket was formed by spreading the subcutaneous connective tissues apart. The contents of each pump was not known to the surgeon or experimental operator. The skin incision was then closed using 7mm staples and the animals were allowed to recover in their home cages.

### Data Analysis

Data are represented as mean ± s.e.m. Gram thresholds (tactile) and mean latencies (thermal) were recorded on paper and entered into Prism (Graphpad Software Inc., LaJolla, CA) for graphing and statistical analysis. For comparison of threshold values over time, a two-way ANOVA with Bonferroni post hoc test was used with a significance level of $p<0.05$.

### Procedure

Animals were trained on the Von Frey Stand and the Hargreaves box Tuesday, Wednesday and Thursday of the previous week before testing. They were allowed to sit on the stand/box for ~30min to get used to being on the apparatus. On Friday, their Pre-Thresholds were tested in both tactile (Von Frey stand) and thermal (Hargreaves). Once Pre-Thresholds were tested the animals were briefly anesthetized and 20μL of 100% CFA was injected into their left hind paw. The animals were allowed to recover and returned to their home cages. On Monday of the next week, mice were tested for Baseline measurements in both tactile and thermal to confirm that the CFA caused sufficient inflammation to lower their thresholds. The mice were then anesthetized and a mini pump was implanted and the animals were allowed to recover. On Tuesday, Wednesday and Thursday, they had their "Day 1", "Day 2" and "Day 3" tactile and thermal thresholds measured. At the end of Day 3, the animals were sacrificed and terminal blood samples obtained.

Plasma ProTx-II concentrations were determined as described in Example 9. The average concentration of ProTx-II was 224 nM.

### Results

ProTX-II exhibited statistically significant efficacy in a mouse model of inflammatory pain following sustained delivery of 228pg/day via osmotic mini-pump. Tactile threshold (Figure 19A) and thermal threshold (Figure 19B) were significantly increased in ProTx-II treated animals. These observations were reproducible across two independent and fully blinded studies. ProTx-II did not did not produce any obvious motor impairment at this efficacious dose. In contrary to published reports (Schmalhofer et al., Mol Pharm 74:1476-1484, 2008; Hacker et al., Proc Natl Acad Sci USA 109:E2018-27), these data demonstrate that systemic administration with sustained exposure to a Nav1.7 selective peptide can provide robust efficacy in inflammatory pain.

Effects of ProTx-II delivered via a mini pump was assessed in mice with spared nerve injury, a model of neuropathic pain, in the tactile (Von Frey) pain assay. ProTx-II was not efficacious in this mouse spared nerve injury model of neuropathic pain following sustained delivery of 228 μg/day of ProTx-II via osmotic mini-pump.

The invention further provides the following numbered embodiments:

1) An isolated Huwentoxin-IV variant comprising a sequence $X_1CX_2X_3X_4FX_5X_6CX_7X_8X_9X_{10}X_{11}X_{12}CCX_{13}X_{14}X_{15}X_{16}X_{17}X_{18}CX_{19}X_{20}X_{21}X_{22}X_{23}$ $X_{24}CKX_{25}X_{26}IX_{27}X_{28}$ (SEQ ID NO: 265); wherein

a) $X_1$, $X_2$, $X_3$, $X_4$, $X_5$, $X_6$, $X_7$, $X_8$, $X_9$, $X_{10}$, $X_{11}$, $X_{12}$, $X_{13}$, $X_{14}$, $X_{15}$, $X_{16}$, $X_{17}$, $X_{18}$, $X_{19}$, $X_{20}$, $X_{21}$, $X_{22}$, $X_{23}$, $X_{24}$, $X_{25}$,

and $X_{26}$ are any amino acid;
b) $X_{27}$ and $X_{28}$ are any amino acid or deleted; and
c) the Huwentoxin-IV variant has an $IC_{50}$ value about $300\times10^{-9}$ M or less for human Nav1.7 (SEQ ID NO: 263), with the proviso that the Huwentoxin-IV variant is not a polypeptide comprising a sequence shown in SEQ ID NO: 1.

2) The isolated Huwentoxin-IV variant of embodiment 1, wherein

a) $X_4$ is Y, V or I;
b) $X_8$ is P or V;
c) $X_{11}$ is D, P or W;
d) $X_{19}$ is S or I;
e) $X_{21}$ is Y, W, A, K or H;
f) $X_{22}$ is T or V;
g) $X_{24}$ is W or K; and
h) $X_{25}$ is W, T, I or Y.

3) The isolated Huwentoxin-IV variant of embodiment 2 comprising the amino acid sequence of SEQ ID NOs: 277, 278, 192, 279, 280, or 3.
4) The isolated Huwentoxin-IV variant of embodiment 2, wherein

a) $X_1$ is K, R, H, D, Y, F, N, Q, S, T, G, L, I, P or E;
b) $X_2$ is R, F, W, N, S or L;
c) $X_3$ is R, H, D, Y, N, Q, L, I, P or E;
d) $X_5$ is R, W, Q, S or K;
e) $X_6$ is R, E, Y, F, V or A;
f) $X_7$ is K, R, E, Y, F, S, V or N;
g) $X_9$ is R, F, Q, V or S;
h) $X_{10}$ is H, D, Y, W, Q, S, T, G, A, V, L, I, P or N;
i) $X_{12}$ is K, R, D, E, Y, W, N, T, A, L or Q;
j) $X_{13}$ is R, Y, Q, S, T, G, L, I, P or K;
k) $X_{14}$ is K, R, Y, F, N, Q, G, A, V, L, I, P or S;
l) $X_{15}$ is R, H, D, Y, W, N, Q, T, V, I, P or S;
m) $X_{16}$ is R, H, D, F, W, N, Q, S, T, G, A, L or K;
n) $X_{17}$ is K, R, Y, F, W, P or L;
o) $X_{18}$ is K, R, T, A, L or V;
p) $X_{20}$ is K, W, G, A, I, R or D;
q) $X_{23}$ is K, H, W, N, G, A, L or R;
r) $X_{26}$ is K, R, Y, F, S, T, G, A, V, L, I or Q;
s) $X_{27}$ is K, R, H, F, W, V, L, I, G or deleted; and
t) $X_{28}$ is R, H, Y, F, W, N, G, V, P, K or deleted.

5) The isolated Huwentoxin-IV variant of embodiment 4, wherein the Huwentoxin-IV variant has an $IC_{50}$ of less than about $160\times10^{-9}$ M for human Nav1.7.
6) The isolated Huwentoxin-IV variant of embodiment 5, wherein the Huwentoxin-IV variant comprises the polypeptide sequence of SEQ ID NOs: 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 241, 242, 243, 244, 245, 246, 247, 248, 249, 250, 251, 252, 253, 277, 278, 279 or 280.
7) An isolated Huwentoxin-IV variant comprising a sequence $X_1CX_2X_3X_4FX_5X_6CX_7X_8X_9X_{10}X_{11}X_{12}CCX_{13}X_{14}X_{15}X_{16}X_{17}X_{18}CX_{19}X_{20}X_{21}X_{22}X_{23}$ $X_{24}CKX_{25}X_{26}IX_{27}X_{28}$ (SEQ ID NO: 265); wherein

a) $X_1$, $X_2$, $X_3$, $X_4$, $X_5$, $X_6$, $X_7$, $X_8$, $X_9$, $X_{10}$, $X_{11}$, $X_{12}$, $X_{13}$, $X_{14}$, $X_{15}$, $X_{16}$, $X_{17}$, $X_{18}$, $X_{19}$, $X_{20}$, $X_{21}$, $X_{22}$, $X_{23}$, $X_{24}$, $X_{25}$, and $X_{26}$ are any amino acid;

b) $X_{27}$ and $X_{28}$ are any amino acid or deleted; and

c) the Huwentoxin-IV variant selectively inhibits Nav1.7, with the proviso that the Huwentoxin-IV variant is not a polypeptide comprising a sequence shown in SEQ ID NO: 1.

8) The isolated Huwentoxin-IV variant of embodiment 7, wherein the Huwentoxin-IV variant comprises the polypeptide sequence of SEQ ID NOs: 5, 7, 12, 13, 16, 21, 25, 45, 46, 48, 55, 57, 58, 60, 61, 72, 74,76, 78, 82, 83, 96, 109, 111, 113, 122, 127, 131, 134, 137, 141, 142, 149, 164, 165, 172, 175, 177, 178, 180, 182, 188, 189, 192, 198, 202, 204, 213, 215, 219, 223, 224, 225, 226, 227, 228, 229, 230, 231, 232, 233, 234, 235, 236, 237, 238, 239 or 240.

9) An isolated Huwentoxin-IV variant comprising the polypeptide sequences shown in SEQ ID NOs: 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 277, 278, 279, 280, 281, 282, 283, 284, 285, 286, 287, 288, 289, 290, 291, 292, 293, 294, 295, 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354 or 355.

10) An isolated polynucleotide encoding the Huwentoxin-IV variant of SEQ ID NOs: 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 277, 278, 279, 280, 281, 282, 283, 284, 285, 286, 287, 288, 289, 290, 291, 292, 293, 294, 295, 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354 or 355.

11) A vector comprising the isolated polynucleotide of embodiment 10.

12) A host cell comprising a vector of embodiment 11.

13) A method of producing the isolated Huwentoxin-IV variant comprising culturing the host cell of embodiment 12 and recovering the Huwentoxin-IV variant by the host cell.

14) A pharmaceutical composition comprising the isolated Huwentoxin-IV variant of embodiment 9 and a pharmaceutically acceptable excipient.

15) A method of treating pain in a subject, comprising peripherally administering to the subject an effective amount of the Huwentoxin-IV variant of embodiment 9 to treat the pain.

16) The Huwentoxin-IV variant of embodiment 9 for use in therapy.

17) A method of alleviating Nav1.7-mediated pain by peripherally administering a therapeutically effective amount of a peptide inhibitor of Nav1.7 to a subject in need thereof for a time sufficient to alleviate the Nav1.7-mediated pain.

18) The method of embodiment 17, wherein the Nav1.7-mediated pain is chronic pain, acute pain, neuropathic pain, nociceptive pain, visceral pain, back pain, post-operative pain, thermal pain, phantom limb pain, or pain associated with inflammatory conditions, primary erythemalgia (PE), paraoxysmal extreme pain disorder (PEPD), osteoarthritis, rheumatoid arthritis, lumbar discectomy, pancreatitis, fibromyalgia, painful diabetic neuropathy (PDN), post-herpetic neuropathy (PHN), trigeminal neuralgia (TN), spinal cord injuries or multiple sclerosis.

19) The method of embodiment 18, wherein the subject is a human.

20) The method of embodiment 19, wherein the peptide inhibitor of Nav1.7 is administered locally to a joint, spinal cord, surgical wound, sites of injury or trauma, peripheral nerve fibers, urogenital organs, or inflamed tissues.

21) The method of embodiment 19, wherein the peptide inhibitor of Nav1.7 is administered using a mini-pump.

22) The method of embodiment 19, wherein the peptide inhibitor of Nav1.7 is Protoxin-II (SEQ ID NO: 356), Huwentoxin-IV (SEQ ID NO: 1), a Protoxin-II variant or a Huwentoxin-IV variant.

23) The method of embodiment 22, wherein the Huwentoxin-IV variant comprises the polypeptide sequence shown in SEQ ID NOs: 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 277, 278, 279, 280, 281, 282, 283, 284, 285, 286, 287, 288, 289, 290, 291, 292, 293, 294, 295, 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354 or 355.

## Claims

**1.** An isolated fusion protein comprising:

(1) an isolated Huwentoxin-IV variant comprising a sequence $X_1CX_2X_3X_4FX_5X_6CX_7X_8X_9X_{10}X_{11}X_{12}CCX_{13}X_{14}X_{15}X_{16}X_{17}X_{18}CX_{19}X_{20}X_{21}X_{22}X_{23}$ $X_{24}CKX_{25}X_{26}IX_{27}X_{28}$ (SEQ ID NO: 265); wherein

a. $X_1$ is E;
b. $X_2$ is L;
c. $X_3$ is R, N, Q or E;
d. $X_4$ is I;
e. $X_5$ is K;
f. $X_6$ is R, or A;
g. X7 is N;
h. $X_8$ is P;
i. $X_9$ is S;
j. $X_{10}$ is N;
k. $X_{11}$ is D;
l. $X_{12}$ is N or Q;
m. $X_{13}$ is K;
n. $X_{14}$ is Q, P or S;
o. $X_{15}$ is N or S;
p. $X_{16}$ is R or K;
q. $X_{17}$ is L;
r. $X_{18}$ is K, T, L or V;
s. $X_{19}$ is S or I;
t. $X_{20}$ is K, W, G, I, R or D;
u. $X_{21}$ is W, K or H;
v. X22 is T;
w. X23 is H, W, N, G, L or R;
x. $X_{24}$ is W or K; and
y. $X_{25}$ is W, T, or Y;
z. $X_{26}$ is Y, F, S, T, G, V, L or Q;

aa. $X_{27}$ is K, R, H, F, W, V, L, I, G or deleted; and
bb. $X_{28}$ is R, H, Y, F, W, N, V, P, K or deleted; and

wherein the Huwentoxin-IV variant has an $IC_{50}$ value of less than $0.03 \times 10^{-6}$ M for human Nav1.7 (SEQ ID NO: 263), with the proviso that the Huwentoxin-IV variant is not a polypeptide comprising a sequence shown in SEQ ID NO: 1; and
(2) a second polypeptide.

**2.** The isolated fusion protein of claim 1 wherein the second polypeptide is selected from the group consisting of a leader sequence, a secretory signal sequence, and a hexahistidine tag.

**3.** An isolated Huwentoxin-IV variant comprising:

(i) an isolated Huwentoxin-IV variant comprising a sequence $X_1CX_2X_3X_4FX_5X_6CX_7X_8X_9X_{10}X_{11}X_{12}CCX_{13}X_{14}X_{15}X_{16}X_{17}X_{18}CX_{19}X_{20}X_{21}X_{22}X_{23}$ $X_{24}CKX_{25}X_{26}IX_{27}X_{28}$ (SEQ ID NO: 265); wherein

a. $X_1$ is E;
b. $X_2$ is L;
c. $X_3$ is R, N, Q or E;
d. $X_4$ is I;
e. $X_5$ is K;
f. $X_6$ is R, or A;
g. X7 is N;
h. $X_8$ is P;
i. $X_9$ is S;
j. $X_{10}$ is N;
k. $X_{11}$ is D;
l. $X_{12}$ is N or Q;
m. $X_{13}$ is K;
n. $X_{14}$ is Q, P or S;
o. $X_{15}$ is N or S;
p. $X_{16}$ is R or K;
q. $X_{17}$ is L;
r. $X_{18}$ is K, T, L or V;
s. $X_{19}$ is S or I;
t. $X_{20}$ is K, W, G, I, R or D;
u. $X_{21}$ is W, K or H;
v. X22 is T;
w. X23 is H, W, N, G, L or R;
x. $X_{24}$ is W or K; and
y. $X_{25}$ is W, T, or Y;
z. $X_{26}$ is Y, F, S, T, G, V, L or Q;
aa. $X_{27}$ is K, R, H, F, W, V, L, I, G or deleted; and
bb. $X_{28}$ is R, H, Y, F, W, N, V, P, K or deleted; and

wherein the Huwentoxin-IV variant has an $IC_{50}$ value of less than $0.03 \times 10^{-6}$ M for human Nav1.7 (SEQ ID NO: 263), with the proviso that the Huwentoxin-IV variant is not a polypeptide comprising a sequence shown in SEQ ID NO: 1, and
(ii) an additional moiety coupled to the Huwentoxin-IV variant.

**4.** The isolated Huwentoxin-IV variant of claim 3 wherein the additional moiety is selected from the group consisting of a polyethylene glycol moiety, a fatty acid, a fatty acid ester, and a carbohydrate.

**5.** A pharmaceutical composition comprising:

(a) the isolated fusion protein of claim 1 or the isolated Huwentoxin-IV variant of claim 3; and
(b) a pharmaceutically acceptable excipient.

**6.** The isolated fusion protein of claim 1, the isolated Huwentoxin-IV variant of claim 3, or the pharmaceutical composition of claim 5 for use in a method of treating Nav1.7-mediated pain in a subject, wherein the method comprises peripherally administering to the subject an effective amount of the fusion protein, the isolated Huwentoxin-IV variant, or the pharmaceutical composition to treat the pain.

**7.** The isolated fusion protein for use, the isolated Huwentoxin-IV variant for use, or the pharmaceutical composition for use of claim 6 wherein the Nav1.7-mediated pain is chronic pain, acute pain, neuropathic pain, nociceptive pain, visceral pain, back pain, post-operative pain, thermal pain, phantom limb pain, or pain associated with inflammatory conditions, primary erythemalgia (PE), paroxysmal extreme pain disorder (PEPD), osteoarthritis, rheumatoid arthritis, lumbar discectomy, pancreatitis, fibromyalgia, painful diabetic neuropathy (PDN), post-herpetic neuropathy (PHN), trigeminal neuralgia (TN), spinal cord injuries or multiple sclerosis.

**8.** The isolated fusion protein for use, the isolated Huwentoxin-IV variant for use, or the pharmaceutical composition for use of claim 6 wherein the isolated fusion protein, the isolated Huwentoxin-IV variant, or the pharmaceutical composition is administered locally to a joint, spinal cord, surgical wound, sites of injury or trauma, peripheral nerve fibers, urogenital organs, or inflamed tissues.

**9.** The isolated fusion protein for use, the isolated Huwentoxin-IV variant for use, or the pharmaceutical composition for use of claim 6 wherein the subject is a human.

**10.** The isolated fusion protein for use, the isolated Huwentoxin-IV variant for use, or the pharmaceutical composition for use of claim 6 wherein the fusion protein, the isolated Huwentoxin-IV variant, or the pharmaceutical composition is administered using a mini-pump.

**11.** The isolated fusion protein of claim 1, the isolated Huwentoxin-IV variant of claim 3, or the pharmaceutical composition of claim 5 for use in therapy.

**12.** An isolated polynucleotide encoding the polypeptide sequence of the fusion protein of claim 1 or 2.

**13.** A vector comprising the isolated polynucleotide of claim 12.

**14.** A host cell comprising the vector of claim 13.

**15.** A method of producing an isolated fusion protein comprising culturing the host cell of claim 14 and recovering the fusion protein produced by the host cell.

| Reference huwentoxin-IV residue | | | Substitution: Basic K | R | H | Acidic D | E | Aromatic Y | F | W | Uncharged Polar N | Q | S | T | Substitution: Nonpolar G | A | V | L | I | P | M | Residue substitutions in variants with $IC_{50}$ ≤300 nM* |
|---|---|---|---|---|---|---|---|---|---|---|---|---|---|---|---|---|---|---|---|---|---|---|
| | | G | | | | | | | | | | | | | | | | | | | | |
| | | P | | | | | | | | | | | | | | | | | | | | |
| | 1 | E | 0.06 | 0.05 | 0.07 | 0.16 | | 0.16 | 0.14 | | 0.07 | 0.09 | 0.11 | 0.12 | 0.08 | | >0.07 | 0.11 | 0.07 | 0.05 | | KRHDYFNQSTGLIPE |
| | 2 | C | | | | | | | | | | | | | | | | | | | | C |
| | 3 | L | | 0.27 | | 1.07 | 3.13 | | 0.16 | 0.15 | 0.11 | | 0.07 | 1.72 | | 0.54 | 0.74 | | | 1.88 | | RFWNSL |
| | 4 | E | | 0.02 | 0.05 | 0.07 | | 0.06 | | | 0.03 | 0.02 | | | | | | 0.08 | 0.20 | 0.11 | | RHDYNQLIPE |
| | 5 | I | | >0.36 | 5.35 | | >0.82 | 0.04 | | 0.43 | 16.80 | | | 4.26 | | 0.40 | 0.16 | | | 4.40 | | YVI |
| | 6 | F | | | >7.38 | >8.74 | | | | | >11.28 | | >0.56 | >0.75 | | 3.38 | 3.48 | 0.57 | | | 1.10 | F |
| | 7 | K | | 0.07 | | | 0.76 | | | 0.06 | 0.44 | 0.30 | 0.14 | 0.86 | | | | | | | | RWQSK |
| | 8 | A | | 0.02 | | | 0.06 | 0.11 | 0.18 | 0.37 | | | 1.24 | | | | 0.26 | | | | | REYFVA |
| | 9 | C | | | | | | | | | | | | | | | | | | | | C |
| | 10 | N | 0.27 | 0.11 | | 0.50 | 0.26 | 0.22 | 0.22 | 0.34 | | 0.68 | 0.20 | | | | 0.14 | | | | | KREYFSVN |
| | 11 | P | 3.57 | 0.49 | | | 7.83 | | 0.62 | 0.42 | | 1.27 | 0.39 | | | | 0.23 | | | | | PV |
| | 12 | S | | 0.13 | 0.35 | | 0.46 | | 0.24 | 2.64 | | 0.26 | | | | | 0.16 | | | | | RFQVS |
| | 13 | N | 0.36 | 0.68 | 0.18 | 0.10 | 0.31 | 0.11 | | 0.07 | | 0.06 | 0.26 | 0.10 | 0.19 | 0.03 | 0.23 | 0.28 | 0.14 | 0.26 | | HDYWQSTGAVLIPN |
| | 14 | D | 1.13 | 10.30 | 1.13 | | 1.41 | >1.27 | | 0.30 | | 0.91 | 0.36 | | 0.72 | 0.78 | 1.23 | 1.01 | >1.23 | 0.28 | | DWP |
| | 15 | Q | 0.24 | 0.04 | 0.36 | 0.18 | 0.23 | 0.07 | | 0.11 | 0.02 | | 0.37 | 0.08 | | 0.10 | 0.75 | 0.26 | | 0.35 | | KRDEYWNTALQ |
| | 16 | C | | | | | | | | | | | | | | | | | | | | C |
| | 17 | C | | | | | | | | | | | | | | | | | | | | C |

FIG. 1A

| Reference huwentoxin-IV residue | | Substitution: Basic | | | Acidic | | Aromatic | | | Uncharged Polar | | | | Substitution: Nonpolar | | | | | | | Residue substitutions in variants with $IC_{50}$ ≤300 nM* |
|---|---|---|---|---|---|---|---|---|---|---|---|---|---|---|---|---|---|---|---|---|---|
| | | K | R | H | D | E | Y | F | W | N | Q | S | T | G | A | V | L | I | P | M | |
| 18 | K | | 0.06 | 0.39 | | 0.40 | 0.04 | 0.36 | 0.46 | | 0.14 | 0.14 | 0.11 | 0.15 | 0.59 | | 0.16 | 0.10 | 0.23 | | RYQSTGLIPK |
| 19 | S | 0.08 | 0.05 | | | | 0.10 | 0.10 | | 0.09 | 0.02 | | | 0.27 | 0.12 | 0.09 | 0.07 | 0.07 | 0.03 | | KRYFNQGAVLIPS |
| 20 | S | | 0.04 | 0.09 | 0.04 | 1.97 | 0.06 | | 0.13 | 0.02 | 0.16 | | 0.09 | 0.76 | | 0.13 | | 0.12 | 0.04 | 0.53 | RHDYWNQTVIPS |
| 21 | K | | 0.02 | 0.04 | 0.30 | | | 0.04 | 0.08 | 0.04 | 0.12 | 0.07 | 0.26 | 0.16 | 0.16 | 1.52 | 0.09 | | | 0.37 | RHDFWNQSTGALK |
| 22 | L | 0.24 | 0.05 | 0.72 | >6.24 | 1.95 | 0.15 | 0.18 | 0.30 | | 2.70 | 1.14 | 0.34 | 1.38 | 0.52 | | | | 0.14 | 1.11 | KRYFWPL |
| 23 | V | 0.30 | 0.15 | 0.40 | 4.70 | 2.90 | 0.39 | 0.46 | | 0.48 | 0.54 | | 0.09 | 1.00 | 0.11 | | 0.13 | | >6.57 | | KRTALV |
| 24 | C | | | | | | | | | | | | | | | | | | | | C |
| 25 | S | 0.61 | | >2.37 | 1.86 | >9.11 | >1.72 | >1.27 | >7.64 | 0.70 | >0.82 | | 3.07 | 3.70 | >0.83 | | | 0.03 | 0.88 | | SI |
| 26 | R | 0.20 | | 0.74 | 1.26 | | | >0.61 | 0.07 | | | | 1.93 | 0.29 | 0.17 | 0.41 | | 0.20 | 1.84 | | KWGAIR |
| 27 | K | | 0.33 | 4.04 | >10.22 | >10.57 | 0.13 | | 0.06 | 1.67 | >17.89 | >2.5 | | 1.51 | 0.26 | | 1.00 | 0.84 | 10.90 | | YWAK |
| 28 | T | 5.41 | | | | 12.50 | | >0.05 | 2.86 | | 0.59 | | | | | 0.21 | 5.69 | | | | TV |
| 29 | R | 0.20 | | 0.06 | 0.95 | >13.31 | >0.13 | | 0.06 | 0.15 | >14.53 | | | 0.21 | 0.26 | >0.13 | 0.29 | >0.71 | | | KHWNGALR |
| 30 | W | 0.10 | >0.49 | >10.14 | >1 | >0.37 | 1.69 | >1 | | >1.66 | >54.93 | >50.86 | >43.5 | >3.12 | >1.8 | >2.5 | >3.2 | >1.41 | >1.07 | | WK |
| 31 | C | | | | | | | | | | | | | | | | | | | | C |
| 32 | K | | | >4.57 | | >2.5 | | >0.81 | 9.12 | >3.51 | >10.76 | >11.85 | >5.6 | >2.5 | 8.86 | >5.95 | >4.56 | >4.56 | >5.63 | | K |
| 33 | Y | 4.31 | | 1.47 | | | | >14.83 | 0.02 | | | 6.90 | 0.07 | 4.35 | 1.11 | 0.34 | | 0.10 | >8.04 | | WTIY |
| 34 | Q | 0.16 | 0.06 | | 5.94 | 0.47 | 0.08 | 0.01 | 1.32 | | | 0.02 | 0.05 | 0.21 | 0.11 | 0.25 | 0.04 | 0.29 | >6.1 | | KRYFSTGAVLIQ |
| 35 | I | | >0.95 | 4.81 | >5.17 | >0.06 | >10.24 | | >2.5 | | | >14.59 | >12 | | 2.58 | | 0.78 | | | | I |
| 36 | G | 0.09 | 0.15 | 0.22 | >11.25 | >5.35 | >3.07 | 0.19 | 0.09 | >3.22 | 1.74 | | 0.58 | | 0.46 | 0.22 | 0.07 | 0.06 | 0.53 | | KRHFWVLIG |
| 37 | K | | 0.03 | 0.10 | 1.17 | >0.65 | 0.19 | 0.05 | 0.11 | 0.27 | 5.45 | 1.47 | 0.37 | 0.09 | | 0.19 | | 0.37 | 0.26 | | RHYFWNGVPK |

*wild type residue included

FIG. 1B

| | | | Substitution | | | | | | | | | | | | | | | | | | |
|---|---|---|---|---|---|---|---|---|---|---|---|---|---|---|---|---|---|---|---|---|---|
| | | | Basic | | | Acidic | | Aromatic | | | Uncharged Polar | | | | Nonpolar | | | | | | |
| | | | K | R | H | D | E | Y | F | W | N | Q | S | T | G | A | V | L | I | P | M |
| Reference huwentoxin-IV residue | | G | | | | | | | | | | | | | | | | | | | |
| | | P | | | | | | | | | | | | | | | | | | | |
| | 1 | E | 0.02 | 0.02 | 0.03 | 0.22 | | 0.07 | 0.07 | | 0.09 | 0.03 | 0.05 | 0.06 | 0.02 | | >0.03 | 0.03 | 0.03 | 0.03 | |
| | 2 | C | | | | | | | | | | | | | | | | | | | |
| | 3 | L | | 0.11 | | 1.60 | 2.90 | | 0.29 | 0.16 | 0.07 | | 0.18 | 0.48 | | 0.66 | 0.41 | | | 0.56 | |
| | 4 | E | | 0.02 | 0.06 | 0.12 | | 0.04 | | | 0.03 | 0.02 | | | | | | 0.11 | 0.19 | 0.15 | |
| | 5 | I | | >0.36 | 2.90 | | >0.82 | 0.07 | | 0.22 | 8.50 | | | 2.46 | | 0.28 | 0.12 | | | 1.17 | |
| | 6 | F | | | 2.20 | >8.74 | | | | | >11.28 | | >0.56 | >0.75 | | 3.40 | >3.61 | 2.23 | | | 6.68 |
| | 7 | K | | 0.03 | | | 0.67 | | | 0.08 | 0.23 | >0.27 | 0.09 | 0.19 | | | | | | | |
| | 8 | A | | 0.01 | | | 0.14 | 0.05 | 0.14 | 0.31 | | | 0.48 | | | | 0.25 | | | | |
| | 9 | C | | | | | | | | | | | | | | | | | | | |
| | 10 | N | 0.24 | 0.09 | | 0.84 | 0.28 | 0.10 | 0.26 | 0.29 | | 0.40 | 0.17 | | | | 0.16 | | | | |
| | 11 | P | 1.65 | >0.43 | | | 9.64 | | 0.44 | 0.34 | | 0.84 | 0.63 | | | | 0.33 | | | | |
| | 12 | S | | 0.17 | 0.32 | | 0.97 | | 0.30 | 0.16 | | 0.07 | | | | | 0.15 | | | | |
| | 13 | N | 0.57 | 0.17 | 0.77 | 0.43 | 0.77 | 0.23 | | 0.37 | | 0.49 | >0.98 | 0.19 | 3.74 | 0.07 | 0.98 | 1.33 | 0.95 | >0.85 | |
| | 14 | D | 3.09 | 11.10 | 5.45 | | 6.86 | >1.27 | | 1.43 | | 7.57 | >1.46 | | >5.18 | 0.35 | 2.99 | >2.5 | >1.23 | 2.71 | |
| | 15 | Q | 0.30 | 0.05 | 0.51 | 1.08 | 2.16 | 0.20 | | 0.79 | 0.06 | | 1.42 | 0.21 | | 0.35 | 4.91 | 1.39 | | 4.63 | |
| | 16 | C | | | | | | | | | | | | | | | | | | | |
| | 17 | C | | | | | | | | | | | | | | | | | | | |

FIG. 2A

| Reference huwentoxin-IV residue | | Substitution | | | | | | | | | | | | | | | | | | |
|---|---|---|---|---|---|---|---|---|---|---|---|---|---|---|---|---|---|---|---|---|
| | | Basic | | | Acidic | | Aromatic | | | Uncharged Polar | | | | Nonpolar | | | | | | |
| | | K | R | H | D | E | Y | F | W | N | Q | S | T | G | A | V | L | I | P | M |
| 18 | K | | 0.08 | 0.76 | | 0.91 | 0.08 | 3.46 | 2.38 | | 0.79 | 0.46 | 0.23 | 0.63 | 0.19 | | 0.72 | 0.32 | 2.40 | |
| 19 | S | 0.36 | 0.05 | | | | 0.20 | 0.31 | | 0.19 | 0.46 | | | 0.33 | 0.19 | 0.15 | 0.19 | 0.14 | 0.09 | |
| 20 | S | | 0.04 | 0.23 | 0.07 | 1.62 | 0.10 | | 0.87 | 0.07 | 0.59 | | 0.19 | 0.64 | | 0.73 | | 0.21 | 0.13 | 0.44 |
| 21 | K | | 0.04 | 0.12 | 0.71 | | | 0.13 | 0.65 | 0.06 | 0.50 | 0.10 | 0.44 | 0.03 | 0.37 | 1.97 | 0.20 | | | 0.35 |
| 22 | L | 1.08 | 0.10 | 2.39 | >6.23 | >7.35 | 0.18 | 0.43 | 2.31 | | >2.09 | 4.82 | 0.74 | 5.93 | >4.08 | | | | 0.25 | 1.41 |
| 23 | V | 1.05 | 0.23 | 1.03 | 6.30 | 6.12 | 0.57 | 1.38 | | 1.44 | 1.33 | | 0.31 | 2.13 | >1.51 | | 0.24 | | >6.57 | |
| 24 | C | | | | | | | | | | | | | | | | | | | |
| 25 | S | 2.58 | | >2.37 | 2.83 | >9.1 | >1.72 | >1.27 | >7.64 | 2.97 | >0.82 | | 5.01 | 2.46 | >0.83 | | | 0.12 | 0.03 | |
| 26 | R | 1.88 | | >6.3 | >5.81 | | | >0.61 | 0.40 | | | | >20.73 | 3.62 | >1.78 | >3.19 | | 1.05 | >3.66 | |
| 27 | K | | 0.04 | >20.3 | >10.22 | >10.57 | 0.10 | | >0.92 | 0.10 | >17.89 | >13.37 | | 0.32 | >2.95 | | 0.25 | >3.86 | >6.79 | |
| 28 | T | >6.64 | | | | 7.71 | | >0.05 | 1.08 | | 1.10 | | | | | 0.43 | >4.53 | | | |
| 29 | R | 0.35 | | 0.37 | >9.59 | >13.31 | >0.13 | | 0.41 | >2.59 | >14.53 | | | 1.59 | 1.03 | >0.13 | >0.9 | >0.71 | | |
| 30 | W | >2.95 | >0.49 | >10.14 | >1 | >0.37 | 36.60 | >1 | | >1.66 | >54.93 | >50.86 | >43.5 | >3.12 | >1.8 | >0.5 | >3.2 | >1.41 | >1.07 | |
| 31 | C | | | | | | | | | | | | | | | | | | | |
| 32 | K | | | >4.57 | | >1.94 | | >0.81 | >7.67 | >3.5 | >10.76 | >11.85 | >5.6 | >3.6 | >7.3 | >5.95 | >4.56 | >4.56 | >5.62 | |
| 33 | Y | 3.26 | | 2.29 | | | | >14.83 | 0.26 | | | 14.40 | 0.70 | 6.79 | 3.26 | 0.98 | | 0.30 | >8.03 | |
| 34 | Q | 0.17 | 0.08 | | 3.49 | >0.85 | 0.15 | >0.06 | 6.87 | | | 0.08 | 0.17 | 1.20 | 0.57 | 0.56 | 0.05 | 0.20 | >6.09 | |
| 35 | I | | >0.95 | >4.91 | >5.17 | >0.06 | >10.24 | | >7.67 | | | >14.59 | >12 | | 3.45 | | 0.82 | | | |
| 36 | G | 0.53 | 0.58 | 0.47 | >11.24 | >5.35 | 1.41 | >0.55 | 0.16 | >3.21 | 10.40 | | >2.85 | | 1.05 | >0.87 | 0.53 | 1.42 | 0.44 | |
| 37 | K | | 0.28 | 0.31 | 0.20 | >0.65 | 0.82 | 0.13 | 0.23 | 0.97 | >17.65 | 10.30 | 2.02 | 0.12 | | 0.63 | | 0.50 | 2.00 | |

*FIG. 2B*

| Reference huwentoxin-IV residues | | | Substitution | | | | | | | | | | | | | | | | | | | Residue substitutions in variants with increased selectivity | Residue substitutions in variants with increased potency and selectivity |
|---|---|---|---|---|---|---|---|---|---|---|---|---|---|---|---|---|---|---|---|---|---|---|---|
| | | | Basic | | | Acidic | | Aromatic | | | Uncharged Polar | | | | Nonpolar | | | | | | | | |
| | | | K | R | H | D | E | Y | F | W | N | Q | S | T | G | A | V | L | I | P | M | | |
| | | G | | | | | | | | | | | | | | | | | | | | | |
| | | P | | | | | | | | | | | | | | | | | | | | | |
| | 1 | E | 0.25 | 0.38 | 0.44 | 1.35 | | 0.41 | 0.54 | | 1.30 | 0.37 | 0.47 | 0.50 | 0.29 | | inact | 0.27 | 0.43 | 0.72 | | | |
| | 2 | C | | | | | | | | | | | | | | | | | | | | | |
| | 3 | L | | 0.43 | | 1.50 | 0.93 | | 1.83 | 1.09 | 0.64 | | 2.63 | 0.28 | | 1.22 | 0.55 | | | 0.30 | | | |
| | 4 | E | | 0.91 | 1.28 | 1.60 | | 0.59 | | | 1.10 | 1.20 | | | | | | 1.28 | 0.92 | 1.37 | | | |
| | 5 | I | | inact | 0.54 | | inact | 1.66 | | 0.52 | 0.51 | | | 0.58 | | 0.71 | 0.72 | | | 0.27 | | | |
| | 6 | F | | | inact | inact | | | | | inact | | inact | inact | | 1.01 | >1.037 | 3.94 | | | 6.07 | VM | |
| | 7 | K | | 0.51 | | | 0.88 | | | 1.36 | 0.52 | >0.891 | 0.61 | 0.22 | | | | | | | | Q | Q |
| | 8 | A | | 0.47 | | | 2.33 | 0.50 | 0.79 | 0.84 | | | 0.39 | | | | 0.93 | | | | | | |
| | 9 | C | | | | | | | | | | | | | | | | | | | | | |
| | 10 | N | 0.89 | 0.84 | | 1.68 | 1.05 | 0.48 | 1.22 | 0.84 | | 0.59 | 0.84 | | | | 1.14 | | | | | | |
| | 11 | P | 0.46 | >0.872 | | | 1.23 | | 0.71 | 0.82 | | 0.66 | 1.61 | | | | 1.44 | | | | | R | |
| | 12 | S | | 1.38 | 0.92 | | 2.12 | | 1.22 | 0.06 | | 0.29 | | | | | 0.93 | | | | | | |
| | 13 | N | 1.60 | 0.26 | 4.36 | 4.18 | 2.46 | 2.03 | | 5.15 | | 7.67 | >3.740 | 1.88 | 19.79 | 2.93 | 4.24 | 4.84 | 6.70 | >3.219 | | WQSGIP | WQSGIP |
| | 14 | D | 2.73 | 1.08 | 4.82 | | 4.88 | inact | | 4.81 | | 8.36 | >4.101 | | >7.154 | 0.45 | 2.42 | >2.475 | inact | 9.72 | | KQSGLP | P |
| | 15 | Q | 1.22 | 1.22 | 1.43 | 6.17 | 9.60 | 2.71 | | 7.44 | 3.44 | | 3.84 | 2.46 | | 3.67 | 6.58 | 5.31 | | 13.23 | | DEWVLP | DEWL |
| | 16 | C | | | | | | | | | | | | | | | | | | | | | |
| | 17 | C | | | | | | | | | | | | | | | | | | | | | |

FIG. 3A

| Reference huwentoxin-IV residue | | Substitution | | | | | | | | | | | | | | | | | | | Residue substitutions in variants with increased selectivity | Residue substitutions in variants with increased potency and selectivity |
|---|---|---|---|---|---|---|---|---|---|---|---|---|---|---|---|---|---|---|---|---|---|---|
| | | Basic | | | Acidic | | Aromatic | | | Uncharged Polar | | | | Nonpolar | | | | | | | | |
| | | K | R | H | D | E | Y | F | W | N | Q | S | T | G | A | V | L | I | P | M | | |
| 18 | K | | 1.39 | 1.95 | | 2.31 | 1.81 | 9.56 | 5.21 | | 5.58 | 3.34 | 2.11 | 4.14 | 0.31 | | 4.65 | 3.24 | 10.61 | | FWQP | Q |
| 19 | S | 4.71 | 1.05 | | | | 2.06 | 3.03 | | 2.08 | 18.39 | | | 1.22 | 1.58 | 1.70 | 2.89 | 2.17 | 3.09 | | Q | Q |
| 20 | S | | 1.09 | 2.40 | 1.97 | 0.82 | 1.81 | | 6.81 | 2.81 | 3.67 | | 2.10 | 0.84 | | 5.60 | | 1.76 | 3.31 | 0.83 | WV | WV |
| 21 | K | | 2.00 | 3.01 | 2.38 | | | 3.33 | 8.52 | 1.39 | 4.28 | 1.53 | 1.71 | 0.19 | 2.31 | 1.30 | 2.15 | | | 0.96 | W | W |
| 22 | L | 4.50 | 2.06 | 3.33 | inact | >3.769 | 1.20 | 2.35 | 7.75 | | >0.774 | 4.23 | 2.18 | 4.30 | >7.846 | | | | 1.76 | 1.27 | EWQA | W |
| 23 | V | 3.49 | 1.52 | 2.61 | 1.34 | 2.11 | 1.47 | 2.99 | | 2.98 | 2.48 | | 3.57 | 2.13 | >13.24 | | 1.79 | | inact | | A | A |
| 24 | C | | | | | | | | | | | | | | | | | | | | C | |
| 25 | S | 4.23 | | inact | 1.52 | inact | inact | inact | inact | 4.24 | inact | | 1.63 | 0.66 | inact | | | 4.55 | 0.03 | | | |
| 26 | R | 9.53 | | >8.467 | >4.611 | | | inact | 5.64 | | | | >10.74 | 12.64 | >10.78 | >7.857 | | 5.20 | >1.989 | | KHDWTGAVIP | KWGAI |
| 27 | K | | 0.13 | >5.024 | inact | inact | 0.77 | | >14.74 | 0.06 | inact | inact | | 0.21 | >11.56 | | 0.25 | >4.587 | >0.622 | | HWAIP | WA |
| 28 | T | >1.227 | | | | 0.62 | | inact | 0.38 | | 1.87 | | | | | 2.06 | >0.796 | | | | KL | |
| 29 | R | 1.81 | | 5.75 | >10.14 | inact | inact | | 6.87 | >17.03 | inact | | | 7.67 | 3.99 | inact | >3.125 | inact | | | HDWNGL | HWNGL |
| 30 | W | >28.64 | inact | inact | inact | inact | 21.66 | inact | | inact | inact | inact | inact | inact | inact | inact | inact | inact | inact | | KY | K |
| 31 | C | | | | | | | | | | | | | | | | | | | | | |
| 32 | K | | | inact | | inact | | inact | >0.841 | inact | inact | inact | inact | inact | >0.823 | inact | inact | inact | inact | | WA | |
| 33 | Y | 0.76 | | 1.56 | | | | inact | 12.70 | | | 2.09 | 9.76 | 1.56 | 2.94 | 2.89 | | 2.88 | inact | | WT | WT |
| 34 | Q | 1.06 | 1.23 | | 0.59 | >1.808 | 2.01 | >4.379 | 5.20 | | | 4.41 | 3.36 | 5.77 | 5.40 | 2.24 | 1.18 | 0.71 | inact | | EFWGA | FGA |
| 35 | I | | inact | >1.020 | inact | inact | inact | | inact | | | inact | inact | | 1.34 | | 1.05 | | | | H | |
| 36 | G | 5.87 | 3.79 | 2.20 | inact | inact | inact | >2.972 | 1.87 | inact | 5.98 | | >4.905 | | 2.26 | >3.936 | 7.16 | 22.71 | 0.82 | | KFQTVLI | KFVLI |
| 37 | K | | 10.70 | 3.14 | 0.17 | inact | 4.37 | 2.60 | 2.04 | 3.54 | >3.238 | 7.01 | 5.42 | 1.40 | | 3.34 | | 1.34 | 7.72 | | RQSTP | RP |

**FIG. 3B**

| Peptide ID | Substitution | SEQ ID NO: | Sequence | $IC_{50}$ (Nav1.7) μM | $IC_{50}$ (Nav1.2) μM | Selectivity |
|---|---|---|---|---|---|---|
| HwTX-IV | Parent Synthetic | 1 | ECLEIFKACNPSNDQCCKSSKLVCSRKTRWCKYQI-NH2 | 0.04 | 0.07 | 1.66 |
| NV1D7 | Parent Recombinant | 2 | GPECLEIFKACNPSNDQCCKSSKLVCSRKTRWCKYQIGK | 0.16 | 0.34 | 2.14 |
| | | | | | | |
| NV1D2163 | E1N,E4R,Y33W,Q34S | 3 | GPNCLRIFKACNPSNDQCCKSSKLVCSRKTRWCKWSIGK | 0.01 | 0.02 | 1.58 |
| NV1D2142 | E1N,E4R,Q34S | 4 | GPNCLRIFKACNPSNDQCCKSSKLVCSRKTRWCKYSIGK | 0.01 | 0.02 | 1.77 |
| NV1D2161 | E1N,E4R,R26K,Q34S | 5 | GPNCLRIFKACNPSNDQCCKSSKLVCSKKTRWCKYSIGK | 0.01 | 0.11 | 8.33 |
| NV1D1780 | Q34F | 6 | GPECLEIFKACNPSNDQCCKSSKLVCSRKTRWCKYFIGK | 0.01 | >0.06 | >4.38 |
| NV1D2148 | E1N,Y33W,G36I | 7 | GPNCLEIFKACNPSNDQCCKSSKLVCSRKTRWCKWQIIK | 0.02 | 0.25 | 16.49 |
| NV1D2153 | E4R,Y33W,Q34S | 8 | GPECLRIFKACNPSNDQCCKSSKLVCSRKTRWCKWSIGK | 0.02 | 0.02 | 1.11 |
| NV1D1376 | E4Q | 9 | GPECLQIFKACNPSNDQCCKSSKLVCSRKTRWCKYQIGK | 0.02 | 0.02 | 1.20 |
| NV1D1783 | Q34S | 10 | GPECLEIFKACNPSNDQCCKSSKLVCSRKTRWCKYSIGK | 0.02 | 0.08 | 4.41 |
| NV1D1546 | Q15N | 11 | GPECLEIFKACNPSNDNCCKSSKLVCSRKTRWCKYQIGK | 0.02 | 0.06 | 3.44 |
| NV1D2143 | E1N,E4R,G36I | 12 | GPNCLRIFKACNPSNDQCCKSSKLVCSRKTRWCKYQIIK | 0.02 | 0.15 | 8.21 |
| NV1D1760 | Y33W | 13 | GPECLEIFKACNPSNDQCCKSSKLVCSRKTRWCKWQIGK | 0.02 | 0.26 | 12.70 |
| NV1D1608 | K21R | 14 | GPECLEIFKACNPSNDQCCKSSRLVCSRKTRWCKYQIGK | 0.02 | 0.04 | 2.00 |
| NV1D1448 | A8R | 15 | GPECLEIFKRCNPSNDQCCKSSKLVCSRKTRWCKYQIGK | 0.02 | 0.01 | 0.47 |
| NV1D2177 | E1N,E4R,R26K,Q34S,G36I | 16 | GPNCLRIFKACNPSNDQCCKSSKLVCSKKTRWCKYSIIK | 0.02 | 0.32 | 14.22 |
| NV1D2164 | E1N,E4R,Y33W,G36I | 17 | GPNCLRIFKACNPSNDQCCKSSKLVCSRKTRWCKWQIIK | 0.02 | 0.05 | 2.32 |
| NV1D1383 | E4R | 18 | GPECLRIFKACNPSNDQCCKSSKLVCSRKTRWCKYQIGK | 0.02 | 0.02 | 0.91 |
| NV1D2147 | E1N,Y33W,Q34S | 19 | GPNCLEIFKACNPSNDQCCKSSKLVCSRKTRWCKWSIGK | 0.02 | 0.01 | 0.57 |
| NV1D1594 | S20N | 20 | GPECLEIFKACNPSNDQCCKSNKLVCSRKTRWCKYQIGK | 0.02 | 0.07 | 2.81 |
| NV1D1569 | S19Q | 21 | GPECLEIFKACNPSNDQCCKQSKLVCSRKTRWCKYQIGK | 0.02 | 0.46 | 18.39 |
| NV1D2125 | E1N,E4R | 22 | GPNCLRIFKACNPSNDQCCKSSKLVCSRKTRWCKYQIGK | 0.03 | 0.03 | 1.10 |

FIG. 4(1)

| | | | | | | |
|---|---|---|---|---|---|---|
| NV1D93 | N13A | 23 | GPECLEIFKACNPSADQCCKSSKLVCSRKTRWCKYQIGK | 0.03 | 0.07 | 2.93 |
| NV1D1660 | S25I | 24 | GPECLEIFKACNPSNDQCCKSSKLVCIRKTRWCKYQIGK | 0.03 | 0.12 | 4.55 |
| NV1D1832 | K37R | 25 | GPECLEIFKACNPSNDQCCKSSKLVCSRKTRWCKYQIGR | 0.03 | 0.28 | 10.70 |
| NV1D1385 | E4N | 26 | GPECLNIFKACNPSNDQCCKSSKLVCSRKTRWCKYQIGK | 0.03 | 0.03 | 1.10 |
| NV1D1581 | S19P | 27 | GPECLEIFKACNPSNDQCCKPSKLVCSRKTRWCKYQIGK | 0.03 | 0.09 | 3.09 |
| NV1D1595 | S20D | 28 | GPECLEIFKACNPSNDQCCKSDKLVCSRKTRWCKYQIGK | 0.04 | 0.07 | 1.97 |
| NV1D1592 | S20R | 29 | GPECLEIFKACNPSNDQCCKSRKLVCSRKTRWCKYQIGK | 0.04 | 0.04 | 1.09 |
| NV1D1544 | Q15R | 30 | GPECLEIFKACNPSNDRCCKSSKLVCSRKTRWCKYQIGK | 0.04 | 0.05 | 1.22 |
| NV1D1790 | Q34L | 31 | GPECLEIFKACNPSNDQCCKSSKLVCSRKTRWCKYLIGK | 0.04 | 0.05 | 1.18 |
| NV1D2155 | E4R,Q34S,G36I | 32 | GPECLRIFKACNPSNDQCCKSSKLVCSRKTRWCKYSIIK | 0.04 | 0.04 | 1.00 |
| NV1D1604 | K21F | 33 | GPECLEIFKACNPSNDQCCKSSFLVCSRKTRWCKYQIGK | 0.04 | 0.13 | 3.33 |
| NV1D1606 | K21H | 34 | GPECLEIFKACNPSNDQCCKSSHLVCSRKTRWCKYQIGK | 0.04 | 0.12 | 3.01 |
| NV1D1597 | S20P | 35 | GPECLEIFKACNPSNDQCCKSPKLVCSRKTRWCKYQIGK | 0.04 | 0.13 | 3.31 |
| NV1D2162 | E1N,E4R,R26K,G36I | 36 | GPNCLRIFKACNPSNDQCCKSSKLVCSKKTRWCKYQIIK | 0.04 | 0.17 | 4.06 |
| NV1D1610 | K21N | 37 | GPECLEIFKACNPSNDQCCKSSNLVCSRKTRWCKYQIGK | 0.04 | 0.06 | 1.39 |
| NV1D1557 | K18Y | 38 | GPECLEIFKACNPSNDQCCYSSKLVCSRKTRWCKYQIGK | 0.04 | 0.08 | 1.81 |
| NV1D1397 | I5Y | 39 | GPECLEYFKACNPSNDQCCKSSKLVCSRKTRWCKYQIGK | 0.04 | 0.07 | 1.66 |
| NV1D2165 | E1N,E4R,Q34S,G36I | 40 | GPNCLRIFKACNPSNDQCCKSSKLVCSRKTRWCKYSIIK | 0.04 | 0.09 | 2.07 |
| NV1D1576 | S19R | 41 | GPECLEIFKACNPSNDQCCKRSKLVCSRKTRWCKYQIGK | 0.05 | 0.05 | 1.05 |
| NV1D1356 | E1P | 42 | GPPCLEIFKACNPSNDQCCKSSKLVCSRKTRWCKYQIGK | 0.05 | 0.03 | 0.72 |
| NV1D1625 | L22R | 43 | GPECLEIFKACNPSNDQCCKSSKRVCSRKTRWCKYQIGK | 0.05 | 0.10 | 2.06 |
| NV1D1381 | E4H | 44 | GPECLHIFKACNPSNDQCCKSSKLVCSRKTRWCKYQIGK | 0.05 | 0.06 | 1.28 |
| NV1D2130 | E4R,R26K | 45 | GPECLRIFKACNPSNDQCCKSSKLVCSKKTRWCKYQIGK | 0.05 | 0.25 | 5.15 |
| NV1D2134 | R26K,Y33W | 46 | GPECLEIFKACNPSNDQCCKSSKLVCSKKTRWCKWQIGK | 0.05 | 0.77 | 15.76 |
| NV1D1828 | K37F | 47 | GPECLEIFKACNPSNDQCCKSSKLVCSRKTRWCKYQIGF | 0.05 | 0.13 | 2.60 |
| NV1D2171 | E4R,R26K,Y33W,G36I | 48 | GPECLRIFKACNPSNDQCCKSSKLVCSKKTRWCKWQIIK | 0.05 | 0.34 | 6.63 |
| NV1D1785 | Q34T | 49 | GPECLEIFKACNPSNDQCCKSSKLVCSRKTRWCKYTIGK | 0.05 | 0.17 | 3.36 |

FIG. 4(2)

| | | | | | | |
|---|---|---|---|---|---|---|
| NV1D1351 | E1R | 50 | GPRCLEIFKACNPSNDQCCKSSKLVCSRKTRWCKYQIGK | 0.05 | 0.02 | 0.38 |
| NV1D1560 | K18R | 51 | GPECLEIFKACNPSNDQCCRSSKLVCSRKTRWCKYQIGK | 0.06 | 0.08 | 1.39 |
| NV1D2133 | E4R,G36I | 52 | GPECLRIFKACNPSNDQCCKSSKLVCSRKTRWCKYQIIK | 0.06 | 0.24 | 4.23 |
| NV1D1590 | S20Y | 53 | GPECLEIFKACNPSNDQCCKSYKLVCSRKTRWCKYQIGK | 0.06 | 0.10 | 1.81 |
| NV1D1441 | A8E | 54 | GPECLEIFKECNPSNDQCCKSSKLVCSRKTRWCKYQIGK | 0.06 | 0.14 | 2.33 |
| NV1D1712 | R29W | 55 | GPECLEIFKACNPSNDQCCKSSKLVCSRKTWWCKYQIGK | 0.06 | 0.41 | 6.87 |
| NV1D1423 | K7W | 56 | GPECLEIFWACNPSNDQCCKSSKLVCSRKTRWCKYQIGK | 0.06 | 0.08 | 1.36 |
| NV1D1680 | K27W | 57 | GPECLEIFKACNPSNDQCCKSSKLVCSRWTRWCKYQIGK | 0.06 | >0.92 | >14.75 |
| NV1D1821 | G36I | 58 | GPECLEIFKACNPSNDQCCKSSKLVCSRKTRWCKYQIIK | 0.06 | 1.42 | 22.71 |
| NV1D1345 | E1K | 59 | GPKCLEIFKACNPSNDQCCKSSKLVCSRKTRWCKYQIGK | 0.06 | 0.02 | 0.25 |
| NV1D1719 | R29H | 60 | GPECLEIFKACNPSNDQCCKSSKLVCSRKTHWCKYQIGK | 0.06 | 0.37 | 5.75 |
| NV1D1505 | N13Q | 61 | GPECLEIFKACNPSQDQCCKSSKLVCSRKTRWCKYQIGK | 0.06 | 0.49 | 7.67 |
| NV1D1380 | E4Y | 62 | GPECLYIFKACNPSNDQCCKSSKLVCSRKTRWCKYQIGK | 0.06 | 0.04 | 0.59 |
| NV1D1784 | Q34R | 63 | GPECLEIFKACNPSNDQCCKSSKLVCSRKTRWCKYRIGK | 0.06 | 0.08 | 1.23 |
| NV1D1580 | S19I | 64 | GPECLEIFKACNPSNDQCCKISKLVCSRKTRWCKYQIGK | 0.07 | 0.14 | 2.17 |
| NV1D2150 | E4R,R26K,Y33W | 65 | GPECLRIFKACNPSNDQCCKSSKLVCSKKTRWCKWQIGK | 0.07 | 0.04 | 0.62 |
| NV1D1582 | S19L | 66 | GPECLEIFKACNPSNDQCCKLSKLVCSRKTRWCKYQIGK | 0.07 | 0.19 | 2.89 |
| NV1D1353 | E1N | 67 | GPNCLEIFKACNPSNDQCCKSSKLVCSRKTRWCKYQIGK | 0.07 | 0.09 | 1.30 |
| NV1D1607 | K21S | 68 | GPECLEIFKACNPSNDQCCKSSSLVCSRKTRWCKYQIGK | 0.07 | 0.10 | 1.53 |
| NV1D1431 | K7R | 69 | GPECLEIFRACNPSNDQCCKSSKLVCSRKTRWCKYQIGK | 0.07 | 0.03 | 0.51 |
| NV1D1367 | L3S | 70 | GPECSEIFKACNPSNDQCCKSSKLVCSRKTRWCKYQIGK | 0.07 | 0.18 | 2.63 |
| NV1D1349 | E1H | 71 | GPHCLEIFKACNPSNDQCCKSSKLVCSRKTRWCKYQIGK | 0.07 | 0.03 | 0.44 |
| NV1D1664 | R26W | 72 | GPECLEIFKACNPSNDQCCKSSKLVCSWKTRWCKYQIGK | 0.07 | 0.40 | 5.64 |
| NV1D1355 | E1I | 73 | GPICLEIFKACNPSNDQCCKSSKLVCSRKTRWCKYQIGK | 0.07 | 0.03 | 0.43 |
| NV1D1504 | N13W | 74 | GPECLEIFKACNPSWDQCCKSSKLVCSRKTRWCKYQIGK | 0.07 | 0.37 | 5.15 |
| NV1D2152 | E4R,R26K,G36I | 75 | GPECLRIFKACNPSNDQCCKSSKLVCSKKTRWCKYQIIK | 0.07 | 0.33 | 4.60 |

FIG. 4(3)

| | | | | | | |
|---|---|---|---|---|---|---|
| NV1D1769 | Y33T | 76 | GPECLEIFKACNPSNDQCCKSSKLVCSRKTRWCKTQIGK | 0.07 | 0.70 | 9.76 |
| NV1D1386 | E4D | 77 | GPECLDIFKACNPSNDQCCKSSKLVCSRKTRWCKYQIGK | 0.07 | 0.12 | 1.60 |
| NV1D1823 | G36L | 78 | GPECLEIFKACNPSNDQCCKSSKLVCSRKTRWCKYQILK | 0.07 | 0.53 | 7.16 |
| NV1D1541 | Q15Y | 79 | GPECLEIFKACNPSNDYCCKSSKLVCSRKTRWCKYQIGK | 0.07 | 0.20 | 2.71 |
| NV1D1781 | Q34Y | 80 | GPECLEIFKACNPSNDQCCKSSKLVCSRKTRWCKYYIGK | 0.08 | 0.15 | 2.01 |
| NV1D1571 | S19K | 81 | GPECLEIFKACNPSNDQCCKKSKLVCSRKTRWCKYQIGK | 0.08 | 0.36 | 4.71 |
| NV1D1600 | K21W | 82 | GPECLEIFKACNPSNDQCCKSSWLVCSRKTRWCKYQIGK | 0.08 | 0.65 | 8.52 |
| NV1D2169 | E1N,Y33W,Q34S,G36I | 83 | GPNCLEIFKACNPSNDQCCKSSKLVCSRKTRWCKWSIIK | 0.08 | 0.50 | 6.38 |
| NV1D2175 | E1N,E4R,R26K,Y33W,Q34S | 84 | GPNCLRIFKACNPSNDQCCKSSKLVCSKKTRWCKWSIGK | 0.08 | 0.18 | 2.25 |
| NV1D1389 | E4L | 85 | GPECLLIFKACNPSNDQCCKSSKLVCSRKTRWCKYQIGK | 0.08 | 0.11 | 1.28 |
| NV1D1358 | E1G | 86 | GPGCLEIFKACNPSNDQCCKSSKLVCSRKTRWCKYQIGK | 0.08 | 0.02 | 0.29 |
| NV1D1545 | Q15T | 87 | GPECLEIFKACNPSNDTCCKSSKLVCSRKTRWCKYQIGK | 0.08 | 0.21 | 2.46 |
| NV1D1572 | S19V | 88 | GPECLEIFKACNPSNDQCCKVSKLVCSRKTRWCKYQIGK | 0.09 | 0.15 | 1.70 |
| NV1D1808 | G36W | 89 | GPECLEIFKACNPSNDQCCKSSKLVCSRKTRWCKYQIWK | 0.09 | 0.16 | 1.87 |
| NV1D1641 | V23T | 90 | GPECLEIFKACNPSNDQCCKSSKLTCSRKTRWCKYQIGK | 0.09 | 0.31 | 3.57 |
| NV1D2160 | E1N,E4R,R26K,Y33W | 91 | GPNCLRIFKACNPSNDQCCKSSKLVCSKKTRWCKWQIGK | 0.09 | 0.19 | 2.24 |
| NV1D1839 | K37G | 92 | GPECLEIFKACNPSNDQCCKSSKLVCSRKTRWCKYQIGG | 0.09 | 0.12 | 1.40 |
| NV1D1344 | E1Q | 93 | GPQCLEIFKACNPSNDQCCKSSKLVCSRKTRWCKYQIGK | 0.09 | 0.03 | 0.37 |
| NV1D2154 | E4R,Y33W,G36I | 94 | GPECLRIFKACNPSNDQCCKSSKLVCSRKTRWCKWQIIK | 0.09 | 0.16 | 1.80 |
| NV1D2173 | E4R,Y33W,Q34S,G36I | 95 | GPECLRIFKACNPSNDQCCKSSKLVCSRKTRWCKWSIIK | 0.09 | 0.18 | 2.03 |
| NV1D1811 | G36K | 96 | GPECLEIFKACNPSNDQCCKSSKLVCSRKTRWCKYQIKK | 0.09 | 0.53 | 5.87 |
| NV1D1578 | S19N | 97 | GPECLEIFKACNPSNDQCCKNSKLVCSRKTRWCKYQIGK | 0.09 | 0.19 | 2.08 |
| NV1D1593 | S20T | 98 | GPECLEIFKACNPSNDQCCKSTKLVCSRKTRWCKYQIGK | 0.09 | 0.19 | 2.10 |
| NV1D1614 | K21L | 99 | GPECLEIFKACNPSNDQCCKSSLLVCSRKTRWCKYQIGK | 0.09 | 0.20 | 2.15 |
| NV1D1591 | S20H | 100 | GPECLEIFKACNPSNDQCCKSHKLVCSRKTRWCKYQIGK | 0.09 | 0.23 | 2.40 |
| NV1D95 | Q15A | 101 | GPECLEIFKACNPSNDACCKSSKLVCSRKTRWCKYQIGK | 0.10 | 0.35 | 3.67 |

FIG. 4(4)

| | | | | | | |
|---|---|---|---|---|---|---|
| NV1D2128 | E1N,Q34S | 102 | GPNCLEIFKACNPSNDQCCKSSKLVCSRKTRWCKYSIGK | 0.10 | 0.25 | 2.58 |
| NV1D1830 | K37H | 103 | GPECLEIFKACNPSNDQCCKSSKLVCSRKTRWCKYQIGH | 0.10 | 0.31 | 3.14 |
| NV1D1574 | S19Y | 104 | GPECLEIFKACNPSNDQCCKYSKLVCSRKTRWCKYQIGK | 0.10 | 0.20 | 2.06 |
| NV1D1564 | K18I | 105 | GPECLEIFKACNPSNDQCCISSKLVCSRKTRWCKYQIGK | 0.10 | 0.32 | 3.24 |
| NV1D1573 | S19F | 106 | GPECLEIFKACNPSNDQCCKFSKLVCSRKTRWCKYQIGK | 0.10 | 0.31 | 3.03 |
| NV1D1514 | N13T | 107 | GPECLEIFKACNPSTDQCCKSSKLVCSRKTRWCKYQIGK | 0.10 | 0.19 | 1.88 |
| NV1D1515 | N13D | 108 | GPECLEIFKACNPSDDQCCKSSKLVCSRKTRWCKYQIGK | 0.10 | 0.43 | 4.18 |
| NV1D1730 | W30K | 109 | GPECLEIFKACNPSNDQCCKSSKLVCSRKTRKCKYQIGK | 0.10 | >2.95 | >28.65 |
| NV1D1772 | Y33I | 110 | GPECLEIFKACNPSNDQCCKSSKLVCSRKTRWCKIQIGK | 0.10 | 0.30 | 2.88 |
| NV1D111 | Q34A | 111 | GPECLEIFKACNPSNDQCCKSSKLVCSRKTRWCKYAIGK | 0.11 | 0.57 | 5.40 |
| NV1D2149 | E1N,Q34S,G36I | 112 | GPNCLEIFKACNPSNDQCCKSSKLVCSRKTRWCKYSIIK | 0.11 | 0.28 | 2.67 |
| NV1D1536 | Q15W | 113 | GPECLEIFKACNPSNDWCCKSSKLVCSRKTRWCKYQIGK | 0.11 | 0.79 | 7.44 |
| NV1D1357 | E1L | 114 | GPLCLEIFKACNPSNDQCCKSSKLVCSRKTRWCKYQIGK | 0.11 | 0.03 | 0.27 |
| NV1D1350 | E1S | 115 | GPSCLEIFKACNPSNDQCCKSSKLVCSRKTRWCKYQIGK | 0.11 | 0.05 | 0.47 |
| NV1D1388 | E4P | 116 | GPECLPIFKACNPSNDQCCKSSKLVCSRKTRWCKYQIGK | 0.11 | 0.15 | 1.37 |
| NV1D1561 | K18T | 117 | GPECLEIFKACNPSNDQCCTSSKLVCSRKTRWCKYQIGK | 0.11 | 0.23 | 2.11 |
| NV1D1445 | A8Y | 118 | GPECLEIFKYCNPSNDQCCKSSKLVCSRKTRWCKYQIGK | 0.11 | 0.05 | 0.50 |
| NV1D1370 | L3N | 119 | GPECNEIFKACNPSNDQCCKSSKLVCSRKTRWCKYQIGK | 0.11 | 0.07 | 0.64 |
| NV1D1465 | N10R | 120 | GPECLEIFKACRPSNDQCCKSSKLVCSRKTRWCKYQIGK | 0.11 | 0.09 | 0.84 |
| NV1D1824 | K37W | 121 | GPECLEIFKACNPSNDQCCKSSKLVCSRKTRWCKYQIGW | 0.11 | 0.23 | 2.04 |
| NV1D102 | V23A | 122 | GPECLEIFKACNPSNDQCCKSSKLACSRKTRWCKYQIGK | 0.11 | >1.51 | >13.25 |
| NV1D1510 | N13Y | 123 | GPECLEIFKACNPSYDQCCKSSKLVCSRKTRWCKYQIGK | 0.11 | 0.23 | 2.03 |
| NV1D1601 | K21Q | 124 | GPECLEIFKACNPSNDQCCKSSQLVCSRKTRWCKYQIGK | 0.12 | 0.50 | 4.28 |
| NV1D98 | S19A | 125 | GPECLEIFKACNPSNDQCCKASKLVCSRKTRWCKYQIGK | 0.12 | 0.19 | 1.58 |
| NV1D1596 | S20I | 126 | GPECLEIFKACNPSNDQCCKSIKLVCSRKTRWCKYQIGK | 0.12 | 0.21 | 1.76 |
| NV1D2159 | Y33W,Q34S,G36I | 127 | GPECLEIFKACNPSNDQCCKSSKLVCSRKTRWCKWSIIK | 0.12 | 0.68 | 5.70 |

*FIG. 4(5)*

| | | | | | | |
|---|---|---|---|---|---|---|
| NV1D1352 | E1T | 128 | GPTCLEIFKACNPSNDQCCKSSKLVCSRKTRWCKYQIGK | 0.12 | 0.06 | 0.50 |
| NV1D2166 | E1N,R26K,Y33W,Q34S | 129 | GPNCLEIFKACNPSNDQCCKSSKLVCSKKTRWCKWSIGK | 0.13 | 0.17 | 1.32 |
| NV1D1496 | S12R | 130 | GPECLEIFKACNPRNDQCCKSSKLVCSRKTRWCKYQIGK | 0.13 | 0.17 | 1.38 |
| NV1D1584 | S20W | 131 | GPECLEIFKACNPSNDQCCKSWKLVCSRKTRWCKYQIGK | 0.13 | 0.87 | 6.81 |
| NV1D2141 | E1N,E4R,Y33W | 132 | GPNCLRIFKACNPSNDQCCKSSKLVCSRKTRWCKWQIGK | 0.13 | 0.13 | 1.03 |
| NV1D1685 | K27Y | 133 | GPECLEIFKACNPSNDQCCKSSKLVCSRYTRWCKYQIGK | 0.13 | 0.10 | 0.77 |
| NV1D1588 | S20V | 134 | GPECLEIFKACNPSNDQCCKSVKLVCSRKTRWCKYQIGK | 0.13 | 0.73 | 5.60 |
| NV1D1646 | V23L | 135 | GPECLEIFKACNPSNDQCCKSSKLLCSRKTRWCKYQIGK | 0.13 | 0.24 | 1.79 |
| NV1D2139 | Q34S,G36I | 136 | GPECLEIFKACNPSNDQCCKSSKLVCSRKTRWCKYSIIK | 0.13 | 0.13 | 1.00 |
| NV1D2218 | N13Q,R29K,K37R | 137 | GPECLEIFKACNPSQDQCCKSSKLVCSRKTKWCKYQIGR | 0.13 | 0.73 | 5.52 |
| NV1D2156 | R26K,Y33W,Q34S | 138 | GPECLEIFKACNPSNDQCCKSSKLVCSKKTRWCKWSIGK | 0.13 | 0.32 | 2.38 |
| NV1D1347 | E1F | 139 | GPFCLEIFKACNPSNDQCCKSSKLVCSRKTRWCKYQIGK | 0.14 | 0.07 | 0.54 |
| NV1D1559 | K18S | 140 | GPECLEIFKACNPSNDQCCSSSKLVCSRKTRWCKYQIGK | 0.14 | 0.46 | 3.34 |
| NV1D1553 | K18Q | 141 | GPECLEIFKACNPSNDQCCQSSKLVCSRKTRWCKYQIGK | 0.14 | 0.79 | 5.58 |
| NV1D1516 | N13I | 142 | GPECLEIFKACNPSIDQCCKSSKLVCSRKTRWCKYQIGK | 0.14 | 0.95 | 6.70 |
| NV1D1630 | L22P | 143 | GPECLEIFKACNPSNDQCCKSSKPVCSRKTRWCKYQIGK | 0.14 | 0.25 | 1.76 |
| NV1D1460 | N10V | 144 | GPECLEIFKACVPSNDQCCKSSKLVCSRKTRWCKYQIGK | 0.14 | 0.16 | 1.14 |
| NV1D1430 | K7S | 145 | GPECLEIFSACNPSNDQCCKSSKLVCSRKTRWCKYQIGK | 0.14 | 0.09 | 0.61 |
| NV1D1622 | L22Y | 146 | GPECLEIFKACNPSNDQCCKSSKYVCSRKTRWCKYQIGK | 0.15 | 0.18 | 1.20 |
| NV1D1359 | L3W | 147 | GPECWEIFKACNPSNDQCCKSSKLVCSRKTRWCKYQIGK | 0.15 | 0.16 | 1.09 |
| NV1D1567 | K18G | 148 | GPECLEIFKACNPSNDQCCGSSKLVCSRKTRWCKYQIGK | 0.15 | 0.63 | 4.14 |
| NV1D1722 | R29N | 149 | GPECLEIFKACNPSNDQCCKSSKLVCSRKTNWCKYQIGK | 0.15 | >2.59 | >17.04 |
| NV1D1817 | G36R | 150 | GPECLEIFKACNPSNDQCCKSSKLVCSRKTRWCKYQIRK | 0.15 | 0.58 | 3.79 |
| NV1D1640 | V23R | 151 | GPECLEIFKACNPSNDQCCKSSKLRCSRKTRWCKYQIGK | 0.15 | 0.23 | 1.52 |
| NV1D1566 | K18L | 152 | GPECLEIFKACNPSNDQCCLSSKLVCSRKTRWCKYQIGK | 0.16 | 0.72 | 4.65 |
| NV1D1615 | K21G | 153 | GPECLEIFKACNPSNDQCCKSSGLVCSRKTRWCKYQIGK | 0.16 | 0.03 | 0.19 |
| NV1D1778 | Q34K | 154 | GPECLEIFKACNPSNDQCCKSSKLVCSRKTRWCKYKIGK | 0.16 | 0.17 | 1.06 |

FIG. 4(6)

| | | | | | | |
|---|---|---|---|---|---|---|
| NV1D2129 | E1N,G36I | 155 | GPNCLEIFKACNPSNDQCCKSSKLVCSRKTRWCKYQIIK | 0.16 | 0.16 | 1.00 |
| NV1D1364 | I3F | 156 | GPECFEIFKACNPSNDQCCKSSKLVCSRKTRWCKYQIGK | 0.16 | 0.29 | 1.83 |
| NV1D1395 | I5V | 157 | GPECLEVFKACNPSNDQCCKSSKLVCSRKTRWCKYQIGK | 0.16 | 0.12 | 0.72 |
| NV1D2145 | E1N,R26K,Q34S | 158 | GPNCLEIFKACNPSNDQCCKSSKLVCSKKTRWCKYSIGK | 0.16 | 0.27 | 1.68 |
| NV1D1585 | S20Q | 159 | GPECLEIFKACNPSNDQCCKSQKLVCSRKTRWCKYQIGK | 0.16 | 0.59 | 3.67 |
| NV1D1354 | E1D | 160 | GPDCLEIFKACNPSNDQCCKSSKLVCSRKTRWCKYQIGK | 0.16 | 0.22 | 1.35 |
| NV1D1348 | E1Y | 161 | GPYCLEIFKACNPSNDQCCKSSKLVCSRKTRWCKYQIGK | 0.16 | 0.07 | 0.41 |
| NV1D100 | K21A | 162 | GPECLEIFKACNPSNDQCCKSSALVCSRKTRWCKYQIGK | 0.16 | 0.37 | 2.31 |
| NV1D1492 | S12V | 163 | GPECLEIFKACNPVNDQCCKSSKLVCSRKTRWCKYQIGK | 0.16 | 0.15 | 0.93 |
| NV1D104 | R26A | 164 | GPECLEIFKACNPSNDQCCKSSKLVCSAKTRWCKYQIGK | 0.17 | >1.78 | >10.79 |
| NV1D1547 | Q15D | 165 | GPECLEIFKACNPSNDDCCKSSKLVCSRKTRWCKYQIGK | 0.18 | 1.08 | 6.17 |
| NV1D1511 | N13H | 166 | GPECLEIFKACNPSHDQCCKSSKLVCSRKTRWCKYQIGK | 0.18 | 0.77 | 4.36 |
| NV1D1444 | A8F | 167 | GPECLEIFKFCNPSNDQCCKSSKLVCSRKTRWCKYQIGK | 0.18 | 0.14 | 0.79 |
| NV1D1621 | L22F | 168 | GPECLEIFKACNPSNDQCCKSSKFVCSRKTRWCKYQIGK | 0.18 | 0.43 | 2.35 |
| NV1D1813 | G36F | 169 | GPECLEIFKACNPSNDQCCKSSKLVCSRKTRWCKYQIFK | 0.19 | >0.55 | >2.98 |
| NV1D1827 | K37V | 170 | GPECLEIFKACNPSNDQCCKSSKLVCSRKTRWCKYQIGV | 0.19 | 0.63 | 3.34 |
| NV1D1829 | K37Y | 171 | GPECLEIFKACNPSNDQCCKSSKLVCSRKTRWCKYQIGY | 0.19 | 0.82 | 4.37 |
| NV1D1519 | N13G | 172 | GPECLEIFKACNPSGDQCCKSSKLVCSRKTRWCKYQIGK | 0.19 | 3.74 | 19.79 |
| NV1D2140 | E1N,E4R,R26K | 173 | GPNCLRIFKACNPSNDQCCKSSKLVCSKKTRWCKYQIGK | 0.19 | 0.29 | 1.51 |
| NV1D1715 | R29K | 174 | GPECLEIFKACNPSNDQCCKSSKLVCSRKTKWCKYQIGK | 0.20 | 0.35 | 1.81 |
| NV1D1667 | R26K | 175 | GPECLEIFKACNPSNDQCCKSSKLVCSKKTRWCKYQIGK | 0.20 | 1.88 | 9.53 |
| NV1D1464 | N10S | 176 | GPECLEIFKACSPSNDQCCKSSKLVCSRKTRWCKYQIGK | 0.20 | 0.17 | 0.84 |
| NV1D1676 | R26I | 177 | GPECLEIFKACNPSNDQCCKSSKLVCSIKTRWCKYQIGK | 0.20 | 1.05 | 5.20 |
| NV1D2158 | R26K,Q34S,G36I | 178 | GPECLEIFKACNPSNDQCCKSSKLVCSKKTRWCKYSIIK | 0.20 | 1.22 | 6.04 |
| NV1D1387 | E4I | 179 | GPECLIIFKACNPSNDQCCKSSKLVCSRKTRWCKYQIGK | 0.20 | 0.19 | 0.92 |
| NV1D1727 | R29G | 180 | GPECLEIFKACNPSNDQCCKSSKLVCSRKTGWCKYQIGK | 0.21 | 1.59 | 7.67 |
| NV1D1727 | R29G | 181 | GPECLEIFKACNPSNDQCCKSSKLVCSRKTGWCKYQIGK | 0.21 | >2.79 | >13.5 |

FIG. 4(7)

| | | | | | | |
|---|---|---|---|---|---|---|
| NV1D1791 | Q34G | 182 | GPECLEIFKACNPSNDQCCKSSKLVCSRKTRWCKYGIGK | 0.21 | 1.20 | 5.77 |
| NV1D1700 | T28V | 183 | GPECLEIFKACNPSNDQCCKSSKLVCSRKVRWCKYQIGK | 0.21 | 0.43 | 2.06 |
| NV1D1815 | G36H | 184 | GPECLEIFKACNPSNDQCCKSSKLVCSRKTRWCKYQIHK | 0.22 | 0.47 | 2.20 |
| NV1D1461 | N10F | 185 | GPECLEIFKACFPSNDQCCKSSKLVCSRKTRWCKYQIGK | 0.22 | 0.26 | 1.22 |
| NV1D1462 | N10Y | 186 | GPECLEIFKACYPSNDQCCKSSKLVCSRKTRWCKYQIGK | 0.22 | 0.10 | 0.48 |
| NV1D1812 | G36V | 187 | GPECLEIFKACNPSNDQCCKSSKLVCSRKTRWCKYQIVK | 0.22 | >0.87 | >3.94 |
| NV1D1537 | Q15E | 188 | GPECLEIFKACNPSNDECCKSSKLVCSRKTRWCKYQIGK | 0.23 | 2.16 | 9.60 |
| NV1D1565 | K18P | 189 | GPECLEIFKACNPSNDQCCPSSKLVCSRKTRWCKYQIGK | 0.23 | 2.40 | 10.61 |
| NV1D1508 | N13V | 190 | GPECLEIFKACNPSVDQCCKSSKLVCSRKTRWCKYQIGK | 0.23 | 0.98 | 4.24 |
| NV1D1476 | P11V | 191 | GPECLEIFKACNVSNDQCCKSSKLVCSRKTRWCKYQIGK | 0.23 | 0.33 | 1.44 |
| NV1D2168 | E1N,R26K,Q34S,G36I | 192 | GPNCLEIFKACNPSNDQCCKSSKLVCSKKTRWCKYSIIK | 0.24 | >7.14 | >30.32 |
| NV1D1619 | L22K | 193 | GPECLEIFKACNPSNDQCCKSSKKVCSRKTRWCKYQIGK | 0.24 | 1.08 | 4.50 |
| NV1D1493 | S12F | 194 | GPECLEIFKACNPFNDQCCKSSKLVCSRKTRWCKYQIGK | 0.24 | 0.30 | 1.22 |
| NV1D1538 | Q15K | 195 | GPECLEIFKACNPSNDKCCKSSKLVCSRKTRWCKYQIGK | 0.24 | 0.30 | 1.22 |
| NV1D2135 | R26K,Q34S | 196 | GPECLEIFKACNPSNDQCCKSSKLVCSKKTRWCKYSIGK | 0.25 | 0.52 | 2.10 |
| NV1D1779 | Q34V | 197 | GPECLEIFKACNPSNDQCCKSSKLVCSRKTRWCKYVIGK | 0.25 | 0.56 | 2.24 |
| NV1D105 | K27A | 198 | GPECLEIFKACNPSNDQCCKSSKLVCSRATRWCKYQIGK | 0.26 | >2.95 | >11.57 |
| NV1D1609 | K21T | 199 | GPECLEIFKACNPSNDQCCKSSTLVCSRKTRWCKYQIGK | 0.26 | 0.44 | 1.71 |
| NV1D1489 | S12Q | 200 | GPECLEIFKACNPQNDQCCKSSKLVCSRKTRWCKYQIGK | 0.26 | 0.07 | 0.29 |
| NV1D107 | R29A | 201 | GPECLEIFKACNPSNDQCCKSSKLVCSRKTAWCKYQIGK | 0.26 | 1.03 | 3.99 |
| NV1D1837 | K37P | 202 | GPECLEIFKACNPSNDQCCKSSKLVCSRKTRWCKYQIGP | 0.26 | 2.00 | 7.72 |
| NV1D1512 | N13S | 203 | GPECLEIFKACNPSSDQCCKSSKLVCSRKTRWCKYQIGK | 0.26 | >0.98 | >3.75 |
| NV1D1550 | Q15L | 204 | GPECLEIFKACNPSNDLCCKSSKLVCSRKTRWCKYQIGK | 0.26 | 1.39 | 5.31 |
| NV1D1443 | A8V | 205 | GPECLEIFKVCNPSNDQCCKSSKLVCSRKTRWCKYQIGK | 0.26 | 0.25 | 0.93 |
| NV1D1458 | N10E | 206 | GPECLEIFKACEPSNDQCCKSSKLVCSRKTRWCKYQIGK | 0.26 | 0.28 | 1.05 |
| NV1D1517 | N13P | 207 | GPECLEIFKACNPSPDQCCKSSKLVCSRKTRWCKYQIGK | 0.26 | >0.85 | >3.22 |
| NV1D1368 | L3R | 208 | GPECREIFKACNPSNDQCCKSSKLVCSRKTRWCKYQIGK | 0.27 | 0.11 | 0.43 |

FIG. 4(8)

| | | | | | | |
|---|---|---|---|---|---|---|
| NV1D1459 | N10K | 209 | GPECLEIFKACKPSNDQCCKSSKLVCSRKTRWCKYQIGK | 0.27 | 0.24 | 0.89 |
| NV1D1583 | S19G | 210 | GPECLEIFKACNPSNDQCCKGSKLVCSRKTRWCKYQIGK | 0.27 | 0.33 | 1.22 |
| NV1D1834 | K37N | 211 | GPECLEIFKACNPSNDQCCKSSKLVCSRKTRWCKYQIGN | 0.27 | 0.97 | 3.54 |
| NV1D1518 | N13L | 212 | GPECLEIFKACNPSLDQCCKSSKLVCSRKTRWCKYQIGK | 0.28 | 1.33 | 4.84 |
| NV1D1533 | D14P | 213 | GPECLEIFKACNPSNPQCCKSSKLVCSRKTRWCKYQIGK | 0.28 | 2.71 | 9.72 |
| NV1D1788 | Q34I | 214 | GPECLEIFKACNPSNDQCCKSSKLVCSRKTRWCKYIIGK | 0.29 | 0.20 | 0.71 |
| NV1D1679 | R26G | 215 | GPECLEIFKACNPSNDQCCKSSKLVCSGKTRWCKYQIGK | 0.29 | 3.62 | 12.64 |
| NV1D1726 | R29L | 216 | GPECLEIFKACNPSNDQCCKSSKLVCSRKTLWCKYQIGK | 0.29 | >0.9 | >3.13 |
| NV1D1611 | K21D | 217 | GPECLEIFKACNPSNDQCCKSSDLVCSRKTRWCKYQIGK | 0.30 | 0.71 | 2.38 |
| NV1D1520 | D14W | 218 | GPECLEIFKACNPSNWQCCKSSKLVCSRKTRWCKYQIGK | 0.30 | 1.43 | 4.81 |
| NV1D1616 | L22W | 219 | GPECLEIFKACNPSNDQCCKSSKWVCSRKTRWCKYQIGK | 0.30 | 2.31 | 7.75 |
| NV1D1635 | V23K | 220 | GPECLEIFKACNPSNDQCCKSSKLKCSRKTRWCKYQIGK | 0.30 | 1.05 | 3.49 |
| NV1D1424 | K7Q | 221 | GPECLEIFQACNPSNDQCCKSSKLVCSRKTRWCKYQIGK | 0.30 | >0.27 | >0.9 |
| NV1D2126 | E1N,R26K | 222 | GPNCLEIFKACNPSNDQCCKSSKLVCSKKTRWCKYQIGK | 0.30 | 0.59 | 1.94 |

FIG. 4(9)

| Peptide ID | Substitution | SEQ ID NO: | Sequence | $IC_{50}$ (Nav1.7) µM | $IC_{50}$ (Nav1.2) µM | Selectivity |
|---|---|---|---|---|---|---|
| HwTX-IV | Parent Synthetic | 1 | ECLEIFKACNPSNDQCCKSSKLVCSRKTRWCKYQI-NH2 | 0.04 | 0.07 | 1.66 |
| NV1D7 | Parent Recombinant | 2 | GPECLEIFKACNPSNDQCCKSSKLVCSRKTRWCKYQIGK | 0.16 | 0.34 | 2.14 |
| | | | | | | |
| NV1D2168 | E1N,R26K,Q34S,G36I | 192 | GPNCLEIFKACNPSNDQCCKSSKLVCSKKTRWCKYSIIK | 0.24 | >7.14 | >30.32 |
| NV1D1730 | W30K | 109 | GPECLEIFKACNPSNDQCCKSSKLVCSRKTRKCKYQIGK | 0.10 | >2.95 | >28.65 |
| NV1D1821 | G36I | 58 | GPECLEIFKACNPSNDQCCKSSKLVCSRKTRWCKYQIIK | 0.06 | 1.42 | 22.71 |
| NV1D1733 | W30Y | 223 | GPECLEIFKACNPSNDQCCKSSKLVCSRKTRYCKYQIGK | 1.69 | 36.60 | 21.66 |
| NV1D1519 | N13G | 172 | GPECLEIFKACNPSGDQCCKSSKLVCSRKTRWCKYQIGK | 0.19 | 3.74 | 19.79 |
| NV1D1569 | S19Q | 21 | GPECLEIFKACNPSNDQCCKQSKLVCSRKTRWCKYQIGK | 0.02 | 0.46 | 18.39 |
| NV1D1722 | R29N | 149 | GPECLEIFKACNPSNDQCCKSSKLVCSRKTNWCKYQIGK | 0.15 | >2.59 | >17.04 |
| NV1D2148 | E1N,Y33W,G36I | 7 | GPNCLEIFKACNPSNDQCCKSSKLVCSRKTRWCKWQIIK | 0.02 | 0.25 | 16.49 |
| NV1D2134 | R26K,Y33W | 46 | GPECLEIFKACNPSNDQCCKSSKLVCSKKTRWCKWQIGK | 0.05 | 0.77 | 15.76 |
| NV1D1680 | K27W | 57 | GPECLEIFKACNPSNDQCCKSSKLVCSRWTRWCKYQIGK | 0.06 | >0.92 | >14.75 |
| NV1D2177 | E1N,E4R,R26K,Q34S,G36I | 16 | GPNCLRIFKACNPSNDQCCKSSKLVCSKKTRWCKYSIIK | 0.02 | 0.32 | 14.22 |
| NV1D102 | V23A | 122 | GPECLEIFKACNPSNDQCCKSSKLACSRKTRWCKYQIGK | 0.11 | >1.51 | >13.25 |
| NV1D1549 | Q15P | 224 | GPECLEIFKACNPSNDPCCKSSKLVCSRKTRWCKYQIGK | 0.35 | 4.63 | 13.23 |
| NV1D1760 | Y33W | 13 | GPECLEIFKACNPSNDQCCKSSKLVCSRKTRWCKWQIGK | 0.02 | 0.26 | 12.70 |
| NV1D1679 | R26G | 215 | GPECLEIFKACNPSNDQCCKSSKLVCSGKTRWCKYQIGK | 0.29 | 3.62 | 12.64 |
| NV1D2136 | R26K,G36I | 225 | GPECLEIFKACNPSNDQCCKSSKLVCSKKTRWCKYQIIK | 0.31 | >3.83 | >12.26 |
| NV1D105 | K27A | 198 | GPECLEIFKACNPSNDQCCKSSKLVCSRATRWCKYQIGK | 0.26 | >2.95 | >11.57 |

FIG. 5(1)

| | | | | | | |
|---|---|---|---|---|---|---|
| NV1D104 | R26A | 164 | GPECLEIFKACNPSNDQCCKSSKLVCSAKTRWCKYQIGK | 0.17 | >1.78 | >10.79 |
| NV1D1673 | R26T | 226 | GPECLEIFKACNPSNDQCCKSSKLVCSTKTRWCKYQIGK | 1.93 | >20.73 | >10.75 |
| NV1D1832 | K37R | 25 | GPECLEIFKACNPSNDQCCKSSKLVCSRKTRWCKYQIGR | 0.03 | 0.28 | 10.70 |
| NV1D1565 | K18P | 189 | GPECLEIFKACNPSNDQCCPSSKLVCSRKTRWCKYQIGK | 0.23 | 2.40 | 10.61 |
| NV1D1723 | R29D | 227 | GPECLEIFKACNPSNDQCCKSSKLVCSRKTDWCKYQIGK | 0.95 | >9.59 | >10.15 |
| NV1D1769 | Y33T | 76 | GPECLEIFKACNPSNDQCCKSSKLVCSRKTRWCKTQIGK | 0.07 | 0.70 | 9.76 |
| NV1D1533 | D14P | 213 | GPECLEIFKACNPSNPQCCKSSKLVCSRKTRWCKYQIGK | 0.28 | 2.71 | 9.72 |
| NV1D1537 | Q15E | 188 | GPECLEIFKACNPSNDECCKSSKLVCSRKTRWCKYQIGK | 0.23 | 2.16 | 9.60 |
| NV1D1556 | K18F | 228 | GPECLEIFKACNPSNDQCCFSSKLVCSRKTRWCKYQIGK | 0.36 | 3.46 | 9.56 |
| NV1D1667 | R26K | 175 | GPECLEIFKACNPSNDQCCKSSKLVCSKKTRWCKYQIGK | 0.20 | 1.88 | 9.53 |
| NV1D1600 | K21W | 82 | GPECLEIFKACNPSNDQCCKSSWLVCSRKTRWCKYQIGK | 0.08 | 0.65 | 8.52 |
| NV1D1671 | R26H | 229 | GPECLEIFKACNPSNDQCCKSSKLVCSHKTRWCKYQIGK | 0.74 | >6.3 | >8.47 |
| NV1D1521 | D14Q | 230 | GPECLEIFKACNPSNQQCCKSSKLVCSRKTRWCKYQIGK | 0.91 | 7.57 | 8.36 |
| NV1D2161 | E1N,E4R,R26K,Q34S | 5 | GPNCLRIFKACNPSNDQCCKSSKLVCSKKTRWCKYSIGK | 0.01 | 0.11 | 8.33 |
| NV1D2143 | E1N,E4R,G36I | 12 | GPNCLRIFKACNPSNDQCCKSSKLVCSRKTRWCKYQIIK | 0.02 | 0.15 | 8.21 |
| NV1D1668 | R26V | 231 | GPECLEIFKACNPSNDQCCKSSKLVCSVKTRWCKYQIGK | 0.41 | >3.19 | >7.86 |
| NV1D101 | L22A | 232 | GPECLEIFKACNPSNDQCCKSSKAVCSRKTRWCKYQIGK | 0.52 | >4.08 | >7.85 |
| NV1D1616 | L22W | 219 | GPECLEIFKACNPSNDQCCKSSKWVCSRKTRWCKYQIGK | 0.30 | 2.31 | 7.75 |
| NV1D1837 | K37P | 202 | GPECLEIFKACNPSNDQCCKSSKLVCSRKTRWCKYQIGP | 0.26 | 2.00 | 7.72 |
| NV1D1727 | R29G | 180 | GPECLEIFKACNPSNDQCCKSSKLVCSRKTGWCKYQIGK | 0.21 | 1.59 | 7.67 |
| NV1D1505 | N13Q | 61 | GPECLEIFKACNPSQDQCCKSSKLVCSRKTRWCKYQIGK | 0.06 | 0.49 | 7.67 |
| NV1D1536 | Q15W | 113 | GPECLEIFKACNPSNDWCCKSSKLVCSRKTRWCKYQIGK | 0.11 | 0.79 | 7.44 |
| NV1D1823 | G36L | 78 | GPECLEIFKACNPSNDQCCKSSKLVCSRKTRWCKYQILK | 0.07 | 0.53 | 7.16 |
| NV1D1535 | D14G | 233 | GPECLEIFKACNPSNGQCCKSSKLVCSRKTRWCKYQIGK | 0.72 | >5.18 | >7.16 |
| NV1D1831 | K37S | 234 | GPECLEIFKACNPSNDQCCKSSKLVCSRKTRWCKYQIGS | 1.47 | 10.30 | 7.01 |
| NV1D1712 | R29W | 55 | GPECLEIFKACNPSNDQCCKSSKLVCSRKTWWCKYQIGK | 0.06 | 0.41 | 6.87 |

*FIG. 5(2)*

| | | | | | | |
|---|---|---|---|---|---|---|
| NV1D1584 | S20W | 131 | GPECLEIFKACNPSNDQCCKSWKLVCSRKTRWCKYQIGK | 0.13 | 0.87 | 6.81 |
| NV1D1516 | N13I | 142 | GPECLEIFKACNPSIDQCCKSSKLVCSRKTRWCKYQIGK | 0.14 | 0.95 | 6.70 |
| NV1D2171 | E4R,R26K,Y33W,G36I | 48 | GPECLRIFKACNPSNDQCCKSSKLVCSKKTRWCKWQIIK | 0.05 | 0.34 | 6.63 |
| NV1D1539 | Q15V | 235 | GPECLEIFKACNPSNDVCCKSSKLVCSRKTRWCKYQIGK | 0.75 | 4.91 | 6.58 |
| NV1D2169 | E1N,Y33W,Q34S,G36I | 83 | GPNCLEIFKACNPSNDQCCKSSKLVCSRKTRWCKWSIIK | 0.08 | 0.50 | 6.38 |
| NV1D1547 | Q15D | 165 | GPECLEIFKACNPSNDDCCKSSKLVCSRKTRWCKYQIGK | 0.18 | 1.08 | 6.17 |
| NV1D1840 | F6M | 236 | GPECLEIMKACNPSNDQCCKSSKLVCSRKTRWCKYQIGK | 1.10 | 6.68 | 6.07 |
| NV1D2158 | R26K,Q34S,G36I | 178 | GPECLEIFKACNPSNDQCCKSSKLVCSKKTRWCKYSIIK | 0.20 | 1.22 | 6.04 |
| NV1D1809 | G36Q | 237 | GPECLEIFKACNPSNDQCCKSSKLVCSRKTRWCKYQIQK | 1.74 | 10.40 | 5.98 |
| NV1D1811 | G36K | 96 | GPECLEIFKACNPSNDQCCKSSKLVCSRKTRWCKYQIKK | 0.09 | 0.53 | 5.87 |
| NV1D1791 | Q34G | 182 | GPECLEIFKACNPSNDQCCKSSKLVCSRKTRWCKYGIGK | 0.21 | 1.20 | 5.77 |
| NV1D1719 | R29H | 60 | GPECLEIFKACNPSNDQCCKSSKLVCSRKTHWCKYQIGK | 0.06 | 0.37 | 5.75 |
| NV1D2159 | Y33W,Q34S,G36I | 127 | GPECLEIFKACNPSNDQCCKSSKLVCSRKTRWCKWSIIK | 0.12 | 0.68 | 5.70 |
| NV1D1664 | R26W | 72 | GPECLEIFKACNPSNDQCCKSSKLVCSWKTRWCKYQIGK | 0.07 | 0.40 | 5.64 |
| NV1D1588 | S20V | 134 | GPECLEIFKACNPSNDQCCKSVKLVCSRKTRWCKYQIGK | 0.13 | 0.73 | 5.60 |
| NV1D1553 | K18Q | 141 | GPECLEIFKACNPSNDQCCQSSKLVCSRKTRWCKYQIGK | 0.14 | 0.79 | 5.58 |
| NV1D2218 | N13Q,R29K,K37R | 137 | GPECLEIFKACNPSQDQCCKSSKLVCSRKTKWCKYQIGR | 0.13 | 0.73 | 5.52 |
| NV1D1833 | K37T | 238 | GPECLEIFKACNPSNDQCCKSSKLVCSRKTRWCKYQIGT | 0.37 | 2.02 | 5.42 |
| NV1D111 | Q34A | 111 | GPECLEIFKACNPSNDQCCKSSKLVCSRKTRWCKYAIGK | 0.11 | 0.57 | 5.40 |
| NV1D1550 | Q15L | 204 | GPECLEIFKACNPSNDLCCKSSKLVCSRKTRWCKYQIGK | 0.26 | 1.39 | 5.31 |
| NV1D1552 | K18W | 239 | GPECLEIFKACNPSNDQCCWSSKLVCSRKTRWCKYQIGK | 0.46 | 2.38 | 5.21 |
| NV1D1776 | Q34W | 240 | GPECLEIFKACNPSNDQCCKSSKLVCSRKTRWCKYWIGK | 1.32 | 6.87 | 5.20 |
| NV1D1676 | R26I | 177 | GPECLEIFKACNPSNDQCCKSSKLVCSIKTRWCKYQIGK | 0.20 | 1.05 | 5.20 |
| NV1D1504 | N13W | 74 | GPECLEIFKACNPSWDQCCKSSKLVCSRKTRWCKYQIGK | 0.07 | 0.37 | 5.15 |
| NV1D2130 | E4R,R26K | 45 | GPECLRIFKACNPSNDQCCKSSKLVCSKKTRWCKYQIGK | 0.05 | 0.25 | 5.15 |

FIG. 5(3)

| Peptide ID | Substitution | SEQ ID NO: | Sequence | $IC_{50}$ (Nav1.7) µM | $IC_{50}$ (Nav1.2) µM | Selectivity |
|---|---|---|---|---|---|---|
| NV1D7 | Parent Recombinant | 2 | GPECLEIFKACNPSNDQCCKSSKLVCSRKTRWCKYQIGK | 0.05 | 0.05 | 0.92 |
| | | | | | | |
| NV1D1383 | E4R | 18 | GPECLRIFKACNPSNDQCCKSSKLVCSRKTRWCKYQIGK | 0.01 | 0.01 | 2.42 |
| NV1D2133 | E4R, G36I | 52 | GPECLRIFKACNPSNDQCCKSSKLVCSRKTRWCKYQIIK | 0.01 | 0.05 | 8.00 |
| NV1D2163 | E1N, E4R, Y33W, Q34S | 3 | GPNCLRIFKACNPSNDQCCKSSKLVCSRKTRWCKWSIGK | 0.01 | 0.02 | 2.46 |
| NV1D1760 | Y33W | 13 | GPECLEIFKACNPSNDQCCKSSKLVCSRKTRWCKWQIGK | 0.01 | 0.01 | 1.90 |
| NV1D2142 | E1N, E4R, Q34S | 4 | GPNCLRIFKACNPSNDQCCKSSKLVCSRKTRWCKYSIGK | 0.01 | 0.03 | 3.49 |
| NV1D2150 | E4R, R26K, Y33W | 65 | GPECLRIFKACNPSNDQCCKSSKLVCSKKTRWCKWQIGK | 0.01 | 0.02 | 1.61 |
| NV1D2165 | E1N, E4R, Q34S, G36I | 40 | GPNCLRIFKACNPSNDQCCKSSKLVCSRKTRWCKYSIIK | 0.01 | 0.06 | 5.00 |
| NV1D2137 | Y33W, Q34S | 241 | GPECLEIFKACNPSNDQCCKSSKLVCSRKTRWCKWSIGK | 0.01 | 0.04 | 2.93 |
| NV1D85 | E4A | 242 | GPECLAIFKACNPSNDQCCKSSKLVCSRKTRWCKYQIGK | 0.02 | 0.03 | 1.36 |
| NV1D1353 | E1N | 67 | GPNCLEIFKACNPSNDQCCKSSKLVCSRKTRWCKYQIGK | 0.02 | 0.03 | 1.24 |
| NV1D1783 | Q34S | 10 | GPECLEIFKACNPSNDQCCKSSKLVCSRKTRWCKYSIGK | 0.03 | 0.04 | 1.41 |
| NV1D82 | E1A | 243 | GPACLEIFKACNPSNDQCCKSSKLVCSRKTRWCKYQIGK | 0.04 | 0.05 | 1.20 |
| NV1D2177 | E1N, E4R, R26K, Q34S, G36I | 16 | GPNCLRIFKACNPSNDQCCKSSKLVCSKKTRWCKYSIIK | 0.04 | 0.87 | 21.15 |
| NV1D1441 | A8E | 54 | GPECLEIFKECNPSNDQCCKSSKLVCSRKTRWCKYQIGK | 0.04 | 0.02 | 0.52 |
| NV1D97 | K18A | 244 | GPECLEIFKACNPSNDQCCASSKLVCSRKTRWCKYQIGK | 0.05 | 0.52 | 10.71 |
| NV1D95 | Q15A | 101 | GPECLEIFKACNPSNDACCKSSKLVCSRKTRWCKYQIGK | 0.05 | 0.07 | 1.35 |
| NV1D107 | R29A | 201 | GPECLEIFKACNPSNDQCCKSSKLVCSRKTAWCKYQIGK | 0.05 | 0.20 | 3.96 |
| NV1D1439 | A8W | 245 | GPECLEIFKWCNPSNDQCCKSSKLVCSRKTRWCKYQIGK | 0.05 | 0.01 | 0.22 |
| NV1D106 | T28A | 246 | GPECLEIFKACNPSNDQCCKSSKLVCSRKARWCKYQIGK | 0.06 | 0.10 | 1.71 |

FIG. 6(1)

| | | | | | | |
|---|---|---|---|---|---|---|
| NV1D2149 | E1N, Q34S, G36I | 112 | GPNCLEIFKACNPSNDQCCKSSKLVCSRKTRWCKYSIIK | 0.07 | 0.11 | 1.55 |
| NV1D99 | S20A | 247 | GPECLEIFKACNPSNDQCCKSAKLVCSRKTRWCKYQIGK | 0.07 | 0.12 | 1.69 |
| NV1D111 | Q34A | 111 | GPECLEIFKACNPSNDQCCKSSKLVCSRKTRWCKYAIGK | 0.09 | 0.11 | 1.21 |
| NV1D98 | S19A | 125 | GPECLEIFKACNPSNDQCCKASKLVCSRKTRWCKYQIGK | 0.10 | 0.05 | 0.48 |
| NV1D93 | N13A | 23 | GPECLEIFKACNPSADQCCKSSKLVCSRKTRWCKYQIGK | 0.10 | 0.15 | 1.56 |
| NV1D114 | K37A | 248 | GPECLEIFKACNPSNDQCCKSSKLVCSRKTRWCKYQIGA | 0.10 | 0.21 | 2.13 |
| NV1D90 | N10A | 249 | GPECLEIFKACAPSNDQCCKSSKLVCSRKTRWCKYQIGK | 0.10 | 0.18 | 1.78 |
| NV1D1505 | N13Q | 61 | GPECLEIFKACNPSQDQCCKSSKLVCSRKTRWCKYQIGK | 0.10 | 0.06 | 0.58 |
| NV1D92 | S12A | 250 | GPECLEIFKACNPANDQCCKSSKLVCSRKTRWCKYQIGK | 0.11 | 0.15 | 1.39 |
| NV1D102 | V23A | 122 | GPECLEIFKACNPSNDQCCKSSKLACSRKTRWCKYQIGK | 0.12 | 0.06 | 0.49 |
| NV1D100 | K21A | 162 | GPECLEIFKACNPSNDQCCKSSALVCSRKTRWCKYQIGK | 0.13 | 0.10 | 0.80 |
| NV1D104 | R26A | 164 | GPECLEIFKACNPSNDQCCKSSKLVCSAKTRWCKYQIGK | 0.14 | 1.87 | 13.55 |
| NV1D88 | K7A | 251 | GPECLEIFAACNPSNDQCCKSSKLVCSRKTRWCKYQIGK | 0.14 | 0.08 | 0.54 |
| NV1D1821 | G36I | 58 | GPECLEIFKACNPSNDQCCKSSKLVCSRKTRWCKYQIIK | 0.14 | 0.22 | 1.56 |
| NV1D105 | K27A | 198 | GPECLEIFKACNPSNDQCCKSSKLVCSRATRWCKYQIGK | 0.19 | 1.22 | 6.29 |
| NV1D86 | I5A | 252 | GPECLEAFKACNPSNDQCCKSSKLVCSRKTRWCKYQIGK | 0.22 | 0.18 | 0.79 |
| NV1D1569 | S19Q | 21 | GPECLEIFKACNPSNDQCCKQSKLVCSRKTRWCKYQIGK | 0.23 | 0.07 | 0.29 |
| NV1D84 | L3A | 253 | GPECAEIFKACNPSNDQCCKSSKLVCSRKTRWCKYQIGK | 0.23 | 0.13 | 0.55 |
| NV1D1667 | R26K | 175 | GPECLEIFKACNPSNDQCCKSSKLVCSKKTRWCKYQIGK | 0.23 | 0.22 | 0.96 |
| NV1D113 | Q36A | 254 | GPECLEIFKACNPSNDQCCKSSKLVCSRKTRWCKYQIAK | 0.39 | 0.32 | 0.83 |
| NV1D87 | F6A | 255 | GPECLEIAKACNPSNDQCCKSSKLVCSRKTRWCKYQIGK | 0.52 | 5.53 | 10.55 |
| NV1D112 | I35A | 256 | GPECLEIFKACNPSNDQCCKSSKLVCSRKTRWCKYQAGK | 0.59 | 0.16 | 0.28 |
| NV1D110 | Y33A | 257 | GPECLEIFKACNPSNDQCCKSSKLVCSRKTRWCKAQIGK | 0.86 | 1.01 | 1.18 |
| NV1D101 | L22A | 232 | GPECLEIFKACNPSNDQCCKSSKAVCSRKTRWCKYQIGK | 0.88 | 3.49 | 3.99 |
| NV1D91 | P11A | 258 | GPECLEIFKACNASNDQCCKSSKLVCSRKTRWCKYQIGK | 1.09 | 1.23 | 1.13 |
| NV1D94 | D14A | 259 | GPECLEIFKACNPSNAQCCKSSKLVCSRKTRWCKYQIGK | 1.33 | 2.34 | 1.76 |
| NV1D103 | S25A | 260 | GPECLEIFKACNPSNDQCCKSSKLVCARKTRWCKYQIGK | 8.24 | 1.89 | 0.23 |
| NV1D108 | W30A | 261 | GPECLEIFKACNPSNDQCCKSSKLVCSRKTRACKYQIGK | Inactive | Inactive | Inactive |
| NV1D109 | K32A | 262 | GPECLEIFKACNPSNDQCCKSSKLVCSRKTRWCAYQIGK | Inactive | Inactive | Inactive |

*FIG. 6(2)*

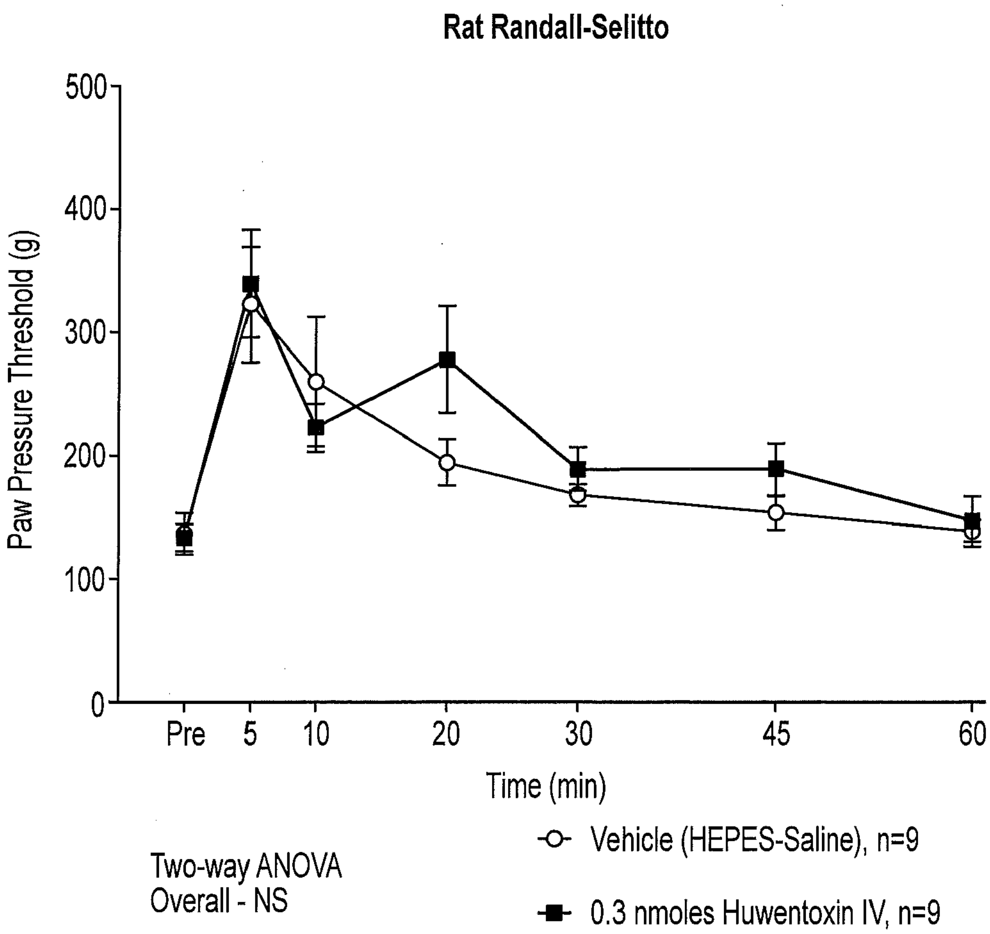
Rat Randall-Selitto
500
400
300
200
100
0
Paw Pressure Threshold (g)
Pre
5
10
20
30
45
60
Time (min)
Two-way ANOVA
Overall - NS
Vehicle (HEPES-Saline), n=9
0.3 nmoles Huwentoxin IV, n=9

*FIG. 7A*

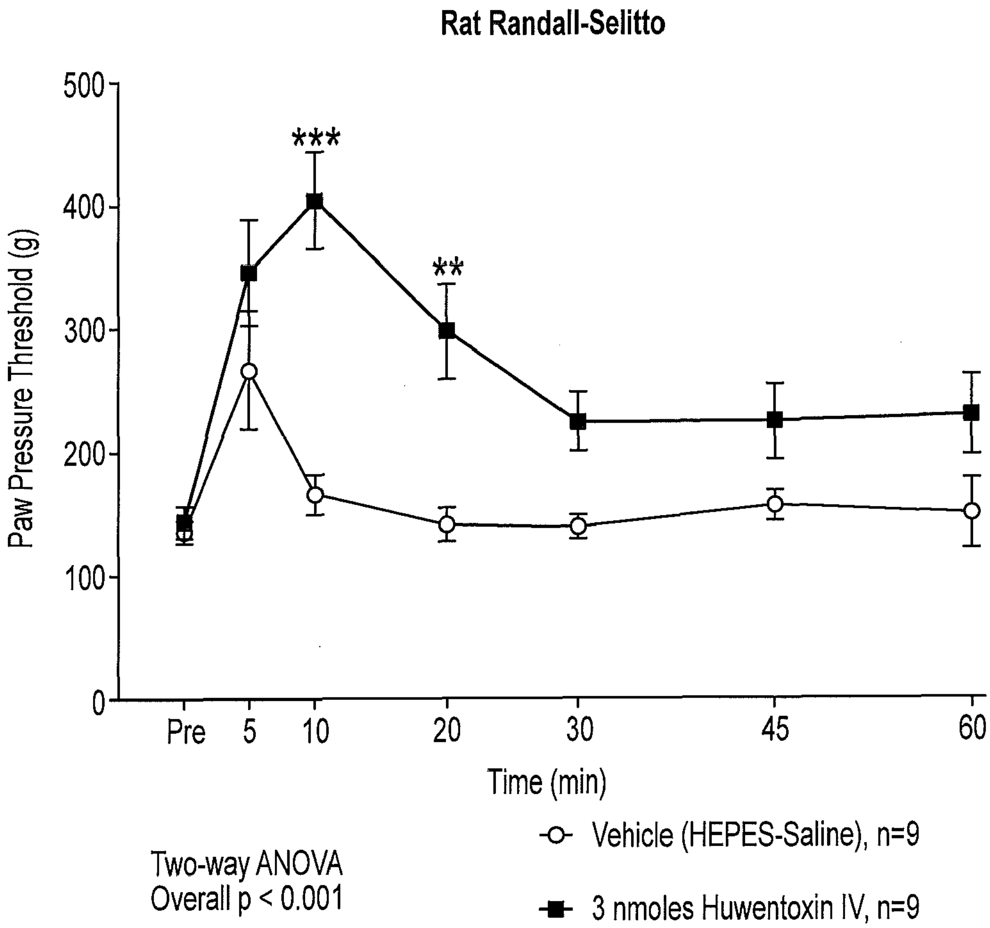
Rat Randall-Selitto
500
400
300
200
100
0
***
**
Paw Pressure Threshold (g)
Pre
5
10
20
30
45
60
Time (min)
Vehicle (HEPES-Saline), n=9
3 nmoles Huwentoxin IV, n=9
Two-way ANOVA
Overall p < 0.001

*FIG. 7B*

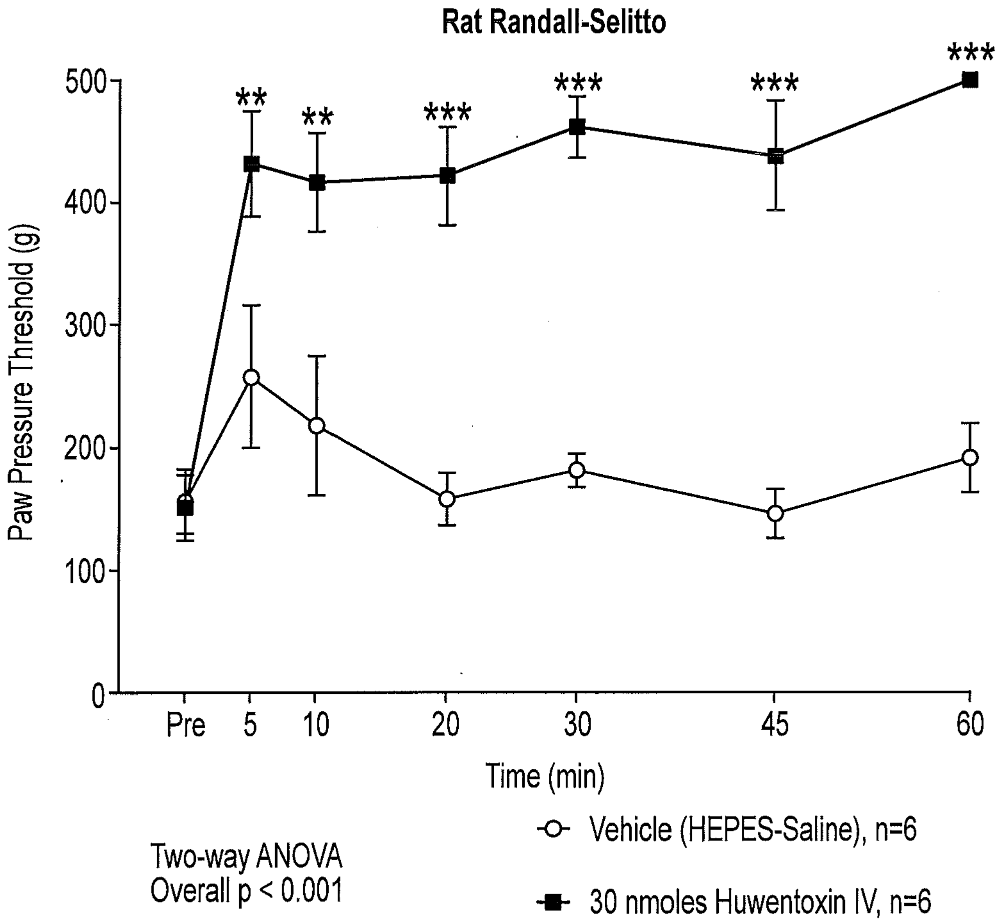
Rat Randall-Selitto
Paw Pressure Threshold (g)
500
400
300
200
100
0
**
**
***
***
***
***
Pre
5
10
20
30
45
60
Time (min)
Two-way ANOVA
Overall p < 0.001
Vehicle (HEPES-Saline), n=6
30 nmoles Huwentoxin IV, n=6

*FIG. 7C*

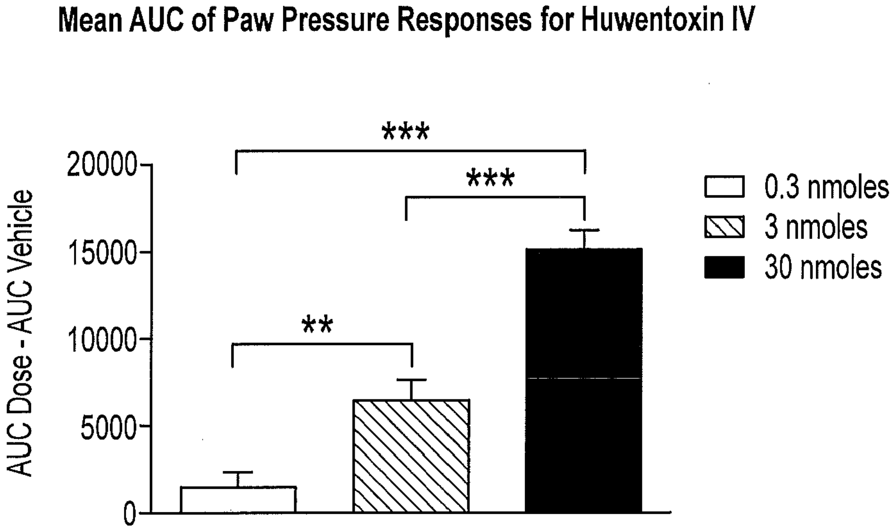
Mean AUC of Paw Pressure Responses for Huwentoxin IV
AUC Dose - AUC Vehicle
20000
15000
10000
5000
0
***
***
**
0.3 nmoles
3 nmoles
30 nmoles

*FIG. 8*

FIG. 9A

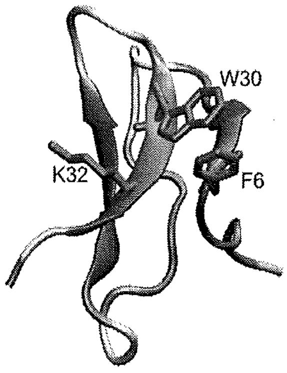


FIG. 9B

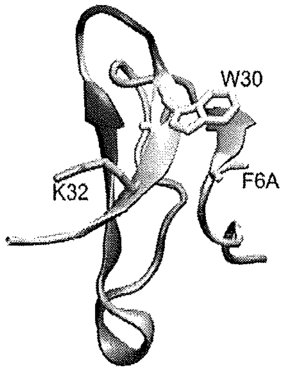


FIG. 9C

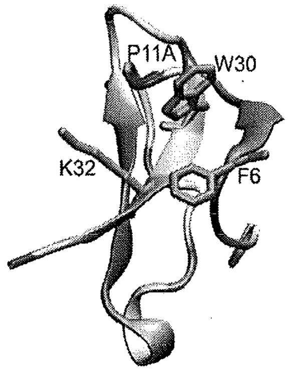


FIG. 9D

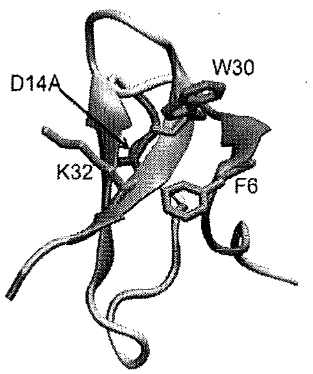


FIG. 9E

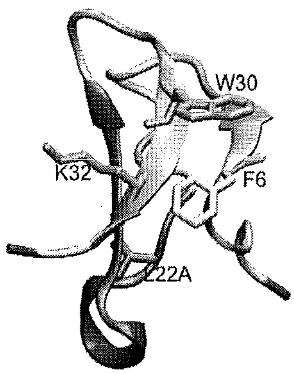


FIG. 9F

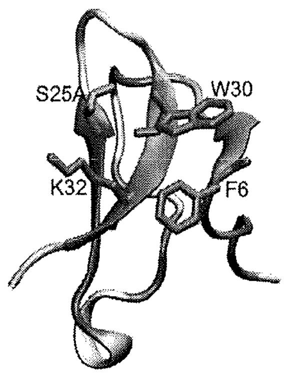


FIG. 9G

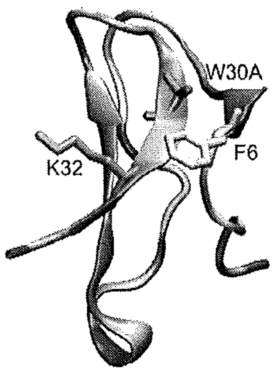


FIG. 9H

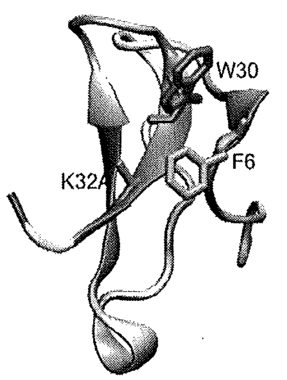


FIG. 9I

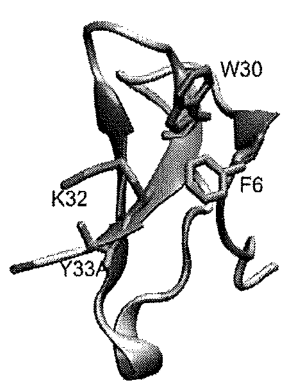

FIG. 10A

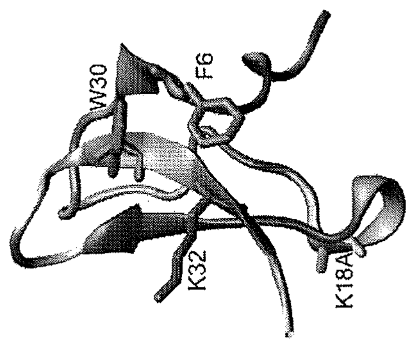


FIG. 10B

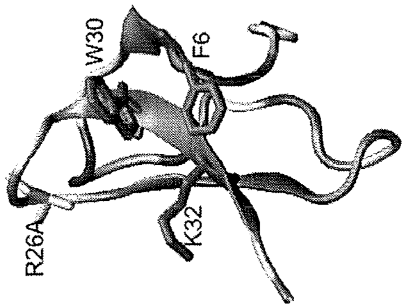


FIG. 10C

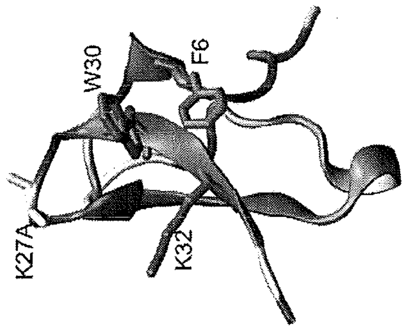

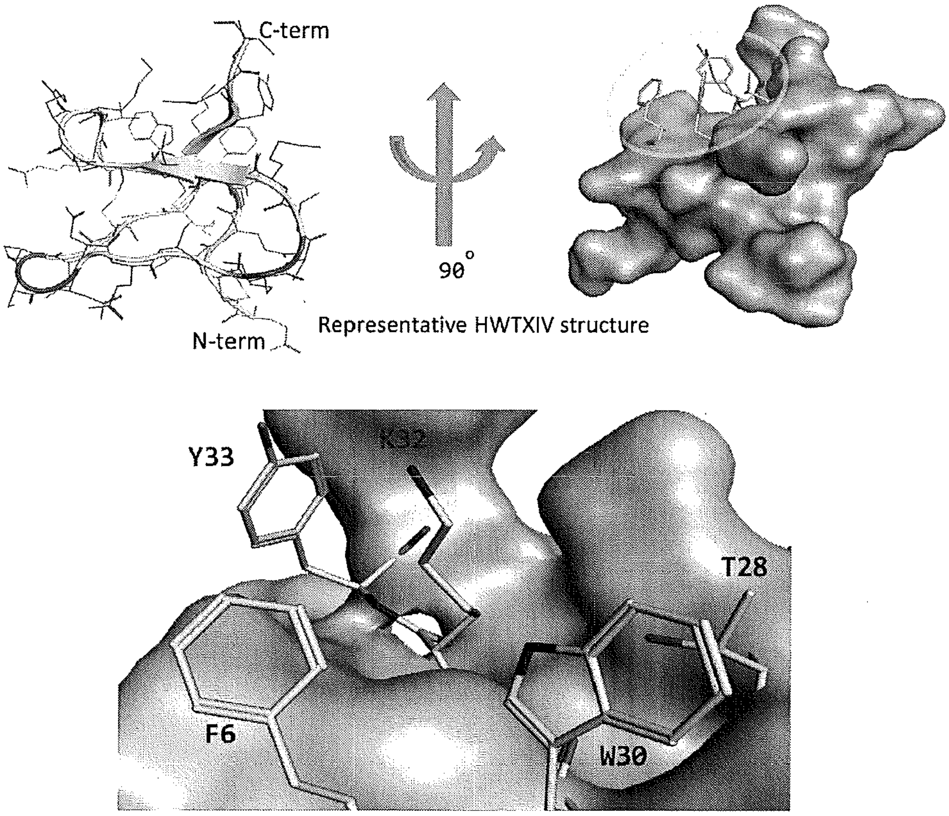
C-term
90°
N-term
Representative HWTXIV structure
Y33
K32
T28
F6
W30

*FIG. 11*

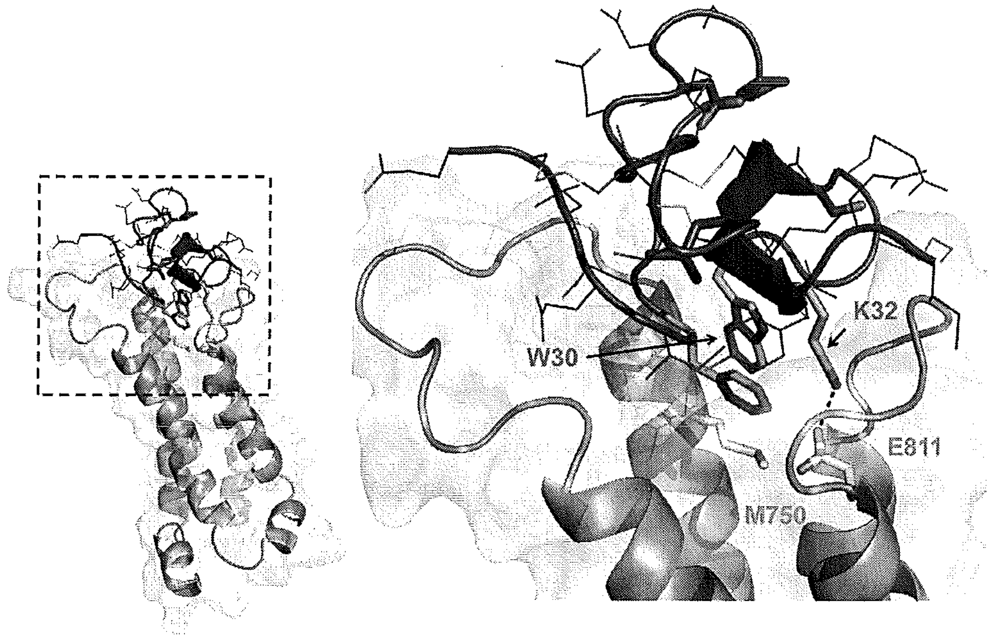


*FIG. 12*

| NV1D2168 Mutants | | Mutation | Sequence |
|---|---|---|---|
| pepide name | SEQ ID NO: | | |
| NV1D2168 | 281 | E1N,R26K,Q34S,G36I | GPNCLEIFKACNPSNDQCCKSSKLVCSKKTRWCKYSIIK |
| NV1G554 | 282 | E1N,R26K,Q34S,G36I,K37F | GPNCLEIFKACNPSNDQCCKSSKLVCSKKTRWCKYSIIF |
| NV1G555 | 283 | E1N,R26K,Q34S,G36I,K37R | GPNCLEIFKACNPSNDQCCKSSKLVCSKKTRWCKYSIIR |
| NV1G556 | 284 | E1N,S25I,R26K,Q34S,G36I | GPNCLEIFKACNPSNDQCCKSSKLVCIKKTRWCKYSIIK |
| NV1G557 | 285 | E1N,K21N,R26K,Q34S,G36I | GPNCLEIFKACNPSNDQCCKSSNLVCSKKTRWCKYSIIK |
| NV1G558 | 286 | E1N,K21H,R26K,Q34S,G36I | GPNCLEIFKACNPSNDQCCKSSHLVCSKKTRWCKYSIIK |
| NV1G559 | 287 | E1N,K21F,R26K,Q34S,G36I | GPNCLEIFKACNPSNDQCCKSSFLVCSKKTRWCKYSIIK |
| NV1G560 | 288 | E1N,K21R,R26K,Q34S,G36I | GPNCLEIFKACNPSNDQCCKSSRLVCSKKTRWCKYSIIK |
| NV1G561 | 289 | E1N,L22R,R26K,Q34S,G36I | GPNCLEIFKACNPSNDQCCKSSKRVCSKKTRWCKYSIIK |
| NV1G563 | 290 | E1N,S19P,R26K,Q34S,G36I | GPNCLEIFKACNPSNDQCCKPSKLVCSKKTRWCKYSIIK |
| NV1G565 | 291 | E1N,K18Y,R26K,Q34S,G36I | GPNCLEIFKACNPSNDQCCYSSKLVCSKKTRWCKYSIIK |
| NV1G566 | 292 | E1N,S20N,R26K,Q34S,G36I | GPNCLEIFKACNPSNDQCCKSNKLVCSKKTRWCKYSIIK |
| NV1G567 | 293 | E1N,S20P,R26K,Q34S,G36I | GPNCLEIFKACNPSNDQCCKSPKLVCSKKTRWCKYSIIK |
| NV1G569 | 294 | E1N,S20R,R26K,Q34S,G36I | GPNCLEIFKACNPSNDQCCKSRKLVCSKKTRWCKYSIIK |
| NV1G550 | 295 | E1N,E4N, A8R,R26K,Q34S,G36I | GPNCLNIFKRCNPSNDQCCKSSKLVCSKKTRWCKYSIIK |
| NV1G551 | 296 | E1N,E4Q, A8R,R26K,Q34S,G36I | GPNCLNIFKRCNPSNDQCCKSSKLVCSKKTRWCKYSIIK |
| NV1G552 | 297 | E1N,E4H, A8Q,R26K,Q34S,G36I | GPNCLHIFKQCNPSNDQCCKSSKLVCSKKTRWCKYSIIK |
| NV1G553 | 298 | E1N,E4Q, A8Q,R26K,Q34S,G36I | GPNCLHIFKQCNPSNDQCCKSSKLVCSKKTRWCKYSIIK |
| NV1G564 | 299 | E1N,Q15R,R26K,Q34S,G36I | GPNCLEIFKACNPSNDRCCKSSKLVCSKKTRWCKYSIIK |
| NV1G570 | 300 | E1N,N13A,R26K,Q34S,G36I | GPNCLEIFKACNPSADQCCKSSKLVCSKKTRWCKYSIIK |
| NV1G571 | 301 | E1N,A8Q,R26K,Q34S,G36I | GPNCLEIFKQCNPSNDQCCKSSKLVCSKKTRWCKYSIIK |
| NV1G572 | 302 | E1N,A8N,R26K,Q34S,G36I | GPNCLEIFKNCNPSNDQCCKSSKLVCSKKTRWCKYSIIK |
| NV1G573 | 303 | E1N,E4Q,R26K,Q34S,G36I | GPNCLQIFKACNPSNDQCCKSSKLVCSKKTRWCKYSIIK |
| NV1G562 | 304 | E1N,Q15N,R26K,Q34S,G36I | GPNCLEIFKACNPSNDNCCKSSKLVCSKKTRWCKYSIIK |
| NV1G568 | 305 | E1N,S19R,R26K,Q34S,G36I | GPNCLEIFKACNPSNDQCCKRSKLVCSKKTRWCKYSIIK |
| NV1G651 | 306 | E1N,S20V,R26K,Q34S,G36I | GPNCLEIFKACNPSNDQCCKSVKLVCSKKTRWCKYSIIK |

*FIG. 13A*

| NV1D2168 Mutants | | hNav1.1 $IC_{50}$ (nM) | | hNav1.2 $IC_{50}$ (nM) | | hNav1.3 $IC_{50}$ (nM) | | hNav1.4 $IC_{50}$ (nM) | | hNav1.5 $IC_{50}$ (nM) | | hNav1.6 $IC_{50}$ (nM) | | hNav1.7 $IC_{50}$ (nM) | |
|---|---|---|---|---|---|---|---|---|---|---|---|---|---|---|---|
| pepide name | SEQ ID NO: | TETRA | QP | TETRA | QP | TETRA | QP | TETRA | QP | TETRA | QP | TETRA | QP | TETRA | QP |
| NV1D2168 | 281 | 187 | 75 | | 871 | | 724 | >1000 | >10000 | >1000 | >10000 | >1000 | 67 | 67 | 41 |
| NV1G554 | 282 | 215 | | | | | | >1000 | | >1000 | | >1000 | | 38 | |
| NV1G555 | 283 | 37 | | | | | | >525 | | >1000 | | >525 | | 34 | |
| NV1G556 | 284 | 166 | | | | | | >1000 | | >1000 | | >1000 | | >1000 | |
| NV1G557 | 285 | 168 | 90 | | 76 | | | >1000 | | >1000 | | >1000 | 88 | 118 | 36 |
| NV1G558 | 286 | 71 | 90 | | 86 | | | >1000 | | >1000 | | 3850 | 91 | 73 | 24 |
| NV1G559 | 287 | >1000 | 81 | | 601 | | | >1000 | | >1000 | | >1000 | 267 | 145 | 17 |
| NV1G560 | 288 | >1000 | 87 | | 85 | | | >1000 | | >1000 | | 2300 | 87 | 55 | 21 |
| NV1G561 | 289 | >1000 | | | | | | >1000 | | >1000 | | 4170 | | >1000 | |
| NV1G563 | 290 | >1000 | 80 | | 44 | | | >1000 | | >1000 | | 2780 | 231 | 203 | 64 |
| NV1G565 | 291 | >1000 | | | | | | >1000 | | >1000 | | >1000 | | 846 | |
| NV1G566 | 292 | >1000 | 89 | | 567 | | | >1000 | | >1000 | | >1000 | 202 | 208 | 32 |
| NV1G567 | 293 | >1000 | | | 41 | | | >1000 | | >1000 | | >1000 | 86 | 446 | 207 |
| NV1G569 | 294 | 704 | | | | | | >1000 | | >1000 | | 206 | | 32 | |
| NV1G550 | 295 | 157 | | | | | | 1370 | | >1000 | | 41 | | 22 | |
| NV1G551 | 296 | 128 | | | | | | 1370 | | >1000 | | 69 | | 34 | |
| NV1G552 | 297 | 360 | | | | | | 2770 | | >1000 | | 145 | | 34 | |
| NV1G553 | 298 | 524 | | | | | | >1000 | | | | 681 | | 162 | |
| NV1G564 | 299 | 833 | | | | | | >1000 | | | | 1110 | | 276 | |
| NV1G570 | 300 | >1000 | | | | | | >1000 | | | | 1190 | | 406 | |
| NV1G571 | 301 | >1000 | | | | | | >1000 | | | | 1200 | | 440 | |
| NV1G572 | 302 | >1000 | | | | | | >1000 | | | | 1340 | | >1000 | |
| NV1G573 | 303 | 579 | | | | | | >1000 | | | | 672 | | 158 | |
| NV1G562 | 304 | | | | | | | | | | | | | | 45.8%* |
| NV1G568 | 305 | | | | | | | | | | | | 36.9%* | | 27 |
| NV1G651 | 306 | | | | | | | | | | | | | | 0%* |
| | | *% inhibiton at single point 100 nM concentration | | | | | | | | | | | | | |

*FIG. 13B*

| NV1D2168 Mutants | | | |
|---|---|---|---|
| | | | |
| ID | SEQ ID NO: | Mutation | Sequence |
| NV1D2163 | 307 | E1N,E4R,Y33W,Q34S | GPNCLRIFKACNPSNDQCCKSSKLVCSRKTRWCKWSIGK |
| NV1G600 | 308 | E1N,E4R,S19Q, Y33W,Q34S | GPNCLRIFKACNPSNDQCCKQSKLVCSRKTRWCKWSIGK |
| NV1G601 | 309 | E1N,E4R,Q15D, Y33W,Q34S | GPNCLRIFKACNPSNDDCCKSSKLVCSRKTRWCKWSIGK |
| NV1G602 | 310 | E1N,E4R,K18Q, Y33W,Q34S | GPNCLRIFKACNPSNDQCCQSSKLVCSRKTRWCKWSIGK |
| NV1G603 | 311 | E1N,E4R,D14S, Y33W,Q34S | GPNCLRIFKACNPSNSQCCKSSKLVCSRKTRWCKWSIGK |
| NV1G604 | 312 | E1N,E4R,D14Q, Y33W,Q34S | GPNCLRIFKACNPSNQQCCKSSKLVCSRKTRWCKWSIGK |
| NV1G605 | 313 | E1N,E4R,Q15P,Y33W,Q34S | GPNCLRIFKACNPSNDPCCKSSKLVCSRKTRWCKWSIGK |
| NV1G607 | 314 | E1N,E4R,K32A,Y33W,Q34S | GPNCLRIFKACNPSNDQCCKSSKLVCSRKTRWCAWSIGK |
| NV1G608 | 315 | E1N,E4R,K32W,Y33W,Q34S | GPNCLRIFKACNPSNDQCCKSSKLVCSRKTRWCWWSIGK |
| NV1G610 | 316 | E1N,E4R,K27A,Y33W,Q34S | GPNCLRIFKACNPSNDQCCKSSKLVCSRATRWCKWSIGK |
| NV1G611 | 317 | E1N,E4R,R26D,Y33W,Q34S | GPNCLRIFKACNPSNDQCCKSSKLVCSDKTRWCKWSIGK |
| NV1G612 | 318 | E1N,E4R,K27H,Y33W,Q34S | GPNCLRIFKACNPSNDQCCKSSKLVCSRHTRWCKWSIGK |
| NV1G613 | 319 | E1N,E4R,R26H,Y33W,Q34S | GPNCLRIFKACNPSNDQCCKSSKLVCSHKTRWCKWSIGK |
| NV1G614 | 320 | E1N,E4R,Y33W,Q34S,K37R | GPNCLRIFKACNPSNDQCCKSSKLVCSRKTRWCKWSIGR |
| NV1G615 | 321 | E1N,E4R,Y33W,Q34S,K37P | GPNCLRIFKACNPSNDQCCKSSKLVCSRKTRWCKWSIGP |
| NV1G616 | 322 | E1N,E4R,K18W,Y33W,Q34S | GPNCLRIFKACNPSNDQCCWSSKLVCSRKTRWCKWSIGK |
| NV1G617 | 323 | E1N,E4R,D14L,Y33W,Q34S | GPNCLRIFKACNPSNLQCCKSSKLVCSRKTRWCKWSIGK |
| NV1G618 | 324 | E1N,E4R,Q15W,Y33W,Q34S | GPNCLRIFKACNPSNDWCCKSSKLVCSRKTRWCKWSIGK |
| NV1G619 | 325 | E1N,E4R,Y33W,Q34S,G36V | GPNCLRIFKACNPSNDQCCKSSKLVCSRKTRWCKWSIVK |
| NV1G620 | 326 | E1N,E4R,R29G,Y33W,Q34S | GPNCLRIFKACNPSNDQCCKSSKLVCSRKTGWCKWSIGK |
| NV1G621 | 327 | E1N,E4R,R29W,Y33W,Q34S | GPNCLRIFKACNPSNDQCCKSSKLVCSRKTWWCKWSIGK |
| NV1G622 | 328 | E1N,E4R,Y33W,Q34S,G36T | GPNCLRIFKACNPSNDQCCKSSKLVCSRKTRWCKWSITK |
| NV1G623 | 329 | E1N,E4R,W30K,Y33W,Q34S | GPNCLRIFKACNPSNDQCCKSSKLVCSRKTRKCKWSIGK |
| NV1G624 | 330 | E1N,E4R,R29L,Y33W,Q34S | GPNCLRIFKACNPSNDQCCKSSKLVCSRKTLWCKWSIGK |
| NV1G625 | 331 | E1N,E4R,W30Y,Y33W,Q34S | GPNCLRIFKACNPSNDQCCKSSKLVCSRKTRYCKWSIGK |
| NV1G626 | 332 | E1N,E4R,R26P,Y33W,Q34S | GPNCLRIFKACNPSNDQCCKSSKLVCSPKTRWCKWSIGK |
| NV1G627 | 333 | E1N,E4R,L22A,Y33W,Q34S | GPNCLRIFKACNPSNDQCCKSSKAVCSRKTRWCKWSIGK |
| NV1G628 | 334 | E1N,E4R,L22W,Y33W,Q34S | GPNCLRIFKACNPSNDQCCKSSKWVCSRKTRWCKWSIGK |
| NV1G629 | 335 | E1N,E4R,T28L,Y33W,Q34S | GPNCLRIFKACNPSNDQCCKSSKLVCSRKLRWCKWSIGK |
| NV1G630 | 336 | E1N,E4R,K27P,Y33W,Q34S | GPNCLRIFKACNPSNDQCCKSSKLVCSRPTRWCKWSIGK |
| NV1G631 | 337 | E1N,E4R,R26V,Y33W,Q34S | GPNCLRIFKACNPSNDQCCKSSKLVCSVKTRWCKWSIGK |
| NV1G632 | 338 | E1N,E4R,R26G,Y33W,Q34S | GPNCLRIFKACNPSNDQCCKSSKLVCSGKTRWCKWSIGK |
| NV1G633 | 339 | E1N,E4R,K21W,Y33W,Q34S | GPNCLRIFKACNPSNDQCCKSSWLVCSRKTRWCKWSIGK |
| NV1G634 | 340 | E1N,E4R,R26I,Y33W,Q34S | GPNCLRIFKACNPSNDQCCKSSKLVCSIKTRWCKWSIGK |
| NV1G635 | 341 | E1N,E4R,K27W,Y33W,Q34S | GPNCLRIFKACNPSNDQCCKSSKLVCSRWTRWCKWSIGK |
| NV1G636 | 342 | E1N,E4R,R26A,Y33W,Q34S | GPNCLRIFKACNPSNDQCCKSSKLVCSAKTRWCKWSIGK |
| NV1G637 | 343 | E1N,E4R,S20W,Y33W,Q34S | GPNCLRIFKACNPSNDQCCKSWKLVCSRKTRWCKWSIGK |
| NV1G638 | 344 | E1N,E4R,Q15E,Y33W,Q34S | GPNCLRIFKACNPSNDECCKSSKLVCSRKTRWCKWSIGK |
| NV1G639 | 345 | E1N,E4R,K18P,Y33W,Q34S | GPNCLRIFKACNPSNDQCCPSSKLVCSRKTRWCKWSIGK |
| NV1G640 | 346 | E1N,E4R,Q15V,Y33W,Q34S | GPNCLRIFKACNPSNDVCCKSSKLVCSRKTRWCKWSIGK |
| NV1G641 | 347 | E1N,E4R,D14G,Y33W,Q34S | GPNCLRIFKACNPSNGQCCKSSKLVCSRKTRWCKWSIGK |
| NV1G642 | 348 | E1N,E4R,K18F,Y33W,Q34S | GPNCLRIFKACNPSNDQCCFSSKLVCSRKTRWCKWSIGK |
| NV1G644 | 349 | E1N,E4R,Y33W,Q34S,K37T | GPNCLRIFKACNPSNDQCCKSSKLVCSRKTRWCKWSIGT |
| NV1G645 | 350 | E1N,E4R,Y33W,Q34S,K37Q | GPNCLRIFKACNPSNDQCCKSSKLVCSRKTRWCKWSIGQ |
| NV1G646 | 351 | E1N,E4R,Y33W,Q34S,K37S | GPNCLRIFKACNPSNDQCCKSSKLVCSRKTRWCKWSIGS |
| NV1G647 | 352 | E1N,E4R,F6M,Y33W,Q34S | GPNCLRIMKACNPSNDQCCKSSKLVCSRKTRWCKWSIGK |
| NV1G648 | 353 | E1N,E4R,K7Q,Y33W,Q34S | GPNCLRIFQACNPSNDQCCKSSKLVCSRKTRWCKWSIGK |
| NV1G649 | 354 | E1N,E4R,P11R,Y33W,Q34S | GPNCLRIFKACNRSNDQCCKSSKLVCSRKTRWCKWSIGK |
| NV1G650 | 355 | E1N,E4R,S19Q,Y33W,Q34S | GPNCLRIFKACNPSNDQCCKQSKLVCSRKTRWCKWSIGK |

*FIG. 14A*

| NV1D2168 Mutants | | | hNav1.6 IC50 (nM) | | | hNav1.7 IC50 (nM) | |
|---|---|---|---|---|---|---|---|
| | | | TETRA | QP | | TETRA | QP |
| ID | SEQ ID NO: | | | | | | |
| NV1D2163 | 307 | | | 98.6%I @ 300 nM | | 14 | 32 |
| NV1G600 | 308 | | | | | 941 | |
| NV1G601 | 309 | | | | | 797 | |
| NV1G602 | 310 | | | | | | 37.7%I @ 100 nM |
| NV1G603 | 311 | | | | | 4490 | |
| NV1G604 | 312 | | | | | | 17.7%I @ 100 nM |
| NV1G605 | 313 | | | | | | -23.7%I @ 100 nM |
| NV1G607 | 314 | | | | | | -3.3%I @ 100 nM |
| NV1G608 | 315 | | | | | | -12.5%I @ 100 nM |
| NV1G610 | 316 | | | 149 | | | 13 |
| NV1G611 | 317 | | | 85 | | | 8 |
| NV1G612 | 318 | | | 47.3%I @ 30 nM | | | 8 |
| NV1G613 | 319 | | | 77.2%I @ 30 nM | | | 5 |
| NV1G614 | 320 | | | 70.3%I @ 100 nM | | | 14 |
| NV1G615 | 321 | | | 66.8%I @ 100 nM | | | 16 |
| NV1G616 | 322 | | | 91.6%I @ 300 nM | | | 25 |
| NV1G617 | 323 | | | 72.8%I @ 300 nM | | | 31 |
| NV1G618 | 324 | | | 14.9%I @ 100 nM | | | 6.1%I @ 30 nM |
| NV1G619 | 325 | | | 86.8%I @ 300 nM | | | 30 |
| NV1G620 | 326 | | | 67.2%I @ 30 nM | | | 9 |
| NV1G621 | 327 | | | 96.2%I @ 100 nM | | | 20 |
| NV1G622 | 328 | | | | | 4720 | |
| NV1G623 | 329 | | | 58.3%I @ 30 nM | | | 7 |
| NV1G624 | 330 | | | | | 406 | |
| NV1G625 | 331 | | | 75.5%I @ 100 nM | | | 13 |
| NV1G626 | 332 | | | 74.1%I @ 300 nM | | | 38 |
| NV1G627 | 333 | | | 77.9%I @ 100 nM | | | 18 |
| NV1G628 | 334 | | | | | 1780 | |
| NV1G629 | 335 | | | 86.0%I @ 300 nM | | | 26 |
| NV1G630 | 336 | | | 74.9%I @ 100 nM | | | 19 |
| NV1G631 | 337 | | | | | | 53 |
| NV1G632 | 338 | | | 57.9%I @ 30 nM | | | 11 |
| NV1G633 | 339 | | | 76.5%I @ 300 nM | | | 46 |
| NV1G634 | 340 | | | 68.3%I @ 100 nM | | | 21 |
| NV1G635 | 341 | | | | | 2180 | |
| NV1G636 | 342 | | | 75.9%I @ 300 nM | | | 24 |
| NV1G637 | 343 | | | | | 1980 | |
| NV1G638 | 344 | | | | | | 80 |
| NV1G639 | 345 | | | | | | -8.6%I @ 30 nM |
| NV1G640 | 346 | | | | | | 17.8%I @ 30 nM |
| NV1G641 | 347 | | | | | | 83 |
| NV1G642 | 348 | | | 75.0%I @ 30 nM | | 65 | 3 |
| NV1G644 | 349 | | | 81.9%I @ 30 nM | | 19 | 2 |
| NV1G645 | 350 | | | 95.6%I @ 30 nM | | 22 | 3 |
| NV1G646 | 351 | | | 92.5%I @ 300 nM | | 66 | 48 |
| NV1G647 | 352 | | | 95.0%I @ 30 nM | | 13 | 3 |
| NV1G648 | 353 | | | | | 131 | |
| NV1G649 | 354 | | | | | 587 | |
| NV1G650 | 355 | | | 81.9%I @ 30 nM | | 19 | 2 |

*FIG. 14B*

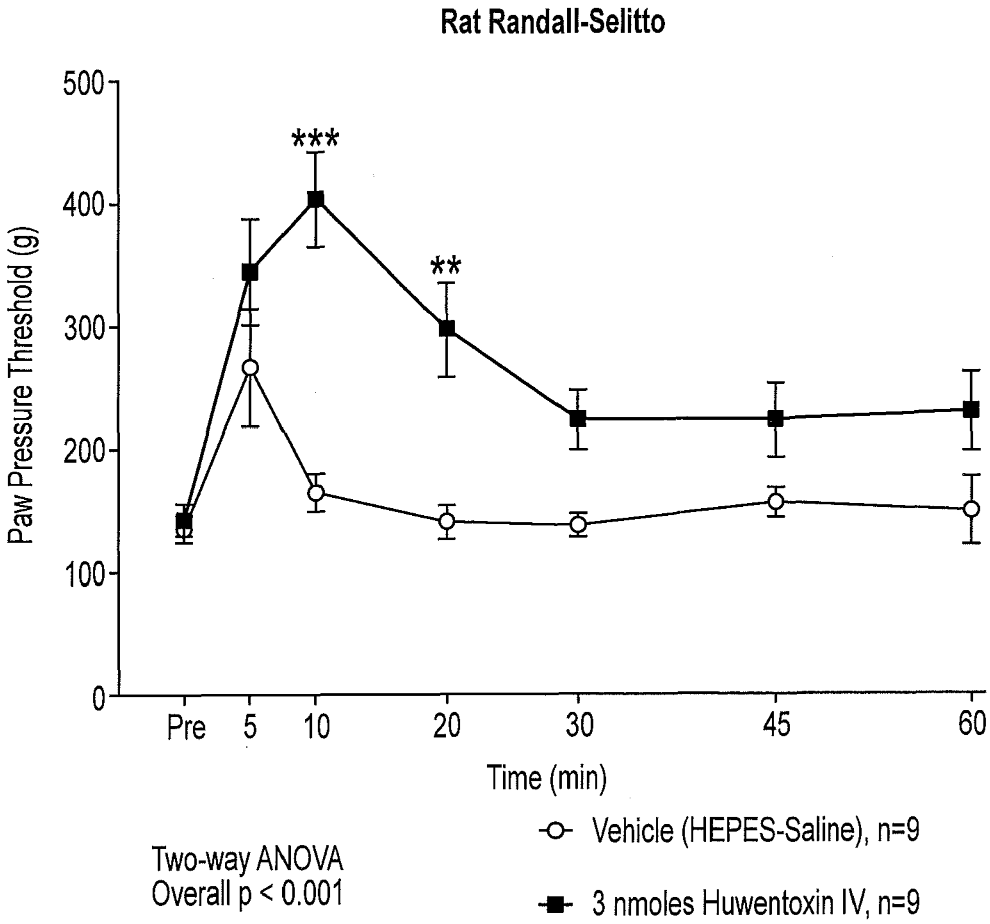
Rat Randall-Selitto
500
400
300
200
100
0
Paw Pressure Threshold (g)
***
**
Pre
5
10
20
30
45
60
Time (min)
Two-way ANOVA
Overall p < 0.001
Vehicle (HEPES-Saline), n=9
3 nmoles Huwentoxin IV, n=9

*FIG. 15A*

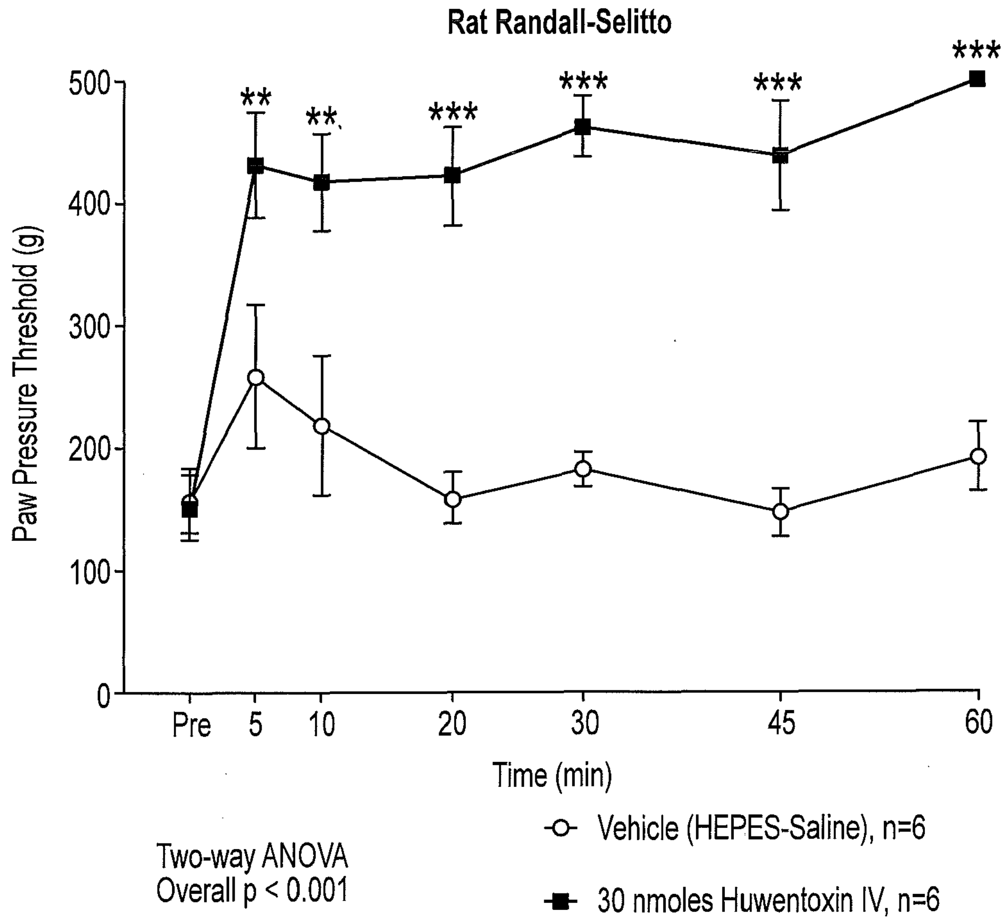
Rat Randall-Selitto
Paw Pressure Threshold (g)
0
100
200
300
400
500
**
**
***
***
***
***
Pre
5
10
20
30
45
60
Time (min)
Two-way ANOVA
Overall p < 0.001
Vehicle (HEPES-Saline), n=6
30 nmoles Huwentoxin IV, n=6

*FIG. 15B*

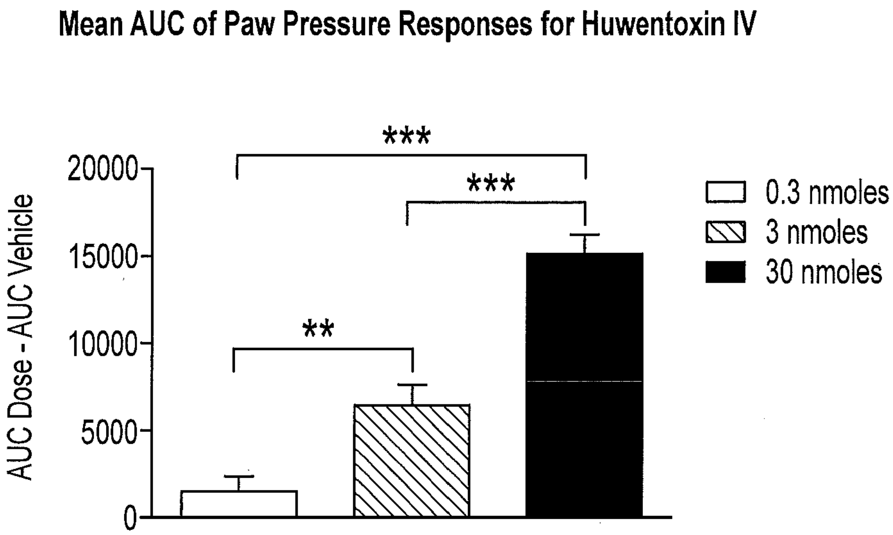
Mean AUC of Paw Pressure Responses for Huwentoxin IV
AUC Dose - AUC Vehicle
20000
15000
10000
5000
0
***
***
**
0.3 nmoles
3 nmoles
30 nmoles

FIG. 15C

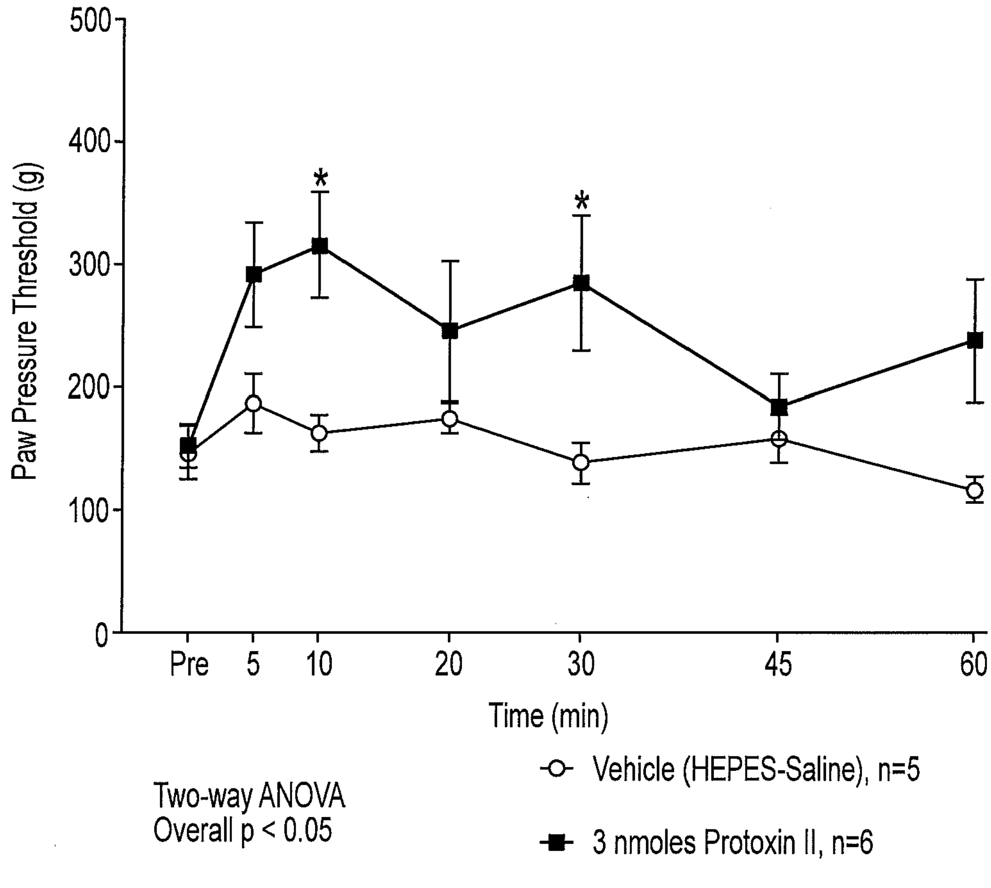
500
400
300
200
100
0
Paw Pressure Threshold (g)
*
*
Pre
5
10
20
30
45
60
Time (min)
Vehicle (HEPES-Saline), n=5
3 nmoles Protoxin II, n=6
Two-way ANOVA
Overall p < 0.05

*FIG. 16A*

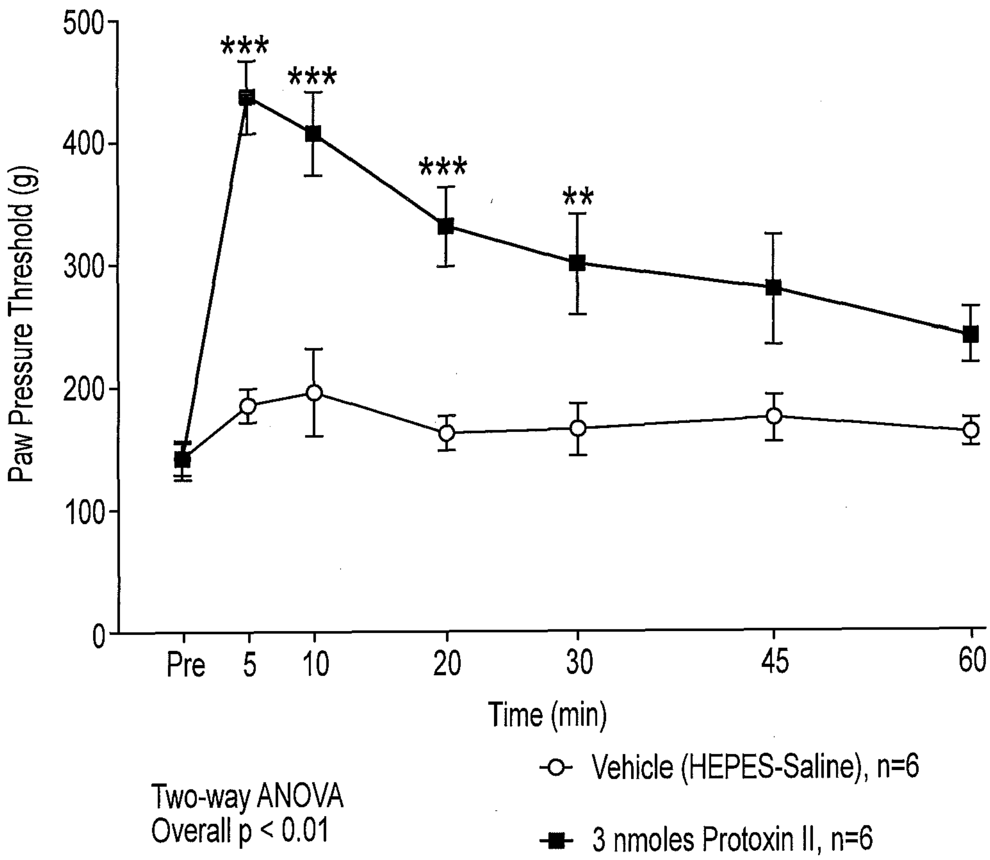
500
400
300
200
100
0
Paw Pressure Threshold (g)
***
***
***
**
Pre
5
10
20
30
45
60
Time (min)
Two-way ANOVA
Overall p < 0.01
Vehicle (HEPES-Saline), n=6
3 nmoles Protoxin II, n=6

*FIG. 16B*

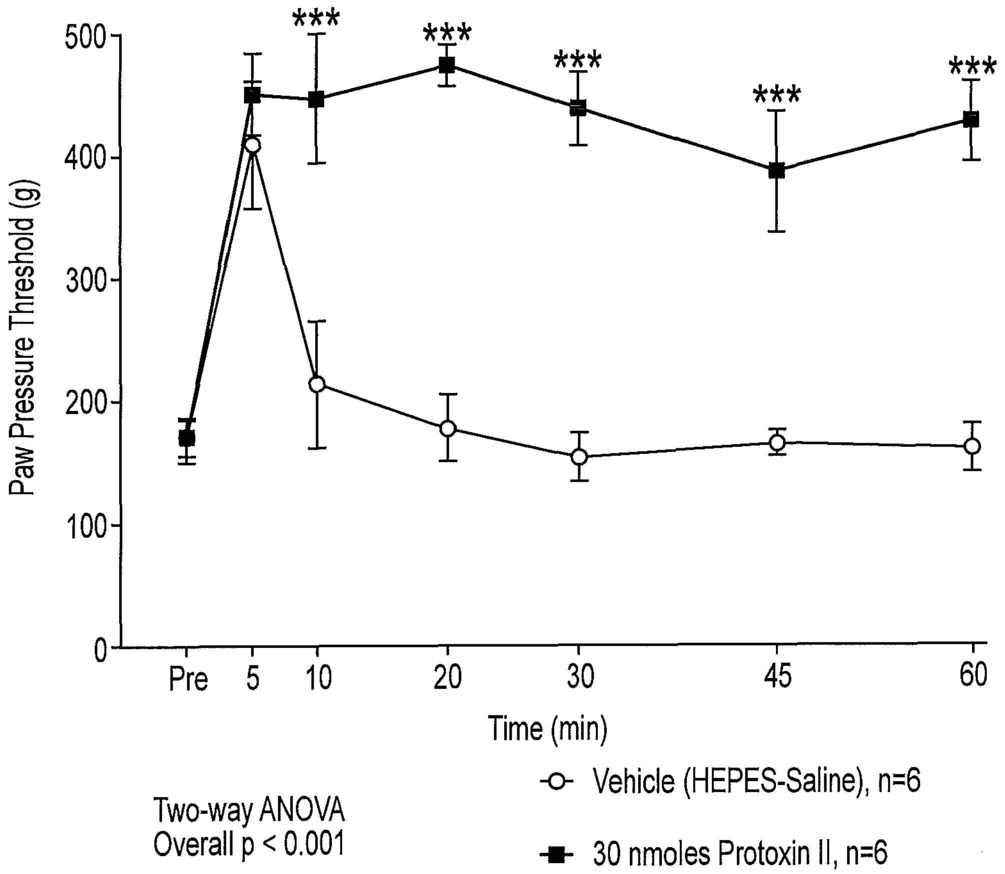

Paw Pressure Threshold (g)
500
400
300
200
100
0
***
***
***
***
***
Pre
5
10
20
30
45
60
Time (min)
Vehicle (HEPES-Saline), n=6
30 nmoles Protoxin II, n=6
Two-way ANOVA
Overall p < 0.001


*FIG. 16C*

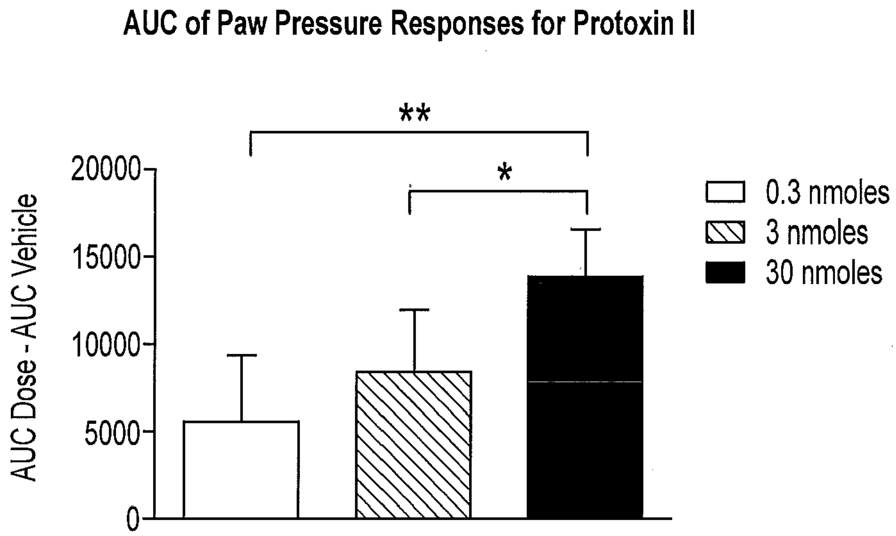
AUC of Paw Pressure Responses for Protoxin II
**
*
20000
15000
10000
5000
0
AUC Dose - AUC Vehicle
0.3 nmoles
3 nmoles
30 nmoles

*FIG. 16D*

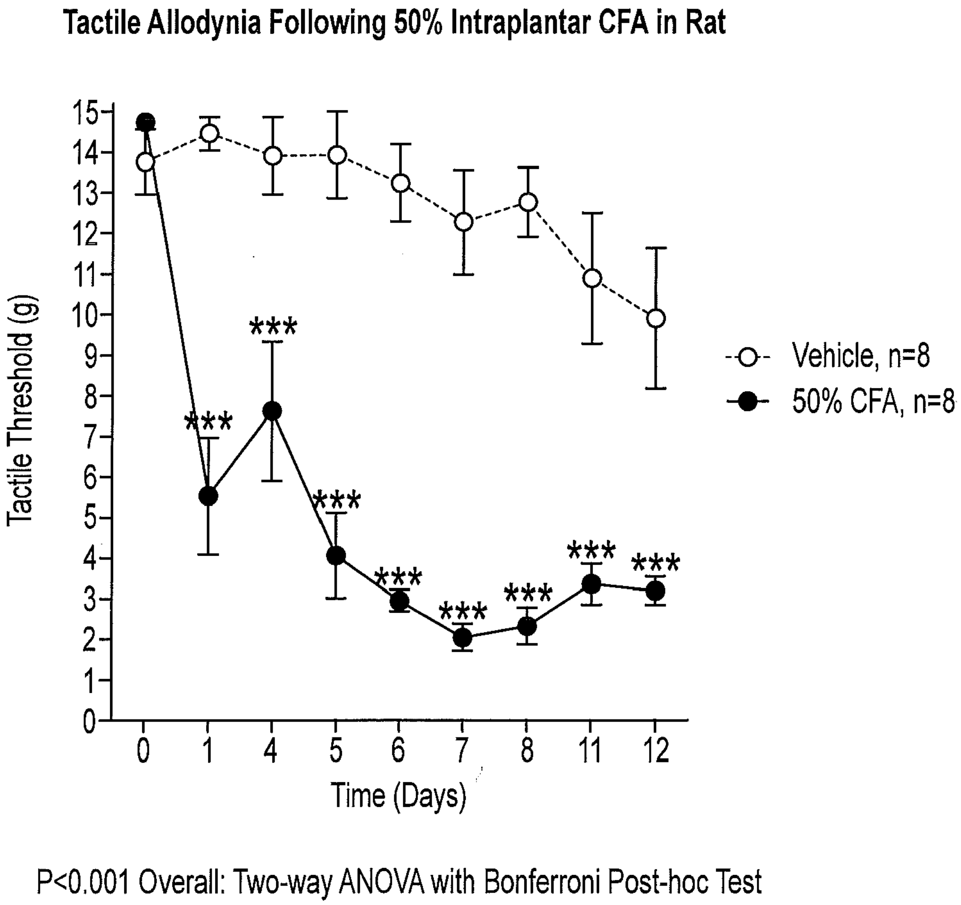
Tactile Allodynia Following 50% Intraplantar CFA in Rat
Tactile Threshold (g)
15
14
13
12
11
10
9
8
7
6
5
4
3
2
1
0
***
0
1
4
5
6
7
8
11
12
Time (Days)
Vehicle, n=8
50% CFA, n=8
P<0.001 Overall: Two-way ANOVA with Bonferroni Post-hoc Test

*FIG. 17A*

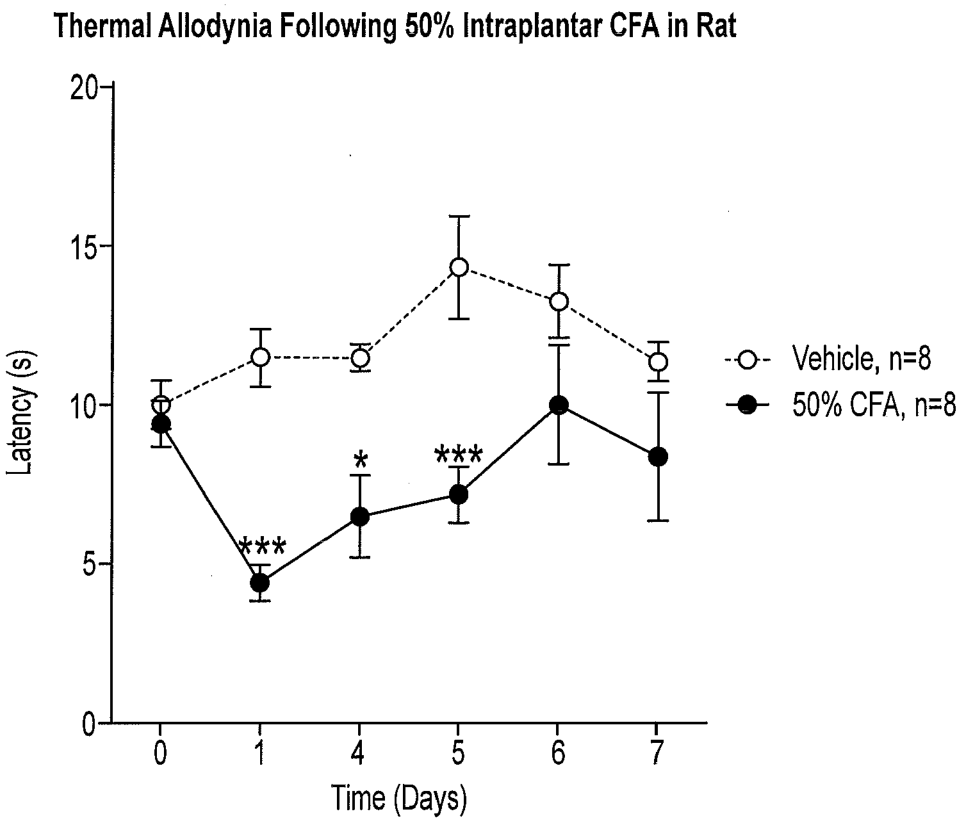


P<0.001 Overall: Two-way ANOVA with Bonferroni Post-hoc Test

*FIG. 17B*

Intraplantar Protoxin II Reduces CFA-Induced Tactile Allodynia

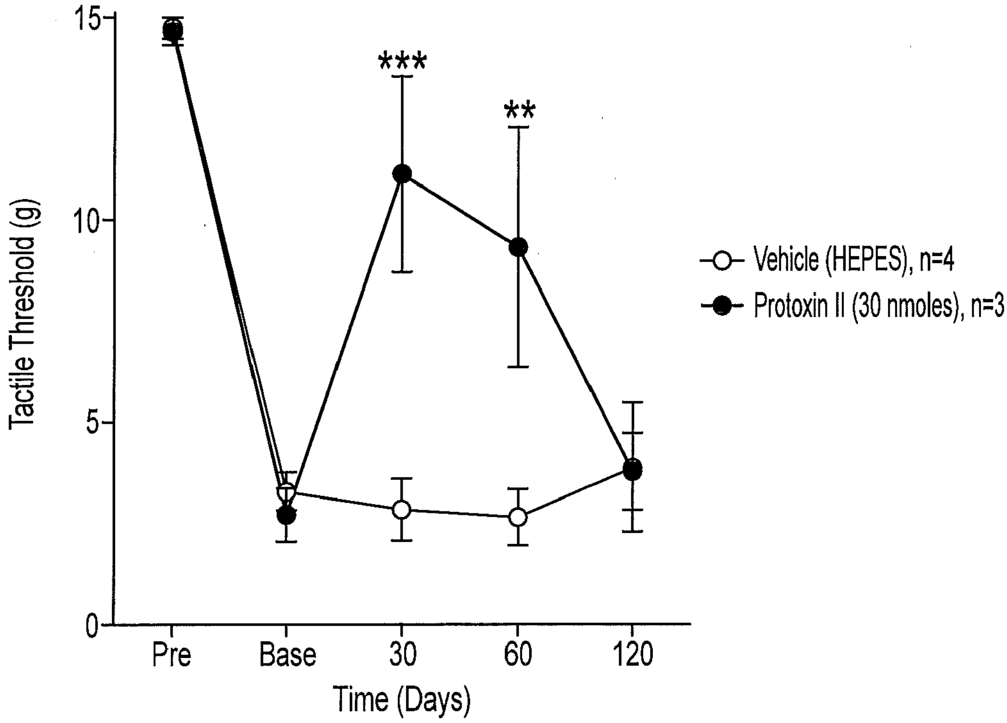


P<0.01 Overall: Two-way ANOVA with Bonferroni Post-hoc Test

*FIG. 17C*

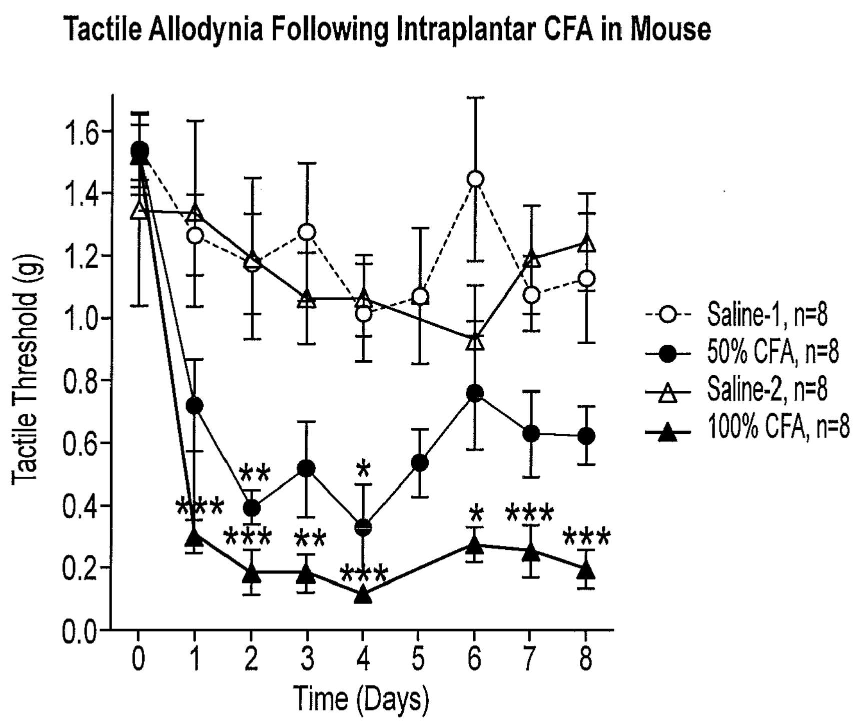


P<0.001 Overall: Two-way ANOVA with Bonferroni Post-hoc Test

*FIG. 18A*

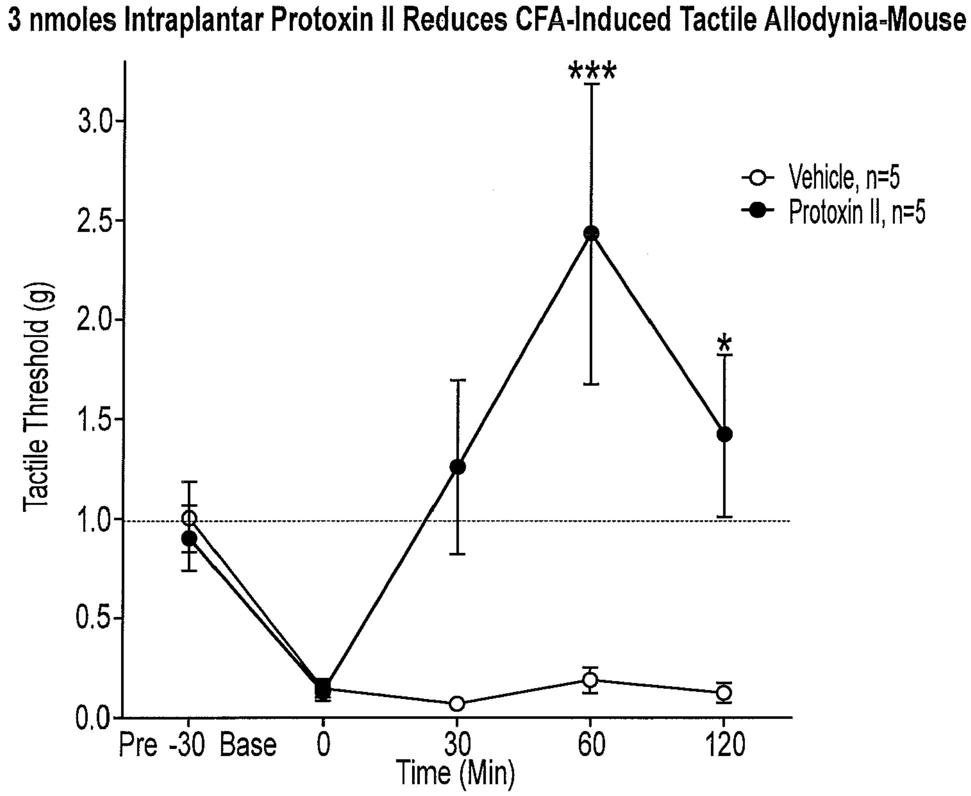


P<0.001 Overall: Two-way ANOVA with Bonferroni Post-hoc Test

*FIG. 18B*

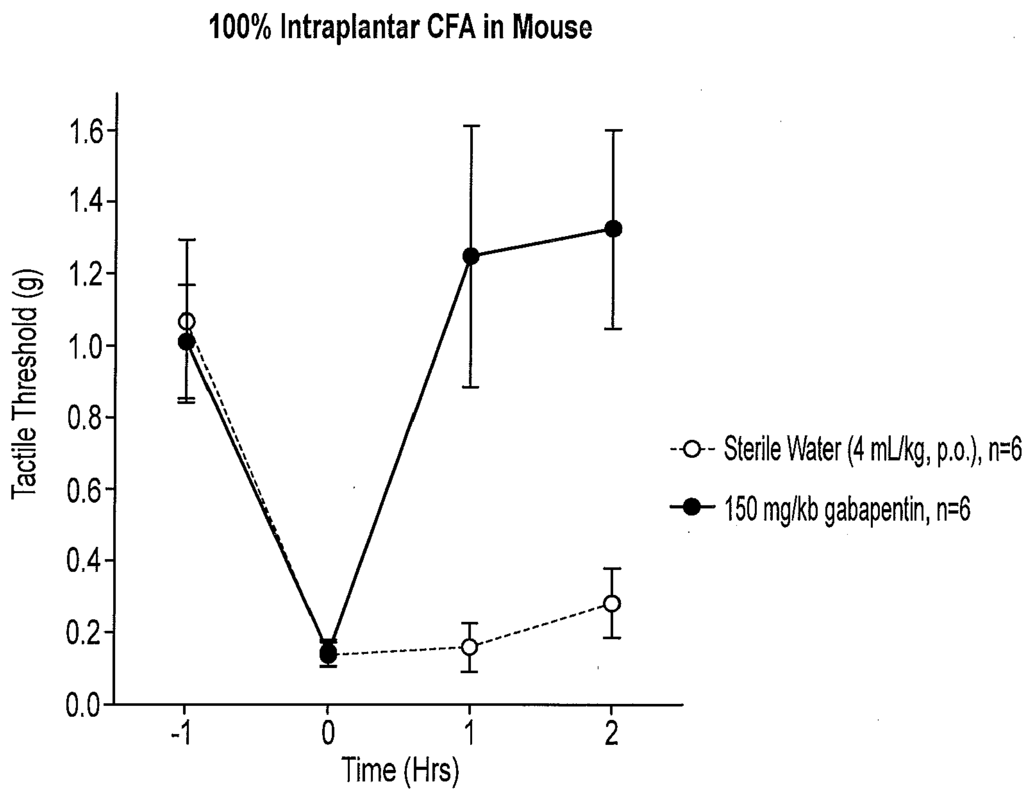


Two-way ANOVA with Bonferroni post hoc, p<0.001

*FIG. 18C*

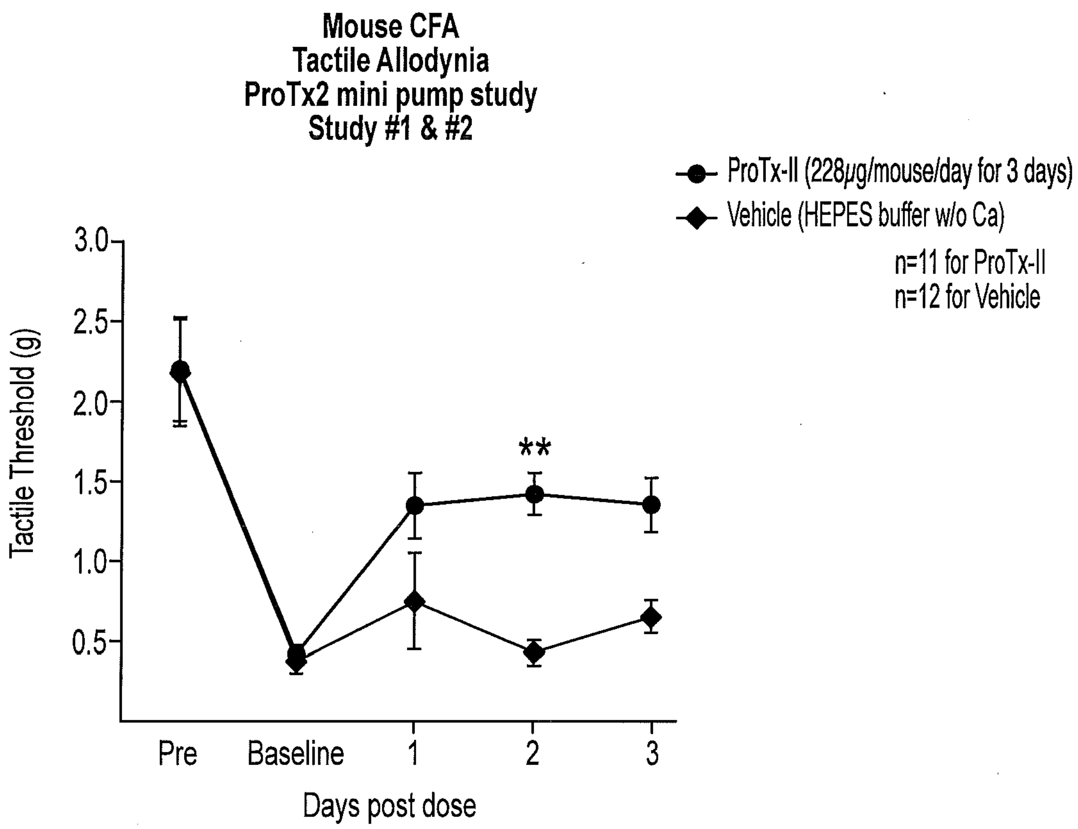
Mouse CFA
Tactile Allodynia
ProTx2 mini pump study
Study #1 & #2
ProTx-II (228μg/mouse/day for 3 days)
Vehicle (HEPES buffer w/o Ca)
n=11 for ProTx-II
n=12 for Vehicle
Tactile Threshold (g)
3.0
2.5
2.0
1.5
1.0
0.5
**
Pre
Baseline
1
2
3
Days post dose

*FIG. 19A*

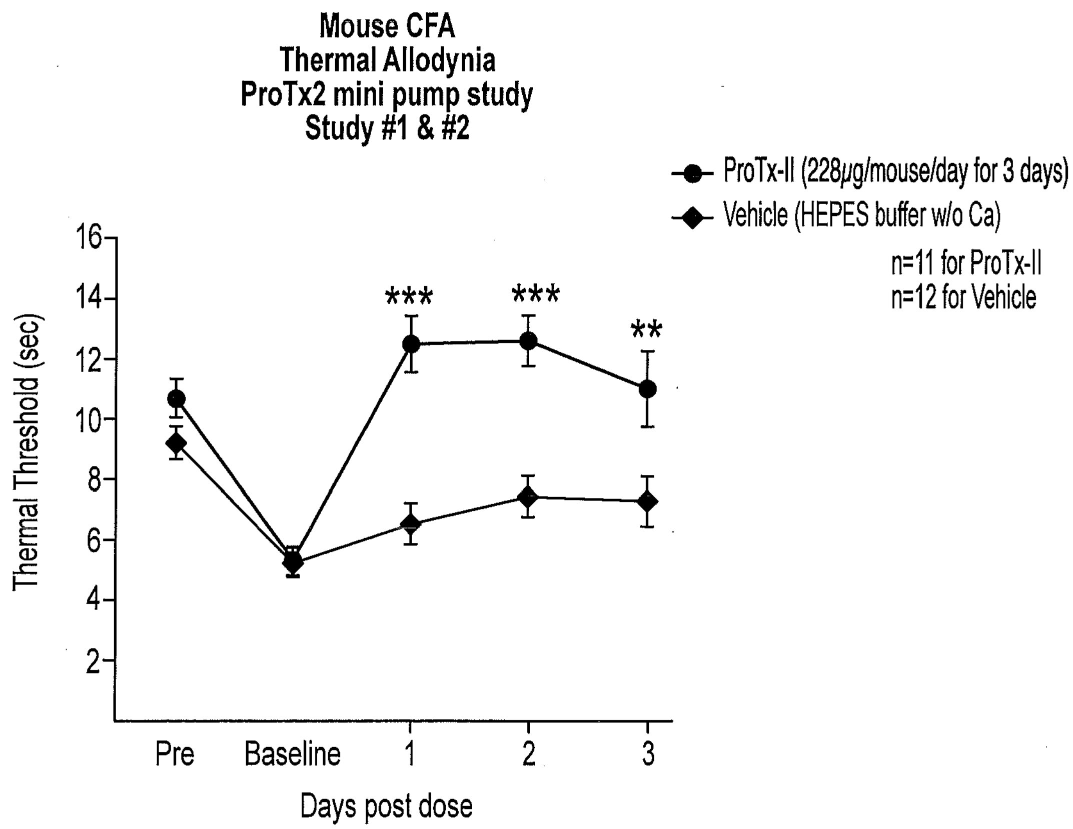
Mouse CFA
Thermal Allodynia
ProTx2 mini pump study
Study #1 & #2
ProTx-II (228µg/mouse/day for 3 days)
Vehicle (HEPES buffer w/o Ca)
n=11 for ProTx-II
n=12 for Vehicle
Thermal Threshold (sec)
16
14
12
10
8
6
4
2
***
***
**
Pre
Baseline
1
2
3
Days post dose

*FIG. 19B*

Europäisches Patentamt
European Patent Office
Office européen des brevets

# EUROPEAN SEARCH REPORT

Application Number
EP 21 18 9885

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | N A Minassian ET AL: "Functional studies of the interaction between Huwentoxin-IV and the voltage-gated sodium channel Na(v)1.7", 41st Annual Meeting of the Society-for-Neuroscience; Washington, DC, USA; November 12 -16, 2011, 12 November 2011 (2011-11-12), XP055244760, Retrieved from the Internet: URL:http://www.abstractsonline.com/Plan/ViewAbstract.aspx?sKey=be720704-3788-440a-b7ae-93ac23c9bfb3&cKey=f7bea491-24bf-4b25-bff3-3fc2e336b397&mKey=8334be29-8911-4991-8c31-32b32dd5e6c8 [retrieved on 2016-01-26] * the whole document * ----- | 1-15 | INV. A61K38/17 |
| A | Z. LIU ET AL: "Isolation and characterization of hainantoxin-IV, a novel antagonist of tetrodotoxin-sensitive sodium channels from the Chinese bird spider Selenocosmia hainana", CMLS CELLULAR AND MOLECULAR LIFE SCIENCES., vol. 60, no. 5, 1 May 2003 (2003-05-01), pages 972-978, XP055244831, DE ISSN: 1420-682X, DOI: 10.1007/s00018-003-2354-x * figures 4, 5 * ----- | 1-15 | |
| | -/-- | | TECHNICAL FIELDS SEARCHED (IPC) |
| | | | C07K |

2

The present search report has been drawn up for all claims

| Place of search | Date of completion of the search | Examiner |
|---|---|---|
| Munich | 17 February 2022 | Lanzrein, Markus |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 21 18 9885

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | SCHMALHOFER W A ET AL: "ProTx-II, a selective inhibitor of NaV1.7 sodium channels, blocks action potential propagation in nociceptors", MOLECULAR PHARMACOLOGY, AMERICAN SOCIETY FOR PHARMACOLOGY AND EXPERIMENTAL THERAPEUTICS, US, vol. 74, no. 5, 1 January 2008 (2008-01-01), pages 1476-1484, XP009120036, ISSN: 0026-895X, DOI: 10.1124/MOL.108.047670 * page 1484, left-hand column, paragraph 2 * ----- | 1-15 | |
| X,P | REVELL JEFFERSON D ET AL: "Potency optimization of Huwentoxin-IV onhNav1.7: A neurotoxin TTX-S sodium-channel antagonist from the venom of the Chinese bird-eating spiderSelenocosmia huwena", PEPTIDES, ELSEVIER, AMSTERDAM, NL, vol. 44, 19 March 2013 (2013-03-19), pages 40-46, XP028551011, ISSN: 0196-9781, DOI: 10.1016/J.PEPTIDES.2013.03.011 * figure 1 * ----- | 1-15 | |
| | | | TECHNICAL FIELDS SEARCHED (IPC) |

2

The present search report has been drawn up for all claims

| Place of search | Date of completion of the search | Examiner |
|---|---|---|
| Munich | 17 February 2022 | Lanzrein, Markus |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................
& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 61781276
- US 83355513
- US 61702538
- US 61648871
- US 6521427 B
- US 6670127 B
- US 20120185956 A
- US 20110124711A1 A
- US 7998980 B
- US 20110065647 A
- WO 2008088422 A
- WO 2012004664 A
- WO 9853847 A
- US 4309989 A
- US 4767402 A

### Non-patent literature cited in the description


- **HÜBNER ; JENTSCH.** *Hum Mol Genet,* 2002, vol. 11, 2435-45
- **YOGEESWARI et al.** *Curr Drug Targets,* 2004, vol. 5, 589-602
- **TFELT-HANSEN et al.** *J Cardiovasc Electrophysiol,* 2010, vol. 21, 107-15
- **CANNON ; BEAN.** *J Clin Invest,* 2010, vol. 120, 80-3
- **CREGG et al.** *J Physiol,* 2010, vol. 588, 1897-904
- **YANG et al.** *J Med Genet,* 2004, vol. 41, 171-4
- **FERTLEMAN et al.** *Neuron,* 2006, vol. 52, 767-74
- **LEGROUX-CRESPEL et al.** *Ann Dermatol Venereol,* 2003, vol. 130, 429-33
- **COX et al.** *Nature,* 2006, vol. 444, 894-8
- **GOLDBERG et al.** *Clin Genet,* 2007, vol. 71, 311-9
- **AHMAD et al.** *Hum Mol Genet,* 2007, vol. 16, 2114-21
- **REIMANN et al.** *Proc Natl Acad Sci USA,* 2010, vol. 107, 5148-53
- **ESTACION et al.** *Ann Neurol,* 2009, vol. 66, 862-6
- **VARGAS-ALARCON et al.** *BMC Musculoskelet Disord,* 2012, vol. 13, 23
- **NASSAR et al.** *Proc Natl Acad Sci USA,* 2004, vol. 101, 12706-11
- **MINETT et al.** *Nat Commun,* 2012, vol. 3, 791
- **PENG et al.** *J Biol Chem,* 2002, vol. 277, 47564-71
- **MIDDLETON et al.** *Biochemistry,* 2002, vol. 41, 14734-47
- **BOSMANS et al.** *Mol Pharmacol,* 2006, vol. 69, 419-29
- **XIAO et al.** *J Biol Chem,* 2008, vol. 283, 27300-13
- **SCHMALHOFER et al.** *Mol Pharm,* 2008, vol. 74, 1476-1484
- **HACKEL.** *PNAS,* 2012, vol. 109 (29), E2018-27
- **MIDDLETON et al.** *Biochemistry,* 2002, vol. 41 (50), 14734-47
- **SCHMALHOFER et al.** *Mol Pharmacol,* 2008, vol. 74, 1476-1484
- **ZHANG et al.** *J Biol Chem,* 2007, vol. 282 (42), 30699-706
- **CLARE et al.** *drug Discovery Today,* 2000, vol. 5, 506-520
- **GINGRAS et al.** *American Pain Society Meeting Abstract,* 2013
- **MUROI et al.** *J Physiol,* 01 December 2011, vol. 589, 5663-76
- **MUROI et al.** *Am J Physiol Regul Integr Comp Physiol,* 10 April 2013
- **HACKEL et al.** *Proc Natl Acad Sci,* 2012, vol. 109, E2018-27
- **DIB-HAJJ et al.** *Nature Rev Neuroscience,* 2013, vol. 14, 49-62
- Remington: The Science and Practice of Pharmacy. Lippincott Williams & Wilkins, 2005
- Remington's Pharmaceutical Science. Mack Publishing Company
- **BAX ; DAVIS.** *Mag. Reson.,* 1985, vol. 65, 355-360
- **JEENER et al.** *J. Chem. Phys.,* 1979, vol. 71, 4546-4553
- **KUMAR et al.** *Biochem. Biophys. Res. Commun.,* 1980, vol. 95, 1-6
- **BODENHAUSEN et al.** *Chem. Phys. Lett.,* 1980, vol. 69, 185-189
- **CAVANAGH et al.** Protein NMR Spectroscopy: Principles and Practice. Academic Press, 1995

- **DELAGLIO et al.** *J. Biomol. NMR,* 1995, vol. 6, 277-293
- **GUNTERT et al.** *J. Mol. Biol.,* 1997, vol. 273, 283-298
- **BERJANSKII et al.** *Nuc. Acid. Res.,* 2006, vol. 34, W63-W69
- **CHEUNG et al.** *J. Mag. Reson.,* 2010, vol. 202, 223-233
- **JAMES et al.** *Journal of Computational Chemistry,* 2005, vol. 26, 1781-1802
- **MACKERELL, JR et al.** *J Comput Chem,* 2004, vol. 25, 1400-1415
- **HUMPHREY et al.** *J. Molec. Graphics,* 1996, vol. 14, 33-38
- **XIAO et al.** *J Biol Chem.,* 09 June 2011, vol. 286, 27301-10
- **SPASSOV et al.** *J. Phys. Chem. B,* 2002, vol. 106, 8726-8738
- **XIAO et al.** *Biol Chem.,* 09 June 2011, vol. 286, 27301-10
- **ZHANG et al.** *J Biol Chem.,* 2007, vol. 282 (42), 30699-706
- **HARGREAVES et al.** *Pain,* 1988, vol. 32, 77-88
- **DIRIG et al.** *J Neurosci. Methods,* 1997, vol. 76, 183-191
- **HACKER et al.** *Proc Natl Acad Sci USA,* vol. 109, E2018-27